# EUROPEAN PATENT SPECIFICATION

(11) **EP 3 634 430 B1**
(45) Date of publication and mention of the grant of the patent: **07.08.2024**
(21) Application number: 18814051.1
(22) Date of filing: 05.06.2018
(51) Int. Cl.: A61K 31/7034, A61K 31/05, A61K 31/09, A61K 31/122, A61K 31/137, A61K 31/14, A61K 31/155, A61K 31/353, A61K 31/366, A61K 31/606, A61P 13/12, A61P 43/00, A61K 47/54, C07C 403/24

(54) **MULTIBIOTIC AGENTS AND METHODS OF USING THE SAME**
MULTIBIOTIKA UND VERWENDUNGSVERFAHREN DAFÜR
AGENTS MULTIBIOTIQUES ET PROCÉDÉS D'UTILISATION DE CEUX-CI

(30) Priority: 05.06.2017 US 201762515409 P; 03.01.2018 US 201862613359 P; 20.02.2018 US 201862632531 P
(43) Date of publication of application: 15.04.2020
(73) Proprietor: Flagship Pioneering Innovations V, Inc., Cambridge, MA 02142 (US)
(72) Inventor: CASEY, Jr., John Patrick, Boston, MA 02114 (US); BERRY, David, Waban, MA 02468 (US); CASTRO, Alfredo, Woburn, MA 01801 (US); TAYLOR, Steven J., Winchester, MA 01890 (US); MASSARI, Ferdinand E., Beverly, MA 01915 (US); PROUDFOOT, John, Newtown, CT 06470 (US); BOGART, Elijah, Cambridge, MA 02139 (US); BRIGGS, Timothy F., Waltham, MA 02453 (US)
(74) Representative: Maiwald GmbH
(86) International application number: PCT/US2018/036113
(87) International publication number: WO 2018/226732

(56) References cited:
- WO-A1-2013/142279
- WO-A1-2016/025668
- WO-A2-2005/000248
- US-A- 5 677 340
- US-A1- 2004 265 345
- US-A1- 2010 310 615
- US-A1- 2012 010 284
- US-A1- 2012 230 955
- US-A1- 2016 213 702
- US-B2- 6 784 177
- US-B2- 8 388 939
- JANIKULA MARK: "Policosanol: A New Treatment for Cardiovascular Disease?", ALTERNATIVE MEDICINE REVIEW: A JOURNAL OF CLINICAL THERAPEUTIC, vol. 7, no. 3, June 2002 (2002-06-01), pages 203 - 217, XP055562247
- LIN ET AL.: "Improving the Stability of Astaxanthin by Microencapsulation in Calcium Alginate Beads", PLOS ONE, vol. 11, no. 4, 19 April 2016 (2016-04-19), pages 1 - 10, XP055562248
- HWANG ET AL.: "Antifungal Effect of (+)-Pinoresinol Isolated from Sambucus williamsii", MOLECULES, vol. 15, no. 5, 14 May 2010 (2010-05-14), pages 3507 - 3516, XP055562252

## Description

### FIELD OF THE INVENTION

The present invention relates to multibiotic agents. The present invention also features compositions containing one or more multibiotic agents and methods of using the multibiotic agents.

### BACKGROUND

The mammalian microbiota can engage in a bidirectional communication with the mammalian host system. While therapeutic approaches taking advantage of the mammalian microbiota have so far largely focused on probiotics (e.g., live microorganisms) as the active agents, small molecules leveraging the bidirectional communication remain largely underutilized.

There is a need for small-molecule based approaches for pharmaceutical and nutraceutical applications leveraging the bidirectional communication between the mammalian host system and the mammalian microbioate.

US 2016/0213702 A1 discloses methods of modulating the abundance of a bacterial taxa in a human subject's gastrointestinal microbiota, the method comprising administering to the human subject a pharmaceutical composition comprising a glycan therapeutic preparation in an amount effective to modulate the abundance of the bacterial taxa, wherein the glycan therapeutic preparation comprises a mixture of branched glycans and wherein the degree of branching is further defined in US 2016/0213702 A1.

US 2012/0230955 A1 refers to methods and compositions, i.e. comprising arabinoxylan preparations, for modulating the specific composition of the intestinal microbiota and the specific interaction of the human intestinal flora with the intestinal surface and the host.

WO 2016/025668 A1 relates to compositions and methods for biodegradable trehalose glycopolymers. The compositions include trehalose-based copolymers having biodegradable bonds, wherein the copolymers can be degradade into non-cytotoxic products.

US 2012/0010284 A1 discloses biodegradable multi-armed (e.g., 2, 3, 4, 5, or 6 arms) oligomers wherein the end groups of these oligomers have been functionalized with biologically active molecules. Further, US 2012/0010284 A1 relates to a therapeutic method for treating a disease in a mammal comprising administering to a mammal in need of such a therapy, an effective amount of an oligomer disclosed therein.

### SUMMARY OF THE INVENTION

In general, the present invention provides compounds, compositions, and methods for modulating human health. Compounds, compositions, and methods of the invention may utilize targeting the host's physiologic compartments to leverage the existing bidirectional communication links between the mammalian microbioata and the mammalian host system.

In one aspect, the invention relates to a multibiotic agent, wherein the multibiotic agent is selected from the group consisting of the compounds of the following structure: 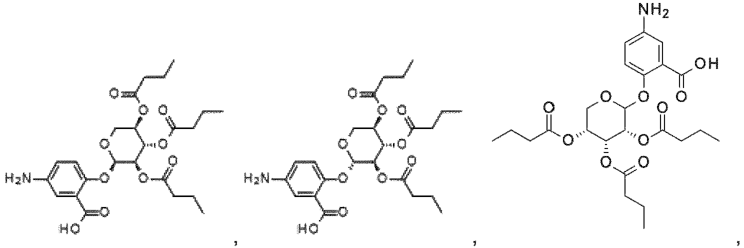 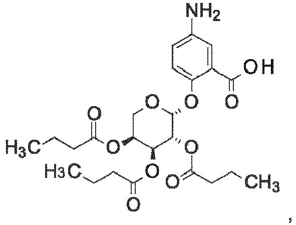 and salts thereof.
In one embodiment, the present invention relates to a multibiotic agent as defined above, wherein the multibiotic agent is a compound of the following structure: 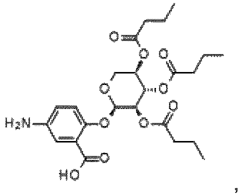 or a salt thereof.
In another embodiment, the multibiotic agent as defined above is a compound of the following structure: 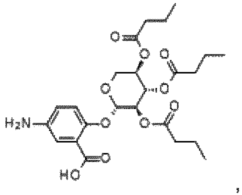 or a salt thereof.
In another embodiment, the multibiotic agent as defined above is a compound of the following structure: 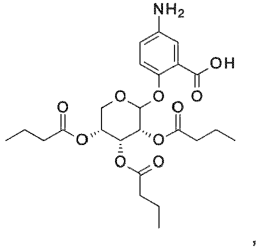 or a salt thereof.
In a further embodiment, the multibiotic agent as defined above is a compound of the following structure: 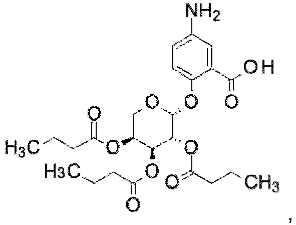 or a salt thereof.

In another aspect, the present invention relates to a pharmaceutical composition comprising the multibiotic agent as defined above and a pharmaceutically acceptable excipient.

In a further aspect, the present invention refers to a nutraceutical composition comprising the multibiotic agent as defined above and a physiologically acceptable excipient.

In another aspect, the present invention relates to the multibiotic agent as defined above for use in delivering a biologically active compound to a target site in a subject.

In another embodiment of said use, the multibiotic agent is administered orally.

In another embodiment of said use, the multibiotic agent is substantially activated by a microbiota of the subject.

Furthermore, the multibiotic agents according to the present disclosure can be further described for illustrative purposes only as outlined below. Any reference in the following disclosure to methods of treatment by therapy or surgery or in vivo diagnosis methods are to be interpreted as references to multibiotic agents, pharmaceutical compositions and medicaments according to the present disclosure for use in those methods.

In one aspect, a multibiotic agent is provided comprising two or more moieties that are independently alcohol cores, amine cores, and acyls. The moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.* In certain embodiments, the multibiotic agent comprises one or more moieties (e.g., alcohol cores, amine cores, and/or acyls) bonded to each other through hydrolyzable *in vivo* bonds, e.g., where at least one of the hydrolyzable *in vivo* bonds is an ester bond. In particular embodiments, the multibiotic agent comprises one or more moieties (e.g., alcohol cores, amine cores, and/or acyls) bonded to each other through hydrolyzable *in vivo* bonds, e.g., where at least one of the hydrolyzable *in vivo* bonds is an amide bond. In further embodiments, the multibiotic agent comprises one or more moieties (e.g., alcohol cores, amine cores, and/or acyls) bonded to each other through hydrolyzable *in vivo* bonds, e.g., where at least one of the hydrolyzable *in vivo* bonds is an azo bond. In yet further embodiments, the multibiotic agent comprises one or more moieties (e.g., alcohol cores, amine cores, and/or acyls) bonded to each other through hydrolyzable *in vivo* bonds, e.g., where at least one of the hydrolyzable *in vivo* bonds is an azo bond. In still further embodiments, the multibiotic agent comprises one or more moieties (e.g., alcohol cores, amine cores, and/or acyls) bonded to each other through hydrolyzable *in vivo* bonds, e.g., where at least one of the hydrolyzable *in vivo* bonds is a glycosidic bond. In some embodiments, the multibiotic agent comprises one or more moieties (e.g., alcohol cores, amine cores, and/or acyls) bonded to each other through hydrolyzable *in vivo* bonds, e.g., where at least one of the hydrolyzable *in vivo* bonds is a carbonate linker. In particular embodiments, the multibiotic agent comprises one or more moieties (e.g., alcohol cores, amine cores, and/or acyls) bonded to each other through hydrolyzable *in vivo* bonds, e.g., where at least one of the hydrolyzable *in vivo* bonds is a carbamate linker.

In the multibiotic agents according to the present disclosure, an alcohol core is covalently linked through hydrolyzable *in vivo* bond(s) to one or more of an acyl, another alcohol core, and an amine core; an amine core is covalently linked through hydrolyzable *in vivo* bond(s) to one or more of an acyl, an alcohol core, and another amine core; and an acyl is covalently linked through hydrolyzable *in vivo* bond(s) to one or more of an alcohol core and an amine core. When an alcohol core is linked to another alcohol core, the linkage is a carbonate linker. When an alcohol core is linked to an amine core, the linkage is an amide bond or a carbamate linker. When an alcohol core is linked to an acyl, the linkage is an ester bond. When an amine core is linked to another amine core, the linkage is an azo bond. When an amine core is linked to an acyl, the linkage is an amide bond. When a multibiotic agent includes a carbohydrate or an oligomer thereof linked to another alcohol core, the linkage is a glycosidic bond.

In further embodiments, a multibiotic agent contains three or more biologically active moieties independently that are alcohol cores, amine cores, or acyls linked by one or more of ester bonds, amide bonds, azo bonds, glycosidic bonds, carbonate linkers, or carbamate linkers. In other embodiments, the at least three biologically active moieties are different.

In further embodiments, a multibiotic agent contains two or more moieties bonded to each other through one or more azo bonds cleavable *in vivo.* In yet further embodiments, when one of the moieties is mesalamine bonded to another moiety through an azo bond, the other moiety bonded to mesalamine through the azo bond is an amino acid, amino acid metabolite, polyamine, glutathione, or folic acid.

In some embodiments, each alcohol core is independently a bile acid, carbohydrate or an oligomer thereof, anhydrosugar alcohol, sugar alcohol, sugar acid, flavonoid, vitamin, lignan, phenolic phytochemical, nucleic acid, carotenoid, curcuminoid, stilbenoid or a multimer thereof, capsinoid, ketone body, pre-ketone body, β-hydroxycarboxylate, ezetimibe, citicoline, antifungal agent, an amino acid metabolite, amino alcohol, mesalamine, pentacyclic triterpenoid, long chain alcohol, hydroxycinnamic acid ester, catechin polyphenol, or telomerase activator. In further embodiments, each amine core is independently an amino acid, amino acid metabolite, amino alcohol, antihyperglycemic biguanide, vitamin, mesalamine, glutathione, or a polyamine. In some embodiments, each acyl is independently an amino acid acyl, bile acid acyl, amino acid metabolite acyl, β-hydroxycarboxylate acyl, ketone body acyl, pre-ketone body acyl, sugar acid acyl, hydroxycinnamic acid ester acyl, phenolic phytochemical acyl, vitamin acyl, pentacyclic triterpenoid acyl, glutathione acyl, α-lipoic acid acyl, mesalamine acyl, polyamine acyl, or picolinate acyl.

In further embodiments, each alcohol core is independently a bile acid, anhydrosugar alcohol, sugar alcohol, sugar acid, vitamin, lignan, flavonoid, phenolic phytochemical, nucleic acid, carotenoid, curcuminoid, capsinoid, β-hydroxyalkanoate, ketone body, pre-ketone body, ezetimibe, citicoline, antifungal agent, an amino acid metabolite, amino alcohol, mesalamine, pentacyclic triterpenoid, hydroxycinnamic acid ester, or telomerase activator. In yet further embodiments, each amine core is independently an amino acid, amino acid metabolite, amino alcohol, antihyperglycemic biguanide, vitamin, mesalamine, glutathione, or a polyamine. In still further embodiments, each acyl is independently an amino acid acyl, bile acid acyl, fatty acid acyl, amino acid metabolite acyl, β-hydrocarboxylate acyl, ketone body acyl, pre-ketone body acyl, sugar acid acyl, hydroxycinnamic acid acyl, phenolic phytochemical acyl, vitamin acyl, pentacyclic triterpenoid acyl, glutathione acyl, α-lipoic acid acyl, mesalamine acyl, polyamine acyl, or picolinate acyl. In other embodiments, when the alcohol core is mesalamine, the acyl is not C₄-C₈ alkanoyl.

In some embodiments, each alcohol core is independently a bile acid, carbohydrate or an oligomer thereof, anhydrosugar alcohol, sugar alcohol, sugar acid, non-catechin flavonoid, vitamin, lignan, phenolic phytochemical, nucleic acid, carotenoid, curcuminoid, pterostilbene, rhapontigenin, piceatannol, pinostilbene, oxyresveratrol, 4-methoxyresveratrol, capsinoid, ezetimibe, citicoline, antifungal agent, an amino acid metabolite, amino alcohol, telomerase activator, mesalamine, or β-hydroxyalkanoate. In some embodiments, each amine core is independently amino acid, amino acid metabolite, vitamin, mesalamine, or a polyamine. In further embodiments, each acyl is independently an amino acid acyl, bile acid acyl, fatty acid acyl, amino acid metabolite acyl, β-hydroxycarboxylate acyl, ketone body acyl, pre-ketone body acyl, sugar acid acyl, hydroxycinnamic acid acyl, phenolic phytochemical acyl, vitamin acyl, pentacyclic triterpenoid acyl, glutathione acyl, α-lipoic acid acyl, or picolinate acyl. In further embodiments, the carbohydrate or an oligomer thereof is arabinose, xylose, galactose, ribose, or glucosinolate, or an oligomer thereof; or lactulose, trehalose, or hyaluronic acid. In yet further embodiments, when the alcohol core is mesalamine, the acyl is not C₄-C₈ alkanoyl.

In other embodiments, each alcohol core is independently a bile acid, carbohydrate or an oligomer thereof, anhydrosugar alcohol, sugar alcohol, sugar acid, non-catechin flavonoid, vitamin, lignan, phenolic phytochemical, nucleic acid, carotenoid, curcuminoid, stilbenoid, capsinoid, ezetimibe, citicoline, antifungal agent, amino acid metabolite, amino alcohol, telomerase activator, mesalamine, or β-hydroxycarboxylate. In some embodiments, each amine core is independently amino acid, amino acid metabolite, vitamin, mesalamine, or a polyamine. In further embodiments, each acyl is independently an amino acid acyl, bile acid acyl, fatty acid acyl, amino acid metabolite acyl, β-hydroxycarboxylate acyl, ketone body acyl, pre-ketone body acyl, sugar acid acyl, hydroxycinnamic acid acyl, phenolic phytochemical acyl, vitamin acyl, pentacyclic triterpenoid acyl, glutathione acyl, α-lipoic acid acyl, or picolinate acyl. In further embodiments, the fatty acid is a saturated medium chain fatty acid, saturated long chain fatty acid, or unsaturated fatty acid.

In some embodiments, the alcohol core is a carbohydrate or an oligomer thereof (e.g., arabinose, xylose, fructose, galactose, ribose, glucosinolate, or an oligomer thereof; or lactulose, hyaluronic acid, or trehalose). In further embodiments, the alcohol core is a flavonoid (e.g., apigenin, naringenin, genistein, quercetin, luteolin, daidzein, equol, or hesperetin). In some embodiments, the alcohol core is a stilbenoid or a multimer thereof (e.g., resveratrol, pterostilbene, rhapontigenin, piceatannol, pinostilbene, oxyresveratrol, 4-methoxyresveratrol, or a viniferin (e.g., α-viniferin, β-viniferin, γ-viniferin, δ-viniferin, or ε-viniferin)). In further embodiments, the alcohol core is a long chain alcohol (e.g., policosanol). In yet further embodiments, the alcohol core is a β-hydroxycarboxylate (e.g., carnitine or β-hydroxyalkanoate).

In other embodiments, at least one acyl is a fatty acid acyl (e.g., a short-chain fatty acid acyl, medium-chain fatty acid acyl, or long-chain fatty acid acyl). In further embodiments, the fatty acid acyl is a saturated fatty acid acyl. In other embodiments, the fatty acid acyl is an unsaturated fatty acid acyl. In yet other embodiments, the fatty acid acyl is a short-chain fatty acid acyl. In still other embodiments, the short-chain fatty acid acyl is formyl, acetyl, propionyl, butyryl, isobutyryl, valeryl, isovaleryl, succinyl, or succin-di-yl. In further embodiments, the fatty acid acyl is a medium-chain fatty acid acyl. In yet further embodiments, the medium-chain fatty acid acyl is hexanoyl, heptanoyl, octanoyl, nonanoyl, decanoyl, or dodecanoyl. In still further embodiments, the fatty acid acyl is a long-chain fatty acid acyl. In some embodiments, the fatty acid acyl is myristyl, palmityl, stearyl, arachidyl, docosanoyl, tetracosanoyl, hexacosanoyl, eicosapentaenoyl, docosahexaenoyl, punicyl, arachidonyl, dihomo-γ-linolenyl, docosapentanoyl, linoleyl, or α-linolenyl.

In other embodiments, the alcohol core is β-hydroxybutyrate. In further embodiments, the alcohol core is a bile acid (e.g., chenodeoxycholic acid, cholic acid, deoxycholic acid, glycocholic acid, glycochenodeoxycholic acid, lithocholic acid, taurocholic acid, taurochenodeoxycholic acid, hyodeoxycholic acid, or ω-muricholic acid). In further embodiments, the alcohol core is a vitamin (e.g., ascorbic acid, retinoic acid, vitamin B5, vitamin D2, vitamin D3, or vitamin E). In yet further embodiments, the alcohol core is a lignan (e.g., enterodiol, enterolactone, pinoresinol, lariciresinol, secoisolariciresinol, matairesinol, or 7-hydroxyenterolactone). In still further embodiments, the alcohol core is a phenolic phytochemical (e.g., a phenalkyl, gossypol, vitamin E, ellagic acid, or ellagic acid analogue). In some embodiments, the alcohol core is a nucleic acid. In further embodiments, the alcohol core is a carotenoid (e.g., fucoxanthin, lutein, zeaxanthin, or astaxanthin). In other embodiments, the alcohol core is a curcuminoid (e.g., curcumin). In further embodiments, the alcohol core is a capsinoid (e.g., capsiate, dihydrocapsiate, or nordihydrocapsiate). In yet further embodiments, the alcohol core is ezetimibe. In still further embodiments, the alcohol core is citicoline. In other embodiments, the alcohol core is an antifungal agent (e.g., an echinocandin (e.g., caspofungin, micafungin, or anidulafungin)). In further embodiments, the alcohol core is an amino acid metabolite (e.g., tyramine). In other embodiments, the alcohol core is an amino alcohol (e.g., choline, mesalamine, or ephedrine). In yet other embodiments, the alcohol core is a telomerase activator. In still other embodiments, the alcohol core is a pentacyclic triterpenoid that is oleanolate or ursolic acid. In some embodiments, the alcohol core is a hydroxycinnamic acid ester (e.g., chlorogenic acid). In further embodiments, the alcohol core is a ketone body (e.g., acetoacetate or β-hydroxybutyrate). In other embodiments, the alcohol core is β-hydroxybutyrate. In further embodiments, the alcohol core is citicoline. In yet further embodiments, the alcohol core is a flavonoid (e.g., apigenin, naringenin, genistein, quercetin, luteolin, daidzein, equol, or hesperetin). In still further embodiments, the alcohol core is an anhydrosugar alcohol (e.g., sorbitan or isosorbide). In other embodiments, the alcohol core is a sugar alcohol (e.g., inositol or erythritol). In yet other embodiments, the alcohol core is a sugar acid (e.g., gluconic acid).

In still other embodiments, the amine core is an amino acid (e.g., alanine, arginine, asparagine, aspartic acid, cysteine, glutamic acid, glutamine, glycine, histidine, isoleucine, leucine, lysine, methionine, phenylalanine, proline, selenocysteine, serine, threonine, tyrosine, tryptophan, ornithine, citrulline, aminobenzoic acid, or taurine). In some embodiments, the amine core is an amino acid metabolite (e.g., tyramine, indole-3-acetic acid, indole-3-propionic acid, or 3-(indole-3-yl)-acrylic acid). In further embodiments, the amine core is an amino alcohol that is mesalamine or ephedrine. In certain embodiments, the amine core is an antifungal agent that is an echinocandin (e.g., caspofungin). In further embodiments, the amine core is an antihyperglycemic biguanide (e.g., metformin). In yet further embodiments, the amine core is glutathione. In still further embodiments, the amine core is mesalamine. In other embodiments, the amine core is a polyamine (e.g., putrescine, spermidine, thermospermine, diaminopropane, N-carbamoylputrescine, cadaverine, homospermidine, norspermine, carboxynorspermidine, carboxyspermidine, norspermidine, spermine, or agmatine).

In further embodiments, the acyl is an amino acid acyl (e.g., alanine acyl, arginine acyl, asparagine acyl, aspartic acid acyl, cysteine acyl, glutamic acid acyl, glutamine acyl, glycine acyl, histidine acyl, isoleucine acyl, leucine acyl, lysine acyl, methionine acyl, phenylalanine acyl, proline acyl, selenocysteine acyl, serine acyl, threonine acyl, tyrosine acyl, tryptophan acyl, ornithine acyl, citrulline acyl, or amino benzoic acid acyl). In some embodiments, the acyl is a bile acid acyl (e.g., chenodeoxycholic acid acyl, cholic acid acyl, deoxycholic acid acyl, glycocholic acid acyl, glycochenodeoxycholic acid acyl, lithocholic acid acyl, taurocholic acid acyl, taurochenodeoxycholic acid acyl, hyodeoxycholic acid, or ω-muricholic acid). In some embodiments, the acyl is a pre-ketone body acyl (e.g., a pre-ketone body of the structure: 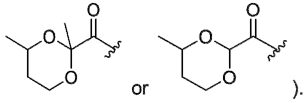 In further embodiments, the acyl is an amino acid metabolite acyl (e.g., indole-3-acetic acid acyl, indole-3-propionic acid acyl, or 3-(indole-3-yl)-acrylic acid acyl). In yet further embodiments, the acyl is a β-hydroxycarboxylate acyl (e.g., β-hydroxybutyrate acyl or carnitine acyl). In still further embodiments, the acyl is a hydroxycinnamic acid acyl (e.g., chlorogenic acid acyl). In some embodiments, the acyl is a phenolic phytochemical acyl (e.g., ellagic acid or ellagic acid analogue). In further embodiments, the acyl is a phenolic phytochemical that is a phenalkyl (e.g., ferulic acid acyl, caffeic acid acyl, or p-coumaric acid acyl). In further embodiments, the acyl is a vitamin acyl (e.g., retinoic acid acyl, vitamin B5 acyl, folic acid acyl, or niacin acyl). In some embodiments, the acyl is a pentacyclic triterpenoid acyl (e.g., ursolic acid acyl or oleanolate acyl). In other embodiments, the acyl is glutathione acyl. In yet other embodiments, the acyl is α-lipoic acid acyl. In still other embodiments, the acyl is picolinate acyl. In further embodiments, the acyl is a sugar acid acyl (e.g., gluconic acid acyl). In yet further embodiments, the acyl is mesalamine acyl. In still further embodiments, the acyl is polyamine acyl (e.g., carboxynorspermidine or carboxyspermidine).

In some embodiments of the invention, the multibiotic agent is capable of being substantially activated by a human microbiota.

In a further embodiment, a method of reducing the level of one or more markers for cardiovascular disorder in a subject is provided by administering to the subject an effective amount of a multibiotic agent comprising two or more moieties that are independently alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In further embodiments, each of the markers is independently the level of cholesterol, triglycerides, lipids, lipoprotein a (LPa), apolipoprotein C3 (Apo-C3), or blood pressure measurement. In further embodiments, the method is for treating the cardiovascular disorder. In some embodiments, a multibiotic agent having cardiovascular health benefits described herein may include an alcohol core that is an amino alcohol, antifungal agent, bile acid, β-hydroxycarboxylate, carbohydrate or an oligomer thereof, carotenoid, stilbenoid, flavonoid, lignan, phenolic phytochemical, long chain alcohol, sugar alcohol, anhydrosugar alcohol, sugar acid, or vitamin. In some embodiments, a multibiotic agent having cardiovascular health benefits described herein may include an amine core that is an amino acid, amino acid metabolite, antifungal agent, antihyperglycemic biguanide, polyamine, or vitamin. In further embodiments, a multibiotic agent having cardiovascular health benefits described herein may include an acyl that is an amino acid acyl, amino acid metabolite acyl, bile acid acyl, fatty acid acyl, β-hydroxycarboxylate acyl, phenolic phytochemical acyl (e.g., phenalkyl acyl or an acyl of ellagic acid or an analogue thereof), sugar acid acyl, or vitamin acyl.

In further embodiments, an amino alcohol is choline. In yet further embodiments, an antifungal agent is an echinocandin (e.g., micafungin, caspofungin, or anidulafungin). In still further embodiments, a bile acid is hyodeoxycholic acid or ω-muricholic acid. In further embodiments, a β-hydroxycarboxylate is carnitine. In further embodiments, a carbohydrate or an oligomer thereof is ribose or trehalose. In some embodiments, a carotenoid is astaxanthin, lutein, or zexanthin. In further embodiments, a stilbenoid is trihydroxystilbene, pterostilbene, rhapontigenin, piceatannol, pinostilbene, oxyresveratrol, or 4-methoxyresveratrol. In yet further embodiments, a flavonoid is catechin, genistein, quercetin, hesperetin, daidzein, equol, or luteolin. In still further embodiments, a long chain alcohol is policosanol. In some embodiments, a lignan is pinoresinol, laricisresinol, secoisolariciresinol, matairesinol, or 7-hydroxyenterolactone. In further embodiments, a phenolic phytochemical is ellagic acid or an analogue thereof (preferably, urolithin A, urolithin B, urolithin C, urolithin D, urolithin E, or urolithin M5). In some embodiments, a phenolic phytochemical is a phenalkyl (preferably, ferulic acid, caffeic acid, or p-coumaric acid). In further embodiments, a sugar alcohol is inositol or erythritol. In yet further embodiments, an anhydrosugar alcohol is sorbitan or isosorbide. In still further embodiments, a sugar acid is gluconic acid (e.g., D-gluconic acid).

In some embodiments, an amino acid is arginine. In further embodiments, an amino acid metabolite is indole-3-propionic acid or 3-(indole-3-yl)-acrylic acid. In some embodiments, an antifungal agent is an echinocandin (e.g., anidulafungin). In further embodiments, an anti hyperglycemic biguanide is metformin. In yet further embodiments, a polyamine is spermidine. In still further embodiments, a vitamin is folic acid.

In some embodiments, an amino acid acyl is arginine. In further embodiments, an amino acid metabolite acyl is indole-3-propionic acid acyl or 3-(indole-3-yl)-acrylic acid acyl. In some embodiments, a bile acid acyl is hyodeoxycholic acid acyl or ω-muricholic acid acyl. In further embodiments, a fatty acid acyl is linoleic acid acyl. In yet further embodiments, a β-hydroxycarboxylate acyl is carnitine acyl. In still further embodiments, a phenolic phytochemical acyl is a phenalkyl acyl (preferably, ferulic acid acyl, caffeic acid acyl, or p-coumaric acid acyl). In some embodiments, a phenolic phytochemical acid is ellagic acid acyl or an analogue thereof (preferably, urolithin A acyl, urolithin B acyl, urolithin C acyl, urolithin D acyl, urolithin E acyl, or urolithin M5 acyl). In further embodiments, a sugar acid acyl is gluconic acid acyl. In further embodiments, a vitamin acyl is vitamin B5 acyl, folate acyl, or niacin acyl.

In a yet further embodiment, a method of increasing insulin sensitivity of a subject, of modulating the level of one or more markers for diabetes in a subject, or of ameliorating one or more symptoms of diabetes in a subject is provided by administering to the subject an effective amount of a multibiotic agent comprising two or more moieties that are alcohol cores, amine cores, or acyls, where the moieties are covalently linked to each other through hydrolyzable *in vivo* bonds that are independently ester bonds, amide bonds, glycosidic bonds, azo bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In further embodiments, a multibiotic agent useful for increasing insulin sensitivity described herein includes an alcohol core that is an antifungal agent, carbohydrate or an oligomer thereof, anhydrosugar alcohol, sugar alcohol, sugar acid, bile acid, vitamin, pentacyclic triterpenoid, carotenoid, catechin polyphenol, amino alcohol, curcuminoid, flavonoid, lignan, phenolic phytochemical, stilbenoid, capsinoid, hydroxycinnamic acid ester, or β-hydroxycarboxylate. In further embodiments, a multibiotic agent useful for increasing insulin sensitivity described herein includes an amine core that is an amino acid metabolite, antihyperglycemic biguanide, or antifungal agent. In some embodiments, a multibiotic agent useful for increasing insulin sensitivity described herein includes an acyl that is an amino acid metabolite acyl, bile acid acyl, fatty acid acyl, α-lipoic acid acyl, β-hydroxycarboxylate acyl, pentacyclic triterpenoid acyl, hydroxycinnamic acid ester acyl, phenolic phytochemical acyl (e.g., a phenalkyl acyl or ellagic acid acyl or an analogue thereof), picolinate acyl, sugar acid acyl, or vitamin acyl.

In some embodiments, an antifungal agent is an echinocandin (e.g., micafungin, caspofungin, or anidulafungin). In some embodiments, a carbohydrate or an oligomer thereof is ribose or trehalose. In further embodiments, an anhydrosugar alcohol is sorbitan or isosorbide. In further embodiments, a sugar alcohol is erythritol or inositol. In yet further embodiments, a bile acid is hyodeoxycholic acid or ω-muricholic acid. In still further embodiments, a vitamin is vitamin B5 or vitamin D3. In other embodiments, a pentacyclic triterpenoid is ursolic acid. In yet other embodiments, a carotenoid is fucoxanthin or astaxanthin. In still other embodiments, a catechin polyphenol is epigallocatechin gallate. In some embodiments, an amino alcohol is ephedrine. In further embodiments, a curcuminoid is curcumin. In further other embodiments, a sugar acid is gluconic acid. In some embodiments, a flavonoid is trihydroxystilbene, pterostilbene, rhapontigenin, piceatannol, pinostilbene, oxyresveratrol, or 4-methoxyresveratrol. In further embodiments, a lignan is secoisolariciresinol, pinoresinol, lariciresinol, matairesinol, or 7-hydroxyenterolactone. In yet further embodiments, a phenolic phytochemical is a phenalkyl (e.g., 4-(4-hydroxyphenyl)butan-2-one, ferrulic acid, caffeic acid, or p-coumaric acid). In still further embodiments, a phenolic phytochemical is ellagic acid or an analogue thereof (e.g., urolithin A, urolithin B, urolithin C, urolithin D, urolithin E, or urolithin M5). In other embodiments, a stilbenoid is trihydroxystilbene, pterostilbene, rhapontigenin, piceatannol, pinostilbene, oxyresveratrol, or 4-methoxyresveratrol. In yet other embodiments, a capsinoid is capsiate. In still other embodiments, a hydroxycinnamic acid ester is chlorogenic acid. In some embodiments, a β-hydroxycarboxylate is carnitine.

In some embodiments, an amino acid metabolite is indole-3-propionic acid or 3-(indole-3-yl)-acrylic acid. In further embodiments, an antihyperglycemic biguanide is metformin. In other embodiments, antifungal agent is an echinocandin (e.g., caspofungin).

In some embodiments, an amino acid metabolite acyl is indole-3-propionic acid acyl or 3-(indole-3-yl)-acrylic acid acyl. In some embodiments, a bile acid acyl is hyodeoxycholic acid or ω-muricholic acid. In further embodiments, a fatty acid acyl is acetyl, propionyl, butyryl, eicosapentanoic acid acyl, docosahexaenoic acid acyl, punicic acid acyl, or α-linolenic acid acyl. In further embodiments, a β-hydroxycarboxylate acyl is carnitine acyl. In yet further embodiments, a pentacyclic triterpenoid acyl is ursolic acid acyl. In still further embodiments, a hydroxycinnamic acid ester acyl is chlorogenic acid acyl. In still further embodiments, a phenolic phytochemical acyl is a phenalkyl acyl (e.g., ferulic acid acyl, caffeic acid acyl, or p-coumaric acid acyl). In some embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In further embodiments, a sugar acid acyl is gluconic acid acyl. In some embodiments, a vitamin acyl is vitamin B5 acyl.

In yet other embodiments, the modulating the level of one or more markers for diabetes is increasing the level of one or more markers for diabetes, where each of the markers is independently insulin level, level of GLP-1, or level of PYY. In still other embodiments, the modulating the level of one or more markers for diabetes is reducing the level of one or more markers for diabetes, where each of the markers is independently blood sugar level or hemoglobin A1c level. In some embodiments, the method is for ameliorating a symptom of diabetes that is peripheral pain, numbness, or a neuropathic symptom. In further embodiments, the method is for treating diabetes in the subject.

In a further embodiment, a method of modulating the level of one or more markers for inflammatory bowel disease in a subject is provided by administering to the subject in need thereof an effective amount of a multibiotic agent comprising two or more moieties that are alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In further embodiments, a multibiotic agent includes an alcohol core that is a bile acid, catechin polyphenol, carbohydrate or an oligomer thereof, curcuminoid, flavonoid, phenolic phytochemical, stilbenoid, sugar alcohol, vitamin, or mesalamine. In further embodiments, a multibiotic agent of the invention includes an alcohol core that is a bile acid, catechin polyphenol, carbohydrate or an oligomer thereof, curcuminoid, flavonoid, phenolic phytochemical, stilbenoid, sugar alcohol, vitamin, mesalamine, ketone body, or pre-ketone body. In some embodiments, a multibiotic agent of the invention includes an amine core that is an antihyperglycemic biguanide, amino acid metabolite or mesalamine. In further embodiments, a multibiotic agent of the invention includes an acyl that is a bile acid acyl, fatty acid acyl, vitamin acyl, amino acid metabolite acyl, phenolic phytochemical acyl, or mesalamine acyl. In yet further embodiments, a multibiotic agent of the invention includes an acyl that is a bile acid acyl, fatty acid acyl, vitamin acyl, amino acid metabolite acyl, phenolic phytochemical acyl, mesalamine acyl, ketone body acyl, or pre-ketone body acyl.

In some embodiments, a bile acid is hyodeoxycholic acid or ω-muricholic acid. In further embodiments, a catechin polyphenol is epigallocatechin gallate. In further embodiments, a carbohydrate or an oligomer thereof is trehalose or ribose. In yet further embodiments, a curcuminoid is curcumin. In still further embodiments, a flavonoid is hesperetin, daidzein, or luteolin. In other embodiments, a phenolic phytochemical is a phenalkyl (e.g., ferulic acid, caffeic acid, or p-coumaric acid). In yet other embodiments, a phenolic phytochemical is ellagic acid or an analogue thereof (e.g., urolithin A, urolithin B, urolithin C, urolithin D, urolithin E, or urolithin M5). In still other embodiments, a stilbenoid is trihydroxystilbene, rhapontigenin, pinostilbene, oxyresveratrol, or 4-methoxyresveratrol. In some embodiments, a sugar alcohol is inositol. In yet further embodiments, a vitamin is vitamin B5.

In some embodiments, an antihyperglycemic biguanide is metformin. In some embodiments, an amino acid metabolite is indole-3-acetic acid, indole-3-propionic acid, or 3-(indole-3-yl)-acrylic acid.

In further embodiments, a bile acid acyl is hyodeoxycholic acid or ω-muricholic acid. In further embodiments, a fatty acid acyl is acetyl, propionyl, butyryl, isobutyryl, valeryl, or isovaleryl. In yet further embodiments, a vitamin acyl is retinoic acid acyl or vitamin B5 acyl. In still further embodiments, an amino acid metabolite acyl is indole-3-acetic acid acyl, indole-3-propionic acid acyl, or 3-(indole-3-yl)-acrylic acid acyl. In other embodiments, a phenolic phytochemical acyl is a phenalkyl acyl (e.g., ferulic acid acyl, caffeic acid acyl, or p-coumaric acid acyl). In yet other embodiments, a phenolic phytochemical acyl is ellagic acid or an analogue thereof (e.g., urolithin A acyl, urolithin B acyl, urolithin C acyl, urolithin D acyl, urolithin E acyl, or urolithin M5 acyl).

In some embodiments, the modulating the level of one or more markers for inflammatory bowel disease is increasing the level of one or more markers for inflammatory bowel disease, where each marker is independently intestinal motility, Treg differentiation, or mucus secretion. In some embodiments, the modulating the level of one or more markers for inflammatory bowel disease is reducing the level of one or more markers for inflammatory bowel disease, where each marker is independently abdominal pain, gastrointestinal inflammation, gastrointestinal permeability, gastrointestinal bleeding, intestinal motility, or frequency of bowel movements. In further embodiments, gastrointestinal inflammation is an inflammation of upper intestine, cecum, ileum, colon, or rectum. In further embodiments, the administering restores gastrointestinal mucosal health. In yet further embodiments, the method is for treating inflammatory bowel disease in a subject.

In a still further embodiment, a method of treating constipation (e.g., constipation secondary to a neurodegenerative disease (e.g., Parkinson's disease)) in a subject is provided by administering to the subject in need thereof an effective amount of a multibiotic agent comprising two or more moieties that are alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In further embodiments, a multibiotic agent includes an alcohol core that is a bile acid, catechin polyphenol, carbohydrate or an oligomer thereof, curcuminoid, flavonoid, phenolic phytochemical, stilbenoid, sugar alcohol, vitamin, or mesalamine. In some embodiments, a multibiotic agent includes an amine core that is an antihyperglycemic biguanide, amino acid metabolite or mesalamine. In further embodiments, a multibiotic agent includes an acyl that is a bile acid acyl, fatty acid acyl, vitamin acyl, amino acid metabolite acyl, phenolic phytochemical acyl, or mesalamine acyl.

In some embodiments, a bile acid is hyodeoxycholic acid or ω-muricholic acid. In further embodiments, a catechin polyphenol is epigallocatechin gallate. In further embodiments, a carbohydrate or an oligomer thereof is trehalose or ribose. In yet further embodiments, a curcuminoid is curcumin. In still further embodiments, a flavonoid is hesperetin, daidzein, or luteolin. In other embodiments, a phenolic phytochemical is a phenalkyl (e.g., ferulic acid, caffeic acid, or p-coumaric acid). In yet other embodiments, a phenolic phytochemical is ellagic acid or an analogue thereof (e.g., urolithin A, urolithin B, urolithin C, urolithin D, urolithin E, or urolithin M5). In still other embodiments, a stilbenoid is trihydroxystilbene, rhapontigenin, pinostilbene, oxyresveratrol, or 4-methoxyresveratrol. In some embodiments, a sugar alcohol is inositol. In yet further embodiments, a vitamin is vitamin B5.

In further embodiments, an antihyperglycemic biguanide is metformin. In some embodiments, an amino acid metabolite is indole-3-acetic acid, indole-3-propionic acid, or 3-(indole-3-yl)-acrylic acid.

In further embodiments, a bile acid acyl is hyodeoxycholic acid or ω-muricholic acid. In further embodiments, a fatty acid acyl is acetyl, propionyl, butyryl, isobutyryl, valeryl, or isovaleryl. In yet further embodiments, a vitamin acyl is retinoic acid acyl or vitamin B5 acyl. In still further embodiments, an amino acid metabolite acyl is indole-3-acetic acid acyl, indole-3-propionic acid acyl, or 3-(indole-3-yl)-acrylic acid acyl. In other embodiments, a phenolic phytochemical acyl is a phenalkyl acyl (e.g., ferulic acid acyl, caffeic acid acyl, or p-coumaric acid acyl). In yet other embodiments, a phenolic phytochemical acyl is ellagic acid or an analogue thereof (e.g., urolithin A acyl, urolithin B acyl, urolithin C acyl, urolithin D acyl, urolithin E acyl, or urolithin M5 acyl).

In some embodiments, the method increases one or more of intestinal motility, mucus secretion, and frequency of bowel movements. In further embodiments, the method reduces one or more of abdominal pain, gastrointestinal inflammation, gastrointestinal permeability, and gastrointestinal bleeding. In some embodiments, gastrointestinal inflammation is an inflammation of upper intestine, cecum, ileum, colon, or rectum. In further embodiments, the administering restores gastrointestinal mucosal health.

In a further embodiment, a method of ameliorating an infectious disease in a subject is provided by administering to the subject in need thereof an effective amount of a multibiotic agent comprising two or more moieties that are independently alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In some embodiments, a multibiotic agent having anti-infectious disease health benefits described herein includes an alcohol core that is an antifungal agent, amino acid metabolite, flavonoid, lignan, phenolic phytochemical, stilbenoid, carbohydrate or an oligomer thereof, or sugar alcohol. In other embodiments, a multibiotic agent having anti-infectious disease health benefits described herein includes an amine core that is an antifungal agent, antihyperglycemic biguanide, or amino acid metabolite. In some embodiments, a multibiotic agent having anti-infectious disease health benefits described herein includes an acyl that is an amino acid metabolite acyl, fatty acid acyl, or phenolic phytochemical acyl.

In some embodiments, an antifungal agent is an echinocandin (e.g., micafungin, caspofungin, or anidulafungin). In some embodiments, a flavonoid is daidzein or luteolin. In further embodiments, a lignan is pinoresinol, lariciresinol, secoisolariciresinol, matairesinol, or 7-hydroxyenterolactone. In yet further embodiments, a phenolic phytochemical is a phenalkyl (e.g., ferulic acid, caffeic acid, or p-coumaric acid). In still further embodiments, a stilbenoid is piceatannol, pinostilbene, oxyresveratrol, or 4-methoxyresveratrol. In other embodiments, a carbohydrate or an oligomer thereof is ribose or trehalose. In yet other embodiments, a sugar alcohol is inositol or erythritol. In still other embodiments, an antihyperglycemic biguanide is metformin. In some embodiments, an amino acid metabolite is indole-3-propionic acid or 3-(indole-3-yl)-acrylic acid.

In some embodiments, an amino acid metabolite acyl is indole-3-propionic acid acyl or 3-(indole-3-yl)-acrylic acid acyl. In further embodiments, a fatty acid acyl is formyl, acetyl, propionyl, butyryl, isobutyryl, valeryl, isovaleryl, hexanoyl, heptanoyl, octanoyl, nonanoyl, decanoyl, dodecanoyl, myristyl, palmityl, stearyl, arachidyl, docosanoyl, tetracosanoyl, or hexacosanoyl. In yet further embodiments, a phenolic phytochemical acyl is a phenalkyl acyl (e.g., ferulic acid acyl, caffeic acid acyl, or p-coumaric acid acyl).

In still further embodiments, the infectious disease is a candida infection or C. difficile infection. In some embodiments, the infectious disease is a vaginal yeast infection. In further embodiments, the administering reduces candida CFU counts. In some embodiments, the candida CFU counts are gastrointestinal, mucosal, oral, or epithelial counts. In other embodiments, the method is for treating an infectious disease.

In a further embodiment, a method of promoting muscle growth in a subject is provided by administering to the subject in need thereof an effective amount of a multibiotic agent comprising two or more moieties that are independently alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In further embodiments, a multibiotic agent having muscle growth health benefits described herein includes an alcohol core that is an antifungal agent, anhydrosugar alcohol, carbohydrate or an oligomer thereof, sugar alcohol, sugar acid, bile acid, flavonoid, lignan, phenolic phytochemical, stilbenoid, vitamin, or pentacyclic triterpenoid. In some embodiments, a multibiotic agent having muscle growth health benefits described herein includes an amine core that is an anti hyperglycemic biguanide, amino acid metabolite, antifungal agent, polyamine, or vitamin. In some embodiments, a multibiotic agent having muscle growth health benefits described herein includes an acyl that is a bile acid acyl, fatty acid acyl, vitamin acyl, pentacyclic triterpenoid acyl, phenolic phytochemical acyl, amino acid acyl, amino acid metabolite acyl, or sugar acid acyl.

In some embodiments, an antifungal agent is an echinocandin (e.g., micafungin, caspofungin, or anidulafungin). In some embodiments, an anhydrosugar alcohol is sorbitan or isosorbide. In further embodiments, a carbohydrate or an oligomer thereof is ribose or trehalose. In yet further embodiments, a sugar alcohol is inositol or erythritol. In still further embodiments, a sugar acid is gluconic acid. In other embodiments, a bile acid is hyodeoxycholic acid or ω-muricholic acid. In yet other embodiments, a flavonoid is daidzein, equol, or luteolin. In still other embodiments, a lignan is pinoresinol, lariciresinol, secoisolariciresinol, matairesinol, or 7-hydroxyenterolactone. In some embodiments, a phenolic phytochemical is ellagic acid or an analogue thereof (e.g., urolithin A, urolithin B, urolithin C, urolithin D, urolithin E, or urolithin M5). In some embodiments, a phenolic phytochemical is a phenalkyl (e.g., ferulic acid, caffeic acid, or p-coumaric acid). In further embodiments, a stilbenoid is piceatannol, pinostilbene, oxyresveratrol, or 4-methoxyresveratrol. In further embodiments, a vitamin is vitamin B5, vitamin D2, or vitamin E. In yet further embodiments, a pentacyclic triterpenoid is ursolic acid. In some embodiments, an antihyperglycemic biguanide is metformin. In further embodiments, an amino acid metabolite is indole-3-propionic acid or 3-(indole-3-yl)-acrylic acid.

In some embodiments, a bile acid acyl is hyodeoxycholic acid acyl or ω-muricholic acid acyl. In some embodiments, a fatty acid acyl is eicosapentaenoic acid acyl or docosahexaenoic acid acyl. In further embodiments, a vitamin acyl is a vitamin B5 acyl. In further embodiments, a pentacyclic triterpenoid acyl is ursolic acid acyl. In yet further embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In still further embodiments, an amino acid acyl is ornithine acyl or citrulline acyl. In other embodiments, an amino acid metabolite acyl is indole-3-propionic acid acyl or 3-(indole-3-yl)-acrylic acid acyl. In yet other embodiments, a sugar acid acyl is gluconic acid acyl.

In a further embodiment, a method of increasing muscle mass or of reducing one or more markers for obesity in a subject is provided by administering to the subject in need thereof an effective amount of a multibiotic agent comprising two or more moieties that are independently alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In some embodiments, a multibiotic agent having muscle mass-increasing health benefits and/or obesity marker(s) reducing effects described herein includes an alcohol core that is a bile acid, flavonoid, pentacyclic triterpenoid, carbohydrate or an oligomer thereof, catechin polyphenol, amino alcohol, curcuminoid, carotenoid, lignan, stilbenoid, phenolic phytochemical, sugar alcohol, hydroxycinnamic acid ester, β-hydroxycarboxylate, ketone body, or pre-ketone body. In some embodiments, a multibiotic agent having muscle mass-increasing health benefits and/or obesity marker(s) reducing effects described herein includes an amine core that is an antihyperglycemic biguanide, antifungal agent, or amino acid metabolite. In some embodiments, a multibiotic agent having muscle mass-increasing health benefits and/or obesity marker(s) reducing effects described herein includes an acyl that is a a bile acid acyl, fatty acid acyl, pentacyclic triterpenoid acyl, catechin polyphenol acyl, phenolic phytochemical acyl, amino alcohol acyl, amino acid metabolite acyl, hydroxycinnamic acid ester acyl, β-hydroxycarboxylate acyl, ketone body acyl, or pre-ketone body acyl.

In some embodiments, a bile acid is chenodeoxycholic acid, cholic acid, deoxycholic acid, glycocholic acid, glycochenodeoxycholic acid, lithocholic acid, taurocholic acid, taurochenodeoxycholic acid, hyodeoxycholic acid, or ω-muricholic acid. In other embodiments, a flavonoid is apigenin, naringenin, daidzein, equol, or luteolin. In some embodiments, a pentacyclic triterpenoid is ursolic acid. In other embodiments, a carbohydrate or an oligomer thereof is ribose or trehalose. In further embodiments, a catechin polyphenol is epigallocatechin gallate. In yet further embodiments, an amino alcohol is ephedrine. In still further embodiments, a curcuminoid is curcumin. In other embodiments, a carotenoid is astaxanthin. In yet other embodiments, a lignan is pinoresinol, laricisiresinol, secoisolariciresinol, matairesinol, or 7-hydroxyenterolactone. In still other embodiments, a stilbenoid is trihydroxystilbene, piceatannol, pinostilbene, oxyresveratrol, or 4-methoxyresveratrol. In some embodiments, a phenolic phytochemical is ellagic acid or an analogure thereof (e.g., urolithin A, urolithin B, urolithin C, urolithin D, urolithin E, or urolithin M5). In other embodiments, a phenolic phytochemical is a phenalkyl (e.g., ferulic acid, caffeic acid, or p-coumaric acid). In some embodiments, a sugar alcohol is inositol or erythritol. In further embodiments, a hydroxycinnamic acid ester is chlorogenic acid. In yet further embodiments, β-hydroxycarboxylate is β-hydroxybutyrate. In further embodiments, a pre-ketone body is butane-1,3-diol or 4-hydroxybutan-2-one. In still further embodiments, a ketone body is β-hydroxybutyrate. In other mebodiments, an antihyperglycemic biguanide is metformin. In yet other embodiments, an antifungal agent is an echinocandsin (e.g., micafungin, caspofungin, or anidulafungin). In still further embodiments, an amino acid metabolite is indole-3-propionic acid or 3-(indole-3-yl)-acrylic acid.

In some embodiments, a bile acid acyl is chenodeoxycholic acid acyl, cholic acid acyl, deoxycholic acid acyl, glycocholic acid acyl, glycochenodeoxycholic acid acyl, lithocholic acid acyl, taurocholic acid acyl, taurochenodeoxycholic acid acyl, hyodeoxycholic acid acyl, or ω-muricholic acid acyl. In other embodiments, a pentacyclic triterpenoid acyl is ursolic acid acyl. In some embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In further embodiments, a phenolic phytochemical acyl is a phenalkyl acyl (e.g., ferulic acid, caffeic acid, or p-coumaric acid). In yet further embodiments, a fatty acid acyl is acetyl, propionyl, butyryl, octanoyl, decanoyl, eicosapentaenoyl, docosahexaenoyl, punicic acid acyl, dihomo-γ-linolenic acid acyl, docosapentanoyl, succinyl, succin-diyl, or α-linolenic acid acyl. In further embodiments, a fatty acid acyl is a medium chain fatty acid acyl (e.g., octanoyl or decanoyl). In still further embodiments, an amino acid metabolite acyl is indole-3-propionic acid acyl or 3-(indole-3-yl)-acrylic acid acyl. In other embodiments, hydroxycinnamic acid ester acyl is chlorogenic acid acyl. In yet other embodiments, β-hydroxycarboxylate acyl is β-hydroxybutyrate acyl. In still other embodiments, ketone body acyl is β-hydroxybutyrate acyl. In some embodiments, pre-ketone body acyl is:

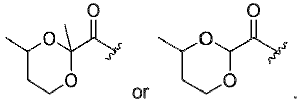

In other embodiments, each marker for obesity is independently total fat percentage, cellular adiposity, rate of weight gain, abdominal fat quantity, or ratio of white to brown fat. In some embodiments, the method is for ameliorating obesity in the subject.

In yet another embodiment, a method of promoting skin health in a subject is provided by administering to the subject in need thereof an effective amount of a multibiotic agent comprising two or more moieties that are independently alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In still another embodiment, a method of ameliorating inflammation in a subject is provided by administering to the subject in need thereof an effective amount of a multibiotic agent comprising two or more moieties that are alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds and/or amide bonds hydrolyzable *in vivo.*

In other embodiments, a multibiotic agent having anti-inflammatory and/or anti-autoimmune health benefits described herein includes an alcohol core that is an an antifungal agent, carbohydrate or an oligomer thereof, anhydrosugar alcohol, sugar alcohol, bile acid, catechin polyphenol, flavonoid, stilbenoid, phenolic phytochemical, or vitamin. In some embodiments, a multibiotic agent having anti-inflammatory and/or anti-autoimmune health benefits described herein includes an amino acid metabolite, antihyperglycemic biguanide, or vitamin. In some embodiments, a multibiotic agent having anti-inflammatory and/or anti-autoimmune health benefits described herein includes an acyl that is an amino acid metabolite acyl, bile acid acyl, fatty acid acyl, phenolic phytochemical acyl, vitamin acyl, amino acid metabolite acyl, or sugar acid acyl.

In some embodiments, an antifungal agent is an echinocandin (e.g., micafungin, caspofungin, or anidulafungin). In other embodiments, a carbohydrate or an oligomer thereof is xylose, ribose, or an oligomer thereof, or trehalose. In some embodiments, an anhydrosugar alcohol is isosorbide or sorbitan. In further embodiments, a sugar alcohol is erythritol or inositol. In yet further embodiments, a bile acid is hyodeoxycholic acid or ω-muricholic acid. In still further embodiments, a catechin polyphenol is epigallocatechin gallate. In some embodiments, a flavonoid is hesperetin, daidzein, equol, or luteolin. In other embodiments, a stilbenoid is trihydroxystilbene, rhapontigenin, piceatannol, pinostilbene, oxyresveratrol, or 4-methoxyresveratrol. In some embodiments, a phenolic phytochemical is ellagic acid or an analogue thereof (e.g., urolithin A, urolithin B, urolithin C, urolithin D, urolithin E, or urolithin M5). In other embodiments, a phenolic phytochemical is a phenalkyl (e.g., ferulic acid, caffeic acid, p-coumaric acid, 6-gingerol, or 6-shogaol). In yet other embodiments, a vitamin is vitamin B5.

In some embodiments, an amino acid metabolite is indole-3-acetic acid, indole-3-propionic acid, or 3-(indole-3-yl)-acrylic acid. In other embodiments, an antihyperglycemic biguanide is metformin.

In some embodiments, an amino acid metabolite acyl is indole-3-acetic acid acyl, indole-3-propionic acid acyl, or 3-(indole-3-yl)-acrylic acid acyl. In further embodiments, a bile acid acyl is hyodeoxycholic acid acyl or ω-muricholic acid acyl. In yet further embodiments, a fatty acid acyl is acetyl, propionyl, or butyryl. In still further embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In other embodiments, a phenolic phytochemical is a phenalkyl acyl (e.g., ferulic acid acyl, caffeic acid acyl, or p-coumaric acid acyl). In yet other embodiments, a vitamin acyl is retinoic acid acyl or vitamin B5 acyl. In still other embodiments, a sugar acid acyl is gluconic acid acyl.

In another embodiment, a method of ameliorating nonalcoholic fatty liver disease or nonalcoholic steatohepatitis in a subject is provided by administering to the subject in need thereof an effective amount of a multibiotic agent comprising two or more moieties that are independently alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers. In some embodiments, the multibiotic agent comprises one or more alcohol cores and/or amine cores bonded to one or more acyls through one or more ester bonds, glycosidic bonds, and/or amide bonds hydrolyzable *in vivo.*

In some embodiments, each alcohol core is independently an amino acid. In other embodiments, each acyl is independently a fatty acid acyl, where the multibiotic agent is not inulin propionate.

In some embodiments, the alcohol core is an amino acid that is glutamine.

In further embodiments, the acyl is a fatty acid acyl.

In yet further embodiments, the method is for treating nonalcoholic fatty liver disease or nonalcoholic steatohepatitis.

In still further embodiments, the multibiotic agent is administered orally or parenterally (e.g., topically).

In some embodiments, the multibiotic agent is substantially activated by a microbiota of the subject.

In yet another embodiment, a topical formulation comprising a multibiotic agent comprising two or more moieties that are independently alcohol cores, amine cores, or acyls, where the moieties are covalently linked to each other through hydrolyzable *in vivo* bonds that are independently ester bonds, amide bonds, azo bonds, glycosidic bonds, carbonate linkers, or carbamate linkers is provided. In some embodiments, the multibiotic agent comprises one or more alcohol cores and/or amine cores bonded to one or more acyls through one or more ester bonds and/or amide bonds hydrolyzable *in vivo.*

In some embodiments, each alcohol core is independently an antifungal agent, vitamin, carotenoid, phenolic phytochemical, polyphenol, or telomerase activator. In other embodiments, each acyl is independently a fatty acid acyl, α-lipoic acid acyl, phenolic phytochemical acyl, sugar acid acyl, amino acid metabolite acyl, vitamin acyl, or glutathione acyl.

In some embodiments, the alcohol core is a vitamin (e.g., ascorbic acid, vitamin B5, or vitamin E). In other embodiments, the alcohol core is a carotenoid (e.g., astaxanthin or zeaxanthin). In yet other embodiments, the alcohol core is a phenolic phytochemical (e.g., phenalkyl, ellagic acid, ellagic acid analogue, or hydroxytyrosol). In still other embodiments, the alcohol core is a stilbenoid multimer (e.g., a viniferin (e.g., α-viniferin, β-viniferin, γ-viniferin, δ-viniferin, or ε-viniferin)). In further embodiments, the alcohol core is a telomerase activator (e.g., cycloastragenol). In other embodiments, the alcohol core is an anhydrosugar alcohol (e.g., sorbitan or isosorbide). In some embdoiments, the alcohol core is a sugar alcohol (e.g., inositol or erythritol). In further embodiments, the alcohol core is a carbohydrate or an oligomer thereof (e.g., ribose or trehalose). In yet further embodiments, the alcohol core is a sugar acid (e.g., gluconic acid). In still further embodiments, the alcohol core is an antifungal agent (e.g., an echinocandin (e.g., micafungin, caspofungin, or anidulafungin)). In some embodiments, the alcohol core is a flavonoid (e.g., hesperetin, daidzein, equol, or luteolin). In other embodiments, the alcohol core is a lignan (e.g., pinoresinol, lariciresinol, secoisolariciresinol, matairesinol, or 7-hydroxyenterolactone).

In some embodiments, the amine core is an amino acid metabolite (e.g., indole-3-propionic acid or 3-(indole-3-yl)-acrylic acid). In further embodiments, the amine core is an antifungal agent (e.g., an echinocandin (e.g., caspofungin)). In yet further embodiments, the amine core is a vitamin (e.g., vitamin B5). In still further embodiments, the amine core is an antihyperglycemic biguanide (e.g., metformin). In other embodimetns, the amine core is glutathione. In yet other embodiments, the acyl is sugar acid acyl (e.g., gluconic acid acyl).

In still other embodiments, the acyl is amino acid metabolite acyl (e.g., indole-3-propionic acid acyl or 3-(indole-3-yl)-acrylic acid). In some embodiments, the acyl is vitamin acyl that is vitamin B5 acyl. In other embodiments, the acyl is a fatty acid acyl (e.g., dihomo-γ-linolenic acid acyl, docosapentanoyl, succinyl, or succin-diyl). In some embodiments, the acyl is α-lipoic acid acyl. In further embodiments, the acyl is a phenolic phytochemical acyl (e.g., ellagic acid acyl or ellagic acid analogue acyl). In yet further embodiments, the acyl is glutathione acyl.

In still further embodiments, the multibiotic agent is substantially activated by a human skin microbiota.

In another aspect of the present invention, provided is a method of delivering a biologically active compound to a target site in a subject. The method includes administering the multibiotic agent disclosed herein to the subject, and the target site comprising an enzyme capable of cleaving the multibiotic agent to release the alcohol core, the amine core, and/or the acyl.

### Definitions

The term "about," as used herein, represents a number that is 90% to 110% of the recited value.

The term "acyl," as used herein, represents a monovalent group of formula -C(O)-R, where R is H, alkyl, alkenyl, aryl, or heteroaryl. The term acyl further encompasses the acyls disclosed herein. Acyl may be optionally substituted as described herein for each group. An acyl is typically a group formed by replacing -OH in a compound -COOH with a valency.

The term "acyloxy," as used herein, represents a monovalent group of formula -OR, where R is acyl. Acyloxy may be optionally substituted as described herein for acyl.

The term "alcoholic oxygen atom," as used herein, refers to a divalent oxygen atom bonded to at least one *sp*³-hybridized carbon atom.

The term "alkanoyl," as used herein, represents a monovalent group formed by replacing a carboxylate hydroxyl in a carboxylic acid with a valency.

The term "alkenyl," as used herein, represents a linear or branched, monovalent hydrocarbon group containing from 1 to 24 carbon atoms and from 1 to 6 carbon-carbon double bonds. In some embodiments, alkenyl contains from 1 to 18 carbon atoms (e.g., from 1 to 12 carbon atoms). Alkenyl may be optionally substituted as described herein for alkyl.

The term "alkoxy," as used herein, represents a monovalent group of formula -OR, where R is alkyl. Alkoxy may be optionally substituted as described herein for alkyl.

The term "alkyl," as used herein, represents a linear or branched, monovalent, saturated hydrocarbon group containing from 1 to 24 carbon atoms. In some embodiments, alkyl contains from 1 to 19 carbon atoms (e.g., from 1 to 12 carbon atoms). Alkyl may be optionally substituted with 1, 2, 3, 4, 5, or 6 substituents that are independently oxo, hydroxy, protected hydroxy, acyloxy, alkoxy, amino, - NHCONH₂, protected amino, optionally substituted aryl, or optionally substituted heteroaryl. In some substituted alkyl groups, one or more substituents may be further substituted with unsubstituted substituents as described for each respective substituent.

The term "alkylene," as used herein refers to a divalent group that is an alkyl, in which one hydrogen atom is replaced with a valency. Alkylene may be optionally substituted as described herein for alkyl. Non-limiting examples of unsubstituted alkylenes include: methylene, ethane-1,1-diyl, ethane-1,2-diayl, propane-1,3-diyl, propane-1,2-diyl, propane-1,2-diyl, butane-1,4-diyl, butane-1,3-dyl, butane-1,2-diyl, 2-methylpropane-1,1-diyl, 2-methylpropane-1,2-diyl, 2-methylpropane-1,3-diyl, 2-methyl-butane-1,1-diyl, 2-methyl-butane-1,2-diyl, 2-methyl-butane-1,3-diyl, 2-methyl-butane-1,4-diyl, 3-methyl-butane-1,1-diyl, 3-methyl-butane-1,2-diyl, and 3-methyl-butane-1,3-diyi.

The term "amide bond," as used herein, refers to a covalent bond between an amino nitrogen atom and a carbonyl group of an acyl in a multibiotic agent.

The term "amino," as used herein, represents -NH₂.

The term "amino acid," as used herein, represents proline, taurine, or a compound having an amino group and a carboxylate or sulfonate group separated by an optionally substituted alkylene or optionally substituted arylene. Amino acids are small molecules and have a molecular weight of < 900 g/mol (preferably, < 500 g/mol). Preferably, when the linker is alkylene, the linker may be optionally substituted as described herein for alkyl. In some embodiments, optionally substituted alkylene is an alkylene substituted with 1 or 2 groups that are independently hydroxyl, thiol, amino, guanidine, carbamoylamino, imidazolyl, indolyl, -SeH, oxo, 4-hydroxyphenyl, phenyl, or -SMe. Non-limiting examples of amino acids include alanine, arginine, asparagine, aspartic acid, cysteine, glutamic acid, glutamine, glycine, histidine, isoleucine, leucine, lysine, methionine, phenylalanine, proline, selenocysteine, serine, threonine, tyrosine, tryptophan, ornithine, citrulline, aminobenzoic acid, taurine, and carglumic acid.

The term "amino acid metabolite," as used herein, represents proteinogenic amino acids, in which the α-amino group is replaced with -OH or -H, in which the 1-carboxyl group is replaced with H, in which he α-(CHNH₂) group is replaced with a carbonyl, in which he α-amino group and β-hydrogen atom are replaced with a double bond, or in which the 1-carboxyl group is replaced with hydroxyl and the α-(CHNH₂) group is replaced with a carbonyl. Alternatively or additionally, amino acid metabolites of proteinogenic amino acids having an aryl or heteroaryl group in a sidechain may include substitution of one or two hydrogen atoms bonded to aromatic carbon atom(s) with -OH. Alternatively or additionally, an amino acid metabolite of tryptophan may be kynurenine. Non-limiting examples of amino acid metabolites include tyramine, indole-3-acetic acid, indole-3-propionic acid, 3-(indole-3-yl)-acrylic acid, kynurenine, 5-hydroxytryptamine, indole-3-pyruvic acid, 3-(indol-3-yl)-2-hydroxypropionic acid, and 2-hydroxyethyl-3-indole.

The term "amino nitrogen atom," as used herein, refers to a trivalent, non-aromatic nitrogen atom, where the first valency of the nitrogen atom is bonded to a carbon atom within the amine core, and (i) the second and the third valencies combine to form a double bond to an amino nitrogen atom in another amine core, or (ii) the second valency is bonded to a hydrogen or carbon atom within the amine core, and the third valency is bonded to a carbonyl group in an acyl or carbamate linker.

The term "anhydrosugar alcohol," as used herein, refers to a singly or doubly dehydrated sugar alcohol. An anhydrosugar alcohol may contain one tetrahydrofuran ring, one tetrahydropyran ring, or two fused tetrahydrofuran rings. Non-limiting examples of anhydrosugar alcohols include sorbitan and isosorbide.

The term "antihyperglycemic biguanide," as used herein, refers to a compound of formula: 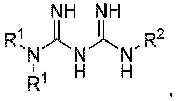 where
each R¹ is independently H or optionally substituted C₁₋₆ alkyl; and
R² is H or a bond to an acyl.
If the antihyperglycemic biguanide is present in a multibiotic agent, R² is a bond to an acyl. Non-limiting examples of antihyperglycemic biguanides include metformin.

The term "aryl," as used herein, is a monovalent or multivalent group consisting of one ring of carbon atoms or two, three, or four fused rings of carbon atoms, provided that at least one of the rings in aryl is π-aromatic. An unsubstituted aryl group typically contains from six to eighteen carbon atoms. An aryl group may be optionally substituted with 1, 2, 3, 4, or 5 substituents that are independently alkyl, hydroxy, protected hydroxy, acyloxy, alkoxy, amino, protected amino, or heteroaryl. The term "aryl" further encompasses monovalent or multivalent groups consisting of alcohol and/or acyl cores disclosed herein that contain at least one optionally substituted aromatic ring bonded to one or more hydroxyl and/or acyl groups, in which at least one hydroxyl group is replaced with a valency. In some substituted aryl groups, one or more substituents may be further substituted with unsubstituted substituents as described for each respective substituent type.

The term "arylene," as used herein, is a divalent group that is an aryl group, in which one hydrogen atom is replaced with a valency. Arylene may be optionally substituted as described herein for aryl. Non-limiting examples of arylenes include phenylene (e.g., 1,2-phenylene, 1,3-phenylene, and 1.4-phenylene).

The term "azo bond," as used herein, refers to a covalent, double bond between an amino nitrogen atom in one amine core and an amino nitrogen atom in another amine core.

The term "bile acid," as used herein, represents a compound of formula: 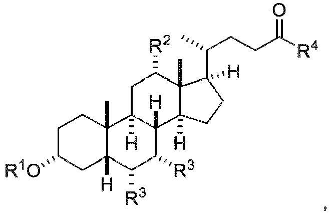 where
R¹ is H, an O-protecting group, or a bond to an acyl;
each of R² and R³ is independently H, hydroxyl, a protected hydroxyl,-OR^{A}, or alkyl (e.g., ethyl);
R⁴ is hydroxyl, a protected hydroxyl, -R^{B}, -NH-CH(R^{C})-CO-R^{D}, or an amino sulfonic acid;
each R^{A}, when present, is independently a bond to an acyl;
R^{B}, when present, is a bond to an alcohol core or an amine core;
R^{C}, when present, is a side chain of a proteinogenic amino acid; and
R^{D}, when present, is hydroxyl, a protected hydroxyl, or a bond to an alcohol core or an amine core.

In some embodiments, bile acid is a compound of formula: 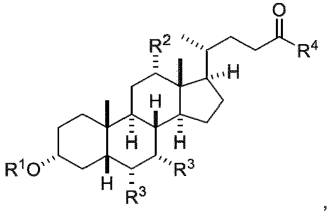 where
R¹ is H, an O-protecting group, or a bond to an acyl;
each of R² and R³ is independently H, hydroxyl, a protected hydroxyl, or -OR^{A};
R⁴ is hydroxyl, a protected hydroxyl, -R^{B}, -NH-CH(R^{C})-CO-R^{D}, or an amino sulfonic acid;
each R^{A}, when present, is independently a bond to an acyl;
R^{B}, when present, is a bond to an alcohol core or an amine core;
R^{C}, when present, is a side chain of a proteinogenic amino acid; and
R^{D}, when present, is hydroxyl, a protected hydroxyl, or a bond to an alcohol core or an amine core.

If the bile acid is present in a multibiotic agent, the bile acid comprises at least one bond to an acyl or at least one bond to an alcohol core or an amine core.

Non-limiting examples of a bile acid include:

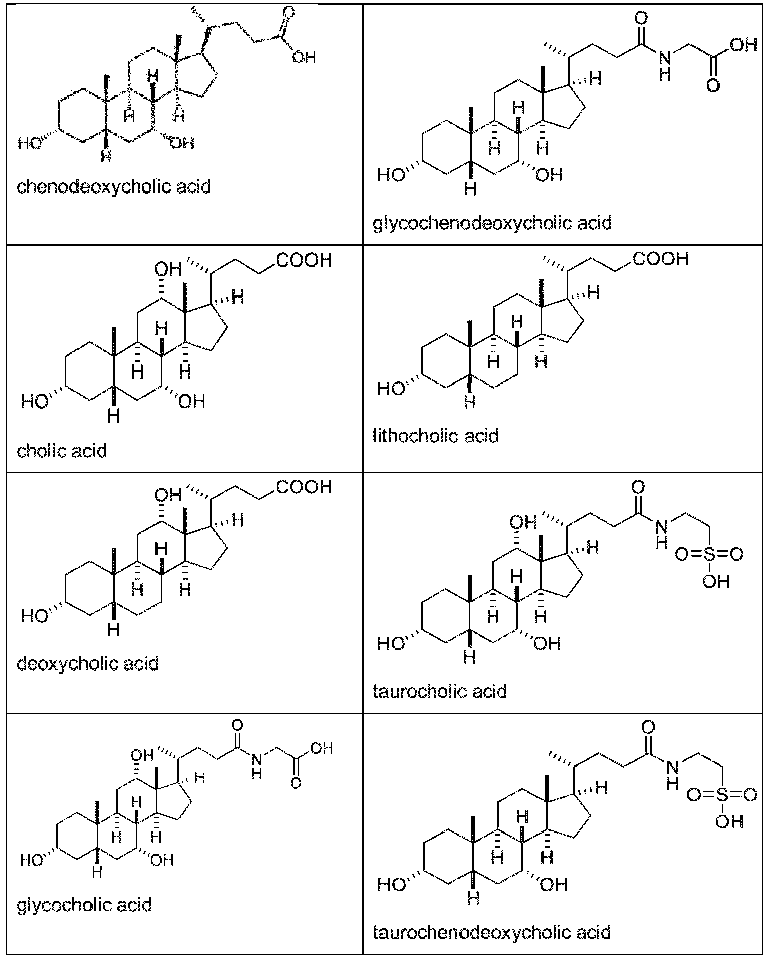

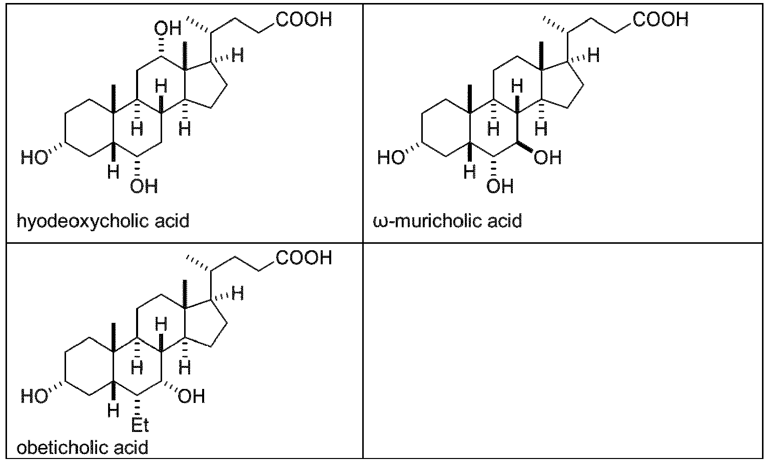

The term "biologically degradable bond," as used herein represents a covalent bond that is enzymatically cleavable. In some embodiments, the bond is stable between pH 2.0 and 8.0, e.g., as measured by the half-life for the bond of at least 6 hours at 37 °C at the defined pH level. Non-limiting examples of biologically degradable bonds include ester bonds, amide bonds, diazo bonds, carbamate linkers, carbonate linkers, and glycosidic bonds.

The term "capsinoid," as used herein, refers to a compound of the structure: 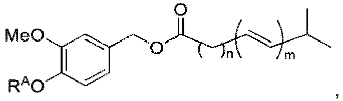 where
n is an integer from 4 to 6;
m is 0 or 1; and
R^{A} is H, O-protecting group, or a bond to an acyl.

If the capsinoid is present in a multibiotic agent, R^{A} is a bond to an acyl.

Non-limiting examples of the capsinoids include:

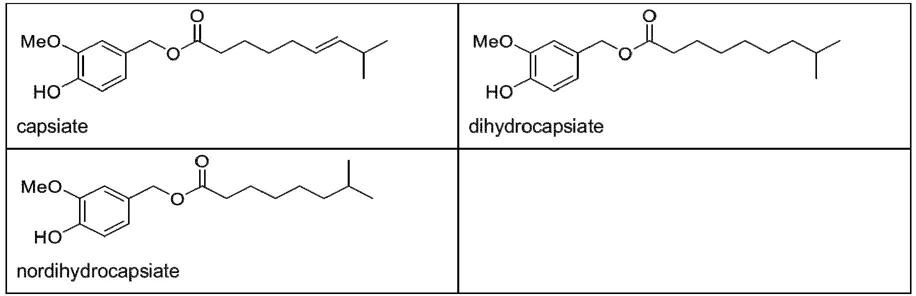

The term "carbamate linker," as used herein, refers to a group R¹-(CO)-R², where R¹ is a bond to an alcoholic or phenolic oxygen atom in one alcohol core, and R² is a bond to an amino nitrogen atom in an amine core.

The term "carbohydrate," as used herein, refers to monosaccharides or glucosinolates known in the art. A monosaccharide may be, e.g., an aldose (e.g., aldopentose, aldohexose, deoxy-aldopentose, deoxy-aldohexose) or ketose (e.g., ketopentose, ketohexose, deoxy-ketopentose, or deoxy-ketohexose). A monosaccharide may be, e.g., a pentose or hexose. Non-limiting examples of carbohydrates include arabinose, xylose, fructose, galactose, glucosinolate, ribose, tagatose, fucose, and rhamnose. Multibiotic agents disclosed herein may include carbohydrate oligomers, e.g., trehalose, lactulose, hyaluronic acid, or an oligomer of arabinose, xylose, fructose, galactose, glucosinolate, ribose, tagatose, fucose, or rhamnose, or a combination thereof.

The term "carbonate linker," as used herein, refers to a group R¹-(CO)-R², where R¹ is a bond to an alcoholic or phenolic oxygen atom in one alcohol core, and R² is a bond to an alcoholic or phenolic oxygen atom in another alcohol core.

The term "carbonyl," as used herein, refers to a divalent group -(CO)-.

The term "carboxylic acid," as used herein, is a short-chain fatty acid, medium-chain fatty acid, long-chain fatty acid, a very long-chain fatty acid, or an unsaturated analogue thereof, or a substituted analogue thereof, or a carboxylic acid of the acyls described herein. A carboxylic acid may be optionally substituted 1, 2, 3, or 4 times with substituents, each of which is independently oxo, hydroxyl, trialkylammonium, phenyl, or -NH₂. An unsubstituted carboxylic acid has a carbon count of 1 to 26. A carboxylic acid may be aliphatic or aromatic. An unsubstituted aliphatic carboxylic acid has a carbon count of 1 to 26 and contains only aliphatic hydrocarbon residues. An unsubstituted aromatic carboxylic acid has a carbon count of 7 to 26 and contains at least one aromatic carbocycle. A carboxylic acid may be a fatty acid as described herein.

The term "carotenoid," as used herein, refers to a compound of the structure: 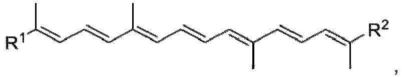 where
R1 is 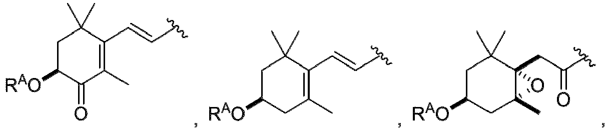 or 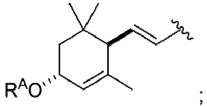
R² is 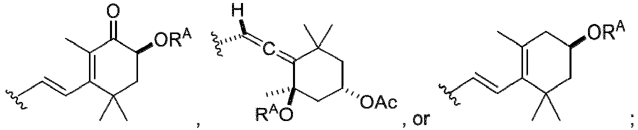
each R^{A} is independently H, an O-protecting group, or a bond to an acyl;

If the carotenoid is present in a multibiotic agent, the carotenoid contains at least one bond to an acyl. Non-limiting examples of carotenoids include:

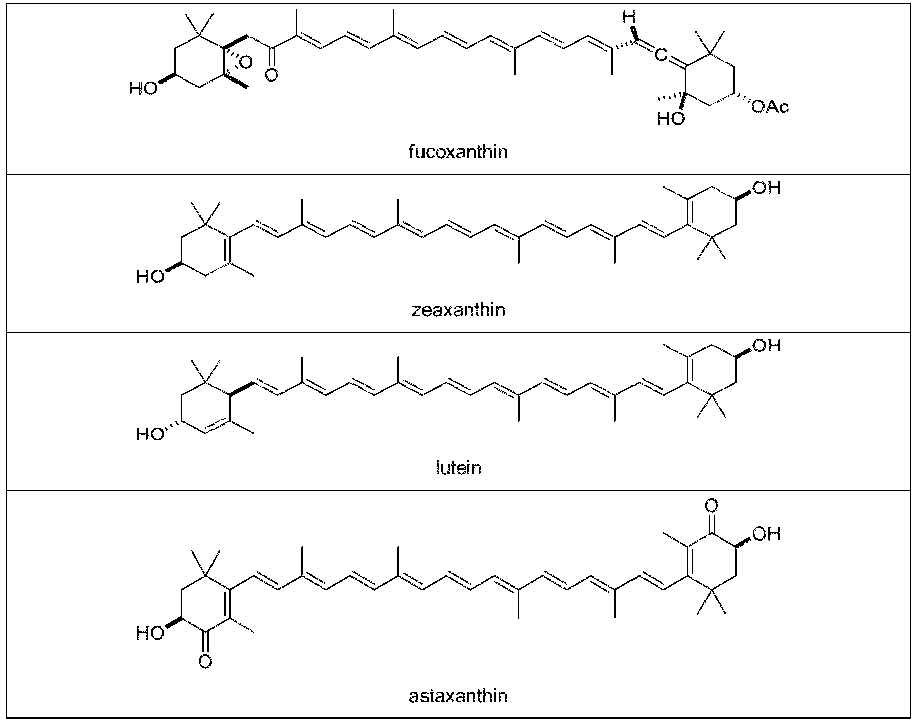

The term "catechin polyphenol," as used herein, refers to a compound having a phenyl chromane core (e.g., catechin, gallocatechin, or epigallocatechin) that is optionally substituted with an optionally substituted benzoate. A catechin polyphenol may be a compound of formula: 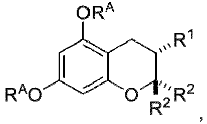 where
each R^{A} is independently H, O-protecting group, or a bond to acyl;
R¹ is -OR^{A} or 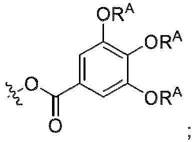
one R² is H and the other R² is 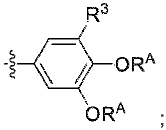 and
R³ is H or -OR^{A}.

If the catechin polyphenol is present in a multibiotic agent, the catechin polyphenol comprises at least one bond to an acyl.

Alternatively, a catechin polyphenol may be a compound of formula: 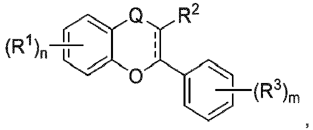 where
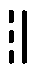 is a single carbon-carbon bond or double carbon-carbon bond;
Q is -CH₂- or -C(O)-;
each R¹ and each R³ is independently H, halogen, -OR^{A}, phosphate, or sulfate;
R² is H or -OR^{A};
each R^{A} is independently a bond to an acyl, H, optionally substituted alkyl, or benzoyl optionally substituted with 1, 2, 3, or 4 substituents independently selected from the group consisting of H, hydroxyl, halogen, optionally substituted alkyl, optionally substituted alkoxy, phosphate, and sulfate; and
each of n and m is independently 1, 2, 3, or 4.

When a catechin polyphenol is present in a multibiotic agent, at least one R^{A} is a bond to an acyl. Non-limiting examples of catechin polyphenols include epigallocatechin gallate. The term "combination therapy" or "administered in combination," as used herein, means that two (or more) different agents or treatments are administered to a subject as part of a defined treatment regimen for a particular disease or condition. The treatment regimen defines the doses and periodicity of administration of each agent such that the effects of the separate agents on the subject overlap. In some embodiments, the delivery of the two or more agents is simultaneous or concurrent and the agents may be co-formulated. In other embodiments, the two or more agents are not co-formulated and are administered in a sequential manner as part of a prescribed regimen. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination may be administered by intravenous injection while a second therapeutic agent of the combination may be administered orally.

The term "curcuminoid," as used herein, refers to a compound of the structure: 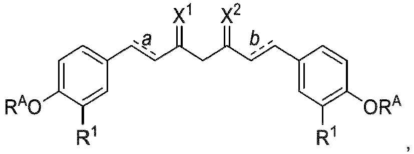 where
each or a and b is independently a single or a double bond;
each of X¹ and X², together with the carbon atom to which each is attached, is independently a carbonyl or -(CH(OR^{A}))-;
each R^{A} is independently H, an O-protecting group, or a bond to an acyl; and
each R¹ is independently H or OMe.

If the curcuminoid is present in a multibiotic agent, the curcuminoid includes at least one bond to an acyl. Non-limiting examples of curcuminoids include:

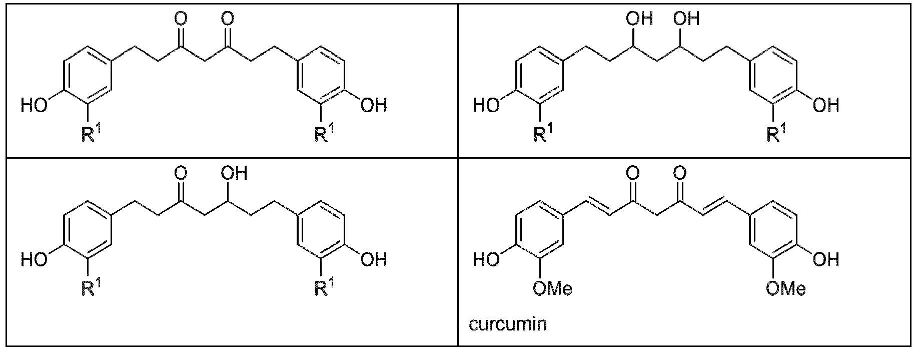

The terms "ellagic acid" and "ellagic acid analogue," as used herein, collectively refer to a compound of the structure: 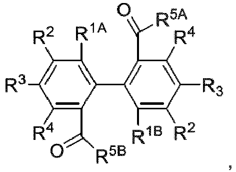 where
each of R², R³, and R⁴ is independently H or -OR^{A};
R^{1A} is H or -OR^{A}, and R^{5A} is -OH or R^{B}, or R^{1A} and R^{5A} combine to form -O-;
R^{1B} is H or -OR^{A}, and R^{5B} is -OH or R^{B}, or R^{1B} and R^{5B} combine to form -O-;
each R^{A} is independently H, O-protecting group, or a bond to an acyl; and
each R^{B} is independently a bond to an alcohol core or an amine core.

When the ellagic acid or its analogue is present in a multibiotic agent, the ellagic acid or its analogue includes at least one bond to an alcohol core, an amine core, or an acyl.

The term "ellagic acid analogue," refers to the compounds of the above structure that are not ellagic acid. The term "ellagic acid" refers to the following two compounds: 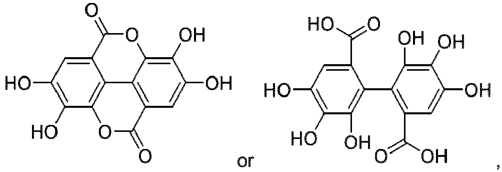 or these compounds within the structure of a multibiotic agent.

Non-limiting examples of ellagic acid analogues include:

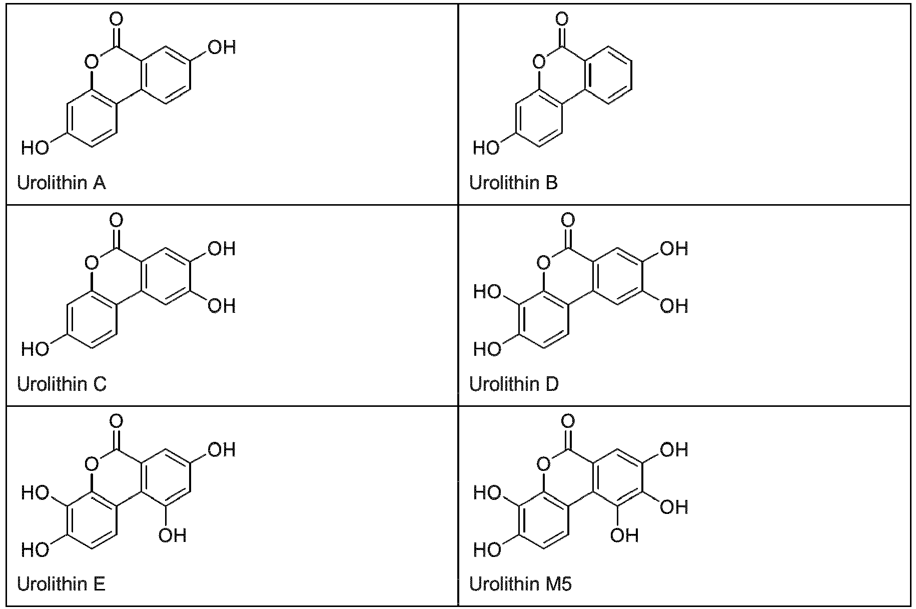

The term "ester bond," as used herein, refers to a covalent bond between an alcoholic or phenolic oxygen atom and a carbonyl group of an acyl in a multibiotic agent.

The term "fatty acid," as used herein, refers to a short-chain fatty acid, a medium chain fatty acid, a long chain fatty acid, a very long chain fatty acid, or an unsaturated analogue thereof, or a phenyl-substituted or methoxycarbonyl-substituted analogue thereof. Short chain fatty acids contain from 1 to 6 carbon atoms, medium chain fatty acids contain from 7 to 13 carbon atoms, long-chain fatty acids contain from 14 to 22 carbon atoms, and a very long-chain fatty acid contains 23 to 26 carbon atoms. A fatty acid may be saturated or unsaturated. An unsaturated fatty acid includes 1, 2, 3, 4, 5, or 6 carbon-carbon double bonds. Preferably, the carbon-carbon double bonds in unsaturated fatty acids have Z stereochemistry. In some embodiments, a fatty acid acyl is a group of the following structure:

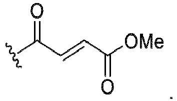

The term "flavonoid," as used herein, refers to flavonoids and isoflavonoids known in the art (e.g., flavans, flavones, flavanones, flavonols, flavanonols, isoflavanes, isoflavones, and/or isoflavonols) that do not include the structure of catechin. A flavonoid may have the structure: 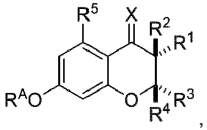 where
X is =O, or X, together with the carbon atom to which it is attached, is -CH₂-;
R¹ is H or -OR^{A};
R² is H, and R³ is H or 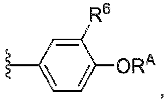 or R² and R³ combine to form a double bond;
R⁴ is H or 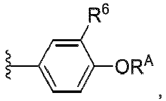
each of R⁵ and R⁶ is independently H or -OR^{A};
   and
each R^{A} is independently H, O-protecting group, or a bond to acyl;
provided that the flavonoid is not catechin.

When the flavonoid is present in a multibiotic agent, the flavonoid includes at least one bond to an acyl. Non-limiting examples of flavonoids include:

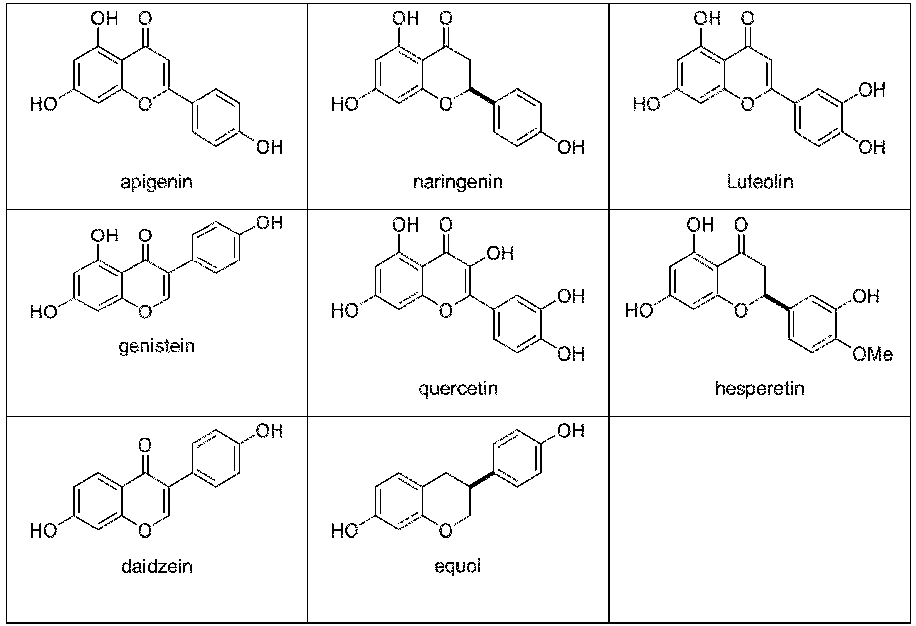

The term "glycosidic bond," as used herein, refers to a covalent bond between an anomeric carbon of a carbohydrate or an oligomer thereof and an oxygen atom of another alcohol core that is not a carbohydrate or an oligomer thereof.

The term "heteroaryl," as used herein, is an aryl group, in which at least one ring carbon atom is replaced with a heteroatom that is oxygen, nitrogen, or sulfur. Heteroaryl typically contains from one to seventeen carbon atoms and from one to six heteroatoms that are independently oxygen, nitrogen, or sulfur. Non-limiting examples of heteroaryls include: indolyl, imidazolyl, and pyridyl.

The term "hydrolysable *in vivo,"* as used herein refers to a covalent bond cleavable under physiological conditions in a human. Alternatively, the term "hydrolysable *in vivo"* refers to a carbonate linker or carbamate linker. The physiological conditions include: pH levels found at a given site in a human, and, in some embodiments, presence of an enzyme (either endogenous or one produced by a bacterium present in the biome). A bond is cleavable under physiological conditions, when at least 25% of a compound are cleaved at the bond within a typical residence time in a physiological compartment. The measurement of the percentage of the compound cleaved at the bond can be carried out using methods known in the art (e.g., liquid chromatography/tandem mass spectrometry), following incubation of the compound in a composition simulating the medium at the physiological compartment (e.g., simulated intestinal fluid for simulating small intestine medium or healthy human fecal matter under anaerobic conditions for simulating large intestine medium). A bond is stable in a physiological compartment, when less than 25% of the compound are cleaved at the bond within a typical residence time in the physiological compartment. Typical residence times for the GI tract are 0.5-2 hours for stomach, 3-5 hours for a small intestine, and 5-48 hours for a large intestine.

The term "β-hydroxyalkanoate," as used herein, is a C₃₋₁₂ aliphatic carboxylic acid residue substituted at β-position with a hydroxyl group. Non-limiting examples of β-hydroxyalkanoates include β-hydroxybutyrate.

The term "β-hydroxycarboxylate," as used herein, is an optionally substituted β-hydroxyalkanoate. A β-hydroxycarboxylate may be a compound of the following structure: 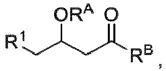 where
R¹ is H or -N⁺Me₃;
R^{A} is H, an O-protecting group, or a bond to an acyl; and
R^{B} is hydroxyl, a protected hydroxyl, or a bond to an alcohol core or an amine core.

When β-hydroxycarboxylate is present in a multibiotic agent, the β-hydroxycarboxylate comprises at least a bond to an acyl or to an alcohol core or an amine core.

Non-limiting examples of β-hydroxycarboxylates include β-hydroxybutyrate and carnitine.

The term "hydroxycinnamic acid ester," as used herein, represents a compound of the structure: 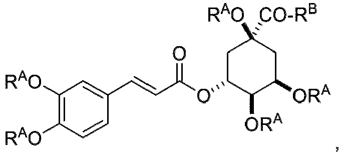 where
each R^{A} is independently H, an O-protecting group, or a bond to an acyl;
R^{B} is hydroxyl, a protected hydroxyl, or a bond to an alcohol core or an amine core;

When the hydroxycinnamic acid ester is present in a multibiotic agent, the hydroxycinnamic acid ester comprises at least one bond to an acyl or to an alcohol core or an amine core.

Non-limiting examples of hydroxycinnamic acid esters include:

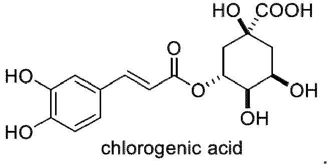

The terms "increasing" and "decreasing," as used herein, refer to modulating resulting in, respectively, greater or lesser amounts, function, or activity of a metric relative to a reference. For example, subsequent to administration of a multibiotic agent described herein, the amount of a marker of inflammation as described herein may be increased or decreased in a subject by at least 5%, 10%, 15%, 20%, 25%, 30%, 35%, 40%, 45%, 50%, 55%, 60%, 65%, 70%, 75%, 80%, 85%, 90%, 95% or 98% or more relative to the amount of the marker prior to administration. Generally, the metric is measured subsequent to administration at a time that the administration has had the recited effect, e.g., at least one week, one month, 3 months, 6 months, after a treatment regimen has begun.

The term "inositol," as used herein, refers to 1,2,3,4,5,6-hexahydroxycyclohexane, an enantiomer thereof, a diastereomer thereof, or a mixture thereof, or a partially alkylated analogue thereof (e.g., inositol alkylated with 1, 2, 3, 4, or 5 C₁₋₆ alkyl groups). Non-limiting examples of inositol include myo-inositol, scyllo-inositol, muco-inositol, D-*chiro*-inositol, L-*chiro*-inositol, neo-inositol, allo-inositol, epi-inositol, or cis-inositol. In some embodiments, inositol is D-*chiro*-inositol.

The term "ketone body," as used herein, refers to β-hydroxybutyric acid or acetoacetic acid.

The term "lignan," as used herein, refers to a compound of the following structure: 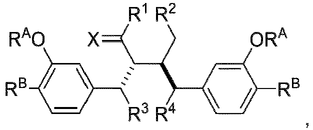 where
X is =O, or X, together with the carbon atom to which it is attached, is -CH₂-;
   (i) R¹ is independently hydroxyl, a protected hydroxyl, or a bond to an alcohol core or an amine core; R² is independently hydroxyl, a protected hydroxyl, or -OR^{A}, and each of R³ and R⁴ is H or
   (ii) R¹ and R² combine to form -O-, R³ is H, and R⁴ is H or -OH;
   (iii) R¹ and R⁴ combine to form -O-, R² and R³ combine to form -O-;
   (iv) R¹ and R⁴ combine to form -O-, R² is -OR^{A}, and R³ is H;
   (v) R¹ is -OR^{A}, R² and R³ combine to form -O-, and R⁴ is H;
each R^{A} is independently H, *O*-protecting group, or a bond to acyl; and
each R^{B} is independently H or -OR^{C}, where R^{C} is H, *O*-protecting group, or a bond to acyl. When the lignan is present in a multibiotic agent, the lignan comprises at least one bond to an acyl or at least one bond to an alcohol core or an amine core.

The term "long chain alcohol," as used herein, refers to an aliphatic alcohol with a carbon count of 24 to 36 (e.g., 24 to 34) or a mixture of such alcohols. Preferably, long chain alcohol is a linear alcohol. Also, preferably, long chain alcohol is a monohydroxy alcohol. Non-limiting examples of long chain alcohol include policosanol.

The term "marker for cardiovascular disorder," as used herein, refers to an observable indicative of the presence, absence, or risk of a cardiovascular disorder. Markers for cardiovascular disorders are known in the art. Non-limiting examples of the markers for cardiovascular disorder include levels of cholesterol, triglycerides, lipids, lipoprotein a (LPa), apolipoprotein C3 (Apo-C3), and blood pressure measurement.

The term "marker for diabetes," as used herein, refers to an observable indicative of the presence, absence, or risk of a diabetes. Non-limiting examples of the markers for diabetes include levels of insulin, GLP-1, PYY, blood sugar, and hemoglobin A1c. A blood sugar level may be a fasting blood sugar level or a blood sugar level after a meal.

The term "marker for inflammatory bowel disease," as used herein, refers to an observable indicative of the presence, absence, or risk of an inflammatory bowel disease. Non-limiting examples of the markers for inflammatory bowel disease include intestinal motility, Treg differentiation, mucus secretion, abdominal pain, gastrointestinal inflammation, gastrointestinal permeability, gastrointestinal bleeding, and frequency of bowel movements.

The term "marker for obesity," as used herein, refers to an observable indicative of the presence, absence, or risk of an obesity. Non-limiting examples of the markers for obesity are total fat percentage, cellular adiposity, rate of weight gain, abdominal fat quantity, and ratio of white to brown fat.

The term "methoxycarbonyl," as used herein, represents a group of formula -COOMe.

The term "4-methyl-1,3-dioxan-2-yl," as used herein, refers to the monovalent group of formula: 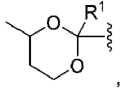 where R¹ is optionally substituted C₁₋₆ alkyl (e.g., methyl).

The term "modulating," as used herein, refers to an observable change in the level of a marker in a subject, as measured using techniques and methods known in the art for the measurement of the marker. Modulating the marker level in a subject may result in a change of at least 1% relative to prior to administration or a control group (e.g., at least 5%, 10%, 15%, 20%, 25%, 30%, 35%, 40%, 45%, 50%, 55%, 60%, 65%, 70%, 75%, 80%, 85%, 90%, 95% or at least 98% or more relative to prior to administration or a control group; e.g., up to 100% relative to prior to administration or a control group). In some embodiments, modulating is increasing the level of a marker in a subject. Increasing the marker level in a subject may result in an increase of at least 1% relative to prior to administration or a control group (e.g., at least 5%, 10%, 15%, 20%, 25%, 30%, 35%, 40%, 45%, 50%, 55%, 60%, 65%, 70%, 75%, 80%, 85%, 90%, 95% or at least 98% or more relative to prior to administration or a control group; e.g., up to 100% relative to prior to administration or a control group). In other embodiments, modulating is decreasing the level of a marker in a subject. Decreasing the marker level in a subject may result in a decrease of at least 1% relative to prior to administration or a control group (e.g., at least 5%, 10%, 15%, 20%, 25%, 30%, 35%, 40%, 45%, 50%, 55%, 60%, 65%, 70%, 75%, 80%, 85%, 90%, 95% or at least 98% or more relative to prior to administration or a control group; e.g., up to 100% relative to prior to administration or a control group). In embodiments in which a parameter is increased or decreased (or reduced) in a subject following a step of administering a composition described herein, the increase or decrease may take place and/or be detectable within a range of time following the administration (e.g., within six hours, 24 hours, 3 days, a week or longer), and may take place and/or be detectable after one or more administrations (e.g., after 2, 3, 4, 5, 6, 7, 8, 9, 10 or more administrations, e.g., as part of a dosing regimen for the subject).

The term "oxo," as used herein, represents divalent group =O.

The term "phenolic phytochemical," as used herein, is a non-catechin phenolic phytochemical known in the art. Non-limiting examples of phenolic phytochemicals include ellagic acid, ellagic acid analogues, vitamin E, and phenalkyls.

The term "pentacyclic triterpenoid," as used herein, refers to a class of pentacyclic triterpenoids recognized in the art. Pentacyclic triterpenoid may be a compound of the following structure: 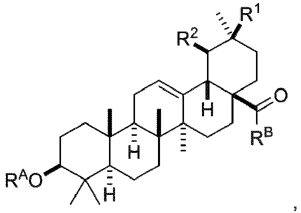 where
each of R¹ and R² is independently H or methyl;
R^{A} is H, an O-protecting group, or a bond to an acyl; and
R^{B} is hydroxyl, a protected hydroxyl, or a bond to an alcohol core or an amine core;
provided that, when the pentacyclic triterpenoid is present in a multibiotic agent, the pentacyclic triterpenoid comprises a bond to an acyl or a bond to an alcohol core or an amine core. Non-limiting examples of pentacyclic triterpenoids include:

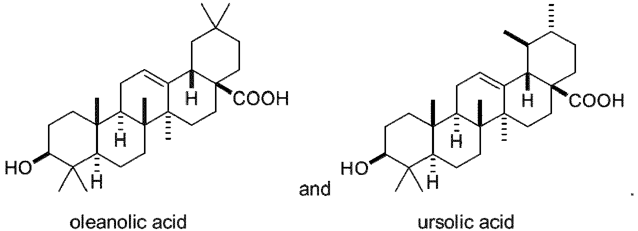

As used herein, a "pharmaceutical composition" or "pharmaceutical preparation" is a composition or preparation, having pharmacological activity or other direct effect in the mitigation, treatment, or prevention of disease, and/or a finished dosage form or formulation thereof and which is suitable for and indicated for human use. As used herein "substantially activated" means that a multibiotic agent preparation described herein is at least 50% cleaved into at least two constituent parts at a target site in a subject's body (e.g., at least 50% hydrolyzed by an enzymatic action present at the target site, e.g., by an enzyme produced by the microbiota at the target site). A substantially activated multibiotic agent may be at least 50%, 60%, 70%, 80%, 90%, 95%, 100% cleaved into at least two constituent parts at a target site in a subject's body. The ability to become active is defined as being chemically stable under a range of pH's, while being transformed into constituents in the presence of the microbiome, its constituents and/or human enzymes.

The term "phenalkyl," as used herein, refers to a compound of the following structure: 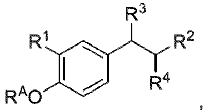 where
R¹ is H, methoxy, or -OR^{A};
R² is -OR^{A}, -CO-R^{B}, 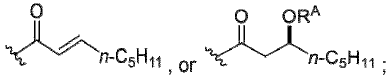
R³ and R⁴ are each H, or R³ and R⁴ combine to form a double bond;
each R^{A} is independently H, an *O*-protecting group, or a bond to an acyl; and
R^{B}, when present, is OH, an -O-(*O*-protecting group), or a bond to an alcohol core or an amine core.

When the phenalkyl is present in a multibiotic agent, the phenalkyl comprises at least one bond to an alcohol core, an amine core, or an acyl.

Non-limiting examples of phenalkyls include:

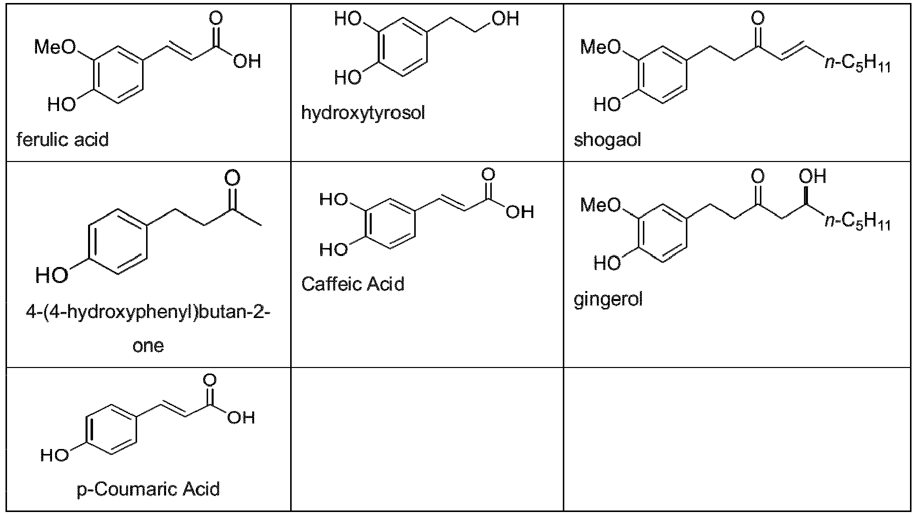

The term "phenolic oxygen atom," as used herein, refers to an oxygen atom bonded to an *sp*²-hybridized carbon atom within a π-aromatic ring.

The term "polyamine," as used herein, represents a compound of the structure: 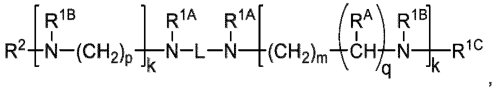 where
L is -(CH₂)ₙ- or cycloalkylene (e.g., cyclohexane-diyl);
each R^{1A} is independently H or a bond to an acyl, or both R^{1A} combine to form an alkylene (e.g., C₂ alkylene);
each R^{1B} is independently H or a bond to an acyl;
R^{1C} is H or a bond to an acyl
R² is H, -CONH₂, -C(NH)NH₂, or a bond to an acyl;
R^{A} is H or -CO-R^{B}, where R^{B} is a bond to an alcohol core or an amine core;
each of n and p is independently an integer from 2 to 6;
m is an integer from 1 to 5; and
each k and q is independently 0 or 1,

In some embodiments, a polyamine is a compound of the following structure: 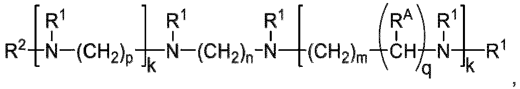 where
each R¹ is independently H or a bond to an acyl;
R² is H, -CONH₂, -C(NH)NH₂, or a bond to an acyl;
R^{A} is H or -CO-R^{B}, where R^{B} is a bond to an alcohol core or an amine core;
each of n and p is independently an integer from 2 to 6;
m is an integer from 1 to 5; and
each k and q is independently 0 or 1,

When the polyamine is present in a multibiotic agent, at least one R¹ or R² is a bond to an acyl, or R^{B} is a bond to an alcohol core or an amine core.

Non-limiting examples of polyamines include:

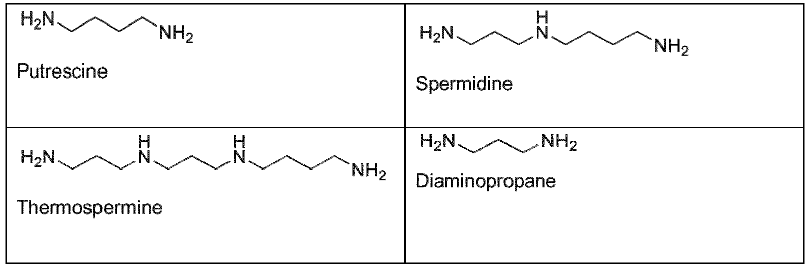

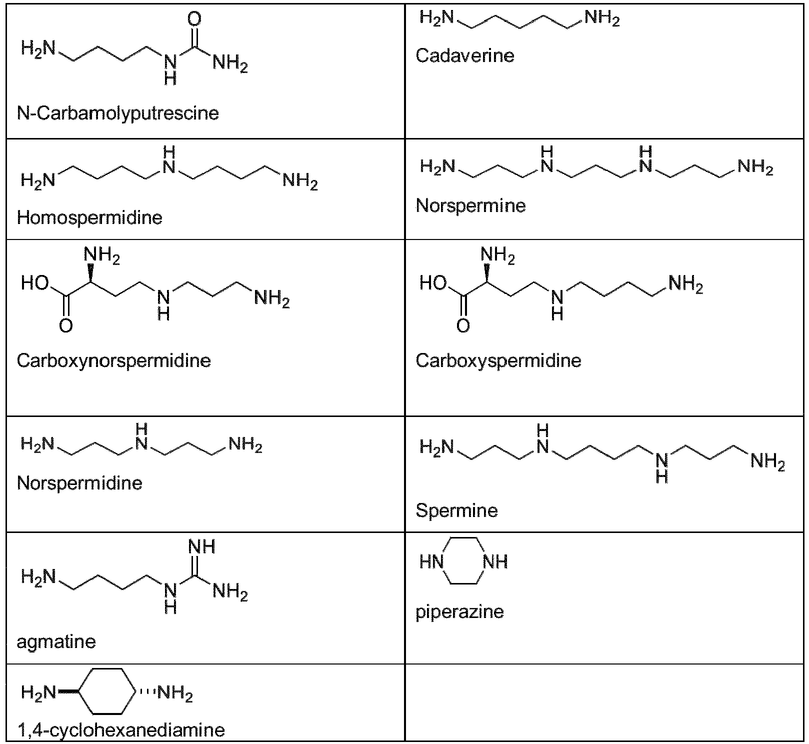

The term "multibiotic agent," as used herein, represents a compound including two or more agents linked through a biologically degradable bond. In some embodiments, multibiotic agents may be referred to as polybiotics.

The term "pre-ketone body," as used herein, represents a compound in which -COOH of a ketone body is replaced with -CH₂OH. The term "pre-ketone body," as used herein, also represents (4-methyl-1,3-dioxan-2-yl)-(alkylene)ₙ-CO-R^{A}, where n is 0 or 1, and R^{A} is -OH, if the pre-ketone body is not part of a multibiotic agent, or a valency if the pre-ketone body is part of a multibotic agent (e.g., as a pre-ketone body acyl). Non-limiting example of a pre-ketone body include butane-1,3-diol, 4-hydroxybutan-2-one, and the following compounds:

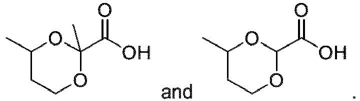

The term "protecting group," as used herein, represents a group intended to protect a functional group (e.g., a hydroxyl, an amino, or a carbonyl) from participating in one or more undesirable reactions during chemical synthesis. The term "O-protecting group," as used herein, represents a group intended to protect an oxygen containing (e.g., phenol, hydroxyl or carbonyl) group from participating in one or more undesirable reactions during chemical synthesis. The term "*N*-protecting group," as used herein, represents a group intended to protect a nitrogen containing (e.g., an amino or hydrazine) group from participating in one or more undesirable reactions during chemical synthesis. Commonly used *O*- and *N-*protecting groups are disclosed in Greene, "Protective Groups in Organic Synthesis," 3rd Edition (John Wiley & Sons, New York, 1999). Exemplary *O*- and *N-*protecting groups include alkanoyl, aryloyl, or carbamyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, *t*-butyldimethylsilyl, tri-*iso-*propylsilyloxymethyl, 4,4'-dimethoxytrityl, isobutyryl, phenoxyacetyl, 4-isopropylpehenoxyacetyl, dimethylformamidino, and 4-nitrobenzoyl.

Exemplary O-protecting groups for protecting carbonyl containing groups include, but are not limited to: acetals, acylals, 1,3-dithianes, 1,3-dioxanes, 1,3-dioxolanes, and 1,3-dithiolanes.

Other O-protecting groups include, but are not limited to: substituted alkyl, aryl, and arylalkyl ethers (e.g., trityl; methylthiomethyl; methoxymethyl; benzyloxymethyl; siloxymethyl; 2,2,2,-trichloroethoxymethyl; tetrahydropyranyl; tetrahydrofuranyl; ethoxyethyl; 1-[2-(trimethylsilyl)ethoxy]ethyl; 2-trimethylsilylethyl; t-butyl ether; p-chlorophenyl, p-methoxyphenyl, p-nitrophenyl, benzyl, p-methoxybenzyl, and nitrobenzyl); silyl ethers (e.g., trimethylsilyl; triethylsilyl; triisopropylsilyl; dimethylisopropylsilyl; t-butyldimethylsilyl; t-butyldiphenylsilyl; tribenzylsilyl; triphenylsilyl; and diphenymethylsilyl); carbonates (e.g., methyl, methoxymethyl, 9-fluorenylmethyl; ethyl; 2,2,2-trichloroethyl; 2-(trimethylsilyl)ethyl; vinyl, allyl, nitrophenyl; benzyl; methoxybenzyl; 3,4-dimethoxybenzyl; and nitrobenzyl).

Other N-protecting groups include, but are not limited to, chiral auxiliaries such as protected or unprotected D, L or D, L-amino acids such as alanine, leucine, phenylalanine, and the like; sulfonyl-containing groups such as benzenesulfonyl, p-toluenesulfonyl, and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyl oxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydroxy carbonyl, t-butyloxycarbonyl, diisopropylmethoxycarbonyl, isopropoxycarbonyl, ethoxycarbonyl, methoxycarbonyl, allyloxycarbonyl, 2,2,2-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl, and the like, arylalkyl groups such as benzyl, triphenylmethyl, benzyloxymethyl, and the like and silyl groups such as trimethylsilyl, and the like.

The term "stilbenoid," as used herein, refers to a hydroxylated or alkoxylated stilbene. A stilbenoid may have the structure: 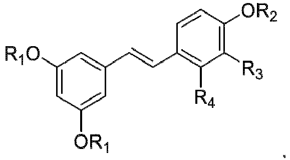 where
each R¹ and R² is independently H, methyl, *O*-protecting group, or a bond to an acyl; and
each R³ and R⁴ is independently H or -OR^{A}, where each R^{A} is independently H, methyl, or a bond to an acyl.

When the stilbenoid is present in a multibiotic agent, at least one of R¹, R², and R^{A} is a bond to an acyl. Non-limiting examples of stilbenoids include:

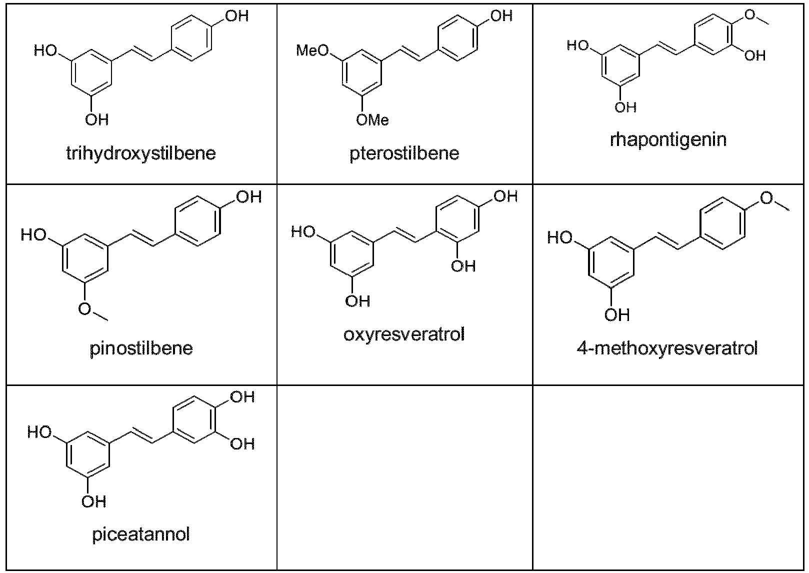

Stilbenoid multimers are known in the art and include viniferins, e.g., α-viniferin, β-viniferin, γ-viniferin, δ-viniferin, and ε-viniferin.

The term "substantially stable," as used herein, refers to multibiotic agents that, when exposed to the conditions of a physiological compartment, undergo less than 25% (e.g., less than 20%, less than 15%, or less than 10%) degradation in at least 4 hours (e.g., at least 6 hours, 8 hours, 12 hours, or 24 hours). The conditions of physiological compartments (e.g., stomach, upper intestine, or colon) may be modeled *in vitro,* e.g., as a simulated gastric fluid assay, simulated intestinal fluid assay, or fecal stability assay.

The term "sugar acid," as used herein, refers to a monosaccharide, in the linear form of which, one or both terminal positions are oxidized to a carboxylic acid. There are four classes of sugar acids: aldonic acid, ulosonic acid, uronic acid, and aldaric acid. Any of the four sugar acid classes may be used in multibiotic agents of the invention. Non-limiting examples of sugar acids include gluconic acid.

The term "sugar alcohol," as used herein, refers to a cyclic or acyclic, saturated hydrocarbon with a carbon count of 4 to 8, each carbon atom of which is substituted with one and only one hydroxyl. If sugar alcohol is acyclic, the hydrocarbon skeleton is linear. Non-limiting examples of sugar alcohols include erythritol, sorbitol, and inositol.

"Treatment" and "treating," as used herein, refer to the medical management of a subject with the intent to improve, ameliorate, stabilize, prevent, or cure a disease, pathological condition, or disorder. This term includes active treatment (treatment directed to improve the disease, pathological condition, or disorder), causal treatment (treatment directed to the cause of the associated disease, pathological condition, or disorder), palliative treatment (treatment designed for the relief of symptoms), preventative treatment (treatment directed to minimizing or partially or completely inhibiting the development of the associated disease, pathological condition, or disorder); and supportive treatment (treatment employed to supplement another therapy).

The term "trialkylammonium," as used herein, is a group of formula -NR₃, where each R is independently a monovalent, saturated hydrocarbon group containing from 1 to 6 carbon atoms. In some embodiments, trialkylammonium is trimethylammonium.

The term "vitamin," as used herein, refers to vitamins, pro-vitamins, vitamin metabolites, and vitamin analogues known in the art. Non-limiting examples of vitamins include vitamin A, vitamin B5, vitamin E, pyridoxine, folic acid, and pralatrexate.

The term "vitamin A," as used herein, refers to retinoic acid (e.g., tretinoin), isotretinoin, or alitretinoin.

The term "vitamin B5," as used herein, refers to pantothenic acid or a derivative thereof, in which -COOH is replaced with -CH₂OH (pantothenol) or -CONH₂ (pantothenamide).

The term "vitamin E," as used herein, refers to tocopherols and tocotrienols. Vitamin E may be a compound of the following structure: 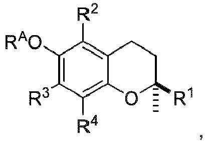 where
R1 is 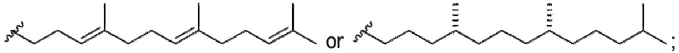
each of R², R³, and R⁴ is independently H or Me; and
R^{A} is H or a bond to an acyl;
When the vitamin E is present in a multibiotic agent, R^{A} is a bond to an acyl.

The multibiotic agents described herein, unless otherwise noted, encompass isotopically enriched compounds (e.g., deuterated compounds), tautomers, and all stereoisomers and conformers (e.g., enantiomers, diastereomers, *E*/*Z* isomers, atropisomers, etc.), as well as racemates thereof and mixtures of different proportions of enantiomers or diastereomers, or mixtures of any of the foregoing forms as well as salts (e.g., pharmaceutically acceptable salts).

### BRIEF DESCRIPTION OF THE DRAWINGS

FIG. is a scheme showing the microbiome-mediated hydrolysis of a multibiotic agent.

### DETAILED DESCRIPTION

The present invention relates to a mulitbiotic agent, wherein the multibiotic agent is selected from the group as defined in the claims.

In more general words, a multibiotic agent can be described as follows for illustrative purposes.

Multibiotic agents may contain two or more moieties, linked to each other through biodegradable covalent bonds (e.g., an ester bond, amide bond, azo bond, glycosidic bonds, carbonate linker, or carbamate linker). The moiety in a multibiotic agent may be, for example, an alcohol core, an acyl, or an amine core (e.g., mesalamine). While not being bound by theory, the multibiotic agents are believed to act in concert with, or in lieu of, the microbiota of a mammalian host to modulate, for example, one or more of: the host's immune system, the neuroendocrine hypothalamus-pituitary-adrenal (HPA) axis, the autonomic (vagus nerve) nervous system, and the enteric nervous system (ENS). In some embodiments, the multibiotic agents of the invention are substantially cleaved at the site of desired action, e.g., by action of an enzyme produced by a constituent of the host microbiota, e.g., by action of one or more (e.g., two, three, or four) enzymes produced by a constituent of the host microbiota at the target site of delivery of the multibiotic agent.

Multibiotic agents are compounds comprising two or more agents linked through a hydrolysable *in vivo* bond (e.g., an ester bond, amide bond, azo bond, glycosidic bonds, carbonate linker, or carbamate linker). The ester bond, amide bond, azo bond, glycosidic bonds, carbonate linker, or carbamate linker may be hydrolysable *in vivo* by the bond-cleaving activity of an enzyme present at a target site in a subject's body (e.g., an enzymatic activity of an endogenous enzyme present at the target delivery site or of an enzyme produced by a microbe present at the target delivery site). The target delivery site may be skin or a compartment within a gastrointestinal (GI) tract (e.g., stomach, small intestine, or large intestine). A multibiotic agent having at least one hydrolysable *in vivo* bond may be stable in one or more physiological compartments, for example, in one or more compartments other than the targeted delivery site (e.g., the GI tract compartments (e.g., stomach, small intestine, or large intestine)). The *in vivo* hydrolysable bond is cleavable in at least one physiological compartment within a typical residence time. Typical residence times for the GI tract are 0.5-2 hours for stomach, 3-5 hours for a small intestine, and 5-48 hours for a large intestine (see Maurer et al., PLoS ONE, 10:e0129076, 2015). In some embodiments, the *in vivo* hydrolysable bond is stable within a pH range of 1.0 to 9.0 (e.g., 2.0 to 8.0, 1.0 to 5.0, 1.0 to 4.0, 2.0 to 4.0, 4.0 to 8.0, or 5.0 to 8.0); these pH ranges may be found in the GI tract. In some embodiments, less than 10% (e.g., less than 15%, less than 20%, or less than 25%) of a multibiotic agent undergo cleavage (e.g., at the ester bond, amide bond, glycosidic bonds, azo bond, carbonate linker, or carbamate linker) *in vivo,* e.g., in one or more of the GI tract regions (e.g., stomach, small intestine, or large intestine). In further embodiments, at least 10% (e.g., at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, or at least 90%) of a multibiotic agent, or substantially all of the multibiotic agent, undergo cleavage (e.g., at the ester bond, amide bond, glycosidic bond, azo bond, carbonate linker, or carbamate linker) *in vivo,* e.g., in one or more of the GI tract regions (e.g., stomach, small intestine, or large intestine). Accordingly, a stable multibiotic agent targeting a Gl tract compartment may undergo cleavage (e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, or at least 90% of the multibiotic agent, or about all of the multibiotic agent, undergoing cleavage) within the residence time at the targeted site.

A multibiotic agent may include one or more (e.g., 1, 2, 3, 4, 5, or 6) alcohol cores, one or more (e.g., 1, 2, 3, 4, 5, or 6) amine cores, and/or one or more (e.g., 1, 2, 3, 4, 5, 6, 7, 8, 9, 10, 11, 12, etc., or as many as need to per-acylate amino nitrogens and/or alcoholic oxygen and/or phenolic oxygens) acyls bonded to each through hydrolyzable *in vivo* bonds that are independently an ester bond, amide bond, azo bond, glycosidic bonds, carbonate linker, and carbamate linker. Certain multibiotic agents comprise one or more alcohol cores and/or amine cores bonded to one or more acyls through one or more ester bonds and/or amide bonds hydrolyzable *in vivo.* In some multibiotic agents, the components may be different. For example, in some multibiotic agents, there may be at least three different components (e.g., three different alcohol core types or one alcohol core type and two different acyl types, etc.) One of skill in the art will recognize that, in a multibiotic agent, when an oxygen atom of an alcohol core is bonded to an acyl carbon atom, the bond is referred to as an ester bond. One of skill in the art will also recognize that, in a multibiotic agent, when an amino nitrogen atom of an amine core is bonded to acyl carbon atom, the bond is referred to as amide bond. Additionally or alternatively, in a multibiotic agent, two alcohol cores may be linked to each other through a carbonate linker or a glycosidic bond (if one of the alcohol cores is a carbohydrate or an oligomer thereof) and that an alcohol core and an amine core may be linked to each other through a carbamate linker. Additionally or alternatively, a multibiotic agent may include amine cores bonded through azo bonds. Azo bonds are nitrogen-nitrogen double bonds formed between an amino nitrogen atom of a first amine core and an amino nitrogen atom of a second amine core.

A moiety included in a multibiotic agent of the invention may have multiple functional groups useful for connectivity to other moieties. Such a moiety, when present in a multibiotic agent, may be referred to as an alcohol core, amine core, or acyl depending on the structure and the context. For example, the structure of mesalamine includes -NH₂, -OH, and -COOH. Mesalamine may be referred to as an amine core, if -NH₂ is used for bonding through an amide bond or carbamate linker to the rest of a multibiotic agent molecule. Additionally or alternatively, mesalamine may be referred to as an alcohol core, if -OH is used for bonding through an ester bond, glycosidic bond, or a carbonate linker to the rest of a multibiotic agent molecule. Additionally or alternatively, mesalamine may be referred to as an acyl, if -COOH is used for bonding through an ester or amide bond to the rest of a multibiotic agent molecule.

In the multibiotic agents, an alcohol core is covalently linked through hydrolyzable *in vivo* bond(s) to one or more of an acyl, another other alcohol core, and an amine core; an amine core is covalently linked through hydrolyzable *in vivo* bond(s) to one or more of an acyl, an alcohol core, and another amine core; and an acyl is covalently linked through hydrolyzable *in vivo* bond(s) to one or more of an alcohol core and an amine core. When an alcohol core is linked to another alcohol core, the linkage is a carbonate linker or, if one of the alcohol cores is a carbohydrate or an oligomer thereof, the linkage may be a glycosidic bond or a carbonate linker. When an alcohol core is linked to an amine core, the linkage is an amide bond or a carbamate linker. When an alcohol core is linked to an acyl, the linkage is an ester bond. When an amine core is linked to another amine core, the linkage is an azo bond. When an amine core is linked to an acyl, the linkage is an amide bond.

### Alcohol Cores

Exemplary a multibiotic agent can include one or more (e.g., 1, 2, 3, 4, 5, or 6) alcohol cores. In some embodiments, each alcohol core is independently a bile acid, carbohydrate or an oligomer thereof, anhydrosugar alcohol, sugar alcohol, sugar acid, polysaccharide, flavonoid (e.g., non-catechin flavonoid), vitamin, lignan, phenolic phytochemical, nucleic acid, carotenoid, curcuminoid, stilbenoid, capsinoid, β-hydroxyalkanoate, ezetimibe, citicoline, antifungal agent, an amino acid metabolite, amino alcohol, mesalamine, pentacyclic triterpenoid, hydroxycinnamic acid ester, or telomerase activator.

In some embodiments, the bile acid is chenodeoxycholic acid, cholic acid, deoxycholic acid, glycocholic acid, glycochenodeoxycholic acid, hyodeoxycholic acid, lithocholic acid, ω-muricholic acid, taurocholic acid, taurochenodeoxycholic acid, or ursodeoxycholic acid. In some embodiments, the carbohydrate or an oligomer thereof is arabinose, xylose, fructose, galactose, ribose, or glucosinolate, or an oligomer thereof, or hyaluronic acid, lactulose, or trehalose. In some embodiments, the flavonoid (e.g., non-catechin flavonoid) is apigenin, naringenin, genistein, quercetin, luteolin, luteolin, daidzein, equol, or hesperetin. In some embodiments, the vitamin is ascorbic acid, retinoic acid, vitamin D2, vitamin D3, or vitamin E. In other embodiments, the amino acid metabolite is tyramine, indole-3-acetic acid, indole-3-propionic acid, 3-(indole-3-yl)-acrylic acid, kynurenine, 5-hydroxytryptamine, indole-3-pyruvic acid, 3-(indol-3-yl)-2-hydroxypropionic acid, or 2-hydroxyethyl-3-indole. In some embodiments, the lignan is enterodiol, enterolactone, pinoresinol, lariciresinol, secoisolariciresinol, matairesinol, or 7-hydroxyenterolactone. In some embodiments, the nucleic acid is adenosine, cytidine, guanosine, 5-methyluridine, uridine, or a polymeric combination thereof. In some embodiments, the stilbenoid is stilbene, monohydroxystillbene, dihydroxystillbene, trihydroxystillbene, rhapontigenin, piceatannol, pinostilbene, oxyresveratrol, or 4-methoxyresveratrol. In some embodiments, the alcohol core is carnitine, choline, tocopherol, ferulate, oleanolate, aminobenzoate, taurine, tyramine, ursolic acid, fucoxanthin, epigallocatechin gallate, ephedrine, astaxanthin, lutein, 4-(4-hydroxyphenyl)butan-2-one, chlorogenic acid, resveratrol, pterostilbene, hesperetin, daidzein, equol, catechin, apigenin, naringenin, genistein, quercetin, luteolin, policosanol, zeaxanthin, ellagic acid, ellagic acid analogue (e.g., urolithin M5, urolithin A, urolithin B, urolithin C, urolithin D, or urolithin E), hydroxytyrosol, a viniferin (e.g., α-viniferin, β-viniferin, γ-viniferin, δ-viniferin, or ε-viniferin), cycloastragenol, gossypol, 6-gingerol, 6-shogaol, beta-hydroxybutyrate, citicoline, mesalamine, echinocandin (e.g., caspofungin, micafungin, or anidulafungin), secoisolariciresinol, enterodiol, enterolactone, pinoresinol, lariciresinol, secoisolariciresinol, matairesinol, 7-hydroxyenterolactone, caffeic acid, p-coumaric acid, or tocotrienol. In some embodiments, the alcohol core is an antifungal agent (e.g., posaconazole). In further embodiments, the alcohol core is an amino alcohol (e.g., fingolimod).

In certain embodiments, the alcohol core may be generally recognized as safe (GRAS). For example, compounds with a potential GRAS designation include: bile acids (cholic acid, glycocholic acid, or taurocholic acid), carbohydrates and oligomers thereof (e.g., arabinose, xylose, lactulose, fructose, galactose, hyaluronic acid, ribose, trehalose, or a glucosinolate), flavonoids (e.g., apigenin naringenin, catechin, lutein, genistein, quercetin, hesperetin, equol, daidzein, or luteolin), vitamins (e.g., ascorbic acid, retinoic acid, vitamin B5, vitamin D₂, vitamin D₃, or vitamin E), lignans (e.g., enterodiol, enterolactone, pinoresinol, lariciresinol, secoisolariciresinol, matairesinol, or 7-hydroxyenterolactone), choline, β-hydroxycarboxylates (e.g., carnitine), ketone bodies (e.g., β-hydroxybutyrate), pre-ketone bodies (e.g., butane-1,3-diol), amino acid metabolites (e.g., tyramine), stilbenoids or multimers thereof (e.g., resveratrol, pterostilbene, rhapontigenin, piceatannol, pinostilbene, 4-methoxyresveratrol, or a viniferin (e.g., α-viniferin, β-viniferin, γ-viniferin, δ-viniferin, or ε-viniferin)), phenolic phytochemicals (e.g., gossypol; phenalkyls, e.g., shogaol, gingerol, hydroxytyrosol, 4-(4-hydroxyphenyl)butan-2-one, ferulic acid, caffeic acid, or p-coumaric acid; ellagic acid or its analogues, e.g., urolithim A, urolithin B, urolithin C, urolithin, D, urolithin E, or urolithin M5; or vitamin E, e.g., tocotrienol), pentacyclic triterpenoids (e.g., oleanolate or ursolic acid), carotenoids (e.g., fucoxanthin, astaxanthin, zeaxanthin, or lutein), capsinoids (e.g., capsiate), curcuminoids (e.g., curcumin), hydroxycinnamic acid esters (e.g., chlorogenic acid), catechin polyphenols (e.g., epigallocatechin gallate), long chain alcohols (e.g., policosanol), and citicoline.

### Acyls

Exemplary a multibiotic agent can include one or more acyls. In some embodiments, each acyl is independently an amino acid acyl, bile acid acyl, amino acid metabolite acyl, β-hydroxycarboxylate acyl, ketone body acyl, pre-ketone body acyl, sugar acid acyl, hydroxycinnamic acid acyl, phenolic phytochemical acyl, vitamin acyl, pentacyclic triterpenoid acyl, glutathione acyl, α-lipoic acid acyl, mesalamine acyl, polyamine acyl, or picolinate acyl. In further embodiments, each acyl is independently an amino acid acyl, bile acid acyl, fatty acid acyl, amino acid metabolite acyl, β-hydroxycarboxylate acyl, hydroxycinnamic acid acyl, phenolic phytochemical acyl, vitamin acyl, pentacyclic triterpenoid acyl, glutathione acyl, α-lipoic acid acyl, or picolinate acyl.

In some embodiments, the amino acid acyl is an acyl of alanine, arginine, asparagine, aspartic acid, cysteine, glutamic acid, glutamine, glycine, histidine, isoleucine, leucine, lysine, methionine, phenylalanine, proline, selenocysteine, serine, threonine, tyrosine, tryptophan, or carglumic acid. In other embodiments, the bile acid acyl is an acyl of chenodeoxycholic acid, cholic acid, deoxycholic acid, glycocholic acid, glycochenodeoxycholic acid, lithocholic acid, taurocholic acid, taurochenodeoxycholic acid, ω-muricholic acid, or obeticholic acid. In further embodiments, the amino acid metabolite acyl is indole-3-acetic acid acyl, indole-3-propionic acid acyl, or 3-(indole-3-yl)-acrylic acid acyl. In further embodiments, the fatty acid acyl is an acyl of formate, acetate, propionate, butyrate, isobutyrate, valerate, isovalerate, hexanoate, heptanoate, octanoate, nonanoate, decanoate, dodecanoate, myristic acid, palmitic acid, stearic acid, arachidic acid, docosanoic acid, tetracosanoic acid, hexacosanoic acid, eicosapentaenoic acid, docosahexaenoic acid, punicic acid, arachidonic acid, dihomo-gamma-linolenate, docosopentanoate, succinic acid, linoleic acid, alpha-linolenic acid, azelaic acid, or a group of formula: 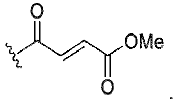 In some embodiments, the acyl is thiosulfate, β-hydroxybutyrate, β-hydroxycarboxylate acyl or oligomer thereof, retinoic acid, indole-3-acetic acid, indole-3-propionic acid, 3-(indole-3-yl)-acrylic acid, ursolic acid, chlorogenic acid, carnitine, folate, niacin, ellagic acid, ellagic acid analogue, glutathione, ornithine, or citrulline. In other embodiments, the acyl is a vitamin acyl (e.g., vitamin A acyl (e.g., tretinoin, isotretinoin, or alitretinoin), vitamin B5 acyl, folic acid acyl, or pralatrexate acyl). In further embodiments, the sugar acid acyl is aldonyl, ulosonyl, uronyl, or aldaryl. In yet further embodiments, a β-hydroxycarboxylate acyl is carnitine acyl or β-hydroxyalkanoate acyl (e.g., β-hydroxybutyrate acyl). In still further embodiments, a pre-ketone body acyl is:

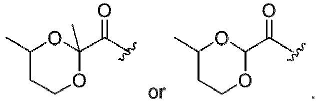

In certain embodiments, the acyl may be generally recognized as safe (GRAS). For example, compounds with a potential GRAS designation include: amino acids (e.g., alanine, arginine, asparagine, aspartic acid, cysteine, glutamic acid, glutamine, glycine, histidine, isoleucine, leucine, lysine, methionine, phenylalanine, proline, selenocysteine, serine, threonine, tyrosine, tryptophan, ornithine, or citrulline), bile acids (e.g., cholic acid, glycocholic acid, or taurocholic acid), fatty acids (e.g., formic acid, acetic acid, propionic acid, butyric acid, isobutyric acid, valeric acid, isovaleric acid, hexanoic acid, heptanoic acid, octanoic acid, nonanoic acid, decanoic acid, myristic acid, palmitic acid, stearic acid, arachidic acid, docosanoic acid, tetracosanoic acid, hexacosanoic acid, eicosapentaenoic acid, docosahexaenoic acid, punicic acid, arachidonic acid, dihomo-gamma-linolenic acid, docosapentaenoic acid, succinic acid, linoleic acid, or alpha-linolenic acid), thiosulfate, ketone bodies (e.g., beta-hydroxy acetic acid or acetoacetic acid), vitamins (e.g., retinoic acid), amino acid metabolite (e.g., indole-3-acetic acid), α-lipoic acid, pentacyclic triterpenoid (e.g., ursolic acid), hydroxycinnamic acid ester (e.g., chlorogenic acid), β-hydroxycarboxylate (e.g., carnitine), vitamin B (e.g., folic acid or niacin), glutathione, picolinic acid, or phenolic phytochemicals (e.g., ellagic acid, ellagic acid analogue, or a phenalkyl (e.g., ferulic acid or caffeic acid)).

### Amine Cores

Exemplary multibiotic agents can include one or more (e.g., 1, 2, 3, 4, 5, or 6) amine cores. In some embodiments, each amine core is independently an amino acid, amino acid metabolite, polyamine, glutathione, vitamin, mesalamine, or antihyperglycemic biguanide. In further embodiments, an amino acid is alanine, arginine, asparagine, aspartic acid, cysteine, glutamic acid, glutamine, glycine, histidine, isoleucine, leucine, lysine, methionine, phenylalanine, proline, selenocysteine, serine, threonine, tyrosine, tryptophan, aminobenzoic acid, glutathione, ornithine, citrulline, or taurine. Polyamines are compounds of the following structure: 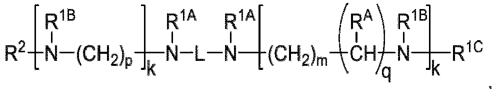 where
L is -(CH₂)ₙ- or cycloalkylene (e.g., cyclohexane-diyl);
each R^{1A} is independently H or a bond to an acyl, or both R^{1A} combine to form an alkylene (e.g., C₂ alkylene);
each R^{1B} is independently H or a bond to an acyl;
R^{1C} is H or a bond to an acyl
R² is H, -CONH₂, -C(NH)NH₂, or a bond to an acyl;
R^{A} is H or-CO-R^{B}, where R^{B} is a bond to an alcohol core or an amine core;
each of n and p is independently an integer from 2 to 6;
m is an integer from 1 to 5; and
each k and q is independently 0 or 1,

In some embodiments, a polyamine is a compound of the following structure: 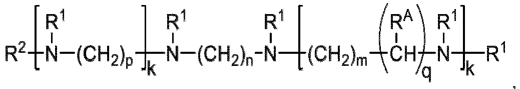 where
each R¹ is independently H or a bond to an acyl;
R² is H, -CONH₂, -C(NH)NH₂, or a bond to an acyl;
R^{A} is H or -CO-R^{B}, where R^{B} is a bond to an alcohol core or an amine core;
each of n and p is independently an integer from 2 to 6;
m is an integer from 1 to 5; and
each k and q is independently 0 or 1,
provided that, when the polyamine is present in a multibiotic agent, at least one R¹ or R² is a bond to an acyl, or R^{B} is a bond to an alcohol core or an amine core.

Examples of the polyamines include:

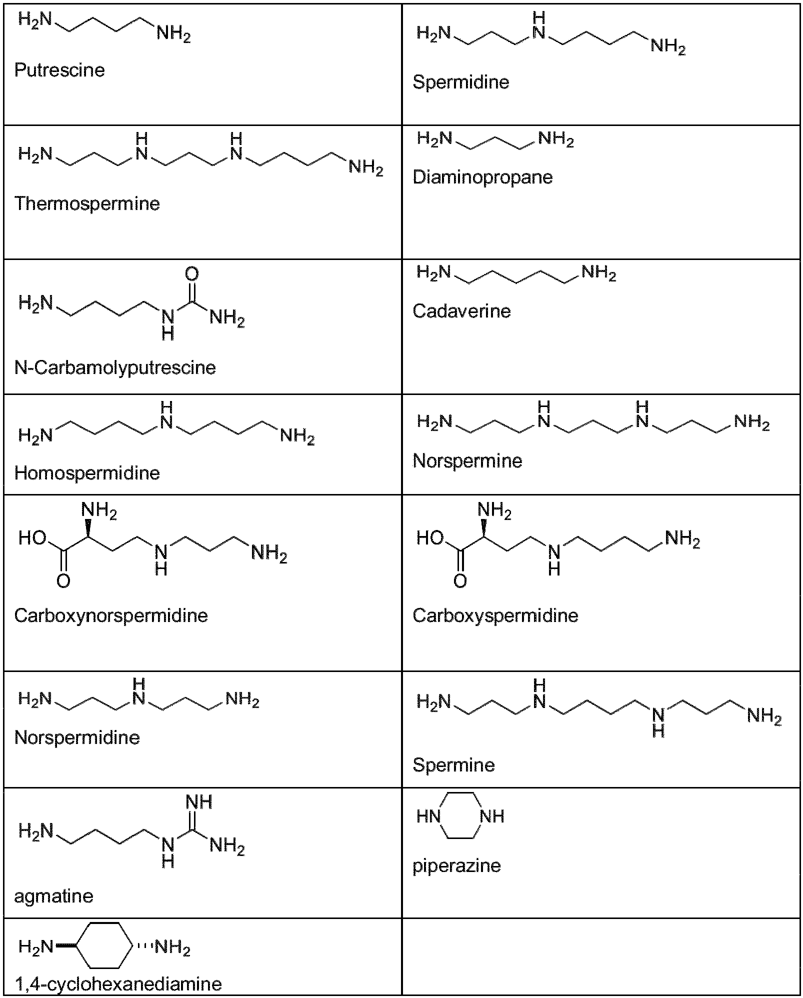

Amine cores capable of forming ester bonds, glycosidic bonds, and/or carbonate linkers include mesalamine, alanine, arginine, aspargine, aspartic acid, cysteine, glutamic acid, glutamine, glycine, histidine, isoleucine, leucine, lysine, methionine, phenylalanine, proline, selenocysteine, serine, threonine, tyrosine, tryptophan, aminobenzoic acid, glutathione, ornithine, citrulline, carboxynorspermidine, carboxyspermidine, glutathione, and vitamins (e.g., folic acid). Amine cores capable of forming azo bonds include mesalamine, alanine, arginine, asparagine, aspartic acid, cysteine, glutamic acid, glutamine, glycine, histidine, isoleucine, leucine, lysine, methionine, phenylalanine, proline, selenocysteine, serine, threonine, tyrosine, tryptophan, aminobenzoic acid, glutathione, ornithine, citrulline, taurine, polyamines, and vitamins (e.g., folic acid). Amine cores capable of forming amide bonds and/or carbamate linkers include mesalamine, alanine, arginine, aspargine, aspartic acid, cysteine, glutamic acid, glutamine, glycine, histidine, isoleucine, leucine, lysine, methionine, phenylalanine, proline, selenocysteine, serine, threonine, tyrosine, tryptophan, aminobenzoic acid, glutathione, ornithine, citrulline, polyamines, and vitamins (e.g., folic acid).

In certain embodiments, the amine core may be generally recognized as safe (GRAS). For example, compounds with a potential GRAS designation include: amino acids (e.g., alanine, arginine, asparagine, aspartic acid, cysteine, glutamic acid, glutamine, glycine, histidine, isoleucine, leucine, lysine, methionine, phenylalanine, proline, selenocysteine, serine, threonine, tyrosine, tryptophan, ornithine, or citrulline), polyamines (e.g., putrescine, spermidine, spermine, diaminopropane, agmatine, or cadaverine), vitamins (e.g., folic acid), and glutathione.

### Hydrolyzable Bonds

Without wishing to be bound by theory, it is believed that multibiotic agents of the invention as defined in the claims can be cleaved by hydrolases or reductases upon delivery to the desired biological compartment. For example, upon oral administration of a multibiotic agent of the invention, the multibiotic agent is delivered to a desired location within the gastrointestinal tract. At such location, the multibiotic agent can be cleaved by a hydrolase or reductase to release the biologically active compounds.

Hydrolases include esterases, amidases, peptidases, and glycosidases. Esterases include acetylesterase, cholinesterase, pectinesterase, thioesterase, phosphatase, biphosphatase, phosphoric diester hydrolase, triphosphoric monoester hydrolase, sulfatase, diphosphoric monoester hydrolase, phosphoric triester hydrolase, exodeoxyribonuclease, exoribonuclease, exonuclease, endonuclease, endodeoxyribonuclease, endoribonuclease, glucosidase, glycosidase, and glycosylase. Reductases include azobenzene reductase, phenylazoxybenzene reductase, nitrate reductase, nitric oxide reductase, nitrite reductase, hyponitrate reductase, and hydroxylamine reductase.

Esterases and azoreductases can be found in those regions of the body colonized by microbiota, such as the distal gastrointestinal tract. In some cases, they can also be found in injured host tissues or those experiencing inflammation.

The methods for preparing multibiotic agents according to the present disclosure are provided for illustrative purposes.

### Methods of Preparing Multibiotic Agents

One of skill in the art is able to adapt reactions and techniques known in the art and/or the synthesis strategies described below to the preparation of a desired multibiotic agent.

Optimum reaction conditions and reaction times may vary depending on the reactants used. Unless otherwise specified, solvents, temperatures, pressures, and other reaction conditions may be readily selected by one of ordinary skill in the art. Specific procedures are provided in the Synthetic Examples section. Typically, reaction progress may be monitored by thin layer chromatography (TLC), if desired, and intermediates and products may be purified by chromatography on silica gel and/or by recrystallization. The appropriately substituted starting materials and intermediates used in the preparation of compounds of the invention are either commercially available or readily prepared by methods known in the art. Examples of the strategies for the preparation of multibiotic agents are illustrated below.

### Preparation of Ester-Containing Multibiotic Agents

Ester-containing multibiotic agents can be prepared using commercially available starting materials, known intermediates, or by using the synthetic methods known in the art (e.g., those described in Wuts, P., Greene's Protective Groups in Organic Synthesis, 5th Ed., Wiley (2014)).

The following exemplary schemes illustrate methods of preparing multibiotic agent esters of the present invention. These methods are not limited to producing the compounds shown, but can be used to prepare a variety of molecules such as the esters described herein. The compounds of the present invention can also be synthesized by methods not explicitly illustrated in the schemes but which are well within the skill of one in the art. The compounds can be prepared using readily available materials or known intermediates.

In the following schemes, Ar is an optionally substituted aryl or an optionally substituted heteroaryl; X is chloro, bromo or iodo; and PG represents a protecting group.

### Ester Preparation Strategy #1 (Acylation)

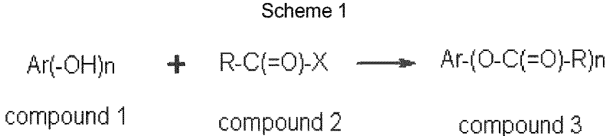

In Scheme 1, a polyphenolic compound, compound 1, where n represents an integer from 1 to 15, is treated with an acylating agent, compound 2, in an appropriate solvent, optionally in the presence of a catalyst. Suitable catalysts include pyridine, dimethylaminopyridine, trimethylamine and the like. The catalyst can be used in quantities ranging from 0.01 to 1.1 equivalents, relative to compound 2. Suitable solvents include methylene chloride, ethyl acetate, diethyl ether, tetrahydrofuran, 1,4-dioxane, 1,2-dimethoxyethane, toluene, combinations thereof and the like. Reaction temperatures range from -10° C. to the boiling point of the solvent used; reaction completion times range from 1 to 96 h. Suitable acylating agents include acyl chlorides, acyl fluorides, acyl bromides, carboxylic acid anhydrides whether symmetrical or not. A suitable acylating agent may also be generated in situ by prior reaction of a carboxylic acid with an activating reagent such as EDC or EEDQ or the like. The acylating agents can be used in quantities ranging from 0.5 to 15 equivalents relative to compound 1.

The product, compound 3, can be purified by methods known in the art.

### Ester Preparation Strategy #2 (Acylation)

In some cases, the polyphenolic compound 1 may contain a functional group, Y, required to remain unreacted in the course of ester formation. In this case, it is appropriate to protect the functional group, Y, in the polyphenolic compound from acylation. This functional group may be an amino group or a hydroxyl group or other functionality with a labile hydrogen attached to a heteroatom. Such polyphenol esters can be prepared according to Scheme 2.

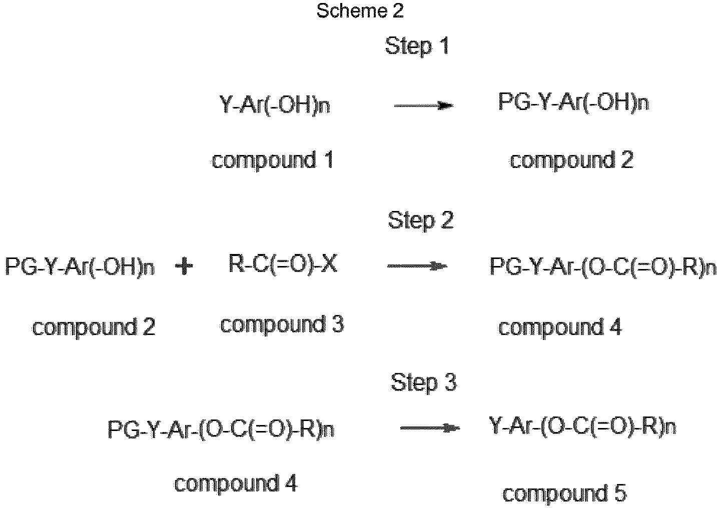

In Scheme 2 Step 1, compound 1, a polyphenolic compound containing a functional group Y with a labile hydrogen in need of protection, is treated with a protecting reagent such as BOC anhydride, benzyoxycarbonyl chloride, FMOC chloride, benzyl bromide and the like in an appropriate solvent, optionally in the presence of a catalyst to provide compound 2 scheme 2. Compound 2 can be purified by methods known in the art.

In Scheme 2 Step 2, compound 2 is treated with an acylating agent, compound 3, in an appropriate solvent, optionally in the presence of a catalyst. Suitable catalysts include pyridine, dimethylaminopyridine, trimethylamine and the like. The catalyst can be used in quantities ranging from 0.01 to 1.1 equivalents, relative to compound 2. Suitable solvents include methylene chloride, ethyl acetate, diethyl ether, tetrahydrofuran, 1,4-dioxane, 1,2-dimethoxyethane, toluene, combinations thereof and the like. Reaction temperatures range from -10° C to the boiling point of the solvent used; reaction completion times range from 1 to 96 h. Suitable acylating agents include acyl chlorides, acyl fluorides, acyl bromides, carboxylic acid anhydrides whether symmetrical or not. A suitable acylating agent may also be generated in situ by prior reaction of a carboxylic acid with an activating reagent such as EDC or EEDQ or the like. The acylating agents can be used in quantities ranging from 0.5 to 15 equivalents, relative to compound 3. Compound 4 can be purified by methods known in the art.

In Scheme 2 Step 3, compound 4 is subjected to conditions that cleave the protecting group, PG.

In the case of a BOC protecting group, the protecting group of compound 4 is removed under acidic conditions to give compound 5 of the invention. Suitable acids include trifluoroacetic acid, hydrochloric acid, p-toluenesulfonic acid and the like.

In the case of an FMOC protecting group, the protecting group of compound 4 is removed under basic conditions to give compound 5 of the invention. Suitable bases include piperidine, triethylamine and the like. Suitable solvents include DMF, NMP dichoromethane and the like. The FMOC group is also removed under non-basic conditions such as by treatment with tetrabutylammonium fluoride trihydrate in a suitable solvent such as DMF. The FMOC group is also removed by catalytic hydrogenation. Suitable catalysts for hydrogenation include 10% palladium-on-charcoal and palladium (II) acetate and the like.

### Suitable solvents for hydrogenation include DMF, ethanol, and the like

In the case of a benzyloxycarbonyl or benzyl protecting group the protecting group of compound 4 is removed by hydrogenation to give compound 5. Suitable catalysts for hydrogenation include 10% Palladium-on-charcoal and Palladium acetate and the like. Suitable solvents for hydrogenation include DMF, ethanol, methanol, ethyl acetate, and the like. The product, compound 5, can be purified by methods known in the art.

### Ester Preparation Strategy #3 (Acylation)

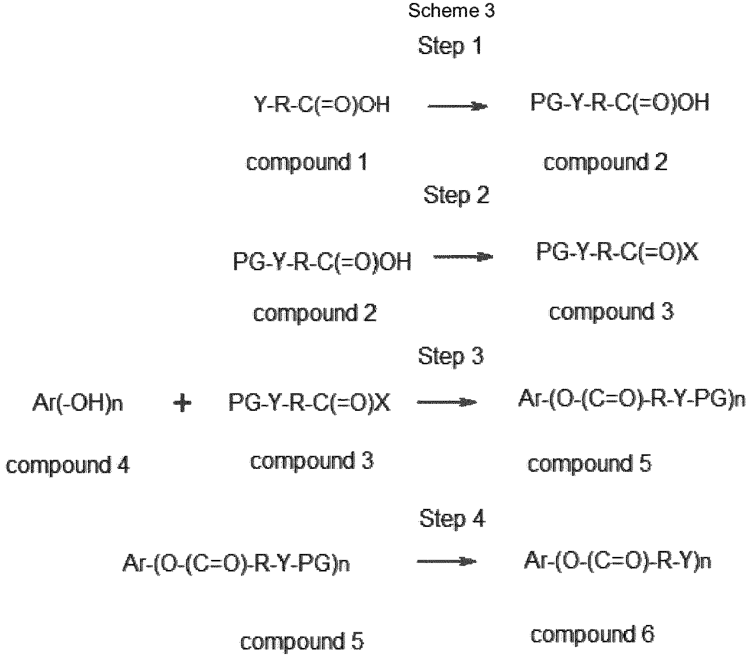

In Scheme 3 Step 1, compound 1, an acyl compound containing a functional group Y with a labile hydrogen in need on protection, is treated with a protecting reagent such as BOC anhydride, benzyoxycarbonyl chloride, FMOC chloride, benzyl bromide and the like in an appropriate solvent, optionally in the presence of a catalyst to provide compound 2 scheme 3. Compound 2 can be purified by methods known in the art.

In Scheme 3 Step 2, compound 2 is treated with an activating reagent such as thionyl chloride, phosphorus oxychloride, EDC or EEDQ or the like to generate the activated acyl compound 3.

In Scheme 3 Step 3, the polyphenol compound 4 is treated with the activated acyl compound 3, in an appropriate solvent, optionally in the presence of a catalyst. Suitable catalysts include pyridine, dimethylaminopyridine, trimethylamine and the like to generate compound 5. The catalyst can be used in quantities ranging from 0.01 to 1.1 equivalents, relative to compound 3. Suitable solvents include methylene chloride, ethyl acetate, diethyl ether, tetrahydrofuran, 1,4-dioxane, 1,2-dimethoxyethane, toluene, combinations thereof and the like. Reaction temperatures range from -10° C. to the boiling point of the solvent used; reaction completion times range from 1 to 96 h. The activated acyl compound 3 can be used in quantities ranging from 0.5 to 15 equivalents relative to compound 4.

In Scheme 3 Step 4, compound 5 is subjected to conditions designed to cleave the protecting group, PG, illustrated in Scheme 2 above. The product, compound 6, can be purified by methods known in the art.

### Ester Preparation Strategy #4 (Acylation)

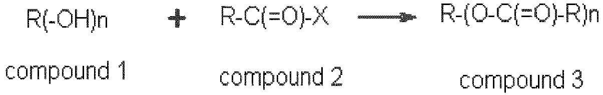

In Scheme 4 Step 1 a poly-ol compound, compound 1, where R represents a non-aromatic cyclic or acyclic moiety and n represents an integer from 1 to 15, is treated with an acylating agent, compound 2, in an appropriate solvent, optionally in the presence of a catalyst. Suitable catalysts include pyridine, dimethylaminopyridine, trimethylamine and the like. The catalyst can be used in quantities ranging from 0.01 to 1.1 equivalents, relative to compound 2. Suitable solvents include methylene chloride, ethyl acetate, diethyl ether, tetrahydrofuran, 1,4-dioxane, 1,2-dimethoxyethane, toluene, combinations thereof and the like. Reaction temperatures range from -10° C. to the boiling point of the solvent used; reaction completion times range from 1 to 96 h. Suitable acylating agents include acyl chlorides, acyl fluorides, acyl bromides, carboxylic acid anhydrides whether symmetrical or not. A suitable acylating agent may also be generated in situ by prior reaction of a carboxylic acid with an activating reagent such as EDC or EEDQ or the like. The acylating agents can be used in quantities ranging from 0.5 to 15 equivalents, relative to compound 1. The product, compound 3, can be purified by methods known in the art.

### Ester Preparation Strategy #5 (Baeyer-Villiger Oxidation)

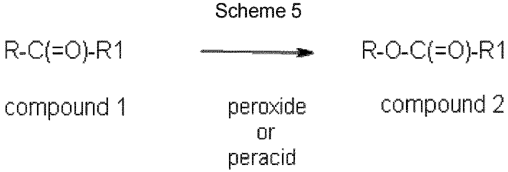

In Scheme 5 Step 1, a ketone compound, compound 1, where R and R1 represent non-aromatic cyclic or acyclic moieties, is treated with a peroxide or peroxyacid agent, such as meta-chloroperbenzoic acid, performic acid, peracetic acid, hydrogen peroxide, tert-butyl hydroperoxide and the like in an appropriate solvent, optionally in the presence of a catalyst. Suitable solvents include methylene chloride, diethyl ether, combinations thereof and the like. Suitable catalysts include BF₃, carboxylic acids and the like. Reaction temperatures range from -10° C. to the boiling point of the solvent used; reaction completion times range from 1 to 96 h. The product, compound 2, can be purified by methods known in the art.

The R and R1 groups of compound 1 in Scheme 5 may optionally include additional ketone functionality that can undergo reaction. In addition the R and R1 groups of compound 1 may form a ring.

### Ester Preparation Strategy #6 (Mitsunobu Reaction)

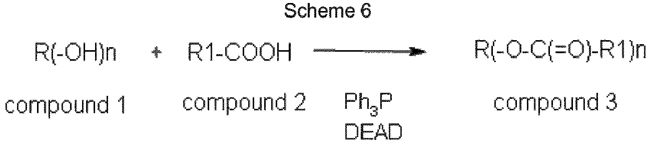

In Scheme 6 Step 1, a mixture of an alcohol compound, compound 1, where R represents a non-aromatic cyclic or acyclic moiety, and a carboxylic acid, compound 2 where R1 represents an alkanoyl group optionally substituted with one or more protected hydroxyl groups or oxo is treated with triphenylphosphine and a diazo compound such as diethylazodicarboxylate (DEAD) and the like in an appropriate solvent. Suitable solvents include methylene chloride, THF, acetonitrile, toluene, diethyl ether, combinations thereof and the like. Reaction temperatures range from -10° C. to the boiling point of the solvent used; reaction completion times range from 1 to 96 h. The product, compound 3 can be purified by methods known in the art.

Where compound 3 is optionally substituted by one or more protected alcohol groups deprotection is accomplished by the methods illustrated in Scheme 2 above.

### Ester preparation strategy #7 (Nucleophilic Alkylation)

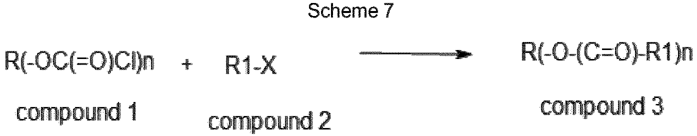

In Scheme 7 Step 1, a chloroformate compound, compound 1, where R represents an aromatic moiety or a non-aromatic cyclic or acyclic moiety, is treated, in an appropriate solvent, with an organometallic compound, compound 2 where R1 represents an alkyl group optionally substituted with one or more protected hydroxyl groups and X represents a metal such as Cu, Zn, Mg which is optionally coordinated by one or more counterions, such as chloride. Suitable solvents include methylene chloride, THF, acetonitrile, toluene, diethyl ether, combinations thereof, and the like. Reaction temperatures range from -10° C. to the boiling point of the solvent used; reaction completion times range from 1 to 96 h. The product, compound 3, can be purified by methods known in the art.

Compound 1 can be prepared from the corresponding alcohol or polyol compounds by standard methods familiar to one skilled in the art.

Where compound 2 is optionally substituted by one or more protected alcohol groups deprotection is accomplished by the methods illustrated in Scheme 2 above.

Further modification of the initial product by methods known in the art and illustrated in the examples below, may be used to prepare additional compounds.

### Ester Preparation Strategy #8 (Acylation)

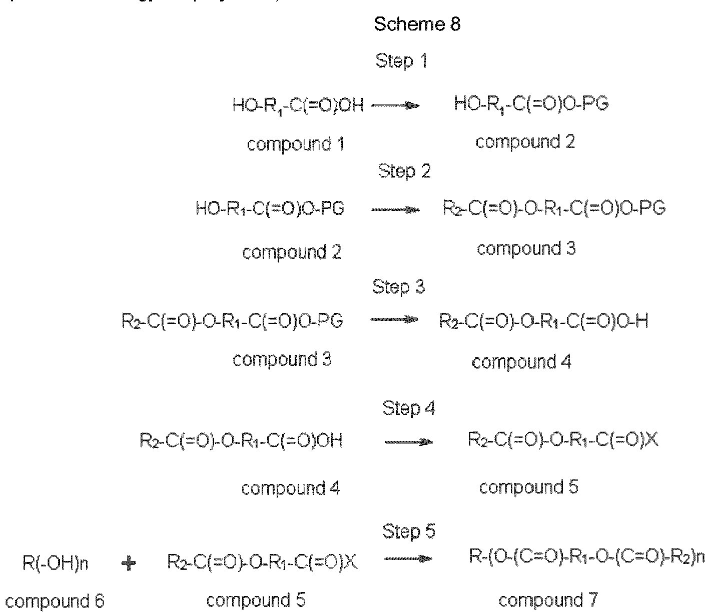

In Scheme 8 Step 1, compound 1, an acyl compound containing a hydroxyl group to be acylated, is treated with a protecting reagent such as benzyl bromide and the like in an appropriate solvent, optionally in the presence of a catalyst to provide compound 2 scheme 8. Compound 2 can be purified by methods known in the art.

In Scheme 8 Step 2, compound 2 is treated with an acylating agent, in an appropriate solvent, optionally in the presence of a catalyst. Suitable catalysts include pyridine, dimethylaminopyridine, trimethylamine and the like. The catalyst can be used in quantities ranging from 0.01 to 1.1 equivalents, relative to compound 2. Suitable solvents include methylene chloride, ethyl acetate, diethyl ether, tetrahydrofuran, 1,4-dioxane, 1,2-dimethoxyethane, toluene, combinations thereof and the like. Reaction temperatures range from -10° C to the boiling point of the solvent used; reaction completion times range from 1 to 96 h. Suitable acylating agents include acyl chlorides, acyl fluorides, acyl bromides, carboxylic acid anhydrides whether symmetrical or not. A suitable acylating agent may also be generated in situ by a reaction of a carboxylic acid with an activating reagent such as EDC or EEDQ or the like. The acylating agents can be used in quantities ranging from 0.5 to 15 equivalents relative to compound 1.

In Scheme 8 Step 3, compound 3 is subjected to conditions that cleave the protecting group, PG. In the case of a benzyl protecting group, the protecting group of compound 3 is removed by hydrogenation to give compound 4. Suitable catalysts for hydrogenation include 10% palladium-on-charcoal and palladium acetate and the like. Suitable solvents for hydrogenation include, DMF, ethanol, methanol, ethyl acetate and the like. The product, compound 4, can be purified by methods known in the art.

In Scheme 8 Step 4, compound 4 is treated with an activating reagent such as thionyl chloride, phosphorus oxychloride, EDC or EEDQ or the like to generate the activated acyl compound 5.

In Scheme 8 Step 5, the poly-hydroxyl compound, compound 6, where R represents an aromatic or an aliphatic cyclic or acyclic core, is treated with the activated acyl compound 5, in an appropriate solvent, optionally in the presence of a catalyst. Suitable catalysts include pyridine, dimethylaminopyridine, trimethylamine and the like to generate compound 5. The catalyst can be used in quantities ranging from 0.01 to 1.1 equivalents, relative to compound 3. Suitable solvents include methylene chloride, ethyl acetate, diethyl ether, tetrahydrofuran, 1,4-dioxane, 1,2-dimethoxyethane, toluene, combinations thereof and the like. Reaction temperatures range from -10° C. to the boiling point of the solvent used; reaction completion times range from 1 to 96 h. The activated acyl compound 5 can be used in quantities ranging from 0.5 to 15 equivalents relative to compound 6.

The product, compound 7, can be purified by methods known in the art.

### Preparation of Azo-Containing Multibiotic agents

Azo-containing multibiotic agents can be prepared using commercially available starting materials, known intermediates, or by using the synthetic methods known in the art. The following exemplary schemes illustrate methods of preparing multibiotic agent azo compounds. These methods are not limited to producing the compounds shown, but can be used to prepare a variety of molecules such as the azo containing compounds described herein. The compounds of the present invention can also be synthesized by methods not explicitly illustrated in the schemes, but which are well within the skill of one in the art. The compounds can be prepared using readily available materials or known intermediates.

### Azo preparation strategy #1

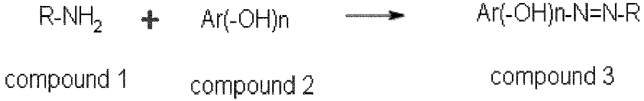

In Azo preparation strategy #1, a compound containing a primary amino group, compound 1 where R represents a an optionally substituted acyclic aliphatic or cyclic aromatic or cyclic aliphatic moiety, dissolved in an aqueous acid, such as hydrochloric acid, is treated at 0°C to 5°C with a nitrosating agent such as sodium nitrite to generate an intermediate diazonium compound. Compound 2, where n represents an integer from 1 to 15, is then added to the intermediate diazonium compound while the pH is maintained between pH6 and pH7 and the temperature is maintained in the range between 0°C and 10°C for up to 24 hours. The product, compound 3 can be purified by methods known in the art.

### Azo preparation strategy #2

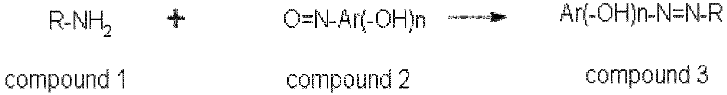

In Azo preparation strategy #2, a compound containing a primary amino group, compound 1 where R represents an optionally substituted acyclic aliphatic or cyclic aromatic or cyclic aliphatic moiety, in a suitable solvent such as ethanol, DMF, 1,4-dioxane and the like, is treated at a temperature up to the boiling point of the solvent with the nitroso compound 2 for 1 to 24 hours. The product, compound 3 can be purified by methods known in the art.

### Azo preparation strategy #3

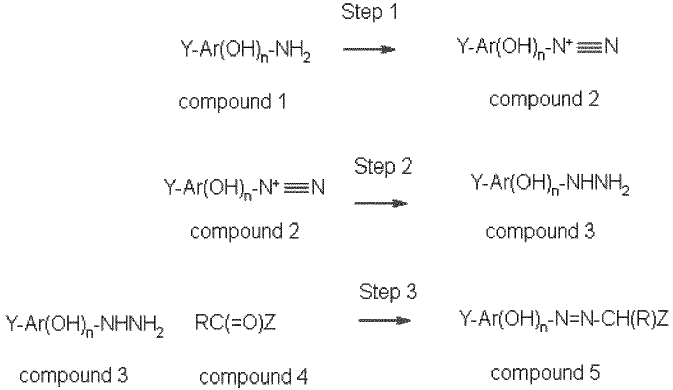

In Azo preparation strategy #3 Step 1, compound 1, where n=1 to 3 and Y is an optional substituent such as a carboxylate or carboxylic acid ester, is transformed to the diazonium compound 2 by reaction with a diazotizing agent such as sodium nitrite or potassium nitrite an acidic aqueous solvent such as aqueous hydrochloric acid at a temperature between 0 °C and 10 °C. The diazonium compound is preferably reacted directly with a reducing agent, such as Sn(II)Cl₂ in aqueous hydrochloric acid to give the hydrazine compound 3 as shown in step 2. The hydrazine product, compound 3, can be purified by methods known in the art.

In Step 3, hydrazine compound 3 is treated with an carbonyl containing compound, compound 4 where Z is optionally a Hdrogen atom, a carboxylate group or a carboxylic acid ester, in an inert solvent such as ethanol, DMF, 1 ,4-dioxane or THF or the like, at a temperature up to the boiling point of the solvent gives the azo compound 5 which can be purified by methods known in the art.

### Amide preparation strategy #1

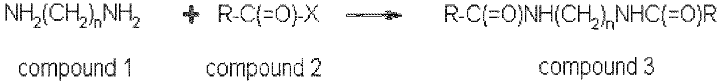

In Amide preparation strategy #1, a polyamine, compound 1 where n represents an integer from 2 to 15, is treated with an acylating agent, compound 2, in an appropriate solvent, optionally in the presence of a catalyst. Suitable catalysts include pyridine, dimethylaminopyridine, trimethylamine and the like. The catalyst can be used in quantities ranging from 0.01 to 1.1 equivalents, relative to compound 2. Suitable solvents include pyridine, methylene chloride, ethyl acetate, diethyl ether, tetrahydrofuran, 1,4-dioxane, 1,2-dimethoxyethane, toluene, combinations thereof and the like. Reaction temperatures range from -10 °C to the boiling point of the solvent used; reaction completion times range from 1 to 96 h. Suitable acylating agents, compound 2, include acyl chlorides, acyl fluorides, acyl bromides, carboxylic acid anhydrides whether symmetrical or not. A suitable acylating agent may also be generated in situ by prior reaction of a carboxylic acid with an activating reagent such as EDC or EEDQ or the like. The acylating agents can be used in quantities ranging from 0.5 to 15 equivalents, relative to compound 1.

The product, compound 3, can be purified by methods known in the art.

### Amide preparation strategy #2

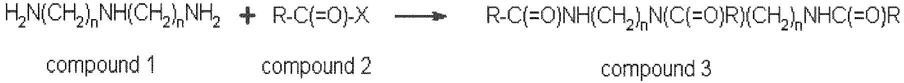

In amide preparation strategy #2, a polyamine, compound 1 where n independently represents an integer from 2 to 15, is treated with an acylating agent, compound 2, in an appropriate solvent, optionally in the presence of a catalyst. Suitable catalysts include pyridine, dimethylaminopyridine, trimethylamine and the like. The catalyst can be used in quantities ranging from 0.01 to 1.1 equivalents, relative to compound 2. Suitable solvents include pyridine, methylene chloride, ethyl acetate, diethyl ether, tetrahydrofuran, 1,4-dioxane, 1,2-dimethoxyethane, toluene, combinations thereof and the like. Reaction temperatures range from -10 °C to the boiling point of the solvent used; reaction completion times range from 1 to 96 h. Suitable acylating agents, compound 2, include acyl chlorides, acyl fluorides, acyl bromides, carboxylic acid anhydrides whether symmetrical or not. A suitable acylating agent may also be generated in situ by prior reaction of a carboxylic acid with an activating reagent such as EDC or EEDQ or the like. The acylating agents can be used in quantities ranging from 0.5 to 15 equivalents, relative to compound 1.

The product, compound 3, can be purified by methods known in the art.

### Amide preparation strategy #3

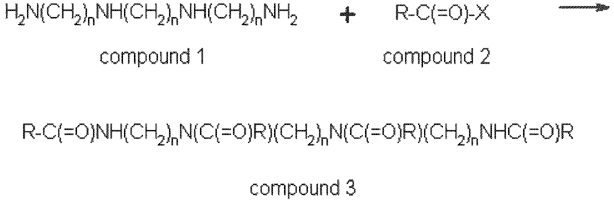

In Amide preparation strategy #3, a polyamine, compound 1 where n independently represents an integer from 2 to 15, is treated with an acylating agent, compound 2, in an appropriate solvent, optionally in the presence of a catalyst. Suitable catalysts include pyridine, dimethylaminopyridine, trimethylamine and the like. The catalyst can be used in quantities ranging from 0.01 to 1.1 equivalents, relative to compound 2. Suitable solvents include pyridine, methylene chloride, ethyl acetate, diethyl ether, tetrahydrofuran, 1,4-dioxane, 1,2-dimethoxyethane, toluene, combinations thereof and the like. Reaction temperatures range from -10 °C to the boiling point of the solvent used; reaction completion times range from 1 to 96 h. Suitable acylating agents, compound 2, include acyl chlorides, acyl fluorides, acyl bromides, carboxylic acid anhydrides whether symmetrical or not. A suitable acylating agent may also be generated in situ by prior reaction of a carboxylic acid with an activating reagent such as EDC or EEDQ or the like. The acylating agents can be used in quantities ranging from 0.5 to 15 equivalents, relative to compound 1.

The product, compound 3, can be purified by methods known in the art.

### Amide preparation strategy #4

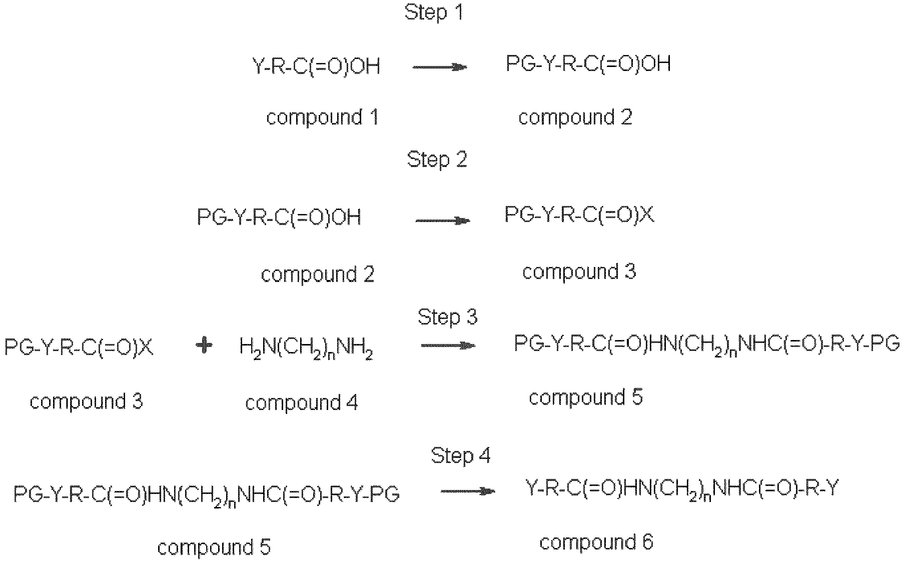

In Amide preparation strategy #4, Step 1, compound 1, an acyl compound containing a functional group Y with a labile hydrogen in need on protection, is treated with a protecting reagent such as BOC anhydride, benzyoxycarbonyl chloride, FMOC chloride, benzyl bromide and the like in an appropriate solvent, optionally in the presence of a catalyst to provide compound 2. Compound 2, can be purified by methods known in the art.

In Step 2, compound 2 is treated with an activating reagent such as thionyl chloride, phosphorus oxychloride, EDC or EEDQ or the like to generate the activated acyl compound 3.

In Step 3, the polyamine compound 4 is treated with the activated acyl compound 3, in an appropriate solvent, optionally in the presence of a catalyst. Suitable catalysts include pyridine, dimethylaminopyridine, trimethylamine and the like to generate compound 5. The catalyst can be used in quantities ranging from 0.01 to 1.1 equivalents, relative to compound 3. Suitable solvents include pyridine, methylene chloride, ethyl acetate, diethyl ether, tetrahydrofuran, 1,4-dioxane, 1,2-dimethoxyethane, toluene, combinations thereof and the like. Reaction temperatures range from -10° C. to the boiling point of the solvent used; reaction completion times range from 1 to 96 h. The activated acyl compound 3 can be used in quantities ranging from 0.5 to 15 equivalents, relative to compound 4.

In Step 4, compound 5 is subjected to conditions designed to cleave the protecting group, PG.

In the case of a BOC protecting group, the protecting group of compound 5 is removed under acidic conditions to give compound 6 of the invention. Suitable acids include trifluoroacetic acid, hydrochloric acid, p-toluenesulfonic acid and the like.

In the case of an FMOC protecting group, the protecting group of compound 5 is removed under basic conditions to give compound 6 of the invention. Suitable bases include piperidine, triethylamine and the like. Suitable solvents include DMF, NMP dichoromethane and the like. The FMOC group is also removed under non-basic conditions such as by treatment with tetrabutylammonium fluoride trihydrate in a suitable solvent such as DMF. The FMOC group is also removed by catalytic hydrogenation. Suitable catalysts for hydrogenation include 10% Palladium-on-charcoal and Palladium acetate and the like. Suitable solvents for hydrogenation include, DMF, ethanol and the like

In the case of a benzyloxycarbonyl or benzyl protecting group the protecting group of compound 5 is removed by hydrogenation to give compound 6 of the invention. Suitable catalysts for hydrogenation include 10% Palladium-on-charcoal and Palladium acetate and the like. Suitable solvents for hydrogenation include, DMF, ethanol, methanol, ethyl acetate and the like.

The product, compound 6, can be purified by methods known in the art.

### Amide preparation strategy #5

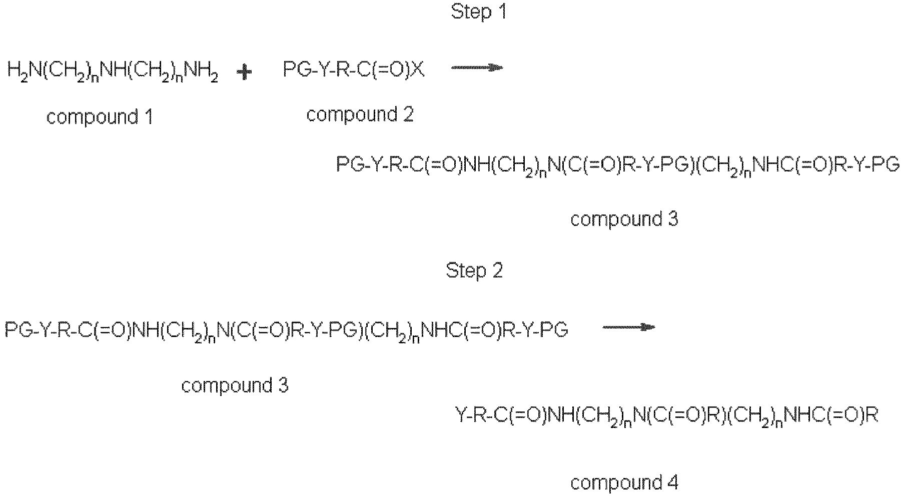

In Amide preparation strategy #5, Step 1, the polyamine, compound 1, is treated with the activated acyl compound 2 (described in amide preparation strategy #4 above), in an appropriate solvent, optionally in the presence of a catalyst. Suitable catalysts include pyridine, dimethylaminopyridine, trimethylamine and the like to generate compound 5. The catalyst can be used in quantities ranging from 0.01 to 1.1 equivalents, relative to compound 2. Suitable solvents include pyridine, methylene chloride, ethyl acetate, diethyl ether, tetrahydrofuran, 1,4-dioxane, 1,2-dimethoxyethane, toluene, combinations thereof and the like. Reaction temperatures range from -10° C. to the boiling point of the solvent used; reaction completion times range from 1 to 96 h. The activated acyl compound 3 can be used in quantities ranging from 0.5 to 15 equivalents, relative to compound 1.

In Step 2, compound 3 is subjected to conditions designed to cleave the protecting group, PG, illustrated in amide preparation strategy #4 above. The product, compound 4, can be purified by methods known in the art.

### Health Benefits

Any references to methods of treatment by therapy or surgery or in vivo diagnosis methods in the following disclosure are to be interpreted as references to multibiotic agents, pharmaceutical compositions and medicaments of the present invention for use in those methods.

### Cardiovascular Health

Accordingly, multibiotic agents disclosed herein may be used for treating a cardiovascular disorder in a subject. Typically, a method of treating a cardiovascular disorder in a subject may include administering a multibiotic agent or a pharmaceutical or nutraceutical composition containing a multibiotic agent to the subject.

Multibiotic agents disclosed herein may be used to provide cardiovascular health benefits to a subject, e.g., to treat high blood pressure, hypercholesterolemia, or hyperlipidemia. In some embodiments, a multibiotic agent described herein improves cholesterol levels (e.g., reduces LDL levels and/or rebalances HDL levels) in a tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In certain embodiments, a multibiotic agent described herein decreases triglycerides and/or lipids in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In particular embodiments, a multibiotic agent described herein reduces lipoprotein(a) levels in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In further embodiments, a multibiotic agent described herein reduces apo-c3 levels in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In yet further embodiments, a multibiotic agent described herein reduces blood pressure in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control).

Components of animal protein products, such as choline and carnitine, can be metabolized by enzymes in gut microbiota to trimethylamine (TMA). TMA can be absorbed through the gut and metabolized by human liver enzymes to trimethylamine-N-oxide (TMAO). TMAO is recognized as an agent contributing to atherosclerotic cardiovascular disease, congestive heart failure and kidney failure (Yamashita et al, Circ J. 79:1882-90, 2015). Additionally, resveratrol (trihydroxystillbene) has shown cardiovascular protective effects mediated by various putative biologic mechanisms including improvement of endothelial function (Zhang et al, Nutr Cancer. 62(1): 1-20, 2010), attenuating mitochondrial oxidative stress (Ungvari et al, Am J Physiol Heart Circ Physiol. 297(5):H1876-81, 2009) and remodeling the gut microbiota to decrease TMAO synthesis (Chen, Biochem Biophys Res Commun. 481(1-2):63-70, 2016).

In an exemplary embodiment, a multibiotic agent containing a combination of the 3,3-dimethyl-1-butanol (DMB) and resveratrol can reduce or inhibit the microbiome catalyzed generation of TMA from dietary choline and carnitine. In certain embodiments, a multibiotic agent of the invention having cardiovascular health benefits described herein may include an alcohol core that is an amino alcohol, antifungal agent, bile acid, β-hydroxycarboxylate, carbohydrate or an oligomer thereof, carotenoid, stilbenoid, flavonoid, lignan, phenolic phytochemical, long chain alcohol, sugar alcohol, anhydrosugar alcohol, sugar acid, or vitamin. In some embodiments, a multibiotic agent of the invention having cardiovascular health benefits described herein may include an amine core that is an amino acid, amino acid metabolite, antifungal agent, anti hyperglycemic biguanide, polyamine, or vitamin. In particular embodiments, a multibiotic agent of the invention having cardiovascular health benefits described herein may include an acyl that is an amino acid acyl, amino acid metabolite acyl, bile acid acyl, fatty acid acyl, β-hydroxycarboxylate acyl, phenolic phytochemical acyl (e.g., phenalkyl acyl or an acyl of ellagic acid or an analogue thereof), sugar acid acyl, or vitamin acyl.

In further embodiments, an amino alcohol is choline. In yet further embodiments, an antifungal agent is an echinocandin (e.g., micafungin, caspofungin, or anidulafungin). In still further embodiments, a bile acid is hyodeoxycholic acid or ω-muricholic acid. In further embodiments, a β-hydroxycarboxylate is carnitine. In some embodiments, a carbohydrate or an oligomer thereof is ribose or trehalose. In some embodiments, a carotenoid is astaxanthin, lutein, or zexanthin. In further embodiments, a stilbenoid is trihydroxystilbene, pterostilbene, rhapontigenin, piceatannol, pinostilbene, oxyresveratrol, or 4-methoxyresveratrol. In yet further embodiments, a flavonoid is catechin, genistein, quercetin, hesperetin, daidzein, equol, or luteolin. In still further embodiments, a long chain alcohol is policosanol. In some embodiments, a lignan is pinoresinol, laricisresinol, secoisolariciresinol, matairesinol, or 7-hydroxyenterolactone. In some embodiments, a phenolic phytochemical is ellagic acid or an analogue thereof (preferably, urolithin A, urolithin B, urolithin C, urolithin D, urolithin E, or urolithin M5). In other embodiments, a phenolic phytochemical is a phenalkyl (preferably, ferulic acid, caffeic acid, or p-coumaric acid). In further embodiments, a sugar alcohol is inositol or erythritol. In yet further embodiments, an anhydrosugar alcohol is sorbitan or isosorbide. In still further embodiments, a sugar acid is gluconic acid (e.g., D-gluconic acid).

In some embodiments, an amino acid is arginine. In other embodiments, an amino acid metabolite is indole-3-propionic acid or 3-(indole-3-yl)-acrylic acid. In some embodiments, an antifungal agent is an echinocandin (e.g., anidulafungin). In further embodiments, an antihyperglycemic biguanide is metformin. In yet further embodiments, a polyamine is spermidine. In still further embodiments, a vitamin is folic acid.

In some embodiments, an amino acid acyl is arginine. In other embodiments, an amino acid metabolite acyl is indole-3-propionic acid acyl or 3-(indole-3-yl)-acrylic acid acyl. In particular embodiments, a bile acid acyl is hyodeoxycholic acid acyl or ω-muricholic acid acyl. In further embodiments, a fatty acid acyl is linoleic acid acyl. In yet further embodiments, a β-hydroxycarboxylate acyl is carnitine acyl. In still further embodiments, a phenolic phytochemical acyl is a phenalkyl acyl (preferably, ferulic acid acyl, caffeic acid acyl, or p-coumaric acid acyl). In some embodiments, a phenolic phytochemical acid is ellagic acid acyl or an analogue thereof (preferably, urolithin A acyl, urolithin B acyl, urolithin C acyl, urolithin D acyl, urolithin E acyl, or urolithin M5 acyl). In other embodiments, a sugar acid acyl is gluconic acid acyl. In further embodiments, a vitamin acyl is vitamin B5 acyl, folate acyl, or niacin acyl.

In some embodiments, a multibiotic agent providing cardiovascular health benefits is hydrolyzed in the small intestine (e.g., as measured using simulated intestinal fluid assay) to release active agents targeting a disease or condition and is substantially stable in the stomach (e.g., as measured using simulated gastric fluid assay). In certain embodiments, a multibiotic agent providing cardiovascular health benefits is substantially stable in the stomach (e.g., as measured using simulated gastric fluid assay) and provides individual components of the multibiotic agent to the colon (e.g., after hydrolysis in the colon). Hydrolysis in the colon may be measured using fecal stability assay as described herein.

### Metabolic Health

Compositions and methods may be used to treat a metabolic disease (e.g., diabetes or obesity) or NAFLD/NASH. Typically, a method of treating a metabolic disorder in a subject may include administering a multibiotic agent or a pharmaceutical or nutraceutical composition containing a multibiotic agent to the subject.

Western diets high in fats and refined carbohydrates are associated with weight gain leading to obesity and risk for metabolic syndrome, type 2 diabetes (DM2) and non-alcoholic fatty liver disease (NAFLD). Consumption of these diets may lead to accumulation of fat in the adipose tissue and liver. This may result in a change in the gut microbiome, elevation of markers of inflammation in the blood, infiltration of inflammatory cells into the liver and impaired glucose tolerance with insulin resistance. In susceptible individuals, these dietary driven changes can lead to outright diabetes with or without NAFLD-related chronic inflammation of the liver which may progress to non-alcoholic steatohepatitis (NASH). NASH can progress to cirrhosis of the liver which can be fatal. DM2, with or without overt liver disease, can cause cardiovascular and ophthalmic disease which can result in blindness, peripheral vascular insufficiency, cardiac disease and premature death. These dietary changes also correlate with changes in the gut microbiome termed dysbiosis. Correcting gut dysbiosis can lead to weight loss and improved glucose tolerance which, longer term, might be expected to abrogate many of the deleterious effects of an unhealthy diet. Metabolic products of the human gut microbiome such as short chain fatty acids (SFCAs) have been demonstrated to have immunomodulatory and metabolic effects upon the human host. In some cases these molecules have been shown to work by binding to short chain fatty acid receptors (Tolhurst, Diabetes. 61(2):364-71, 2002; Chambers, Proc Nutr Soc. 74(3):328-36, 2015) In other cases the benefit has been thought to work via mechanisms such as peroxisome proliferator-activator receptor gamma (PPAR- gamma) or inhibition of histone deacetylase (HDACi). The polyphenol epigallocatechin-3-gallate (EGCG), a natural dietary component, has anti-inflammatory and immunomodulatory effects proposed to be mediated by several mechanisms such as suppression of cytokine production and inflammatory cell recruitment (Han, Exp Mol Med. 35(2):136-9, 2003; Qin, J Immunol. 186(6):3693-700, 2011) and induction of regulatory T cells (Wong, Immunol Lett. 139(1-2):7-13, 2011).

In some embodiments, a multibiotic agent described herein decreases fat percentage or cellular adiposity in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In some embodiments, a multibiotic agent described herein increases glucose tolerance in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In certain embodiments, a multibiotic agent described herein decreases weight or rate of weight gain in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In particular embodiments, a multibiotic agent described herein decreases abdominal fat or back fat in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In further embodiments, a multibiotic agent described herein decreases ratio of white vs. brown fat in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In yet further embodiments, a multibiotic agent described herein increases muscle mass in a tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In still further embodiments, a multibiotic agent described herein decreases blood sugar in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In other embodiments, a multibiotic agent described herein decreases hemoglobin A1c in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In yet other embodiments, a multibiotic agent described herein increases insulin sensitivity or levels of insulin in a tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In still other embodiments, a multibiotic agent described herein decreases peripheral pain, numbness, or other neuropathic symptoms in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In certain embodiments, a multibiotic agent described herein increases GLP-1 or PYY in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In particular embodiments, a multibiotic agent described herein decreases liver adiposity or liver fat percentage in a tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control. In further embodiments, a multibiotic agent described herein decreases liver fibrosis or cirrhosis in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control).

The markers described herein may be measured using methods known in the art. For example, glucose tolerance may be assessed using an oral glucose tolerance test (OGTT) described at MedlinePlus (medlineplus.gov). In this test, a subject drinks a liquid containing a predetermined amount of glucose (typically, 75 g of glucose), and blood glucose level is then measured at 15 minutes, 30 minutes, 60 minutes, 90 minutes, 120 minutes, 150 minutes, and 180 minutes after the glucose dosing. Insulin sensitivity can be measuring using an insulin clamp, for example, as described in Farrnnini and Mari, J. Hypertens., 16:895-906, 1998. Lipogenesis may be measured using a hepatic de novo lipogenesis test, for example, as described in Rabol et al., Proc. Nat. Acad. Sci., 108:13705-13709, 2011. This test assesses the incorporation of deuterium into plasma very-low-density lipoprotein triglyceride (VLDL) during administration of deuterium-labeled water.

In an exemplary embodiment, a multibiotic agent comprising EGCG covalently bonded to one or more molecules of the SFCA groups (e.g., acetate or butyrate) biodegrades in the distal small intestine or colon and releases components that modulate metabolic markers, e.g., decrease blood sugar (e.g., fasting blood sugar level or blood sugar level after a meal), modulate insulin response, and/or reduce hemoglobin A1c.

In an exemplary embodiment, a multibiotic agent comprising one or more SCFA groups (e.g., butyrate) covalently bonded to curcumin that biodegrades in the distal small intestine or large intestine could represent a novel treatment for NAFLD/NASH (e.g., that abrogates the lipid accumulation in the liver and/or decreases inflammation).

In an exemplary embodiment, a multibiotic agent comprises SCFA (e.g., butyrate) and curcumin.

Proteins may also be catabolized to produce ammonia *in vivo.* A metabolic disturbance characterizes by an excess ammonia is known as hyperammonemia. Hyperammonemia is a dangerous condition that may lead to brain injury and death. Certain multibiotic agents of the invention may be used to treat hyperammonemia or to reduce the blood ammonia level in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). Typically, methods of treating hyperammonemia or of reducing the blood ammonia in a subject in need thereof include administration of a multibiotic agent to the subject. Multibiotic agents that may be used in these methods include an acyl that is carglumic acid acyl.

In exemplary embodiments, a multibiotic agent of the invention having metabolic health benefits described herein includes an alcohol core that is an antifungal agent, carbohydrate or an oligomer thereof, anhydrosugar alcohol, sugar alcohol, sugar acid, bile acid, vitamin, pentacyclic triterpenoid, carotenoid, catechin polyphenol, amino alcohol, curcuminoid, flavonoid, lignan, phenolic phytochemical, stilbenoid, capsinoid, hydroxycinnamic acid ester, or β-hydroxycarboxylate. In further exemplary embodiments, a multibiotic agent having metabolic health benefits described herein includes an amine core that is an amino acid metabolite, antihyperglycemic biguanide, or antifungal agent. In some embodiments, a multibiotic agent having metabolic health benefits described herein includes an acyl that is an amino acid, amino acid metabolite acyl, bile acid acyl, fatty acid acyl, α-lipoic acid acyl, β-hydroxycarboxylate acyl, pentacyclic triterpenoid acyl, hydroxycinnamic acid ester acyl, phenolic phytochemical acyl (e.g., a phenalkyl acyl or ellagic acid acyl or an analogue thereof), picolinate acyl, sugar acid acyl, or vitamin acyl.

In some embodiments, an antifungal agent is an echinocandin (e.g., micafungin, caspofungin, or anidulafungin). In certain embodiments, a carbohydrate or an oligomer thereof is ribose or trehalose. In some embodiments, an anhydrosugar alcohol is sorbitan or isosorbide. In further embodiments, a sugar alcohol is erythritol or inositol. In yet further embodiments, a bile acid is hyodeoxycholic acid or ω-muricholic acid. In still further embodiments, a vitamin is vitamin B5 or vitamin D3. In other embodiments, a pentacyclic triterpenoid is ursolic acid. In yet other embodiments, a carotenoid is fucoxanthin or astaxanthin. In still other embodiments, a catechin polyphenol is epigallocatechin gallate. In some embodiments, an amino alcohol is ephedrine. In certain embodiments, a curcuminoid is curcumin. In some other embodiments, a sugar acid is gluconic acid. In other embodiments, a flavonoid is trihydroxystilbene, pterostilbene, rhapontigenin, piceatannol, pinostilbene, oxyresveratrol, or4-methoxyresveratrol. In further embodiments, a lignan is secoisolariciresinol, pinoresinol, lariciresinol, matairesinol, or 7-hydroxyenterolactone. In yet further embodiments, a phenolic phytochemical is a phenalkyl (e.g., 4-(4-hydroxyphenyl)butan-2-one, ferrulic acid, caffeic acid, or p-coumaric acid). In still further embodiments, a phenolic phytochemical is ellagic acid or an analogue thereof (e.g., urolithin A, urolithin B, urolithin C, urolithin D, urolithin E, or urolithin M5). In other embodiments, a stilbenoid is trihydroxystilbene, pterostilbene, rhapontigenin, piceatannol, pinostilbene, oxyresveratrol, or 4-methoxyresveratrol. In yet other embodiments, a capsinoid is capsiate. In still other embodiments, a hydroxycinnamic acid ester is chlorogenic acid. In some embodiments, a β-hydroxycarboxylate is carnitine.

In further embodiments, an amino acid metabolite is indole-3-propionic acid or 3-(indole-3-yl)-acrylic acid. In other embodiments, an antihyperglycemic biguanide is metformin. In other embodiments, antifungal agent is an echinocandin (e.g., caspofungin).

In some embodiments, an amino acid acyl is carglumic acid acyl. In other embodiments, an amino acid metabolite acyl is indole-3-propionic acid acyl or 3-(indole-3-yl)-acrylic acid acyl. In some embodiments, a bile acid acyl is hyodeoxycholic acid or ω-muricholic acid. In further embodiments, a fatty acid acyl is acetyl, propionyl, butyryl, eicosapentanoic acid acyl, docosahexaenoic acid acyl, punicic acid acyl, or α-linolenic acid acyl. In further embodiments, a β-hydroxycarboxylate acyl is carnitine acyl. In yet further embodiments, a pentacyclic triterpenoid acyl is ursolic acid acyl. In still further embodiments, a hydroxycinnamic acid ester acyl is chlorogenic acid acyl. In still further embodiments, a phenolic phytochemical acyl is a phenalkyl acyl (e.g., ferulic acid acyl, caffeic acid acyl, or p-coumaric acid acyl). In some embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In some embodiments, a sugar acid acyl is gluconic acid acyl. In other embodiments, a vitamin acyl is vitamin B5 acyl.

In further exemplary embodiments, a multibiotic agent having anti-obesity health benefits described herein includes an alcohol core that is a bile acid, flavonoid, pentacyclic triterpenoid, carbohydrate or an oligomer thereof, catechin polyphenol, amino alcohol, curcuminoid, carotenoid, lignan, stilbenoid, phenolic phytochemical, sugar alcohol, hydroxycinnamic acid ester, β-hydroxycarboxylate, ketone body, or pre-ketone body. In some embodiments, a multibiotic agent of the invention having anti-obesity health benefits described herein includes an amine core that is an antihyperglycemic biguanide, antifungal agent, or amino acid metabolite. In some embodiments, a multibiotic agent of the invention having anti-obesity health benefits described herein includes an acyl that is a a bile acid acyl, fatty acid acyl, pentacyclic triterpenoid acyl, catechin polyphenol acyl, phenolic phytochemical acyl, amino alcohol acyl, amino acid metabolite acyl, hydroxycinnamic acid ester acyl, β-hydroxycarboxylate acyl, ketone body acyl, or pre-ketone body acyl.

In some embodiments, a bile acid is chenodeoxycholic acid, cholic acid, deoxycholic acid, glycocholic acid, glycochenodeoxycholic acid, lithocholic acid, taurocholic acid, taurochenodeoxycholic acid, hyodeoxycholic acid, or ω-muricholic acid. In other embodiments, a flavonoid is apigenin, naringenin, daidzein, equol, or luteolin. In some embodiments, a pentacyclic triterpenoid is ursolic acid. In further embodiments, a carbohydrate or an oligomer thereof is ribose or trehalose. In further embodiments, a catechin polyphenol is epigallocatechin gallate. In yet further embodiments, an amino alcohol is ephedrine. In still further embodiments, a curcuminoid is curcumin. In other embodiments, a carotenoid is astaxanthin. In yet other embodiments, a lignan is pinoresinol, laricisiresinol, secoisolariciresinol, matairesinol, or 7-hydroxyenterolactone. In still other embodiments, a stilbenoid is piceatannol, pinostilbene, oxyresveratrol, or 4-methoxyresveratrol. In some embodiments, a phenolic phytochemical is ellagic acid or an analogure thereof (e.g., urolithin A, urolithin B, urolithin C, urolithin D, urolithin E, or urolithin M5). In other embodiments, a phenolic phytochemical is a phenalkyl (e.g., ferulic acid, caffeic acid, or *p*-coumaric acid). In some embodiments, a sugar alcohol is inositol or erythritol. In further embodiments, a hydroxycinnamic acid ester is chlorogenic acid. In yet further embodiments, β-hydroxycarboxylate is β-hydroxybutyrate. In further embodiments, a pre-ketone body is butane-1,3-diol or 4-hydroxybutan-2-one. In still further embodiments, a ketone body is β-hydroxybutyrate. In other mebodiments, an antihyperglycemic biguanide is metformin. In yet other embodiments, an antifungal agent is an echinocandsin (e.g., micafungin, caspofungin, or anidulafungin). In still further embodiments, an amino acid metabolite is indole-3-propionic acid or 3-(indole-3-yl)-acrylic acid.

In some embodiments, a bile acid acyl is chenodeoxycholic acid acyl, cholic acid acyl, deoxycholic acid acyl, glycocholic acid acyl, glycochenodeoxycholic acid acyl, lithocholic acid acyl, taurocholic acid acyl, taurochenodeoxycholic acid acyl, hyodeoxycholic acid acyl, ω-muricholic acid acyl, or obeticholic acid acyl. In other embodiments, a pentacyclic triterpenoid acyl is ursolic acid acyl. In further embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In further embodiments, a phenolic phytochemical acyl is a phenalkyl acyl (e.g., ferulic acid, caffeic acid, or p-coumaric acid). In yet further embodiments, a fatty acid acyl is acetyl, propionyl, butyryl, eicosapentaenoyl, docosahexaenoyl, punicic acid acyl, dihomo-γ-linolenic acid acyl, docosapentanoyl, succinyl, succin-diyl, or α-linolenic acid acyl. In further embodiments, a fatty acid acyl is a medium chain fatty acid acyl (e.g., octanoyl or decanoyl). In still further embodiments, an amino acid metabolite acyl is indole-3-propionic acid acyl or 3-(indole-3-yl)-acrylic acid acyl. In other embodiments, hydroxycinnamic acid ester acyl is chlorogenic acid acyl. In yet other embodiments, β-hydroxycarboxylate acyl is β-hydroxybutyrate acyl. In still other embodiments, ketone body acyl is β-hydroxybutyrate acyl. In further embodiments, a pre-ketone body acyl is a group of the structure:

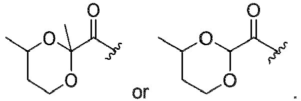

In some exemplary embodiments, a multibiotic agent providing metabolic health benefits (e.g., used for the treatment of type 2 diabetes or NAFLD/NASH) is hydrolyzed in the small intestine (e.g., as measured using simulated intestinal fluid assay) to release active agents targeting a disease or condition and is substantially stable in the stomach (e.g., as measured using simulated gastric fluid assay). In other exemplary embodiments, a multibiotic agent providing metabolic health benefits is substantially stable in the stomach (e.g., as measured using simulated gastric fluid assay) and provides individual components of the multibiotic agent to the colon (e.g., after hydrolysis in the colon). Hydrolysis in the colon may be measured using fecal stability assay as described herein.

### Bowel Health

Compositions and methods of the invention may be used to improve bowel health, e.g., to treat irritable bowel syndrome (IBS), Inflammatory bowel disease (IBD) (e.g., ulcerative colitis orCrohn's disease), chronic diarrhea, or constipation.

In some embodiments, a multibiotic agent described herein decreases gastrointestinal inflammation (upper intestine, cecum, ileum, colon, rectum in a tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In other embodiments, a multibiotic agent described herein decreases abdominal pain in a tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In further embodiments, a multibiotic agent described herein decreases gastrointestinal permeability in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In further embodiments, a multibiotic agent described herein increases intestinal motility or frequency of bowel movements in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In yet further embodiments, a multibiotic agent described herein decreases intestinal motility or frequency of bowel movements in a subject in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In still further embodiments, a multibiotic agent described herein decreases GI bleeding in a tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In other embodiments, a multibiotic agent described herein increases TReg differentiation in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In yet other embodiments, a multibiotic agent described herein improves gut tissue (e.g., endothelial tissue) morphology in a cell, tissue or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In still other embodiments, a multibiotic agent described herein decreases or increases mucus secretion or improves mucosal health in a gastrointestinal cell, tissue or in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control).

Mesalamine (5-amino-salicylic acid) is an established treatment for ulcerative colitis. When dosed orally in conjunction with an enema containing butyric acid, the improvement in clinical condition was better than mesalamine alone but only in the region where the rectally administered butyrate could reach (Vernia 2013). In an exemplary embodiment, a multibiotic agent described herein comprised of mesalamine covalently linked to one or more butyric acid provides a product that is biodegraded in the colon.

In some embodiments, a multibiotic agent having bowel health benefits described herein includes an alcohol core that is a bile acid, catechin polyphenol, carbohydrate or an oligomer thereof, curcuminoid, flavonoid, phenolic phytochemical, stilbenoid, sugar alcohol, vitamin, or mesalamine. In other embodiments, a multibiotic agent having bowel health benefits described herein includes an amine core that is an antihyperglycemic biguanide, amino acid metabolite or mesalamine. In further embodiments, a multibiotic agent having bowel health benefits described herein includes an acyl that is a bile acid acyl, fatty acid acyl, vitamin acyl, amino acid metabolite acyl, phenolic phytochemical acyl, or mesalamine acyl.

In some embodiments, a bile acid is hyodeoxycholic acid or ω-muricholic acid. In other embodiments, a catechin polyphenol is epigallocatechin gallate. In further embodiments, a carbohydrate or an oligomer thereof is trehalose or ribose. In yet further embodiments, a curcuminoid is curcumin. In still further embodiments, a flavonoid is hesperetin, daidzein, or luteolin. In other embodiments, a phenolic phytochemical is a phenalkyl (e.g., ferulic acid, caffeic acid, or *p*-coumaric acid). In yet other embodiments, a phenolic phytochemical is ellagic acid or an analogue thereof (e.g., urolithin A, urolithin B, urolithin C, urolithin D, urolithin E, or urolithin M5). In still other embodiments, a stilbenoid is trihydroxystilbene, rhapontigenin, pinostilbene, oxyresveratrol, or 4-methoxyresveratrol. In certain embodiments, a sugar alcohol is inositol. In yet further embodiments, a vitamin is vitamin B5.

In some embodiments, an antihyperglycemic biguanide is metformin. In some embodiments, an amino acid metabolite is indole-3-acetic acid, indole-3-propionic acid, or 3-(indole-3-yl)-acrylic acid.

In other embodiments, a bile acid acyl is hyodeoxycholic acid or ω-muricholic acid. In further embodiments, a fatty acid acyl is acetyl, propionyl, butyryl, isobutyryl, valeryl, or isovaleryl. In yet further embodiments, a vitamin acyl is retinoic acid acyl or vitamin B5 acyl. In still further embodiments, an amino acid metabolite acyl is indole-3-acetic acid acyl, indole-3-propionic acid acyl, or 3-(indole-3-yl)-acrylic acid acyl. In other embodiments, a phenolic phytochemical acyl is a phenalkyl acyl (e.g., ferulic acid acyl, caffeic acid acyl, or p-coumaric acid acyl). In yet other embodiments, a phenolic phytochemical acyl is ellagic acid or an analogue thereof (e.g., urolithin A acyl, urolithin B acyl, urolithin C acyl, urolithin D acyl, urolithin E acyl, or urolithin M5 acyl).

In some embodiments, a multibiotic agent providing bowel health benefits (e.g., used for the treatment of an inflammatory bowel disease (e.g., Crohn's disease)) is hydrolyzed in the small intestine (e.g., as measured using simulated intestinal fluid assay) to release active agents targeting a disease or condition and is substantially stable in the stomach (e.g., as measured using simulated gastric fluid assay). In some embodiments, a multibiotic agent providing bowel health benefits (e.g., used for the treatment of an irritable bowel syndrome, inflammatory bowel disease (e.g., ulcerative colitis or Crohn's disease), chronic diarrhea, or constipation (e.g., constipation secondary to a neurodegenerative disorder (e.g., Parkinson's disease))) is substantially stable in the stomach (e.g., as measured using simulated gastric fluid assay) and provides individual components of the multibiotic agent to the colon (e.g., after hydrolysis in the colon). Hydrolysis in the colon may be measured using fecal stability assay as described herein.

### Infectious Diseases

Multibiotic agents disclosed herein may be used for treating infectious diseases in a subject. Typically, a method of treating infectious disease in a subject may include administering a multibiotic agent or a pharmaceutical or nutraceutical composition containing a multibiotic agent to the subject.

Metabolic products of the microbiome have been shown to restrict grown and colonization of invading pathogens by creating a hostile localized microenvironment (McKenney, Pathog Dis. 74(5), doi: 10.1093/femspd/ftw051, 2016; Weingarden, PLoS One, 11(1): e0147210, 2016). Examples of metabolites impacting pathogens (e.g., Candidiasis) include polyphenols derived from peel extracts of Punica granatum (Madugula, J Clin Diagn Res. 11(1):ZC114-ZC117, 2017), and dietary tryptophan metabolites (Zelante, Immunity. 39(2):372-85, 2013). In some cases, these molecules have been shown to work by signaling through the aryl hydrocarbon receptors (AhR). Short chain fatty acids such as acetate have been shown to promote defensive functions on host epithelial cells, and thereby provide protection from lethal infection. Short, medium, and long chain fatty acids exhibit diverse patterns of inhibition against oral bacteria with some specificity related to fatty acid chain length. In addition, secondary bile acids generated by the gut microbiome and present in the feces after fecal microbiome transplant are sufficient to control the germination and growth of C. difficile infection. Multibiotic agents disclosed herein may be used to provide health benefits to the subject with an infectious disease, e.g., to treat oral, epithelial mucosal, vulvovaginal, or GI Candida.

In some embodiments, a multibiotic agent described herein decreases non-culture based markers of candidiasis markers, e.g., Candida albicans germ tube antibody (CAGTA), Platelia Candida mannan antigens (MN), anti-mannan antibodies (AMN) and (1→3) -β-D-glucan (BDG) in a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In some embodiments, a multibiotic agent described herein decreases Candida CFUs in a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In other embodiments, a multibiotic agent described herein decreases the number of patients who develop a significant drug-related adverse event, and increase the number of patients with a favorable overall response. (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control).

In some embodiments, a multibiotic agent described herein increases the levels of one or more secondary bile acids in the stool of treated animals or human subjects (e.g., to at least 0.001%, 0.002%, 0.003%, 0.004%, or at least 0.005% (e.g., 0.001% to 0.01% or 0.001% to 0.005%) or to at least 0.5 mM, 1.0 mM, 1.5 mM, or 2.0 mM (e.g., 0.5 mM to 4.0 mM or 0.5 mM to 2.0 mM). In some embodiments, a multibiotic agent described herein decreases the colony forming units (CFU) of C. difficile in the stool of treated animals infected with C. difficile (e.g., by at least 10², 10³, 10⁴,10⁵, or 10⁶ CFU/g stool). In other embodiments, a multibiotic agent described herein increases the clinical cure rate or decreases the clinical recurrence rate of human subjects infected with C. difficile.

In some embodiments, a multibiotic agent having anti-infectious disease health benefits described herein includes an alcohol core that is an antifungal agent, amino acid metabolite, flavonoid, lignan, phenolic phytochemical, stilbenoid, carbohydrate or an oligomer thereof, or sugar alcohol. In some embodiments, a multibiotic agent having anti-infectious disease health benefits described herein includes an amine core that is an antifungal agent, antihyperglycemic biguanide, or amino acid metabolite. In some embodiments, a multibiotic agent having anti-infectious disease health benefits described herein includes an acyl that is an amino acid metabolite acyl, fatty acid acyl, or phenolic phytochemical acyl.

In other embodiments, an antifungal agent is an echinocandin (e.g., micafungin, caspofungin, or anidulafungin). In some embodiments, a flavonoid is daidzein or luteolin. In further embodiments, a lignan is pinoresinol, lariciresinol, secoisolariciresinol, matairesinol, or 7-hydroxyenterolactone. In yet further embodiments, a phenolic phytochemical is a phenalkyl (e.g., ferulic acid, caffeic acid, or *p-*coumaric acid). In still further embodiments, a stilbenoid is piceatannol, pinostilbene, oxyresveratrol, or 4-methoxyresveratrol. In other embodiments, a carbohydrate or an oligomer thereof is ribose or trehalose. In yet other embodiments, a sugar alcohol is inositol or erythritol. In still other embodiments, an antihyperglycemic biguanide is metformin. In some embodiments, an amino acid metabolite is indole-3-propionic acid or 3-(indole-3-yl)-acrylic acid.

In some embodiments, an amino acid metabolite acyl is indole-3-propionic acid acyl or 3-(indole-3-yl)-acrylic acid acyl. In further embodiments, a fatty acid acyl is formyl, acetyl, propionyl, butyryl, isobutyryl, valeryl, isovaleryl, hexanoyl, heptanoyl, octanoyl, nonanoyl, decanoyl, dodecanoyl, myristyl, palmityl, stearyl, arachidyl, docosanoyl, tetracosanoyl, or hexacosanoyl. In yet further embodiments, a phenolic phytochemical acyl is a phenalkyl acyl (e.g., ferulic acid acyl, caffeic acid acyl, or p-coumaric acid acyl).

In some embodiments, a multibiotic agent providing bowel health benefits is hydrolyzed in the small intestine (e.g., as measured using simulated intestinal fluid assay) to release active agents targeting a disease or condition and is substantially stable in the stomach (e.g., as measured using simulated gastric fluid assay). In some embodiments, a multibiotic agent providing anti-infectious disease health benefits (e.g., used for the treatment of C. difficile infection) is substantially stable in the stomach (e.g., as measured using simulated gastric fluid assay) and provides individual components of the multibiotic agent to the colon (e.g., after hydrolysis in the colon). Hydrolysis in the colon may be measured using fecal stability assay as described herein.

### Muscle Growth

Compositions and methods of the invention may be used to improve muscle growth and fitness (e.g., to treat sarcopenia or muscle atrophy). Typically, a method of treating sarcopenia or muscle atrophy in a subject may include administering a multibiotic agent or a pharmaceutical or nutraceutical composition containing a multibiotic agent to the subject.

The metabolic active state of gut microorganisms is critical in conferring benefits to host growth health and longevity (Lenaerts, J. Gerontol A Bio Sci Med Sci 2008). Several microbiome metabolites have been shown to correlate with muscle health, including 3-hydroxy isovaleric acid, shown for inhibiting muscle wasting (Stratton, J Clin Nutr 2006;84:384-8.), and various SCFA including butyrate, acetate that are enriched in the microbiome of athletes (Barton et al., Gut 2017;0:1-9.). The polyphenol ellagitannin has been shown clinically to improve recovery of isometric strength when dosed over a nine-day period vs control (Trombold, Medicine and Science in sports and exercise, 493, 2010). The exact mechanism by which microbiome derived metabolites influence muscle growth and health is still unclear, however SCFA are known to activate AMP Kinase that serves to introduce mitochondriogenesis. In addition, the metabolite urolithin A, a microbial metabolite of ellagitannins has been shown to promote microbial diversity, and enhance skeletal muscles oxidative capacity and the selective autophagy of mitochondria (Mitophagy) (Ryu, Nature Medicine, 22:879-888, 2016). A multibiotic agent comprised of urolithin-butyrate could biodegrade in the distal small intestine or colon potentially providing high levels of butyrate as well as the immunomodulatory properties of urolithin directly into the distal gut where the components are thought to interact with the immune system.

In further embodiments, a multibiotic agent described herein modifies C-reactive protein, interleukin-6, tumor necrosis factor-α, insulin-like growth factor-1, oxidized low-density lipoproteins, carotenoids, procollagen type III N-terminal peptide, gene and/or protein expression for mitophagy and/or autophagy, and/or α-tocopherolin a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In yet further embodiments, a multibiotic agent described herein modifies albumin, hemoglobin, creatinine in a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In still further embodiments, a multibiotic agent described herein modifies muscle or fat free mass using dual energy X-ray absorptiometry (DXA), electrical impedance myography (EIM), or computed tomography (CT) in a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In other embodiments, a multibiotic agent described herein reduces composite outcome measures of disease progression in patients, e.g., Participant Lean Body Mass, Bilateral Leg Press (BLP) Measurement, Gait Speed, Knee Extension Maximum Isokinetic Strength, and performance on a 400 meter walk (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control).

In some embodiments, a multibiotic agent having muscle growth health benefits described herein includes an alcohol core that is an antifungal agent, anhydrosugar alcohol, carbohydrate or an oligomer thereof, sugar alcohol, sugar acid, bile acid, flavonoid, lignan, phenolic phytochemical, stilbenoid, vitamin, or pentacyclic triterpenoid. In other embodiments, a multibiotic agent having muscle growth health benefits described herein includes an amine core that is an anti hyperglycemic biguanide, amino acid metabolite, antifungal agent, polyamine, or vitamin. In some embodiments, a multibiotic agent having muscle growth health benefits described herein includes an acyl that is a bile acid acyl, fatty acid acyl, vitamin acyl, pentacyclic triterpenoid acyl, phenolic phytochemical acyl, amino acid acyl, amino acid metabolite acyl, or sugar acid acyl.

In some embodiments, an antifungal agent is an echinocandin (e.g., micafungin, caspofungin, or anidulafungin). In some embodiments, an anhydrosugar alcohol is sorbitan or isosorbide. In further embodiments, a carbohydrate or an oligomer thereof is ribose or trehalose. In yet further embodiments, a sugar alcohol is inositol or erythritol. In still further embodiments, a sugar acid is gluconic acid. In other embodiments, a bile acid is hyodeoxycholic acid or ω-muricholic acid. In yet other embodiments, a flavonoid is daidzein, equol, or luteolin. In still other embodiments, a lignan is pinoresinol, lariciresinol, secoisolariciresinol, matairesinol, or 7-hydroxyenterolactone. In some embodiments, a phenolic phytochemical is ellagic acid or an analogue thereof (e.g., urolithin A, urolithin B, urolithin C, urolithin D, urolithin E, or urolithin M5). In other embodiments, a phenolic phytochemical is a phenalkyl (e.g., ferulic acid, caffeic acid, or p-coumaric acid). In some embodiments, a stilbenoid is piceatannol, pinostilbene, oxyresveratrol, or 4-methoxyresveratrol. In further embodiments, a vitamin is vitamin B5, vitamin D2, or vitamin E. In yet further embodiments, a pentacyclic triterpenoid is ursolic acid. In some embodiments, an antihyperglycemic biguanide is metformin. In certain embodiments, an amino acid metabolite is indole-3-propionic acid or 3-(indole-3-yl)-acrylic acid.

In some embodiments, a bile acid acyl is hyodeoxycholic acid acyl or ω-murichoiic acid acyl. In other embodiments, a fatty acid acyl is eicosapentaenoic acid acyl or docosahexaenoic acid acyl. In further embodiments, a vitamin acyl is a vitamin B5 acyl. In further embodiments, a pentacyclic triterpenoid acyl is ursolic acid acyl. In yet further embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In still further embodiments, an amino acid acyl is ornithine acyl or citrulline acyl. In other embodiments, an amino acid metabolite acyl is indole-3-propionic acid acyl or 3-(indole-3-yl)-acrylic acid acyl. In yet other embodiments, a sugar acid acyl is gluconic acid acyl.

In some embodiments, a multibiotic agent having muscle mass-increasing health benefits described herein includes an alcohol core that is a bile acid, flavonoid, pentacyclic triterpenoid, carbohydrate or an oligomer thereof, catechin polyphenol, amino alcohol, curcuminoid, carotenoid, lignan, stilbenoid, phenolic phytochemical, sugar alcohol, hydroxycinnamic acid ester, β-hydroxycarboxylate, ketone body, or pre-ketone body. In other embodiments, a multibiotic agent having muscle mass-increasing health benefits described herein includes an amine core that is an antihyperglycemic biguanide, antifungal agent, or amino acid metabolite. In some embodiments, a multibiotic agent having muscle mass-increasing health benefits described herein includes an acyl that is a a bile acid acyl, fatty acid acyl, pentacyclic triterpenoid acyl, catechin polyphenol acyl, phenolic phytochemical acyl, amino alcohol acyl, amino acid metabolite acyl, hydroxycinnamic acid ester acyl, β-hydroxycarboxylate acyl, ketone body acyl, or pre-ketone body acyl.

In some embodiments, a bile acid is chenodeoxycholic acid, cholic acid, deoxycholic acid, glycocholic acid, glycochenodeoxycholic acid, lithocholic acid, taurocholic acid, taurochenodeoxycholic acid, hyodeoxycholic acid, or ω-muricholic acid. In other embodiments, a flavonoid is apigenin, naringenin, daidzein, equol, or luteolin. In some embodiments, a pentacyclic triterpenoid is ursolic acid. In certain embodiments, a carbohydrate or an oligomer thereof is ribose or trehalose. In further embodiments, a catechin polyphenol is epigallocatechin gallate. In yet further embodiments, an amino alcohol is ephedrine. In still further embodiments, a curcuminoid is curcumin. In other embodiments, a carotenoid is astaxanthin. In yet other embodiments, a lignan is pinoresinol, laricisiresinol, secoisolariciresinol, matairesinol, or 7-hydroxyenterolactone. In still other embodiments, a stilbenoid is trihydroxystilbene, piceatannol, pinostilbene, oxyresveratrol, or 4-methoxyresveratrol. In some embodiments, a phenolic phytochemical is ellagic acid or an analogure thereof (e.g., urolithin A, urolithin B, urolithin C, urolithin D, urolithin E, or urolithin M5). In other embodiments, a phenolic phytochemical is a phenalkyl (e.g., ferulic acid, caffeic acid, or p-coumaric acid). In some embodiments, a sugar alcohol is inositol or erythritol. In further embodiments, a hydroxycinnamic acid ester is chlorogenic acid. In yet further embodiments, β-hydroxycarboxylate is β-hydroxybutyrate. In further embodiments, a pre-ketone body is butane-1 ,3-diol or 4-hydroxybutan-2-one. In still further embodiments, a ketone body is β-hydroxybutyrate. In other mebodiments, an antihyperglycemic biguanide is metformin. In yet other embodiments, an antifungal agent is an echinocandsin (e.g., micafungin, caspofungin, or anidulafungin). In still further embodiments, an amino acid metabolite is indole-3-propionic acid or 3-(indole-3-yl)-acrylic acid.

In some embodiments, a bile acid acyl is chenodeoxycholic acid acyl, cholic acid acyl, deoxycholic acid acyl, glycocholic acid acyl, glycochenodeoxycholic acid acyl, lithocholic acid acyl, taurocholic acid acyl, taurochenodeoxycholic acid acyl, hyodeoxycholic acid acyl, or ω-muricholic acid acyl. In other embodiments, a pentacyclic triterpenoid acyl is ursolic acid acyl. In some embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In further embodiments, a phenolic phytochemical acyl is a phenalkyl acyl (e.g., ferulic acid, caffeic acid, or *p*-coumaric acid). In yet further embodiments, a fatty acid acyl is acetyl, propionyl, butyryl, octanoyl, decanoyl, eicosapentaenoyl, docosahexaenoyl, punicic acid acyl, dihomo-γ-linolenic acid acyl, docosapentanoyl, succinyl, succin-diyl, or α-linolenic acid acyl. In further embodiments, a fatty acid acyl is a medium chain fatty acid acyl (e.g., octanoyl or decanoyl). In still further embodiments, an amino acid metabolite acyl is indole-3-propionic acid acyl or 3-(indole-3-yl)-acrylic acid acyl. In other embodiments, hydroxycinnamic acid ester acyl is chlorogenic acid acyl. In yet other embodiments, β-hydroxycarboxylate acyl is β-hydroxybutyrate acyl. In still other embodiments, ketone body acyl is β-hydroxybutyrate acyl. In some embodiments, pre-ketone body acyl is:

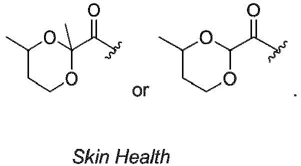

Compositions and methods of the invention may be used to improve skin health, e.g., to treat acne, rosacean and atopic eczema. Typically, a method of treating skin disease in a subject may include administering a multibiotic agent or a pharmaceutical or nutraceutical composition containing a multibiotic agent to the subject.

The causes of atopic eczema and acne are not well understood and idiopathic. Diet and gastrointestinal disease can impact skin, and dermatoses are strongly correlated with certain GI diseases. The "hygiene hypothesis" seeks to explain the marked increased in allergic diseases in western cultures because of minimization of exposure to a diverse range of microbes. Characterization of gut microbiota from suffers of atopic eczema show a scientifically lower bacteria diversity when compared to healthy controls (Abrahamsson, J Allergy Clin Immunol. 129(2):434-40, 2012). Specifically, a lower number of species containing lipopolysaccharide molecules correlated in infants with atopic eczema. Mechanisms linking microbiota metabolites to improved skin health has been hypothesis to be as a result of increase serum levels of the anti-inflammatory cytokine IL10, and the decreased level of IL17 that is demonstrated in probiotic fed mice (Levkovich, PLoS One, 8(1):e53867, 2013). Studies in human volunteers also support the role of the gut microbe L. paracasei, as circulating TGF-β and skin sensitivity as measured by trans-epidermal water loss correlate with microbial diversity. An array of metabolites from foods such as fruits and green teas have been shown to reduce skin inflammation due to their ability to scavenge free radicals and prevent transepidermal water loss (Tundis, Curr. Med. Chem. 22:1515-1538, 2015). In some embodiments, methods described herein may be used to treat a skin disease or to modulate a skin disease marker described herein. Examples of a skin disease include acne, rosacea, and atopic eczema.

In some embodiment, a multibiotic agent described herein modifies inflammatory and autoimmune markers, e.g., IL1a, IL2, IL4, IL10, IL17, CRP, TGF-beta, serum thymus and activation-regulated chemokine, cathelicidin, IgE, eicosanoids in a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In other embodiments, a multibiotic agent described herein modifies mechanistic markers of skin health, e.g., protease activity in stratum corneum, transepidermal water loss (TEWL), sebum diacylglycerols in a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In further embodiments, a multibiotic agent described herein reduces composite outcome measures of disease progression in patients, e.g., six sign atopic dermatitis severity score (SASSAD), Number of Pruritus Events, Eczema Area and Severity Index (EASI), Parent's Index of Quality of Life - Atopic Dermatitis (PIQoL-AD), Change from Baseline in Inflammatory and Non-Inflammatory Lesion Counts and Their Totals (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control).

In some embodiments, a multibiotic agent having skin health benefits described herein includes an alcohol core that is an anhydrosugar alcohol, sugar alcohol, carbohydrate or an oligomer thereof, sugar acid, antifungal agent, vitamin, flavonoid, lignan, carotenoid, phenolic phytochemical, stilbenoid or a multimer thereof, or telomerase activator. In other embodiments, a multibiotic agent having skin health benefits described herein includes an amine core that is an amino acid metabolite, antifungal agent, vitamin, antihyperglycemic biguanide, or glutathione. In some embodiments, a multibiotic agent having skin health benefits described herein includes an acyl that is a fatty acid acyl, α-lipoic acid acyl, phenolic phytochemical acyl, sugar acid acyl, amino acid metabolite acyl, vitamin acyl, or glutathione acyl.

In some embodiments, an anhydrosugar alcohol is sorbitan or isosorbide. In some embodiments, sugar alcohol is inositol or erythritol. In further embodiments, a carbohydrate or an oligomer thereof is ribose or trehalose. In yet further embodiments, a sugar acid is gluconic acid. In still further embodiments, an antifungal agent is an echinocandin (e.g., micafungin, casponfungin, or anidulafundin). In other embodiments, a vitamin is ascorbic acid, vitamin B5, or vitamin E. In yet other embodiments, a flavonoid is hesperetin, daidzein, equol, or luteolin. In still other embodiments, a lignan is pinoresinol, lariciresinol, secoisolariciresinol, matairesinol, or 7-hydroxyenterolactone. In further embodiments, a carotenoid is zeaxanthin or astaxanthin. In yet further embodiments, a phenolic phytochemical is ellagic acid or an analogue thereof (e.g., urolithin A, urolithin B, urolithin C, urolithin D, urolithin E, or urolithin M5). In still further embodiments, a phenolic phytochemical is a phenalkyl (e.g., ferulic acid, caffeic acid, or p-coumaric acid). In further embodiments, a phenolic phytochemical is hydroxytyrosol. In other embodiments, a stilbenoid or a multimer thereof is a viniferin (e.g., α-viniferin, β-viniferin, γ-viniferin, δ-viniferin, or ε-viniferin). In other embodiments, a telomerase activator is cycloastrogenol.

In some embodiments, an amino acid metabolite is indole-3-propionic acid or 3-(indole-3-yl)-acrylic acid. In other embodiments, an antifungal agent is an echinocandin (e.g., micafungin, caspofungin, or anidulafungin) or posaconazole. In some embodiments, a vitamin is vitamin B5. In other embodiments, an antihyperglycemic biguanide is metformin.

In further embodiments, a fatty acid acyl is dihomo-γ-linolenic acid acyl, docosapentanoyl, succinyl, succin-diyl, or azelaic acid acyl. In yet further embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In still further embodiments, sugar acid acyl is gluconic acid acyl. In other embodiments, an amino acid metabolite acyl is indole-3-propionic acid acyl or 3-(indole-3-yl)-acrylic acid acyl. In yet other embodiments, a vitamin acyl is vitamin B5 acyl or vitamin A acyl (e.g., retinoic acid acyl, isotretinoin acyl, or alitretinoin acyl).

### Anti-inflammatory and Autoimmunity

Multibiotic agents disclosed herein may be used for treating an autoimmune disorder in a subject. Typically, a method of treating an autoimmune disorder in a subject may include administering a multibiotic agent or a pharmaceutical or nutraceutical composition containing a multibiotic agent to the subject.

Metabolic products of the microbiome have been demonstrated to interact with the host immune system in several ways. The metabolites can have effects quite remote to the gastrointestinal tract including bidirectional interactions with the central nervous system. Examples include SCFA interacting with free fatty acid receptors and tryptophan metabolites interacting with the aryl hydrocarbon receptor (AHR). A multibiotic agent comprised of EGCG octa- (indole acetate) could biodegrade in the distal small intestine or colon potentially providing high levels of the AHR agonist indole acetate as well as the immunomodulatory properties of EGCG directly into the distal gut where the components are thought to interact with the immune system. Dietary short-chain fatty acids may impact autoimmunity by expanding regulatory T cells and suppression of the JNK1/P38 pathway (Haghikia Immunity, 43:819-29, 2015).

Multibiotic agents disclosed herein may be used to provide autoimmune health benefits to the subject, e.g., to treat an autoimmune disorder (e.g., multiple sclerosis). Examples of autoimmune diseases include an inflammatory bowel disease, Addison's disease, alopecia areata, ankylosing spondylitis, antiphospholipid syndrome, hemolytic anemia, autoimmune hepatitis, Behcet's disease, Berger's disease, bullous pemphigoid, cardiomyopathy, celiac sprue, chronic fatigue immune dysfunction syndrome (CFIDS), chronic inflammatory demyelinating polyneuropathy, Churg-Strauss syndrome, cicatricial pemphigoid, cold agglutinin disease, type 1 diabetes, discoid lupus, essential mixed cryoglobulinemia, Graves' disease, Guillain-Barre syndrome, Hashimoto's thyroiditis, hypothyroidism, autoimmune lymphoproliferative syndrome (ALPS), idiopathic pulmonary fibrosis, idiopathic thrombocytopenia purpura (ITP), juvenile arthritis, lichen planus, lupus erythematosus, Meniere's disease, mixed connective tissue disease, multiple sclerosis, myasthenia gravis, pemphigus vulgaris, pernicious anemia, polychondritis, autoimmune polyglandular syndromes, polymyalgia rheumatica, polymyositis, dermatomyositis, primary agammaglobulinemia, primary biliary cirrhosis, psoriasis, psoriatic arthritis, Raynaud's phenomenon, Reiter's syndrome, rheumatic fever, rheumatoid arthritis, sarcoidosis, scleroderma, Sjögren's syndrome, stiff-man syndrome, Takayasu arteritis, giant cell arteritis, ulcerative colitis, uveitis, vasculitis, and granulomatosis with polyangiitis. Certain multibiotic agents of the invention may be used to treat autoimmune disease or to modulate an autoimmunity marker in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In some embodiments, the autoimmune disease is an inflammatory bowel disease. In other embodiments, the autoimmune disease is multiple sclerosis. Typically, methods of treating hyperammonemia or of reducing the blood ammonia in a subject in need thereof include administration of a multibiotic agent to the subject. Multibiotic agents that may be used in these methods include an acyl that is carglumic acid acyl and an alcohol core or amine core described herein.

In some embodiments, a multibiotic agent described herein decreases inflammatory and autoimmune markers, e.g., MMP9, INFγ, IL17, ICAM, CXCL13, 8-iso-PGF2α in a cell, tissue, fluid or subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In other embodiments, a multibiotic agent described herein improves CNS lesions, e.g., gadolinium-enhanced lesions observed by MRI (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In some embodiments, a multibiotic agent described herein decreases body pain, muscle stiffness or other neuropathic symptoms in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control). In further embodiments, a multibiotic agent described herein reduces composite outcome measures of disease progression in a subject, e.g., Expanded Disability Status Scale (EDSS) or Numerical Rating Scale (NRS) in a subject (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to prior to administration or a control).

In some embodiments, a multibiotic agent having anti-inflammatory and/or anti-autoimmune health benefits described herein includes an alcohol core that is an an antifungal agent, carbohydrate or an oligomer thereof, anhydrosugar alcohol, sugar alcohol, bile acid, catechin polyphenol, flavonoid, stilbenoid, phenolic phytochemical, vitamin, or amino alcohol. In other embodiments, a multibiotic agent having anti-inflammatory and/or anti-autoimmune health benefits described herein includes an amino acid metabolite, antihyperglycemic biguanide, vitamin, or amino alcohol. In some embodiments, a multibiotic agent having anti-inflammatory and/or anti-autoimmune health benefits described herein includes an acyl that is an amino acid metabolite acyl, bile acid acyl, fatty acid acyl, phenolic phytochemical acyl, vitamin acyl, amino acid metabolite acyl, or sugar acid acyl.

In some embodiments, an antifungal agent is an echinocandin (e.g., micafungin, caspofungin, or anidulafungin). In other embodiments, a carbohydrate or an oligomer thereof is xylose, ribose, or an oligomer thereof, or trehalose. In some embodiments, an anhydrosugar alcohol is isosorbide or sorbitan. In further embodiments, a sugar alcohol is erythritol or inositol. In yet further embodiments, a bile acid is hyodeoxycholic acid or ω-muricholic acid. In still further embodiments, a catechin polyphenol is epigallocatechin gallate. In some embodiments, a flavonoid is hesperetin, daidzein, equol, or luteolin. In other embodiments, a stilbenoid is trihydroxystilbene, rhapontigenin, piceatannol, pinostilbene, oxyresveratrol, or4-methoxyresveratrol. In some embodiments, a phenolic phytochemical is ellagic acid or an analogue thereof (e.g., urolithin A, urolithin B, urolithin C, urolithin D, urolithin E, or urolithin M5). In other embodiments, a phenolic phytochemical is a phenalkyl (e.g., ferulic acid, caffeic acid, p-coumaric acid, 6-gingerol, or 6-shogaol). In yet other embodiments, a vitamin is vitamin B5. In further embodiments, an amino alcohol is fingolimod.

In some embodiments, an amino acid metabolite is indole-3-acetic acid, indole-3-propionic acid, or 3-(indole-3-yl)-acrylic acid. In other embodiments, an antihyperglycemic biguanide is metformin. In further embodiments, an amino alcohol is fingolimod.

In some embodiments, an amino acid metabolite acyl is indole-3-acetic acid acyl, indole-3-propionic acid acyl, or 3-(indole-3-yl)-acrylic acid acyl. In further embodiments, a bile acid acyl is hyodeoxycholic acid acyl or ω-muricholic acid acyl. In yet further embodiments, a fatty acid acyl is acetyl, propionyl, butyryl, or a group of formula: 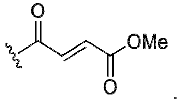 In still further embodiments, a phenolic phytochemical acyl is ellagic acid acyl or an analogue thereof (e.g., urolithin A acyl, urolithin B acyl, urolithin C acyl, urolithin D acyl, urolithin E acyl, or urolithin M5 acyl). In other embodiments, a phenolic phytochemical is a phenalkyl acyl (e.g., ferulic acid acyl, caffeic acid acyl, or *p*-coumaric acid acyl). In yet other embodiments, a vitamin acyl is retinoic acid acyl or vitamin B5 acyl. In still other embodiments, a sugar acid acyl is gluconic acid acyl.

In embodiments relating to multiple sclerosis, a multibiotic agent may include, e.g., at least one fingolimod or a group of formula:

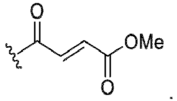

In some embodiments, a multibiotic agent providing autoimmunity health benefits (e.g., used for the treatment of multiple sclerosis or an inflammatory bowel disease) is hydrolyzed in the small intestine (e.g., as measured using a simulated intestinal fluid assay) to release active agents targeting a disease or condition and is substantially stable in the stomach (e.g., as measured using a simulated gastric fluid assay). In other embodiments, a multibiotic agent providing autoimmunity health benefits (e.g., used for the treatment of an inflammatory bowel disease (e.g., ulcerative colitis)) is substantially stable in the stomach (e.g., as measured using simulated gastric fluid assay) and provides individual components of the multibiotic agent to the colon (e.g., after hydrolysis in the colon). Hydrolysis in the colon may be measured using fecal stability assay as described herein.

### Oncology and Cancer Supportive Care

Multibiotic agents disclosed herein may be used for treating cancer in a subject, or as cancer supportive care in a subject. Typically, a method of treating cancer in a subject may include administering a multibiotic agent or a pharmaceutical or nutraceutical composition containing a multibiotic agent to the subject. In some embodiments, the cancer is renal cancer, melanoma, or non-small cell lung cancer.

The gut microbiome influences metabolism, inflammation and the adaptive immune response which can modulate the progression of cancer and host response to anticancer therapies (Roy, Nat Rev Cancer 17:271-285, 2017). Transfer of fecal material from patients responsive to cancer therapy into germ free mice enables these animals susceptible to cancer therapy. Butyrate, a microbiota metabolite has been implicated in colorectal cancer prevention and is hypothesized to inhibit several HDACs as well as act as a ligand for GPR109a which has been implicated in tumor suppression (Singh, Immunity 40:128-39, 2014). In vitro, butyrate exerts anti-proliferative and anti-cancer effects in numerous cell lines. Many polyphenols, and their metabolites have also been shown to be supportive of cancer therapy. EGCG inhibits the proliferation of many tumor types in culture by inhibiting neovascularization promoted by VEGF and other growth factors present in numerous cancer cell lines.

The gut microbiome has also been shown to generate metabolites that may reduce chemotherapy induced peripheral neuropathy. Isoflavones and their metabolites prevent the shrinkage of neurons and inhibit edema in rats treated with oxaliplatin (Azevevdo, Mol. Pain, 9:53, 2013) suggested to work via antioxidant and anti-inflammatory properties of the polyphenols. Curcumin and its metabolites also diminished neuortensin plasma levels in rats, and protect the sciatic nerve from damage (Moundhri, J Med Toxicol, 9:25-33, 2013).

Ketone bodies such as beta-hydroxybutyrate and acetoacetate are generated by the liver typically under conditions of fasting or very low carbohydrate intake but can also be exogenously supplemented. In animal models as well as in vitro cancer models, ketogenic diets or supplemented ketones have slowed the progression of tumors or decreased the viability of tumor cells. Several human studies have suggested that ketogenic diets may decrease the adverse events related to cancer chemotherapy and/or serve as cancer adjuvant therapy by improving the outcomes of cancer chemotherapy.

Multibiotic agents disclosed herein may be used to treat cancer or support recovery after chemotherapy treatment, e.g., to treat cancer (e.g., colorectal cancer, glioblastoma, or breast cancer), to reduce chemotherapy induced neuropathy, neutropenia, thrombocytopenia, or anemia, or to modulate one or more cancer markers. Examples of cancers include non-small cell lung cancer, squamous cell carcinoma of the head and neck, classical Hodgkin's lymphoma, urothelial carcinoma, melanoma, renal cell carcinoma, hepatocellular carcinoma, Merkel cell carcinoma, carcinomas with microsatellite instability, colorectal cancer, small intestine cancer, acute lymphoblastic leukemia, acute myeloid leukemia, adrenocortical carcinoma, Kaposi sarcoma, primary CNS lymphoma, anal cancer, astrocytoma, glioblastoma, bladder cancer, Ewing sarcoma, osteosarcoma, non-Hodgkin lymphoma (e.g., peripheral T cell lymphoma), breast cancer, brain tumor, cervical cancer, bile duct cancer, chronic lymphocytic leukemia, chronic myelogenous leukemia, gallbladder cancer, gastrointestinal stromal tumor, ovarian cancer, testicular cancer, multiple myeloma, neuroblastoma, pancreatic cancer, parathyroid cancer, prostate cancer, rectal cancer, and Wilms tumor.

In some embodiments, a multibiotic agent described herein reduces the viability of tumor cells in in vitro assays or decreases tumor burden in an animal model of cancer. In some embodiments, a multibiotic agent described herein reduces the amount of pain and or supportive medication used by a patient, e.g., change in duration of opioid medication or decreases the need for recombinant human granulocyte colony-stimulating factor analogs in a subject (e.g., at least 5%, 10%, 15%, 20%, 25%, 30%, 35%, 40%, 45%, 50%, 55%, 60%, 65%, 70%, 75%, 80%, 85%, 90%, 95%, or 98% or more relative to a control group). In other embodiments, a multibiotic agent described herein reduces the incidence of adverse events in patients, e.g., change in a patient-reported outcome of CIPN, patients' pain intensity score, percentage of patients stopping chemotherapy due to sensory peripheral neuropathy, or percentage of patients requiring a decrease in chemotherapy dose intensity due to adverse events (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to a control group). In some embodiments, a multibiotic agent described herein improves composite outcome measures of disease progression in patients, e.g., objective response rate, progression free survival, overall survival, response rate in subjects (e.g., at least 5%, 10%, 15%, 20%, 25%, 30%, 35%, 40%, 45%, 50%, 55%, 60%, 65%, 70%, 75%, 80%, 85%, 90%, 95% or 98% or more relative to a control group). In some embodiments, a multibiotic agent described herein increases a cancer marker, e.g., CD4⁺CD25⁺ Treg cell (e.g., CD4⁺CD25⁺Foxp3⁺ Treg cell) count, cytotoxic T cell count, Tₕ1 cell count, interferon γ (IFNy) level, interleukin-17 (IL17) level, or intercellular adhesion molecule (ICAM) level in a subject (e.g., at least 5%, 10%, 15%, 20%, 25%, 30%, 35%, 40%, 45%, 50%, 55%, 60%, 65%, 70%, 75%, 80%, 85%, 90%, 95%, or 98% or more relative to a control group or to the level prior to administration or a control). In other embodiments, a multibiotic agent described herein reduces a cancer marker, e.g., NFκB level, MMP9 level, 8-iso-PGF2α level, or CXCL13 level in a subject (e.g., at least 5%, 10%, 15%, 20%, 25%, 30%, 35%, 40%, 45%, 50%, 55%, 60%, 65%, 70%, 75%, 80%, 85%, 90%, 95%, or 98% or more relative to a control group or to the level prior to administration or a control). In further embodiments, a multibiotic agent described herein modulates (increases or decreases) a cancer marker, e.g., Tₕ1 cell count, IgA level, or iNOS level in a subject (e.g., at least 5%, 10%, 15%, 20%, 25%, 30%, 35%, 40%, 45%, 50%, 55%, 60%, 65%, 70%, 75%, 80%, 85%, 90%, 95%, or 98% or more relative to a control group or to the level prior to administration or a control). An attendant doctor or nurse practitioner can determine whether an increase or a decrease in the Tₕ1 cell count, IgA level, or iNOS level is desired.

In some embodiments, a multibiotic agent having activity useful for treating cancer in a subject, modulating a cancer marker, or as cancer supportive care in a subject includes at least one alcohol core that is a catechin polyphenol (e.g., epigallocatechin gallate) or a nucleic acid (e.g., nelarabine). In other embodiments, a multibiotic agent having activity useful for treating cancer in a subject, modulating a cancer marker, or as cancer supportive care in a subject includes at least one amine core that is a nucleic acid (e.g., nelarabine). In further embodiments, a multibiotic agent having activity useful for treating cancer in a subject, modulating a cancer marker, or as cancer supportive care in a subject includes at least one acyl that is a fatty acid acyl (e.g., butyryl) or a vitamin acyl (e.g., isotretinoin acyl, alitretinoin acyl, or pralatrexate acyl).

The cancer markers may be measured using methods known in the art. For example, blood sample analyses may be performed to measure a CD4⁺CD25⁺ Treg cell (e.g., CD4⁺CD25⁺Foxp3⁺ Treg cell) count, cytotoxic T cell count, Tₕ1 level, NFκB level, inducible nitric oxide synthase (iNOS) level, matrix metallopeptidase 9 (MMP9) level, interferon γ (IFNγ) level, interleukin-17 (IL17) level, intercellular adhesion molecule (ICAM) level, CXCL13 level, and 8-iso-prostaglandin F_{2α} (8-iso-PGF2α) level.

Multibiotic agents may be administered alone or in combination with a chemotherapeutic or an anti-cancer immunotherapeutic agent (e.g., a checkpoint inhibitor). For example, such combination therapy may be used for the treatment of renal cancer, melanoma, or non-small cell lung cancer. In a example, a multibiotic agent disclosed herein may be co-administered to a subject with a PD1/PDL1 inhibitor (e.g., pembrolizumab, nivolumab, avelumab, duvalumab, atezolizumab, AMP-225 (from GlaxoSmithKline), AMP-514 (from GlaxoSmithKline), PDR001 (from Novartis), or BMS-936559 (from Bristol Myers Squibb)), CTLA4 inhibitor (e.g., ipilimumab), or IDO inhibitor (e.g., epacadostat, navoximod, or BMS-986205 (from Bristol Myers Squibb)). Preferably, a multibiotic agent disclosed herein may be co-administered to a subject with a PD1/PDL1 inhibitor (e.g., pembrolizumab, nivolumab, avelumab, duvalumab, atezolizumab, AMP-225 (from GlaxoSmithKline), AMP-514 (from GlaxoSmithKline), PDR001 (from Novartis), or BMS-936559 (from Bristol Myers Squibb)) or CTLA4 inhibitor (e.g., ipilimumab).

In some embodiments, a multibiotic agent providing anti-cancer health benefits is hydrolyzed in the small intestine (e.g., as measured using simulated intestinal fluid assay) to release active agents targeting a disease or condition and is substantially stable in the stomach (e.g., as measured using simulated gastric fluid assay). In other embodiments, a multibiotic agent providing anti-cancer health benefits (e.g., to treat colorectal cancer) is substantially stable in the stomach (e.g., as measured using simulated gastric fluid assay) and provides individual components of the multibiotic agent to the colon (e.g., after hydrolysis in the colon). Hydrolysis in the colon may be measured using fecal stability assay as described herein.

### Pharmaceutical and Nutraceutical Compositions

The multibiotic agents disclosed herein may be formulated into pharmaceutical or nutraceutical compositions for administration to human subjects in a biologically compatible form suitable for administration in vivo. Pharmaceutical and nutraceutical compositions typically include a multibiotic agent as described herein and a physiologically acceptable excipient (e.g., a pharmaceutically acceptable excipient).

The multibiotic agents described herein can also be used in the form of the free acid/base, in the form of salts, zwitterions, or as solvates. All forms are within the scope of the invention. The multibiotic agents, salts, zwitterions, solvates, or pharmaceutical or nutraceutical compositions thereof, may be administered to a patient in a variety of forms depending on the selected route of administration, as will be understood by those skilled in the art. The multibiotic agents described herein may be administered, for example, by oral, parenteral, buccal, sublingual, nasal, rectal, patch, pump, or transdermal administration, and the pharmaceutical or nutraceutical compositions formulated accordingly. Parenteral administration includes intravenous, intraperitoneal, subcutaneous, intramuscular, transepithelial, nasal, intrapulmonary, intrathecal, rectal, and topical modes of administration. Parenteral administration may be by continuous infusion over a selected period of time.

For human use, a multibiotic agent disclosed herein can be administered alone or in admixture with a pharmaceutical or nutraceutical carrier selected regarding the intended route of administration and standard pharmaceutical practice. Pharmaceutical and nutraceutical compositions for use in accordance with the present invention thus can be formulated in a conventional manner using one or more physiologically acceptable carriers comprising excipients and auxiliaries that facilitate processing of multibiotic agents disclosed herein into preparations which can be used pharmaceutically.

This invention also includes pharmaceutical and nutraceutical compositions which can contain one or more physiologically acceptable carriers. In making the pharmaceutical or nutraceutical compositions of the invention, the active ingredient is typically mixed with an excipient, diluted by an excipient or enclosed within such a carrier in the form of, for example, a capsule, sachet, paper, or other container. When the excipient serves as a diluent, it can be a solid, semisolid, or liquid material (e.g., normal saline), which acts as a vehicle, carrier or medium for the active ingredient. Thus, the compositions can be in the form of tablets, powders, lozenges, sachets, cachets, elixirs, suspensions, emulsions, solutions, syrups, and soft and hard gelatin capsules. As is known in the art, the type of diluent can vary depending upon the intended route of administration. The resulting compositions can include additional agents, e.g., preservatives. Nutraceutical compositions may be administered enterally (e.g., orally). A nutraceutical composition may be a nutraceutical oral formulation (e.g., a tablet, powder, lozenge, sachet, cachet, elixir, suspension, emulsion, solution, syrup, or soft or hard gelatin capsule), food additive (e.g., a food additive as defined in 21 C.F.R. § 170.3), food product (e.g., food for special dietary use as defined in 21 C.F.R. § 105.3), or dietary supplement (e.g., where the active agent is a dietary ingredient (e.g., as defined in 21 U.S.C. § 321(ff))). Active agents can be used in nutraceutical applications and as food additive or food products. Non-limiting examples of compositions including an active agent of the invention are a bar, drink, shake, powder, additive, gel, or chew.

The excipient or carrier is selected on the basis of the mode and route of administration. Suitable pharmaceutical carriers, as well as pharmaceutical necessities for use in pharmaceutical formulations, are described in Remington: The Science and Practice of Pharmacy, 21st Ed., Gennaro, Ed., Lippencott Williams & Wilkins (2005), a well-known reference text in this field, and in the USP/NF (United States Pharmacopeia and the National Formulary). Examples of suitable excipients are lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents, e.g., talc, magnesium stearate, and mineral oil; wetting agents; emulsifying and suspending agents; preserving agents, e.g., methyl- and propylhydroxy-benzoates; sweetening agents; and flavoring agents. Other exemplary excipients are described in Handbook of Pharmaceutical Excipients, 6th Edition, Rowe et al., Eds., Pharmaceutical Press (2009).

These pharmaceutical and nutraceutical compositions can be manufactured in a conventional manner, e.g., by conventional mixing, dissolving, granulating, dragee-making, levigating, emulsifying, encapsulating, entrapping, or lyophilizing processes. Methods well known in the art for making formulations are found, for example, in Remington: The Science and Practice of Pharmacy, 21st Ed., Gennaro, Ed., Lippencott Williams & Wilkins (2005), and Encyclopedia of Pharmaceutical Technology, eds. J. Swarbrick and J. C. Boylan, 1988-1999, Marcel Dekker, New York. Proper formulation is dependent upon the route of administration chosen. The formulation and preparation of such compositions is well-known to those skilled in the art of pharmaceutical and nutraceutical formulation. In preparing a formulation, the multibiotic agent can be milled to provide the appropriate particle size prior to combining with the other ingredients. If the multibiotic agent is substantially insoluble, it can be milled to a particle size of less than 200 mesh. If the multibiotic agent is substantially water soluble, the particle size can be adjusted by milling to provide a substantially uniform distribution in the formulation, e.g., about 40 mesh.

### Dosages

The dosage of the multibiotic agent used in the methods described herein, or pharmaceutically acceptable salts thereof, or pharmaceutical or nutraceutical compositions thereof, can vary depending on many factors, e.g., the pharmacodynamic properties of the multibiotic agent; the mode of administration; the age, health, and weight of the recipient; the nature and extent of the symptoms; the frequency of the treatment, and the type of concurrent treatment, if any; and the clearance rate of the multibiotic agent in the subject to be treated. One of skill in the art can determine the appropriate dosage based on the above factors. The multibiotic agents used in the methods described herein may be administered initially in a suitable dosage that may be adjusted as required, depending on the clinical response. In general, a suitable daily dose of a multibiotic agent disclosed herein will be that amount of the multibiotic agent that is the lowest dose effective to produce a therapeutic effect. Such an effective dose will generally depend upon the factors described above.

A multibiotic agent disclosed herein may be administered to the patient in a single dose or in multiple doses. When multiple doses are administered, the doses may be separated from one another by, for example, 1-24 hours, 1-7 days, 1-4 weeks, or 1-12 months. The multibiotic agent may be administered according to a schedule or the multibiotic agent may be administered without a predetermined schedule. It is to be understood that, for any particular subject, specific dosage regimes should be adjusted over time according to the individual need and the professional judgment of the person administering or supervising the administration of the compositions.

The multibiotic agents may be provided in a unit dosage form. In some embodiments, the unit dosage form may be an oral unit dosage form (e.g., a tablet, capsule, suspension, liquid solution, powder, crystals, lozenge, sachet, cachet, elixir, syrup, and the like) or a food product serving (e.g., the active agents may be included as food additives or dietary ingredients). In certain embodiments, the unit dosage form is designed for administration of at least one multibiotic agent disclosed herein, where the total amount of an administered multibiotic agent is from 1 mg to 500 g, 100 mg to 250 g, 1 g to 100 g, 5 g to 80 g, 10 g to 70 g, or from 10 g to 60 g. In other embodiments, the multibiotic agent is consumed at a rate of from 1 g to 200 g a day, 10 g to 100 g a day, 15 g to 80 g a day, or from 15 g to 50 g a day, or more. In certain embodiments, the unit dosage form is designed for administration of at least one multibiotic agent disclosed herein, where the total amount of an administered multibiotic agent(s) is from 0.1 g to 10 g (e.g., 0.5 g to 9 g, 0.5 g to 8 g, 0.5 g to 7 g, 0.5 g to 6 g, 0.5 g to 5 g, 0.5 g to 1 g, 0.5 g to 1.5 g, 0.5 g to 2 g, 0.5 g to 2.5 g, 1 g to 1.5 g, 1 g to 2 g, 1 g to 2.5 g, 1.5 g to 2 g, 1.5 g to 2.5 g, or 2 g to 2.5 g). In other embodiments, the multibiotic agent is consumed at a rate of 0.1 g to 10 g per day (e.g., 0.5 g to 9 g, 0.5 g to 8g, 0.5 g to 7 g, 0.5 g to 6 g, 0.5 g to 5 g, 0.5 g to 1 g per day, 0.5 g to 1.5 g per day, 0.5 g to 2 g per day, 0.5 g to 2.5 g per day, 1 g to 1.5 g per day, 1 g to 2 g per day, 1 g to 2.5 g per day, 1.5 g to 2 g per day, 1.5 g to 2.5 g per day, or 2 g to 2.5 g per day) or more. The attending physician ultimately will decide the appropriate amount and dosage regimen, an effective amount of the multibiotic agent disclosed herein may be, for example, a total daily dosage of, e.g., between 0.1 g and 10 g of any of the multibiotic agent described herein. Alternatively, the dosage amount can be calculated using the body weight of the patient.

In the methods of the invention, the time period during which multiple doses of a multibiotic agent disclosed herein are administered to a patient can vary. For example, in some embodiments doses of the multibiotic agents are administered to a patient over a time period that is 1-7 days; 1-12 weeks; or 1-3 months. In other embodiments, the multibiotic agents are administered to the patient over a time period that is, for example, 4-11 months or 1-30 years. In yet other embodiments, the multibiotic agents disclosed herein are administered to a patient at the onset of symptoms. In any of these embodiments, the amount of the multibiotic agent that is administered may vary during the time period of administration. When a multibiotic agent is administered daily, administration may occur, for example, 1, 2, 3, 4, 5, 6, 7, 8, 9, 10, 11, or 12 times per day.

### Formulations

A multibiotic agent described herein may be administered to a subject with a pharmaceutically acceptable diluent, carrier, or excipient, in unit dosage form. Conventional pharmaceutical practice may be employed to provide suitable formulations or compositions to administer the multibiotic agent to subjects suffering from a disorder. Administration may begin before the patient is symptomatic.

Exemplary routes of administration of the multibiotic agents disclosed herein or pharmaceutical or nutraceutical compositions thereof, used in the present invention include oral, sublingual, buccal, transdermal, intradermal, intramuscular, parenteral, intravenous, intra-arterial, intracranial, subcutaneous, intraorbital, intraventricular, intraspinal, intraperitoneal, intranasal, inhalation, and topical administration. The multibiotic agents desirably are administered with a physiologically acceptable carrier (e.g., a pharmaceutically acceptable carrier). Pharmaceutical formulations of the multibiotic agents described herein formulated for treatment of the disorders described herein are also part of the present invention. In some preferred embodiments, the multibiotic agents disclosed herein are administered to a subject orally. In other preferred embodiments, the multibiotic agents disclosed herein are administered to a subject topically.

### Formulations for Oral Administration

The pharmaceutical and nutraceutical compositions contemplated by the invention include those formulated for oral administration ("oral unit dosage forms"). Oral unit dosage forms can be, for example, in the form of tablets, capsules, a liquid solution or suspension, a powder, or liquid or solid crystals, which contain the active ingredient(s) in a mixture with physiologically acceptable excipients (e.g., pharmaceutically acceptable excipients). These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other physiologically acceptable excipients (e.g., pharmaceutically acceptable excipients) can be colorants, flavoring agents, plasticizers, humectants, buffering agents, and the like.

Formulations for oral administration may also be presented as chewable tablets, as hard gelatin capsules where the active ingredient is mixed with an inert solid diluent (e.g., potato starch, lactose, microcrystalline cellulose, calcium carbonate, calcium phosphate or kaolin), or as soft gelatin capsules where the active ingredient is mixed with water or an oil medium, for example, peanut oil, liquid paraffin, or olive oil. Powders, granulates, and pellets may be prepared using the ingredients mentioned above under tablets and capsules in a conventional manner using, e.g., a mixer, a fluid bed apparatus or a spray drying equipment.

Controlled release compositions for oral use may be constructed to release the active drug by controlling the dissolution and/or the diffusion of the active drug substance. Any of a number of strategies can be pursued in order to obtain controlled release and the targeted plasma concentration versus time profile. In one example, controlled release is obtained by appropriate selection of various formulation parameters and ingredients, including, e.g., various types of controlled release compositions and coatings. Examples include single or multiple unit tablet or capsule compositions, oil solutions, suspensions, emulsions, microcapsules, microspheres, nanoparticles, patches, and liposomes. In certain embodiments, compositions include biodegradable, pH, and/or temperature-sensitive polymer coatings.

Dissolution or diffusion controlled release can be achieved by appropriate coating of a tablet, capsule, pellet, or granulate formulation of multibiotic agents, or by incorporating the multibiotic agent into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methyl methacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated methylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

The liquid forms in which the multibiotic agents and compositions of the present invention can be incorporated for administration orally include aqueous solutions, suitably flavored syrups, aqueous or oil suspensions, and flavored emulsions with edible oils, e.g., cottonseed oil, sesame oil, coconut oil, or peanut oil, as well as elixirs and similar pharmaceutical and nutraceutical vehicles.

### Formulations for Buccal Administration

Dosages for buccal or sublingual administration typically are 0.1 to 500 mg per single dose as required. In practice, the physician determines the actual dosing regimen which is most suitable for an individual patient, and the dosage varies with the age, weight, and response of the particular patient. The above dosages are exemplary of the average case, but individual instances exist where higher or lower dosages are merited, and such are within the scope of this invention.

For buccal administration, the compositions may take the form of tablets, lozenges, etc. formulated in a conventional manner. Liquid drug formulations suitable for use with nebulizers and liquid spray devices and electrohydrodynamic (EHD) aerosol devices will typically include a multibiotic agent disclosed herein with a pharmaceutically acceptable carrier. Preferably, the pharmaceutically acceptable carrier is a liquid, e.g., alcohol, water, polyethylene glycol, or a perfluorocarbon. Optionally, another material may be added to alter the aerosol properties of the solution or suspension of multibiotic agents disclosed herein. Desirably, this material is liquid, e.g., an alcohol, glycol, polyglycol, or a fatty acid. Other methods of formulating liquid drug solutions or suspension suitable for use in aerosol devices are known in the art (see, e.g., U.S. Pat. Nos. 5,112,598 and 5,556,611).

### Formulations for Nasal or Inhalation Administration

The multibiotic agents may also be formulated for nasal administration. Compositions for nasal administration also may conveniently be formulated as aerosols, drops, gels, and powders. The formulations may be provided in a single or multidose form. In the case of a dropper or pipette, dosing may be achieved by the patient administering an appropriate, predetermined volume of the solution or suspension. In the case of a spray, this may be achieved, for example, by means of a metering atomizing spray pump.

The multibiotic agents may further be formulated for aerosol administration, particularly to the respiratory tract by inhalation and including intranasal administration. The multibiotic agents for nasal or inhalation administration will generally have a small particle size for example on the order of five (5) microns or less. Such a particle size may be obtained by means known in the art, for example by micronization. The active ingredient is provided in a pressurized pack with a suitable propellant, e.g., a chlorofluorocarbon (CFC), for example, dichlorodifluoromethane, trichlorofluoromethane, or dichlorotetrafluoroethane, or carbon dioxide, or other suitable gas. The aerosol may conveniently also contain a surfactant, e.g., lecithin. The dose of drug may be controlled by a metered valve. Alternatively, the active ingredients may be provided in a form of a dry powder, e.g., a powder mix of the multibiotic agent in a suitable powder base, e.g., lactose, starch, starch derivatives (e.g., hydroxypropylmethyl cellulose), and/or polyvinylpyrrolidine (PVP). The powder carrier will form a gel in the nasal cavity. The powder composition may be presented in unit dose form for example in capsules or cartridges of e.g., gelatin or blister packs from which the powder may be administered by means of an inhaler.

Aerosol formulations typically include a solution or fine suspension of the active substance in a physiologically acceptable aqueous or non-aqueous solvent and are usually presented in single or multidose quantities in sterile form in a sealed container, which can take the form of a cartridge or refill for use with an atomizing device. Alternatively, the sealed container may be a unitary dispensing device, e.g., a single dose nasal inhaler or an aerosol dispenser fitted with a metering valve which is intended for disposal after use. Where the unit dosage form comprises an aerosol dispenser, it will contain a propellant, which can be a compressed gas, e.g., compressed air or an organic propellant, e.g., fluorochlorohydrocarbon. The aerosol unit dosage forms can also take the form of a pump-atomizer.

### Formulations for Parenteral Administration

The multibiotic agents described herein for use in the methods of the invention can be administered in a pharmaceutically acceptable parenteral (e.g., intravenous or intramuscular) formulation as described herein. The pharmaceutical formulation may also be administered parenterally (intravenous, intramuscular, subcutaneous or the like) in unit dosage forms or formulations containing conventional, non-toxic pharmaceutically acceptable carriers and adjuvants. In particular, formulations suitable for parenteral administration include aqueous and non-aqueous sterile injection solutions which may contain anti-oxidants, buffers, bacteriostats, and solutes which render the formulation isotonic with the blood of the intended recipient; and aqueous and non-aqueous sterile suspensions which may include suspending agents and thickening agents. For example, to prepare such a composition, the multibiotic agents disclosed herein may be dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution and isotonic sodium chloride solution. The aqueous formulation may also contain one or more preservatives, for example, methyl, ethyl or n-propyl p-hydroxybenzoate. Additional information regarding parenteral formulations can be found, for example, in the United States Pharmacopeia-National Formulary (USP-NF).

The parenteral formulation can be any of the five general types of preparations identified by the USP-NF as suitable for parenteral administration:
(1) "Drug Injection:" a liquid preparation that is a drug substance (e.g., a multibiotic agent disclosed herein or a solution thereof);
(2) "Drug for Injection:" the drug substance (e.g., a multibiotic agent disclosed herein) as a dry solid that will be combined with the appropriate sterile vehicle for parenteral administration as a drug injection;
(3) "Drug Injectable Emulsion:" a liquid preparation of the drug substance (e.g., a multibiotic agent disclosed herein) that is dissolved or dispersed in a suitable emulsion medium;
(4) "Drug Injectable Suspension:" a liquid preparation of the drug substance (e.g., a multibiotic agent disclosed herein) suspended in a suitable liquid medium; and
(5) "Drug for Injectable Suspension:" the drug substance (e.g., a multibiotic agent disclosed herein) as a dry solid that will be combined with the appropriate sterile vehicle for parenteral administration as a drug injectable suspension.

Exemplary formulations for parenteral administration include solutions of the multibiotic agents prepared in water suitably mixed with a surfactant, e.g., hydroxypropylcellulose. Dispersions can also be prepared in glycerol, liquid polyethylene glycols, DMSO and mixtures thereof with or without alcohol, and in oils. Under ordinary conditions of storage and use, these preparations may contain a preservative to prevent the growth of microorganisms. Conventional procedures and ingredients for the selection and preparation of suitable formulations are described, for example, in Remington: The Science and Practice of Pharmacy, 21st Ed., Gennaro, Ed., Lippencott Williams & Wilkins (2005) and in The United States Pharmacopeia: The National Formulary (USP 36 NF31), published in 2013.

Formulations for parenteral administration may, for example, contain excipients, sterile water, or saline, polyalkylene glycols, e.g., polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the multibiotic agents or biologically active agents within multibiotic agents. Other potentially useful parenteral delivery systems for multibiotic agents include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

The parenteral formulation can be formulated for prompt release or for sustained/extended release of the multibiotic agent. Exemplary formulations for parenteral release of the multibiotic agent include: aqueous solutions, powders for reconstitution, cosolvent solutions, oil/water emulsions, suspensions, oil-based solutions, liposomes, microspheres, and polymeric gels.

The following examples are meant to illustrate the invention. Examples 20, 21, 117 and 121 are examples of the invention. The remaining examples are disclosed below for illustrative purposes.

### EXAMPLES

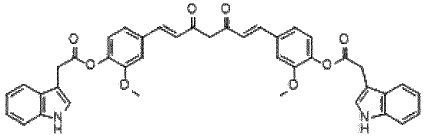

### Example 1: [4-[(1E,6E)-7-[4-[2-(1H-indol-3-yl)acetyl]oxy-3-methoxy-phenyl]-3,5-dioxo-hepta-1,6-dienyl]-2-methoxy-phenyl] 2-(1H-indol-3-yl)acetate

A mixture of curcumin (3 g, 8.14 mmol, 1 equiv.), 2-(1H-indol-3-yl)acetic acid (7.13 g, 40.72 mmol, 5 equiv.), EDCI (7.49 g, 39.09 mmol, 4.8 equiv.), and 4-dimethylaminopyridine (4.78 g, 39.09 mmol, 4.8 equiv.) in THF (100 mL) was degassed and purged with N₂ three times, and then the mixture was stirred at 15 °C for 3 h under N₂ atmosphere. The reaction mixture was mixed with brine (100 mL) and extracted with EtOAc (100 mL×3). The organic layer was dried with anhydrous Na₂SO₄, filtered, and concentrated in vacuo. The residue was purified by column purification (petroleum ether:ethyl acetate = 10:1 to 3:1) and concentrated to afford the crude product. The crude product was further purified by recrystallization with EtOAc (20 mL) to give the pure product. (638 mg, 935 mmol, 11% yield, 96.39% purity) LC/MS: (M+H⁺): 683.2

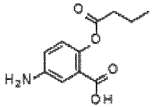

### Example 2: 5-amino-2-butanoyloxy-benzoic acid

### Step 1:

To a mixture of 5-amino-2-hydroxy-benzoic acid (3 g, 19.59 mmol, 1 equiv.) in methanol (50 mL) was added Boc₂O (4.28 g, 19.59 mmol, 4.50 mL, 1 equiv.) in one portion at 15 °C under N₂. The mixture was stirred at 15 °C for 5 h. The residue was poured into water (100 mL). The aqueous phase was extracted with EtOAc (100 mL), and the organic phase was dried with anhydrous Na₂SO₄, filtered, and concentrated in vacuo. The residue was used in next step without further purification. 5-(tert-butoxycarbonylamino)-2-hydroxy-benzoic acid (4 g, crude) as crude was obtained.

### Step 2:

To a solution of 5-(tert-butoxycarbonylamino)-2-hydroxy-benzoic acid (4 g, 15.79 mmol, 1 equiv.) and triethylamine (119.87 mg, 1.18 mmol, 164.88 µL, 1 equiv.) in THF (30 mL) was added butanoyl chloride (126.22 mg, 1.18 mmol, 123.74 µL, 1 equiv.) drop-wise at 0°C, while the temperature was maintained below 0 °C. The reaction mixture was warmed to 15 °C and stirred for 2 h. The reaction was quenched by slow addition of ice, and then the mixture was extracted with EtOAc (100 mL). The organic phase was dried over anhydrous Na₂SO₄, filtered, and concentrated in vacuo. The residue was purified by recrystallization with EtOAc (20 mL) to give the pure 2-butanoyloxy-5- (tert-butoxycarbonylamino)benzoic acid (1.5 g, 4.64 mmol, 29.37% yield) as white solid.

### Step 3:

A solution of 2-butanoyloxy-5-(tert-butoxycarbonylamino)benzoic acid (1.5 g, 4.64 mmol, 1 equiv.) in HCl-EtOAc (20 mL, 4 M) was stirred at 15 °C for 1 h. The mixture was filtered to obtain the product 5-amino-2-butanoyloxy-benzoic acid as off-white solid (0.74 g, 2.76 mmol, 59.58% yield, 96.993% purity, HCl salt). LC/MS: (M+H⁺) 224.1

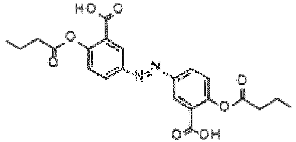

### Example 3: 2-butanoyloxy-5-[(E)-(4-butanoyloxy-3-carboxy-phenyl)azo]benzoic acid

A solution of [2-carboxy-4-[(E)-(3-carboxy-4-sodiooxy-phenyl)azo]phenoxy] sodium (2 g, 5.78 mmol, 1 equiv.), butanoyl chloride (2.46 g, 23.11 mmol, 2.41 mL, 4 equiv.), and NaOH (462.12 mg, 11.55 mmol, 2 equiv.) in DMF (100 mL) was stirred at 50°C for 0.5 h. The solid was filtered, water (150 mL) was added to the filtrate, and the mixture was filtered again. The resulting solids filter cake was dried in vacuo. 2-butanoyloxy-5-[(E)-(4-butanoyloxy-3-carboxy-phenyl)azo]benzoic acid (0.8 g) was obtained as brown solid. LC/MS: (M+H⁺): 443.1

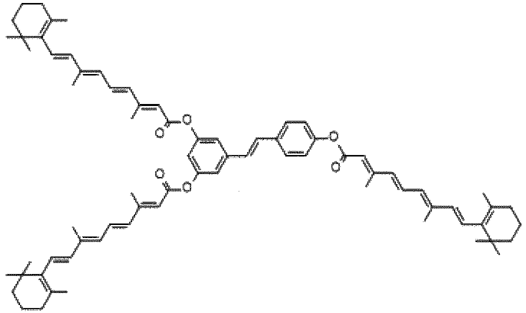

### Example 4: [4-[(E)-2-[3,5-bis[[(2E,4E,6E,8E)-3,7-dimethyl-9-(2,6,6-trimethylcyclohexen-1-yl)nona-2,4,6,8-tetraenoyl]oxy]phenyl]vinyl]phenyl] (2E,4E,6E,8E)-3,7-dimethyl-9-(2,6,6-trimethylcyclohexen-1-yl)nona-2,4,6,8-tetraenoate

A solution of (2E,4E,6E,8E)-3,7-dimethyl-9-(2,6,6-trimethylcyclohexen-1-yl) nona-2,4,6,8-tetraenoic acid (1 g, 3.33 mmol, 1 equiv.), 5-[(E)-2-(4-hydroxyphenyl) vinyl]benzene-1,3-diol (151.94 mg, 665.70 mmol, 0.2 equiv.), *N*,*N'*-dicyclohexylcarbodiimide (686.76 mg, 3.33 mmol, 673.30 mL, 1 equiv.), and 4-dimethylaminopyridine (40.66 mg, 332.85 mmol, 0.1 equiv.) in CH₂Cl₂ (40 mL) was stirred at 20°C for 10 h. The solvent was removed in vacuo, and the crude product was purified by silica gel chromatography using Petroleum ether/Ethyl acetate=20:1-5:1 as a mobile phase. The title product (0.6 g, 557.88 mmol, 16.76% yield) was obtained as yellow solid. ¹H NMR: (400 MHz, CDCl₃): δ 7.4 (m, 2H), 7.03-7.1 (m, 12H), 6.07-6.1 (m, 5H), 6.27-6.33 (m, 5H), 5.9 (br s, 3H), 2.35 (brs, 8H), 1.93-1.97 (m, 15H), 1.65 (m, 8H), 1.54-1.55 (m, 8H), 1.39- 1.42 (m, 6H) 0.9-1.0 (m, 18H) ppm

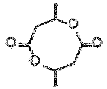

### Example 5: 4,8-dimethyl-1,5-dioxocane-2,6-dione

### Step 1:

A solution of methyl 4-chloro-3-oxo-butanoate (20 g, 132.84 mmol, 1 equiv.) and NaOAc (21.79 g, 265.67 mmol, 2 equiv.) in DMF (150 mL) was stirred at 20°C for 10 h. The solvent was removed in vacuo, and the crude product was purified by silica gel chromatography (Petroleum ether/Ethyl acetate/CH₂Cl₂=30:1:1-10:1:1). The product (dimethyl 2,5-dioxocyclohexane-1,4-dicarboxylate; 5 g, 21.91 mmol, 16.49% yield) was obtained as a yellow solid.

### Step 2:

A solution of dimethyl 2,5-dioxocyclohexane-1,4-dicarboxylate (5 g, 21.91 mmol, 1 equiv.), K₂CO₃ (7.57 g, 54.78 mmol, 2.5 equiv.) and Mel (15.55 g, 109.55 mmol, 6.82 mL, 5 equiv.) in acetone (200 mL) was stirred at 70°C for 20 h. The solid was filtered, and the filtrate was concentrated in vacuo. The crude product was purified by silica gel chromatography using Petroleum ether/Ethyl acetate (30:1-5:1) as a mobile phase. The product (dimethyl 1,4-dimethyl-2,5-dioxocyclohexane-1,4-dicarboxylate; 2 g, 7.80 mmol, 35.62% yield) was obtained as a yellow solid.

### Step 3:

To a solution of dimethyl 1,4-dimethyl-2,5-dioxocyclohexane-1,4-dicarboxylate (9 g, 35.12 mmol, 1 equiv.) in methanol (1 mL) and H₂O (200 mL) was added conc. H₂SO₄ (504.00 g, 5.14 mol, 273.91 mL, 146.31 equiv.) dropwise. The mixture was stirred at 100°C for 5 h. The pH of the mixture was adjusted to ca. 6-7 by the addition of aq. NaOH (6 N), then the mixture was extracted with EtOAc (100 mL×3). The combined organic phase was washed with brine (100 mL), dried over anhydrous Na₂SO₄, filtered, and concentrated in vacuo. The crude product was purified by silica gel chromatography (Petroleum ether/Ethyl acetate=30:1-1:1). Compound 2,5-dimethylcyclohexane-1,4-dione (2 g, crude) was obtained as yellow solid.

### Step 4:

To a solution of 2,5-dimethylcyclohexane-1,4-dione (2 g, 14.27 mmol, 1 equiv.) in dichloromethane (150 mL) was added m-CPBA (8.69 g, 42.80 mmol, 85% purity, 3 equiv.) in one portion under N₂, then the mixture was stirred at 20 °C in the dark for 48 h. The mixture was filtered, and the filtrate was washed with aq. K₂CO₃ (50 mL × 2) and aq. NaS₂O₃ (50 mL). The organic layer was separated, dried over Na₂SO₄, and concentrated in vacuo. The crude product was mixed with Petroleum ether: Ethyl acetate=50:1 (100 mL) and stirred for 10 min. The solid was separated out by filtration and dried in vacuum. Compound 4,8-dimethyl-1,5-dioxocane-2,6-dione (0.7 g, 4.07 mmol, 28.50% yield, racemate) is obtained as white solid. ¹H NMR (400 MHz, CDCl₃): δ 1.45 ppm (d, 6H), 2.6 (dd, 4H), 5.3 (m, 1H) ppm

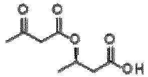

### Example 6: (3R)-3-(3-oxobutanoyloxy)butanoic acid

### Step 1:

To a solution of [(3R)-3-hydroxybutanoyl]oxysodium (3 g, 23.79 mmol, 1 equiv.) in DMF (40 mL) was added bromomethylbenzene (4.48 g, 26.17 mmol, 3.11 mL, 1.1 equiv.) drop-wise at 0°C under N₂. The reaction mixture was warmed to 15°C and stirred at 15°C for 6 h, at which time the residue was poured into water (80 mL). The aqueous phase was extracted with ethyl acetate (100 mL). The organic phase was washed with brine (80 mL), dried over anhydrous Na₂SO₄, filtered, and concentrated in vacuo. The residue was purified by silica gel chromatography (Petroleum ether/Ethyl acetate=1:0, 5/1) to afford benzyl (3R)-3-hydroxybutanoate (3.82 g, 19.65 mmol, 82.60% yield) as white oil.

### Step 2:

A mixture of tert-butyl 3-oxobutanoate (5 g, 31.61 mmol, 5.24 mL, 1 equiv.) and trifluoroacetic acid (36.04 g, 316.07 mmol, 23.40 mL, 10 equiv.) in dichloromethane (20 mL) was degassed and purged with N₂ 3 times, and the mixture was stirred at 20 °C for 0.5 h. The reaction mixture was concentrated under reduced pressure to afford 3-oxobutanoic acid (2.36 g, 23.12 mmol, 73.14% yield) as a colorless oil and used in the next step directly.

### Step 3:

A mixture of 3-oxobutanoic acid (1.31 g, 12.87 mmol, 1 equiv.), benzyl (3R)-3-hydroxybutanoate (2.5 g, 12.87 mmol, 1 equiv.), 4-dimethylaminopyridine (786.25 mg, 6.44 mmol, 0.5 equiv.), and EDCI (3.70 g, 19.31 mmol, 1.5 equiv.) in dichloromethance (50 mL) was stirred at 20 °C for 5 h. The reaction mixture was concentrated under reduced pressure to give a residue, which was separated between H₂O (15 mL) and EtOAc (15 mL). The organic phase was separated, dried over Na₂SO₄, filtered, and concentrated under reduced pressure to give a residue. The residue was purified by column chromatography (SiO₂, Petroleum ether/Ethyl acetate=20/1 to 10:1) to obtain benzyl (3R)-3-(3-oxobutanoyloxy)butanoate (1.62 g, 5.82 mmol, 45.22% yield) as a yellow oil.

### Step 4:

To a solution of benzyl (3R)-3-(3-oxobutanoyloxy)butanoate (1.3 g, 4.67 mmol, 1 equiv.) in THF (100 mL) is added Pd/C (10%, 800 mg) under N₂ atmosphere. The suspension was degassed and purged with H₂ 3 times. The mixture was stirred under H₂ (15 Psi) at 20 °C for 10 h. The reaction mixture was filtered, and the filtrate was concentrated under reduced pressure to afford a residue. The residue was purified by column chromatography (SiO₂, Petroleum ether/Ethyl acetate=2:1). (3R)-3-(3-oxobutanoyloxy) butanoic acid (769 mg, 3.33 mmol, 71.31% yield, 81.51% purity) was obtained as a gray oil. LCMS: (M+H⁺): 189.1

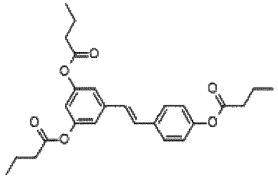

### Example 7: [4-[(E)-2-[3,5-di(butanoyloxy)phenyl]vinyl]phenyl] butanoate

To a solution of 5-[(E)-2-(4-hydroxyphenyl)vinyl]benzene-1,3-diol (3 g, 13.14 mmol, 1 equiv.) and K₂CO₃ (4.54 g, 32.86 mmol, 2.5 equiv.) in acetonitrile (50 mL) was added butanoyl chloride (5.60 g, 52.58 mmol, 5.49 mL, 4 equiv.). The mixture was stirred at 20 °C for 10 h. The reaction mixture was filtered, and the filtrate was concentrated under reduced pressure to give a residue that was purified by column chromatography (SiO₂, Petroleum ether/Ethyl acetate=20/1 to 10:1). [4-[(E)-2-[3,5-di(butanoyloxy) phenyl]vinyl]phenyl] butanoate was obtained as a white solid. LC/MS: (M+NH₄⁺): 456.2

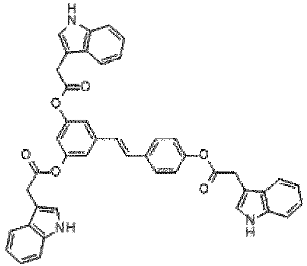

### Example 8: [4-[(E)-2-[3,5-bis[[2-(1H-indol-3-yl)acetyl]oxy]phenyl]vinyl]phenyl] 2-(1H-indol-3-yl)acetate

A mixture of 5-[(E)-2-(4-hydroxyphenyl)vinyl]benzene-1,3-diol (977.16 mg, 4.28 mmol, 1 equiv.), 2-(1H-indol-3-yl)acetic acid (3 g, 17.12 mmol, 4 equiv.), 4-dimethylaminopyridine (2.09 g, 17.12 mmol, 4 equiv.) and EDCI (3.28 g, 17.12 mmol, 4 equiv.) in DMF (100 mL) was stirred at 20 °C for 10 h. The reaction mixture was partitioned between H₂O (100 mL) and EtOAc (100 mL). The organic phase was separated, washed with brine (50 mL), dried over anhydrous Na₂SO₄, filtered, and concentrated under reduced pressure to give a residue. The residue was purified by prep-HPLC (column: Phenomenex luna(2) C18 250*50 10u; mobile phase: [water(0.1% trifluoroacetic acid)-acetonitrile];B%: 50%-80%,20min) and concentrated under reduced pressure to give a residue, which was further separated between H₂O (20 mL) and EtOAc (20 mL). The organic phase was separated, dried over Na₂SO₄, filtered, and concentrated under reduced pressure to afford a light brown solid. LC/MS:(M+H⁺):700.3

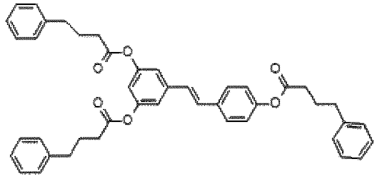

### Example 9: [4-[(E)-2-[3,5-bis(4-phenylbutanoyloxy)phenyl]vinyl]phenyl] 4-phenylbutanoate

### Step 1

To a solution of 4-phenylbutanoic acid (5 g, 30.45 mmol, 1 equiv.) in dichloromethane (50 mL) was added SOCl₂ (10.87 g, 91.35 mmol, 6.63 mL, 3 equiv.) at 0 °C. The mixture is stirred at 20 °C for 10 h. The reaction mixture was concentrated under reduced pressure to give a residue that was dissolved in toluene (15 mL). The solution was concentrated under reduced pressure to afford 4-phenylbutanoyl chloride (4.32 g, crude) as a yellow oil, which was used in next step directly.

### Step 2

To a solution of 5-[(E)-2-(4-hydroxyphenyl)vinyl]benzene-1,3-diol (0.278 g, 1.22 mmol, 1 equiv.) and K₂CO₃ (420.89 mg, 3.05 mmol, 2.5 equiv.) in acetonitrile (30 mL) was added 4-phenylbutanoyl chloride (1.00 g, 5.48 mmol, 4.5 equiv.). The mixture was stirred at 20 °C for 10 h, the reaction mixture was filtered, and the filtrate was concentrated under reduced pressure to give a residue. The residue was combined with another batch and purified by column chromatography (SiO₂, Petroleum ether/Ethyl acetate=20/1 to 8:1) to afford [4-[(E)-2-[3, 5-bis (4-phenylbutanoyloxy) phenyl] vinyl] phenyl] 4-phenylbutanoate (367mg, 93.51% purity) as a white solid. LC/MS (M+NH₄⁺): 684.3

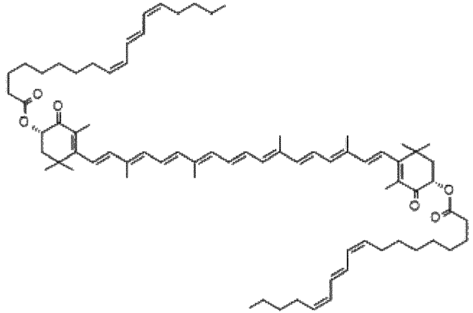

### Example 10: [(1S)-3,5,5-trimethyl-2-oxo-4-[(1E,3E,5E,7E,9E,11E,13E,15E,17E)-3,7,12,16-tetramethyl-18-[(4S)-2,6,6-trimethyl-4-[(9Z,11E,13Z)-octadeca-9, 11, 13-trienoyl]oxy-3-oxo-cyclohexen-1-yl]octadeca-1,3,5,7,9,11,13,15,17-nonaenyl]cyclohex-3-en-1-yl](9Z,11E,13Z)-octadeca-9,11,13-trienoate

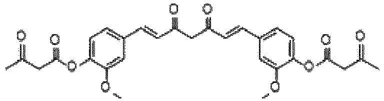

### Example 11: [2-methoxy-4-[(1E,6E)-7-[3-methoxy-4-(3-oxobutanoyloxy)phenyl]-3,5-dioxo-hepta-1,6-dienyl]phenyl] 3-oxobutanoate

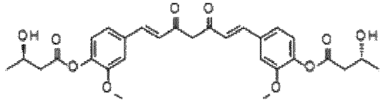

### Example 12: [4-[(1E,6E)-7-[4-[(3R)-3-hydroxybutanoyl]oxy-3-methoxy-phenyl]-3,5-dioxo-hepta-1,6-dienyl]-2-methoxy-phenyl] (3R)-3-hydroxybutanoate

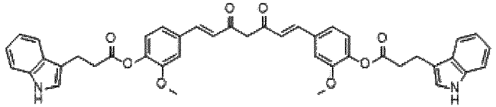

### Example 13: [4-[(1E,6E)-7-[4-[3-(1H-indol-3-yl)propanoyloxy]-3-methoxy-phenyl]-3,5-dioxo-hepta-1,6-dienyl]-2-methoxy-phenyl] 3-(1H-indol-3-yl)propanoate

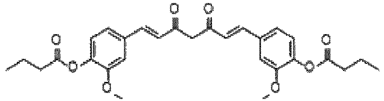

### Example 14: [4-[(1E,6E)-7-(4-butanoyloxy-3-methoxy-phenyl)-3,5-dioxo-hepta-1,6-dienyl]-2-methoxyphenyl] butanoate

To a solution of (1E,6E)-1,7-bis(4-hydroxy-3-methoxy-phenyl) hepta-1,6-diene-3,5-dione (3 g, 8.14 mmol, 1 equiv.) and Na₂CO₃ (1.29 g, 12.22 mmol, 3.40 mL, 1.5 equiv.) in acetonitrile (50 mL) was added butanoyl chloride (2.17 g, 20.36 mmol, 2.13 mL, 2.5 equiv.). The mixture was stirred at 20 °C for 10 h. The reaction mixture was concentrated under reduced pressure, and the residue was purified by column chromatography (SiO2, Petroleum ether/Ethyl acetate=2/1 to 1:1). [4-[(1E,6E)-7-(4-butanoyloxy-3-methoxy-phenyl)-3,5-dioxo-hepta-1,6-dienyl]-2-methoxy-phenyl] butanoate (1.5 g, 2.85 mmol, 35.02% yield, 96.70% purity) was obtained as a yellow powder. LC/MS: (M+H⁺):509.2

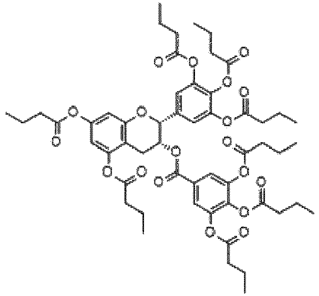

### Example 15: [(2R,3R)-5,7-di(butanoyloxy)-2-[3,4,5-tri(butanoyloxy)phenyl]chroman-3-yl] 3,4,5-tri(butanoyloxy)benzoate

Butyryl chloride (6.03 mL) was added to a stirring solution of epigallo catechin gallate (2.0 g) and pyridine (6.28 mL) in dichloromethane (20 mL) over 2 h between -5 °C to 5 °C. The resulting mixture was stirred overnight at room temperature. The reaction mixture was then diluted with dichloromethane (100 mL), washed sequentially with water (50 mL), 2N HCl (50 mL), saturated sodium bicarbonate (50 mL), and brine. The organic layer was evaporated in vacuo, and the resulting crude material was purified by flash chromatography by 30% ethyl acetate / heptane to give product (800 mg, 18%). ¹H NMR (CDCl₃): δ 7.6 (s, 2H), 7.22 (s, 2H), 6.78 (s, 1H), 6.6 (s, 1H), 5.62 (t, 1H), 5.18 (s, 1H), 2.98-3.02 (m, 2H), 2.4-2.6 (m, 16H), 1.6-1.8 (m, 16H), 0.92-1.02 (m, 24H) ppm

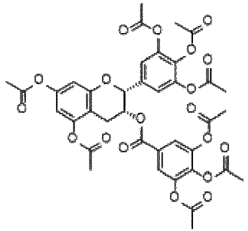

### Example 16: [(2R,3R)-5,7-diacetoxy-2-(3,4,5-triacetoxyphenyl)chroman-3-yl] 3,4,5-triacetoxybenzoate

Acetic anhydride (6.1mL) was added dropwise to epigallo catechin gallate (2.0 g) in pyridine (20mL) at 0°C, and the resulting mixture was stirred overnight at room temperature. Water was added to the reaction mixture, and the solid was filtered and washed with 1N HCl (10 mL) and heptane (20 mL). The solid was then dissolved in dichloromethane and passed through a silica gel filter column with dichloromethane as a mobile phase to get product (1.0 g, 28%). ¹H NMR (CDCl₃): δ 7.6 (s, 2H), 7.2 (s, 2H), 6.75 (s, 1H), 6.6 (s, 1H), 5.6 (t, 1H), 5.19 (s, 1H), 2.98-3.02 (m, 2H), 2.18-2.28 (m, 24H) ppm

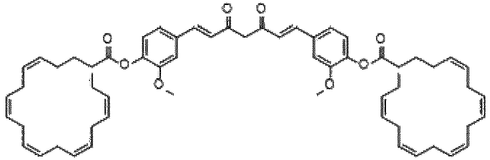

### Example 17: [4-[(1E,6E)-7-[4-[(5Z,8Z,11Z,14Z,17Z)-icosa-5,8,11,14,17-pentaenoyl]oxy-3-methoxyphenyl]-3,5-dioxo-hepta-1,6-dienyl]-2-methoxy-phenyl] (5Z,8Z,11Z,14Z,17Z)-icosa-5,8,11,14,17-pentaenoate was prepared

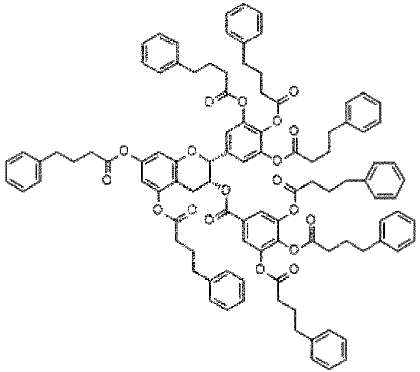

### Example 18: [(2R,3R)-5,7-bis(4-phenylbutanoyloxy)-2-[3,4,5-tris(4-phenylbutanoyloxy)phenyl]chroman-3-yl] 3,4,5-tris(4-phenylbutanoyloxy)benzoate

### Step 1:

To a solution of 4-phenylbutanoic acid (3 g, 18.27 mmol, 1 equiv.) and SOCl₂ (10.87 g, 91.35 mmol, 6.63 mL, 5 equiv.) in dichloromethane (50 mL) is added one drop of DMF, then the mixture stirred at 20°C for 5 h. The solvent is removed in vacuum and toluene (20 mL) added to the mixture. The mixture is concentrated in vacuo to afford 4-phenylbutanoyl chloride (3.5 g, crude).

### Step 2:

To a solution of [(2R,3R)-5,7-dihydroxy-2-(3,4,5-trihydroxyphenyl)chroman-3-yl] 3,4,5-trihydroxybenzoate (1 g, 2.18 mmol, 1 equiv.) and K₂CO₃ (4.52 g, 32.72 mmol, 15 equiv.) in acetonitrile (100 mL) was added a solution of 4-phenylbutanoyl chloride (7.97 g, 43.63 mmol, 20 equiv.) in acetonitrile (10 mL), then the mixture was stirred at 20°C for 10 h. The mixture was filtered, and the filtrate was concentrated in vacuum. The crude product was purified by silica gel chromatography (petroleum ether/ethyl acetate=20:1-1:1) to afford [(2R,3R)-5,7-bis(4-phenylbutanoyloxy)-2-[3,4,5-tris(4-phenyl butanoyloxy)phenyl] chroman-3-yl],4,5-tris(4-phenylbutanoyloxy)benzoate (2.2 g, 1.28 mmol, 58.73% yield, 94.8% purity) as a white solid. LC/MS(M+H₃O⁺):1645.1

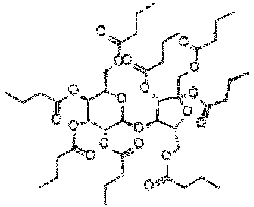

### Example 19: [(2R,3S,4S,5R,6S)-3,4,5-tri(butanoyloxy)-6-[(2R,3R,4S,5S)-4,5-di(butanoyloxy)-2,5-bis(butanoyloxymethyl)tetrahydrofuran-3-yl]oxy-tetrahydropyran-2-yl]methyl butanoate

Butyryl chloride (12.1, 9equiv) in dichloromethane (100 mL, 20 vol) was added dropwise to a stirred solution of lactulose (5 g, 1 equiv) in anhydrous pyridine (21.2 mL, 18 equiv) under nitrogen over a period of 1-2 hours, maintaining the temperature at -5 to 5 °C. The reaction mixture was allowed to warm to room temperature and stirred at room temperature for 18 hours. The resultant mixture was diluted with 100 mL (20 vol) of dichloromethane and washed with water (100 mL, 20 vol). The organic phase was separated and washed with 2N HCl (2 × 100 mL), saturated sodium bicarbonate solution (100 mL, 20 vol), and half brine (100 mL, 20 vol). The organic layer was dried over magnesium sulfate, filtered and concentrated under reduced presurre. The crude product was purified by silica gel column chromatography eluted with 5-20% EtOAc/heptanes to afford the product L8B as light yellow oil. Yield: 8 g (65%). ¹H NMR (CDCl₃): δ 4.9-5.4 (m, 5H), 4.3-4.8 (m, 4H), 3.8-4.2 (m, 4 H) 2.1-2.4 (m, 16H),1.5-1.7(m, 16H) 0.8-1.5 (m, 24H) ppm

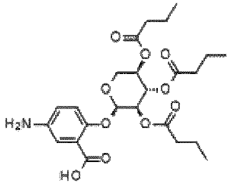

### Example 20: 5-amino-2-[(2R,3R,4S,5R)-3,4,5-tri(butanoyloxy)tetrahydropyran-2-yl]oxy-benzoic acid

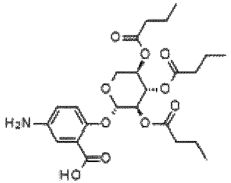

### Example 21: 5-amino-2-[(2S,3R,4S,5R)-3,4,5-tri(butanoyloxy)tetrahydropyran-2-yl]oxy-benzoic acid

### Step 1

5-Amino salicylic acid (10.0 g) was dissolved in a mixture of dioxane (100 mL), water (100 mL), and NaOH (2.60 g), and the resulting solution was cooled in an ice-bath. Di-tert-butyl dicarbonate (Boc anhydride) (15.60 g) was added, and the mixture was warmed to room temperature and stirred for 1.0 h. The solution was concentrated to 60 mL, diluted with ethyl acetate (100 mL), and the resulting mixture was cooled in an ice-bath. The mixture was acidified with aq. KHSO₄ to pH 2-3. The aqueous layer was extracted with EtOAc. The organic phase was washed with water, brine, dried over Na₂SO₄, filtered, and concentrated to afford 5-(tert-butoxycarbonylamino)-2-hydroxy-benzoic acid (7.0 g, 42%).

### Step 2

5-(tert-butoxycarbonylamino)-2-hydroxy-benzoic acid (3 g) was dissolved in DMF, and the resulting solution was cooled to 0 ⁰C. 1,1'-Carbonyldiimidazole (CDI) was added, and the mixture was stirred at room temperature for 2 h. Then, tert-butylalcohol (1.7 g) and DBU (2.1 g) were added. The reaction was stirred at room temperature overnight. The reaction mixture was poured onto ice-water, and the solid product, tert-butyl 5-(tert-butoxycarbonylamino)-2-hydroxy-benzoate, was collected by filtration (3.0 g, 81.9%).

### Step 3

To a mixture of tert-butyl 5-(tert-butoxycarbonylamino)-2-hydroxy-benzoate, [(3R,4S,5R)-4,5-di(butanoyloxy)-6-hydroxy-tetrahydropyran-3-yl] butanoate (1.2 g) and triphenylphosphene (1.2 g) in THF (50 mL) was added di-t-butyl azodicarboxylate (DTAD) (1.1 g) DTAD, and the mixture was stirred overnight at room temperature. The product was purified by reverse phase chromatography using acetonitrile-water to afford tert-butyl 5-(tert-butoxycarbonylamino)-2-[(3R,4S,5R)-3,4,5-tri(butanoyloxy)tetrahydropyran-2-yl]oxy-benzoate as sticky solid (0.6 g, 30.0%).

### Step 4

Tert-butyl 5-(tert-butoxycarbonylamino)-2-[(3R,4S,5R)-3,4,5-tri(butanoyloxy)tetrahydropyran-2-yl]oxy-benzoate (600 mg) was added to 4M HCl in dioxane (15 mL) and stirred at room temperature overnight. After the consumption of the starting material, the organic phase was evaporated, and the residue was co-evaporated with heptane and dichloromethane twice more. The solid obtained was dried under high vacuum to afford compound the title product as dark brown solid (200 mg, 43.8%). Fractionation of the product afforded two anomeric isomers (the compounds of Examples 20 and 21). ¹H NMR (DMSO d6): Isomer 1: δ 7.62 (d, 1H), 7.45(dd, 1H), 7.38 (d, 1H), 6 (d, 1H), 5.6(t, 1H), 5.0-5.1(m,1H), 4.7-4.75 (m, 1H), 3.6-3.8 (m, 1H), 3.45-3.6 (1H), 2.1-2.3 (m, 6H), 1.4-1.6 (m, 6H), 0.75-0.85 (m, 9H). Isomer 2: δ 7.82 (d, 1H), 7.5(dd, 1H), 7.05 (d, 1H), 5.5 (d, 1H), 5.3 (t, 1H), 5.1-5.15 (m,1H), 4.9-5.0 (m, 1H), 4.0-4.08 (m, 1H), 3.7-3.8 (1H), 2.1-2.3 (m, 6H), 1.4-1.6 (m, 6H), 0.75-0.85 (m, 9H) ppm

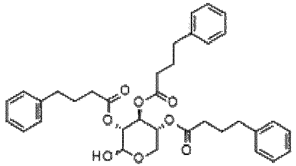

### Example 22: [(3R,4S,5R)-6-hydroxy-4,5-bis(4-phenylbutanoyloxy)tetrahydropyran-3-yl] 4-phenyl butanoate

### Step 1

Oxalyl chloride (206.4 g, 7 equiv) was added dropwise into a solution of 4-phenylbutyric acid (267 g, 7 equiv) in CH₂Cl₂ (350 mL, 10 vol) and catalytic DMF at 0-5 °C. The mixture was stirred at RT for 1 hour then added dropwise to a stirred solution of D-(+)-xylose (35 g, 1 equiv) in anhydrous pyridine (170 mL, 9 equiv) under nitrogen over a period of ~2 hours, maintaining the temperature at -5 to 5 °C. The reaction mixture was allowed to warm to room temperature and stirred at room temperature for 18 hours. The resultant mixture was diluted with 1.4 L (40 vol) of dichloromethane and washed with water (1.4 L, 40 vol). The organic phase was separated and washed with 2N HCl (2 × 25 vol), saturated sodium bicarbonate solution (700 mL, 20 vol), and half brine (700 mL, 20 vol). The organic layer was concentrated under reduced presurre and the crude product purified by silica gel column chromatography (2 kg silica gel) eluted with 5-20% EtOAc/heptanes to afford the product xylose tetraphenylbutyrate as a colorless oil as a ~91:9 mixture of α /β epimers by HPLC analysis at 220 nm. Yield: 149 g (87%).

### Step 2

Ammonium hydroxide (202 mL, 5 equiv) was added slowly to a mixture of xylose tetraphenylbutyrate ( 237 g, 1 equiv) in acetonitrile (1.77 L, 7.5 vol) at room temperature and the mixture stirred at room temperature for 6 hours. TLC and HPLC analysis indicated incomplete reaction. The reaction was stopped at this point to prevent further decomposition as indicated from TLC analysis. MTBE (1.9 L, 8 vol) was added and the mixture stirred for 15 minutes. The organic layer was separated and concentrated under reduced pressure to remove most of the MTBE and acetonitrile. The residue was diluted with water (1.2 L, 5 vol) and extracted with MTBE (1.9 L, 8 vol). The organic layer was separated, dried ove MgSO₄, filtered and concentrated under reduced presurre. The crude product was purified by silica gel column chromatography (2 kg silica gel) eluted with 10-50% EtOAc/heptanes to afford the product xylose triphenylbutyrate as a waxy solid as a ~64:36 mixture of α /β epimers by HPLC analysis at 220 nm. Yield: 68 g (36%). ¹H NMR (CDCl₃): δ 7.0-7.2 (m, 15H) 5.5 (dd, 1H), 5.4 (m, 1H), 4.8-5.0 (m, 2H), 4.1 (brs, 1 H), 3.8, (dd, 2H), 2.5-2.6 (m, 6H), 2.2-2.3 (m, 6H), 1.8-0.9 (m, 6H) ppm

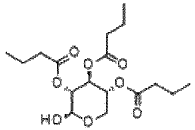

### Example 23: [(3R,4S,5R)-4,5-di(butanoyloxy)-6-hydroxy-tetrahydropyran-3-yl] butanoate

### Step 1

Butyryl chloride (298 g, 7 equiv) in dichloromethane (600 mL, 10 vol) was added dropwise to a stirred solution of D-(+)-xylose (60 g, 1 equiv) in anhydrous pyridine (285 g, 9 equiv) under nitrogen over a period of ~2 hours, maintaining the temperature at -5 to 5 °C. The reaction mixture was allowed to warm to room temperature and stirred at room temperature for 18 hours. The resultant orange mixture was diluted with 1.2 L (20 vol) of dichloromethane and washed with water (1.2 L, 20 vol). The organic phase was separated and washed with 2N HCl (2 × 15 vol), saturated sodium bicarbonate solution (900 mL, 15 vol), and half brine (10 vol). The organic layer was concentrated under reduced presurre and the crude product purified by silica gel column chromatography (2 kg silica gel) eluted with 5-15% EtOAc/heptanes to afford the product xylose tetrabutyrate as a colorless oil as a ~90:10 mixture of α /β epimers by HPLC analysis at 210 nm. Yield: 157 g (91%).

### Step 2:

Ammonium hydroxide (145 mL, 5 equiv) was added slowly to a mixture of xylose tetrabutyrate (100 g, 1 equiv) in acetonitrile (750 mL, 7.5 vol) at room temperature and the mixture stirred at room temperature for 3 hours. TLC and HPLC analysis indicated incomplete reaction. The reaction was stopped at this point to prevent further decomposition as indicated from TLC analysis. MTBE (1 L, 10 vol) was added and the mixture stirred for 15 minutes. The organic layer was separated and concentrated under reduced pressure to remove most of the MTBE and acetonitrile. The residue was diluted with water (1 L, 10 vol) and extracted with MTBE (1.5 L, 15 vol). The organic layer was separated, dried over MgSO₄, filtered and concentrated under reduced presurre. The crude product was purified by silica gel column chromatography (1.5 kg silica gel) eluted with 10-40% EtOAc/heptanes to afford the product xylose tributyrate (X3B) as a waxy solid as a ~67:33 mixture of α /β epimers by HPLC analysis at 210 nm. Yield: 30 g (36%). ¹H NMR (CDCl₃): δ 5.5 (t, 1H), 5.2-5.3 (m, 1H), 4.8-5.0 (m, 2H), 4.1 (brs, 1 H), 3.8, (dd, 2H), 2.2-2.3 (m, 6H),1.5-1.6 (m, 6H), 0.87-0.91 (m, 9H) ppm

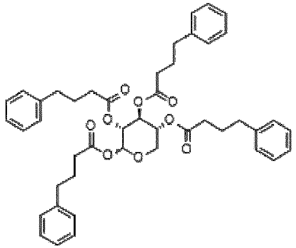

### Example 24: [(3R,4S,5R)-4,5,6-tris(4-phenylbutanoyloxy)tetrahydropyran-3-yl] 4-phenylbutanoate

Oxalyl chloride (206.4 g, 7 equiv.) was added dropwise into a solution of 4-phenylbutyric acid (267 g, 7 equiv.) in CH₂Cl₂ (350 mL, 10 vol.) and catalytic DMF at 0-5 °C. The mixture was stirred at RT for 1 hour then added dropwise to a stirred solution of D-(+)-xylose (35 g, 1 equiv) in anhydrous pyridine (170 mL, 9 equiv.) under nitrogen over a period of ~2 hours, maintaining the temperature at -5 to 5 °C. The reaction mixture was allowed to warm to room temperature and stirred at room temperature for 18 hours. The resultant mixture was diluted with 1.4 L (40 vol) of dichloromethane and washed with water (1.4 L, 40 vol.). The organic phase was separated and washed with 2N HCl (2 × 25 vol), saturated sodium bicarbonate solution (700 mL, 20 vol.), and half brine (700 mL, 20 vol.). The organic layer was concentrated under reduced presurre and the crude product purified by silica gel column chromatography (2 kg silica gel) eluted with 5-20% EtOAc/heptanes to afford the product xylose tetraphenylbutyrate as a colorless oil as a ~91:9 mixture of α /β epimers by HPLC analysis at 220 nm. Yield: 149 g (87%).

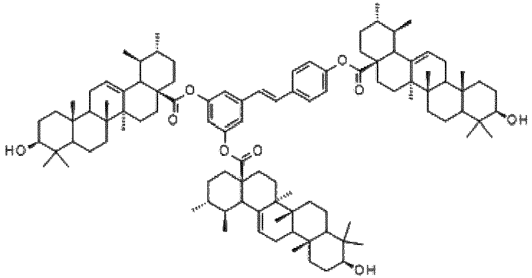

### Example 25: [4-[(E)-2-[3,5-bis[[(1S,2R,4aS,6aS,6bR,10S,12aR)-10-hydroxy-1,2,6a,6b,9,9,12a-heptamethyl-2,3,4,5,6,6a,7,8,8a,10,11,12,13,14b-tetradecahydro-1H-picene-4a-carbonyl]oxy]phenyl]vinyl]phenyl] (1R,2S,4aR,6aR,6bS,10R,12aS)-10-hydroxy-1,2,6a,6b,9,9,12a-heptamethyl-2,3,4,5,6,6a,7,8,8a,10,11,12,13,14b-tetradecahydro-1H-picene-4a-carboxylate

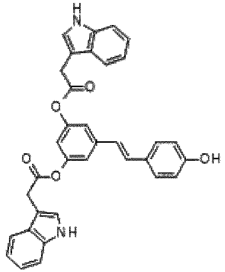

### Example 26: [3-[(E)-2-(4-hydroxyphenyl)vinyl]-5-[2-(1H-indol-3-yl)acetyl]oxy-phenyl] 2-(1H-indol-3-yl)acetate

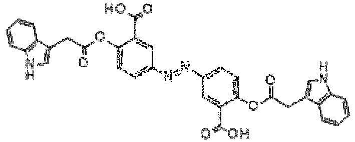

### Example: 27 5-[(E)-[3-carboxy-4-[2-(1H-indol-3-yl)acetyl]oxy-phenyl]azo]-2-[2-(1H-indol-3-yl)acetyl]oxy-benzoic acid

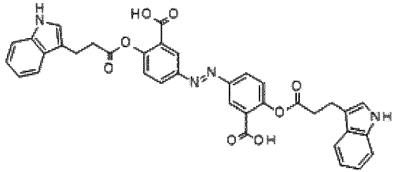

### Example 28: 5-[(E)-[3-carboxy-4-[3-(1H-indol-3-yl)propanoyloxy]phenyl]azo]-2-[3-(1H-indol-3-yl)propanoyloxy]benzoic acid

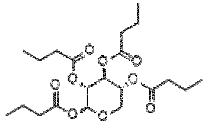

### Example 29: [(3R,4S,5R)-4,5,6-tri(butanoyloxy)tetrahydropyran-3-yl] butanoate

Butyryl chloride (298 g, 7 equiv) in dichloromethane (600 mL, 10 vol) was added dropwise to a stirred solution of D-(+)-xylose (60 g, 1 equiv) in anhydrous pyridine (285 g, 9 equiv) under nitrogen over a period of ~2 hours, maintaining the temperature at -5 to 5 °C. The reaction mixture was allowed to warm to room temperature and stirred at room temperature for 18 hours. The resultant orange mixture was diluted with 1.2 L (20 vol) of dichloromethane and washed with water (1.2 L, 20 vol). The organic phase was separated and washed with 2N HCl (2 × 15 vol), saturated sodium bicarbonate solution (900 mL, 15 vol), and half brine (10 vol). The organic layer was concentrated under reduced presurre and the crude product purified by silica gel column chromatography (2 kg silica gel) eluted with 5-15% EtOAc/heptanes to afford the product xylose tetrabutyrate as a colorless oil as a ~90:10 mixture of α /β epimers by HPLC analysis at 210 nm. Yield: 157 g (91%). ¹H NMR (CDCl₃): δ 6.3 (d, 1H), 5.5 (m, 1H), 5.0-5.1 (m, 2H), 3.6-3.9, (dd, 2H), 2.2-2.3 (m, 8H),1.5-1.6 (m, 8H), 0.91-1.0 (m, 12H) ppm

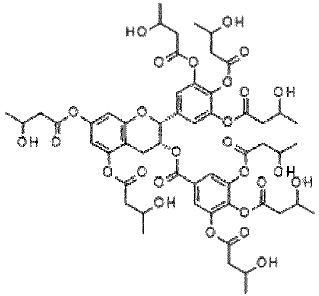

### Example 30: [(2R,3R)-5,7-bis(3-hydroxybutanoyloxy)-2-[3,4,5-tris(3-hydroxybutanoyloxy)phenyl]chroman-3-yl] 3,4,5-tris(3-hydroxybutanoyloxy)benzoate

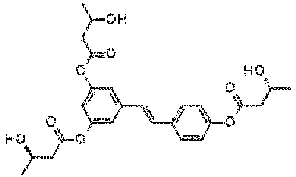

### Example 31: [4-[(E)-2-[3,5-bis[[(3R)-3-hydroxybutanoyl]oxy]phenyl]vinyl]phenyl] (3R)-3-hydroxybutanoate

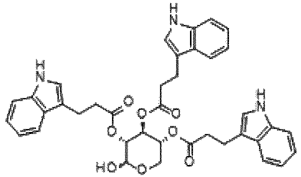

### Example 32: [(3R,4S,5R)-6-hydroxy-4,5-bis[3-(1H-indol-3-yl)propanoyloxy]tetrahydropyran-3-yl] 3-(1H-indol-3-yl)propanoate

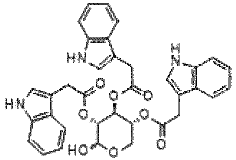

### Example 33: [(3R,4S,5R)-6-hydroxy-4,5-bis[[2-(1H-indol-3-yl)acetyl]oxy]tetrahydropyran-3-yl] 2-(1H-indol-3-yl)acetate

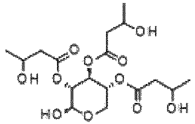

### Example 34: [(3R,4S,5R)-6-hydroxy-4,5-bis(3-hydroxybutanoyloxy)tetrahydropyran-3-yl] 3-hydroxybutanoate

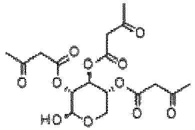

### Example 35: [(3R,4S,5R)-6-hydroxy-4,5-bis(3-oxobutanoyloxy)tetrahydropyran-3-yl] 3-oxobutanoate

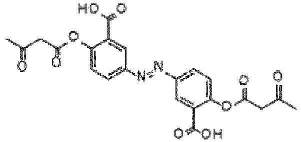

### Example 36: 5-[(E)-[3-carboxy-4-(3-oxobutanoyloxy)phenyl]azo]-2-(3-oxobutanoyloxy)benzoic acid

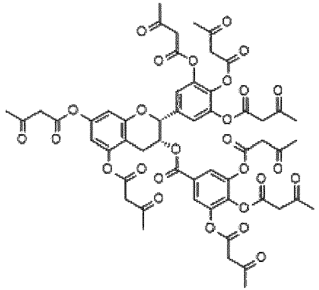

### Example 37: [(2R,3R)-5,7-bis(3-oxobutanoyloxy)-2-[3,4,5-tris(3-oxobutanoyloxy)phenyl]chroman-3-yl] 3,4,5-tris(3-oxobutanoyloxy)benzoate

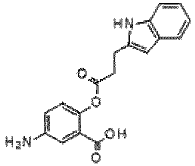

### Example 38: 5-amino-2-[3-(1H-indol-2-yl)propanoyloxy]benzoic acid

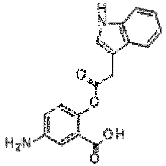

### Example 39: 5-amino-2-[2-(1H-indol-3-yl)acetyl]oxy-benzoic acid

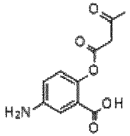

### Example 40: 5-amino-2-(3-oxobutanoyloxy)benzoic acid

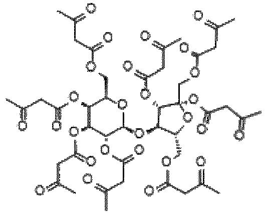

### Example 41: [(2R,3S,4S,5R,6S)-6-[(2R,3R,4S,5S)-4,5-bis(3-oxobutanoyloxy)-2,5-bis(3-oxobutanoyloxymethyl)tetrahydrofuran-3-yl]oxy-3,4,5-tris(3-oxobutanoyloxy)tetrahydropyran-2-yl]methyl 3-oxobutanoate

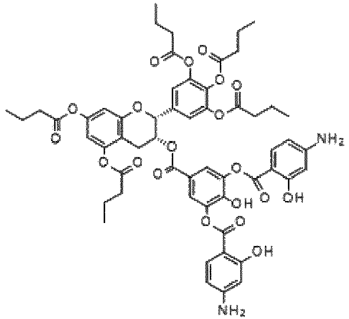

### Example 42: [(2R,3R)-5,7-di(butanoyloxy)-2-[3,4,5-tri(butanoyloxy)phenyl]chroman-3-yl] 3,5-bis[(4-amino-2-hydroxy-benzoyl)oxy]-4-hydroxy-benzoate

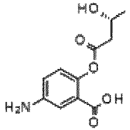

### Example 43: 5-amino-2-[(3R)-3-hydroxybutanoyl]oxy-benzoic acid

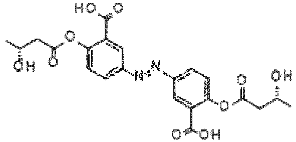

### Example 44: 5-[(E)-[3-carboxy-4-[(3R)-3-hydroxybutanoyl]oxy-phenyl]azo]-2-[(3R)-3-hydroxybutanoyl]oxy-benzoic acid

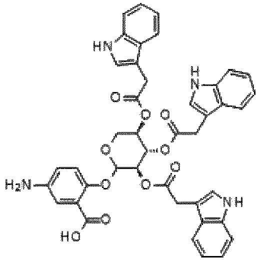

### Example 45: 5-amino-2-[(3R,4S,5R)-3,4,5-tris[[2-(1H-indol-3-yl)acetyl]oxy]tetrahydropyran-2-yl]oxy-benzoic acid

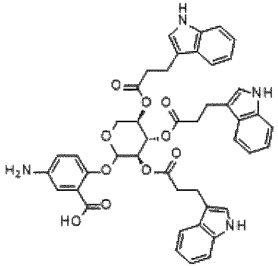

### Example 46: 5-amino-2-[(3R,4S,5R)-3,4,5-tris[3-(1H-indol-3-yl)propanoyloxy]tetrahydropyran-2-yl]oxy-benzoic acid

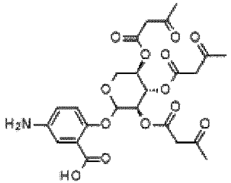

### Example 47: 5-amino-2-[(3R,4S,5R)-3,4,5-tris(3-oxobutanoyloxy)tetrahydropyran-2-yl]oxy-benzoic acid

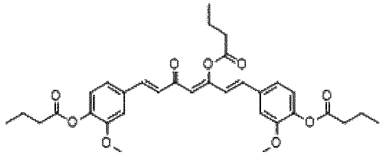

### Example 48: [4-[(1E,3Z,6E)-3-butanoyloxy-7-(4-butanoyloxy-3-methoxy-phenyl)-5-oxo-hepta-1,3,6-trienyl]-2-methoxy-phenyl] butanoate

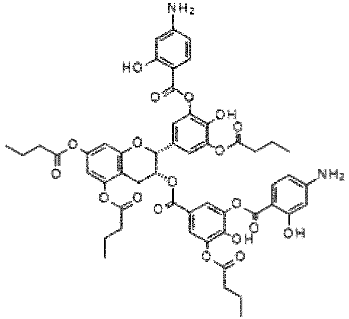

### Example 49: [(2R,3R)-2-[3-(4-amino-2-hydroxy-benzoyl)oxy-5-butanoyloxy-4-hydroxy-phenyl]-5,7-di(butanoyloxy)chroman-3-yl] 3-(4-amino-2-hydroxy-benzoyl)oxy-5-butanoyloxy-4-hydroxy-benzoate

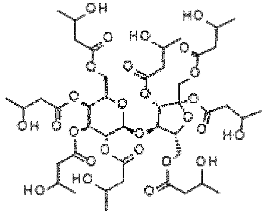

### Example 50: [(2R,3S,4S,5R,6S)-6-[(2R,3R,4S,5S)-4,5-bis(3-hydroxybutanoyloxy)-2,5-bis(3-hydroxybutanoyloxymethyl)tetrahydrofuran-3-yl]oxy-3,4,5-tris(3-hydroxybutanoyloxy)tetrahydropyran-2-yl]methyl 3-hydroxybutanoate

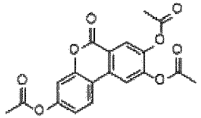

### Example 51: (8,9-diacetoxy-6-oxo-benzo[c]chromen-3-yl) acetate

To a mixture of 3,8,9-trihydroxybenzo[c]chromen-6-one (0.3 g) and acetic anhydride (0.5 g) in CH₂Cl₂ (10 mL) was added triethylamine (0.37 g). The mixture was stirred at 25 °C for 10 hours. The reaction mixture was quenched by addition of water (10 mL) and extracted three times with EtOAc (10 mL). The combined organic layers were washed with brine (20 mL), dried over Na₂SO₄, filtered and concentrated under reduced pressure. The residue was purified by column chromatography (SiO₂, petroleum ether / ethyl acetate, 3:1 to 1:1) to give (8,9-diacetoxy-6-oxo-benzo[c]chromen-3-yl) acetate as a gray solid. LCMS: 371.0 (M+H⁺) ¹H NMR (400 MHz, CDCl₃): δ 8.214 (s, 1H), 7.937 (d, 1H), 7.926 (s, 1H), 7.181 -7.127 (m, 2H), 2.381 (s, 3H), 2.361 (s, 3H), 2.353 (s, 3H) ppm

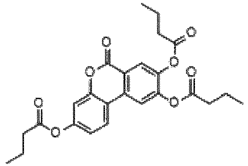

### Example 52: 8,9-di(butanoyloxy)-6-oxo-benzo[c]chromen-3-yl] butanoate

To a mixture of 3,8,9-trihydroxybenzo[c]chromen-6-one (0.3 g) and butanoyl chloride (0.52 g) in acetonitrile (10 mL) was added K₂CO₃ (0.68 g). The mixture was stirred at 45°C for 10 hours. The reaction mixture was quenched with water (10 mL) and extracted three times with EtOAc (10 mL). The combined organic layers were washed with brine (20 mL), dried over Na₂SO₄, filtered and concentrated under reduced pressure. The residue was combined with another batch for purification by column chromatography (SiO₂, petroleum ether / ethyl acetate, 10:1 to 1:1) to give 8,9-di(butanoyloxy)-6-oxo-benzo[c]chromen-3-yl] butanoate (0.45 g) as a gray solid. LCMS: 455.2 (M+H⁺) ¹H NMR (400 MHz, CDCl₃): δ 8.199 (s, 1H), 7.939 (d, 1H), 7.921 (s, 1H), 7.170 - 7.115 (m, 2H), 2.622 - 2.573 (m, 6H), 1.829 - 1.806 (6H, m), 1.102 - 1.056 (m, 9H) ppm

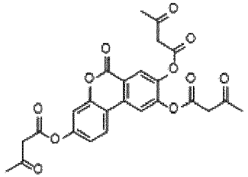

### Example 53: [6-oxo-8,9-bis(3-oxobutanoyloxy)benzo[c]chromen-3-yl] 3-oxobutanoate

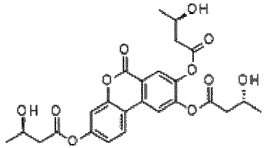

### Example 54: [8,9-bis[[(3R)-3-hydroxybutanoyl]oxy]-6-oxo-benzo[c]chromen-3-yl] (3R)-3-hydroxybutanoate

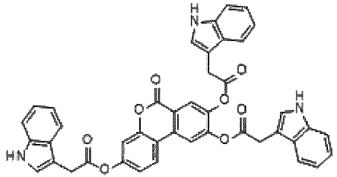

### Example 55: [8,9-bis[[2-(1H-indol-3-yl)acetyl]oxy]-6-oxo-benzo[c]chromen-3-yl] 2-(1H-indol-3-yl)acetate

A mixture of 3,8,9-trihydroxybenzo[c]chromen-6-one (0.3 g), 2-(1H-indol-3-yl)acetic acid (0.86 g), 4-dimethylaminopyridine (0.060 g) and *N*,*N*'-dicyclohexylcarbodiimide (1.01 g) in DMF (20 mL) was stirred at 25 °C for 10 hours. The reaction mixture was filtered and concentrated under reduced pressure. The residue was purified by reverse phase prep-HPLC (C18, water (0.1% trifluoroacetic acid)-acetonitrile gradient) to give [8,9-bis[[2-(1H-indol-3-yl)acetyl]oxy]-6-oxo-benzo[c]chromen-3-yl]2-(1H-indol-3-yl)acetate (0.127 g) as a brown solid. ¹H NMR (400 MHz, DMSO-d6): δ 11.079 - 11.038 (m, 3H), 8.371 - 8.333 (m, 2H), 8.071 (s, 1H), 7.7 - 6.9 (m, 17H), 4.09 (s, 2H), 3.838 (s, 2H), 3.77 (s, 2H) ppm

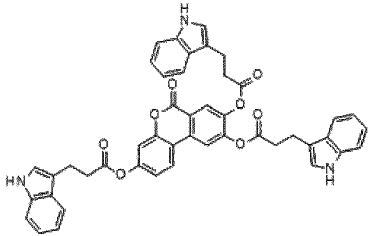

### Example 56: [8,9-bis[3-(1H-indol-3-yl)propanoyloxy]-6-oxo-benzo[c]chromen-3-yl] 3-(1H-indol-3-yl)propanoate

A mixture of 3,8,9-trihydroxybenzo[c]chromen-6-one (0.3 g), 3-(1H-indol-3-yl)propanoic acid (0.93 g), 4-dimethylaminopyridine (0.06) and *N*,*N'*-dicyclohexylcarbodiimide (1.02 g) in DMF (20 mL) was stirred at 25 °C for 10 hours. The reaction mixture was filtered, and the filtrate was concentrated under reduced pressure. The residue was purified by reverse phase prep-HPLC (C18, water (0.1% trifluoroacetic acid)-acetonitrile gradient) to give [8,9-bis[3-1H-indol-3-yl)propanoyloxy]-6-oxo-benzo[c]chromen-3-yl]3-(1H-indol-3-yl) propanoate (0.048 g, 4.8%) as a brown solid. ¹H NMR (400 MHz, DMSO-d6): δ 10.872 (m, 3H), 8.250 - 8.233 (m, 2H), 8.087 (s, 1H), 7.55 - 7.06 (m, 17H), 3.317 - 2.912 (m, 12H) ppm

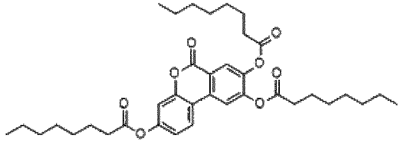

### Example 57: [8,9-di(octanoyloxy)-6-oxo-benzo[c]chromen-3-yl] octanoate

To a solution of 3,8,9-trihydroxybenzo[c]chromen-6-one (0.3 g) in acetonitrile (10 mL) was added K₂CO₃ (0.68 g) followed by octanoyl chloride (0.8 g). The resulting mixture was stirred at 50 °C for 24 hours. Additional octanoyl chloride (0.8 g) was added and the mixture was stirred at 50 °C for 12 hours. The reaction mixture was quenched by addition of water (10 mL) and extracted three times with ethyl acetate (10 mL). The combined organic layers were dried over Na₂SO₄, filtered and concentrated under reduced pressure. The residue was purified by column chromatography (SiO₂, petroleum ether / ethyl acetate, 9:1 to 1:1) to give [8,9-di(octanoyloxy)-6-oxo-benzo[c]chromen-3-yl]octanoate (0.45 g, 55.5%) as a white solid. ¹H NMR (400 MHz, CDCl₃): δ 8.186 (s, 1H), 7.926 (d, 1H), 7.908 (s, 1H), 7.157 - 7.096 (m, 2H), 2.621 - 2.573 (m, 6H), 1.79 - 1.75 (6H, m), 1.5 - 1.25 (m, 24H), 0.916 - 0.878 (m, 9H) ppm

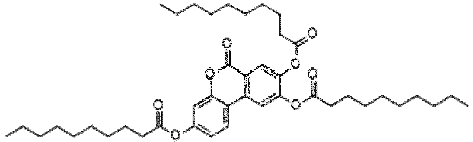

### Example 58: [8,9-di(decanoyloxy)-6-oxo-benzo[c]chromen-3-yl] decanoate

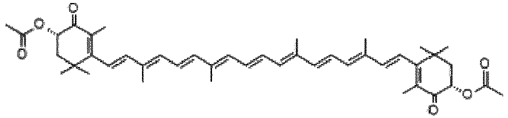

### Example 59: [(1S)-4-[(1E,3E,5E,7E,9E,11E,13E,15E,17E)-18-[(4S)-4-acetoxy-2,6,6-trimethyl-3-oxo-cyclohexen-1-yl]-3,7,12,16-tetramethyl-octadeca-1,3,5,7,9,11,13,15,17-nonaenyl]-3,5,5-trimethyl-2-oxo-cyclohex-3-en-1-yl] acetate

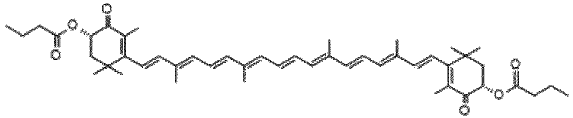

### Example 60: [(1S)-4-[(1E,3E,5E,7E,9E,11E,13E,15E,17E)-18-[(4S)-4-butanoyloxy-2,6,6-trimethyl-3-oxo-cyclohexen-1-yl]-3,7,12,16-tetramethyl-octadeca-1,3,5,7,9,11,13,15,17-nonaenyl]-3,5,5-trimethyl-2-oxo-cyclohex-3-en-1-yl] butanoate

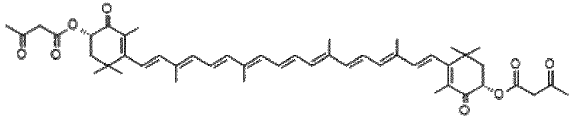

### Example 61: [(1S)-3,5,5-trimethyl-2-oxo-4-[(1E,3E,5E,7E,9E,11E,13E,15E,17E)-3,7,12,16-tetramethyl-18-[(4S)-2,6,6-trimethyl-3-oxo-4-(3-oxobutanoyloxy)cyclohexen-1-yl]octadeca-1 ,3,5,7,9,11,13,15,17-nonaenyl]cyclohex-3-en-1-yl] 3-oxobutanoate

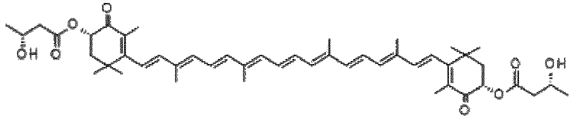

### Example 62: [(1S)-4-[(1E,3E,5E,7E,9E,11E,13E,15E,17E)-18-[(4S)-4-[(3R)-3-hydroxybutanoyl]oxy-2,6,6-trimethyl-3-oxo-cyclohexen-1-yl]-3,7,12,16-tetramethyl-octadeca-1,3,5,7,9,11,13,15,17-nonaenyl]-3,5,5-trimethyl-2-oxo-cyclohex-3-en-1-yl] (3R)-3-hydroxybutanoate

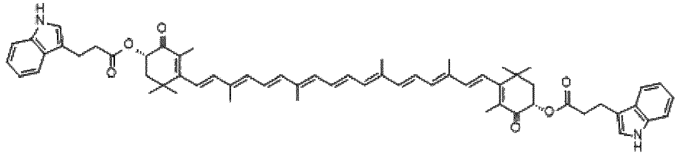

### Example 63: [(1S)-4-[(1E,3E,5E,7E,9E,11E,13E,15E,17E)-18-[(4S)-4-[3-(1H-indol-3-yl)propanoyloxy]-2,6,6-trimethyl-3-oxo-cyclohexen-1-yl]-3,7,12,16-tetramethyl-octadeca-1,3,5,7,9,11,13,15,17-nonaenyl]-3,5,5-trimethyl-2-oxo-cyclohex-3-en-1-yl] 3-(1H-indol-3-yl)propanoate

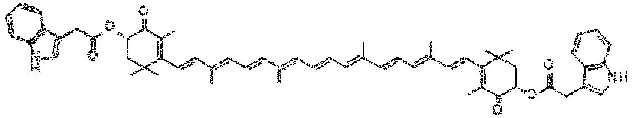

### Example64: [(1S)-4-[(1E,3E,5E,7E,9E,11E,13E,15E,17E)-18-[(4S)-4-[2-(1H-indol-3-yl)acetyl]oxy-2,6,6-trimethyl-3-oxo-cyclohexen-1-yl]-3,7,12,16-tetramethyl-octadeca-1,3,5,7,9,11,13,15,17-nonaenyl]-3,5,5-trimethyl-2-oxo-cyclohex-3-en-1-yl] 2-(1H-indol-3-yl)acetate

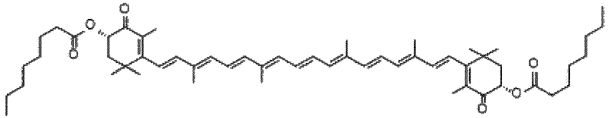

### Example 65: [(1S)-3,5,5-trimethyl-2-oxo-4-[(1E,3E,5E,7E,9E,11E,13E,15E,17E)-3,7,12,16-tetramethyl-18-[(4S)-2,6,6-trimethyl-4-octanoyloxy-3-oxo-cyclohexen-1-yl]octadeca-1,3,5,7,9,11,13,15,17-nonaenyl]cyclohex-3-en-1-yl] octanoate

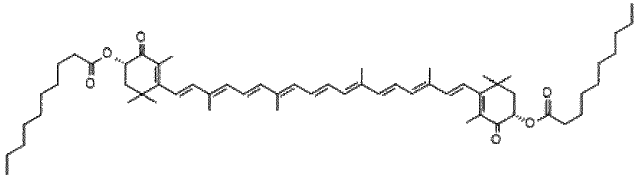

### Example 66: [(1S)-4-[(1E,3E,5E,7E,9E,11E,13E,15E,17E)-18-[(4S)-4-decanoyloxy-2,6,6-trimethyl-3-oxo-cyclohexen-1-yl]-3,7,12,16-tetramethyl-octadeca-1,3,5,7,9,11,13,15,17-nonaenyl]-3,5,5-trimethyl-2-oxo-cyclohex-3-en-1-yl] decanoate

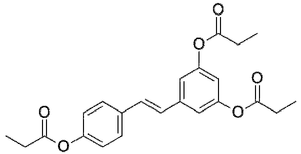

### Example 67: [4-[(E)-2-[3,5-di(propanoyloxy)phenyl]vinyl]phenyl] propanoate

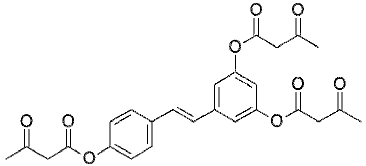

### Example 68: [4-[(E)-2-[3,5-bis(3-oxobutanoyloxy)phenyl]vinyl]phenyl] 3-oxobutanoate

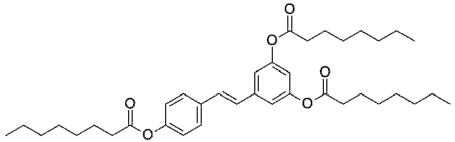

### Example 69: [4-[(E)-2-[3,5-di(octanoyloxy)phenyl]vinyl]phenyl] octanoate

To a mixture of 5-[(E)-2-(4-hydroxyphenyl)vinyl]benzene-1,3-diol (1 g) and K₂CO₃(1.8 g) in acetonitrile (30 mL) was added octanoyl chloride (2.14 g) at 25°C. The mixture was stirred at 25°C for 10 hours. Additional octanoyl chloride (1.42 g) was added and the mixture was stirred at 25°C for 10 hours. The reaction mixture was concentrated under reduced pressure and the residue was purified by reverse phase prep-HPLC (C18, water(0.05%HCI)-acetonitrile gradient) to give [4-[(E)-2-[3,5-di(octanoyloxy)phenyl]vinyl]phenyl] octanoate (0.53 g, 20%) as colorless oil. ¹H NMR (400 MHz, CDCl₃): δ 7.481 (m, 2H), 7.107 - 6.956 (m, 6H), 6.809, (m, 1H), 2.564 (m, 6H), 1.781 (m, 6H), 1.383 (m, 24H), 0.894 (m, 9H) ppm

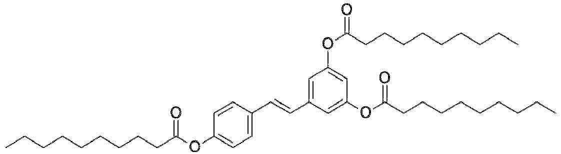

### Example 70: [4-[(E)-2-[3,5-di(decanoyloxy)phenyl]vinyl]phenyl] decanoate

To the solution of resveratrol (1 g) and K₂CO₃ (3.0 g) in acetonitrile (50 mL) was added decanoyl chloride (5.85 g) dropwise. Then the mixture was stirred at 25°C for 16 h. The mixture was filtered and concentrated in and the residue was purified by silica gel chromatography (petroleum ether / EtOAc, 10:1) and then prep-TLC (petroleum ether / EtOAc, 5:1) to give the product (0.062 g, 2%) as yellow oil. LCMS: 691.3 (M+H⁺) ¹H NMR (400 MHz, CDCl₃): δ 7.479 (m, 2H), 7.110 - 6.954 (m, 6H), 6.802, (m, 1H), 2.562 (m, 6H), 1.759 (m, 6H), 1.422 - 1.322 (m, 36H), 0.894 (m, 9H) ppm

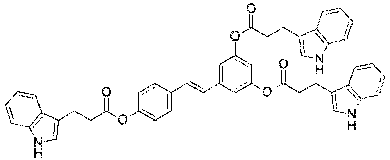

### Example 71: [4-[(E)-2-[3,5-bis[3-(1H-indol-3-yl)propanoyloxy]phenyl]vinyl]phenyl] 3-(1H-indol-3-yl)propanoate

The solution of resveratrol (1 g), 3-(1H-indol-3-yl)propanoic acid (2.74 g), EDCI (2.77 g) and 4-dimethylaminopyridine (1.77 g,) in DMF (100 mL) was stirred at 25°Cfor 10 hours. Water (100 mL) was added to the mixture and the aqueous phase was extracted three times with ethyl acetate (100 mL). The combined organic phase was washed with brine (100 mL), dried with anhydrous Na₂SO₄, filtered and concentrated in vacuum. The crude product was purified by silica gel chromatography (petroleum ether / ethyl acetate, 10:1 to 1:3) to give the product (0.5 g, 15%) as brown oil. LCMS: 742.3 (M+H⁺) ¹H NMR (400 MHz, CDCl₃): δ 10.851 (m, 3H), 7.57 - 7.55 (m, 5H), 7.34 - 7.35, (m, 3H), 7.21 - 6.98 (m, 15H), 6.71 (m, 1H), 3.100 - 3.064 (m, 6H), 2.965 - 2.928 (m, 6H) ppm

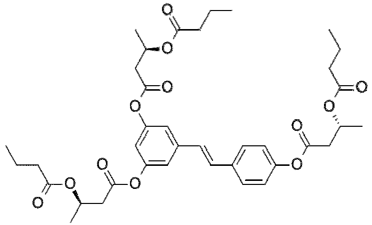

### Example 72: [4-[(E)-2-[3,5-bis[[(3R)-3-butanoyloxybutanoyl]oxy]phenyl]vinyl]phenyl] (3R)-3-butanoyloxy-butanoate

### Step 1: Benzyl (3R)-3-hydroxybutanoate

To a solution of [(3R)-3-hydroxybutanoyl]oxysodium (50 g) in DMF (500 mL) was added dropwise bromomethylbenzene (67.8 g) at 25 °C. Then the mixture was stirred at 60 °C for 12 h. Water (800 mL) was added to the reaction mixture, and extracted with EtOAc (550 mL). The organic layer was washed with brine (230 mL) and dried over Na₂SO₄, filtered and concentrated. The residue was purified by flash silica gel chromatography (petroleum ether/ethyl acetate = 100/1 to 40/1) to give benzyl (3R)-3-hydroxybutanoate (57 g, 66.6%) as colorless oil which was used directly in the next step.

### Step 2: Benzyl (3R)-3-butanoyloxybutanoate

To a solution of pyridine (55.7 g) in CH₂Cl₂ (570 mL) was added benzyl (3R)-3-hydroxybutanoate (57 g) and 4-dimethylaminopyridine (1.15 g) at 25 °C. Butanoyl chloride (43.8 g) was added dropwise to the mixture under N₂ and then stirred at 25°C for 12 h. The mixture was concentrated, the residue was diluted with EtOAc (300 mL) and the organic layer was washed with H₂O (550 mL), brine (270 mL), dried over Na₂SO₄, filtered and concentrated. The residue was purified by flash silica gel chromatography (petroleum ether / ethyl acetate, 100:1 to 70:1) to give benzyl (3R)-3-butanoyloxybutanoate (54 g, 62.6%) as a colorless oil.
LCMS: 265.1 (M+H⁺)

### Step 3:

To a suspension of Pd/C 10% (9 g) in EtOAc (1300 mL) was added benzyl (3R)-3-butanoyloxybutanoate (54 g) at 25 °C. The reaction mixture was stirred at 25 °C under H₂ (15 Psi) for 4 h. The mixture was filtered and concentrated to give (3R)-3-butanoyloxybutanoic acid (30 g) as colorless oil.

### Step 4: [4-[(E)-2-[3,5-bis[[(3R)-3-butanoyloxybutanoyl]oxy]phenyl]vinyl]phenyl] (3R)-3-butanoyloxy-butanoate

To a solution of 5-[(E)-2-(4-hydroxyphenyl)vinyl]benzene-1,3-diol (0.25 g) and (3R)-3-butanoyloxybutanoic acid (0.76 g) in CH₂Cl₂ (7.5 mL) was added *N,N*'-dicyclohexylcarbodiimide (0.29 g) in CH₂Cl₂ (5 mL). 4-dimethylaminopyridine (0.040 g) was added to the mixture at 25 °C, and the mixture was stirred for 12 h. The mixture was cooled to 0 °C, petroleum ether (10 mL) was added and the mixture was stirred for 15 min, then filtered and concentrated. The residue was dissolved with EtOAc (5 mL), washed with 0.5 N HCl (18 mL) and brine (8 mL), dried over Na₂SO₄, filtered and concentrated. The residue was purified by reverse phase prep-HPLC (C18; water (0.05%HCl)-acetonitrile gradient) to give [4-[(E)-2-[3,5-bis[[(3R)-3-butanoyloxybutanoyl]oxy]phenyl]vinyl]phenyl] (3R)-3-butanoyloxy-butanoate (0.060 g, 7%) as a colorless oil. LCMS: 697.4 (M+H⁺) ¹H NMR (400 MHz, CDCl₃): δ 7.494 (m,2H), 7.12 - 7.042 (m, 6H), 6.824 (m, 1H), 5.428, (m, 3 H), 2.909 - 2.785 (m, 6H), 2.303 (m, 6H), 1.696 - 1.658 (m, 6H), 1.527 (d, 9H), 0.956 (t, 9H) ppm

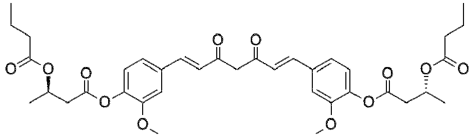

### Example 73: [4-[(1E,6E)-7-[4-[(3R)-3-butanoyloxybutanoyl]oxy-3-methoxy-phenyl]-3,5-dioxo-hepta-1,6-dienyl]-2-methoxy-phenyl] (3R)-3-butanoyloxybutanoate

### Step 1: [(1R)-3-chloro-1-methyl-3-oxo-propyl] butanoate

To a solution of (3R)-3-butanoyloxybutanoic acid (1 g) in CH₂Cl₂ (10 mL) was added dropwise (COCI)₂ (3.64 g) under N₂. Then DMF (0.004 g, 4.4 µL) was added to the mixture. The reaction was stirred at 25 °C for 12 h. The mixture was concentrated under reduced pressure to give [(1R)-3-chloro-1-methyl-3-oxo-propyl] butanoate (0.80 g) as yellow oil which was used directly in the next step.

### Step 2:

To a solution of curcumin (0.05 g) in acetonitrile (1 mL) was added Na₂CO₃ (0.043 g,) at 25 °C. (1R)-3-chloro-1-methyl-3-oxo-propyl] butanoate (0.13 g) was added dropwise to the mixture. The reaction was stirred at 25 °C for 5 h. The reaction mixture was filtered and concentrated and the residue was purified by prep-TLC (petroleum ether / ethyl acetate, 1:1) to give [4-[(1E,6E)-7-[4-[(3R)-3-butanoyloxybutanoyl]oxy-3-methoxy-phenyl]-3,5-dioxo-hepta-1,6-dienyl]-2-methoxy-phenyl] (3R)-3-butanoyloxybutanoate as a brown oil (0.04 g, 41%). LCMS: 703.2 (M+Na⁺) ¹H NMR (400 MHz, CDCl₃): δ 7.625 (d, 2H), 7.175 - 7.049 (m, 6H), 6.574 (m, 2H), 5.867 (s, 1H), 5.464 - 5.384 (m, 2H), 3.872 (s, 6H), 2.960 - 2.903 (m, 2 H), 2.816 - 2.778 (m, 2 H), 2.300 (m, 4H), 1.69 (m, 4H), 1.410 (d, 6H), 0.958 (t, 6H) ppm

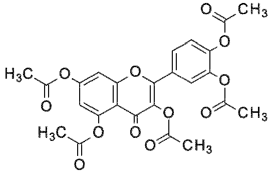

### Example 74: [2-acetoxy-4-(3,5,7-triacetoxy-4-oxo-chromen-2-yl)phenyl] acetate

To a mixture of 2-(3,4-dihydroxyphenyl)-3,5,7-trihydroxy-chromen-4-one (1 g) and acetic anhydride (2.36 g) in THF (40 mL) was added K₂CO₃ (3.2 g) at 25°C, then the mixture was stirred at 55 °C for 12 h. Additional acetic anhydride was added (3 eq) and the mixture and stirred for another 3 h. The reaction mixture was concentrated in vacuum and purified by reverse phase prep-HPLC (C18; water(0.05%HCI)-acetonitrile gradient) to give [2-acetoxy-4-(3,5,7-triacetoxy-4-oxo-chromen-2-yl)phenyl] acetate (0.837 g, 49%) as a white solid. LCMS: 513.2 (M+H⁺) ¹H NMR (400 MHz, CDCl₃): δ 7.742 - 7.703 (m, 2H), 7.373 -7.346 (m, 2H), 6.888 (s, 1H), 2.443, (s, 3H), 2.356 (s, 6H), 2.350(s, 6H) ppm

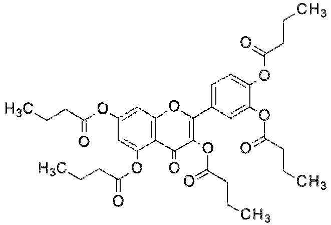

### Example 75: [2-butanoyloxy-4-[3,5,7-tri(butanoyloxy)-4-oxo-chromen-2-yl]phenyl]butanoate

To a mixture of 2-(3,4-dihydroxyphenyl)-3,5,7-trihydroxy-chromen-4-one (1 g) and butanoyl chloride (3.53 g) in THF (40 mL) was added triethylamine (3.35 g) at 25°C, then the mixture was stirred at 55°C for 12 h. The reaction mixture was concentrated in vacuum and purified by reverse phase prep-HPLC (C18, water(0.05%HCI)-acetonitrile gradient) to give [2-butanoyloxy-4-[3,5,7-tri(butanoyloxy)-4-oxo-chromen-2-yl]phenyl]butanoate (1.13 g, 52% yield) as a colorless solid. LCMS: 653.3 (M+H⁺) ¹H NMR (400 MHz, CDCl₃): δ 7.666 - 7.608 (m, 2H), 7.292 - 7.210 (m, 2H), 6.880 (s, 1H), 2.542 (t, 2H), 2.535 - 2.484 (m, 8H), 1.753 (m, 10H), 1.020 -0.997 (m, 12H), 0.949 (t, 3H) ppm

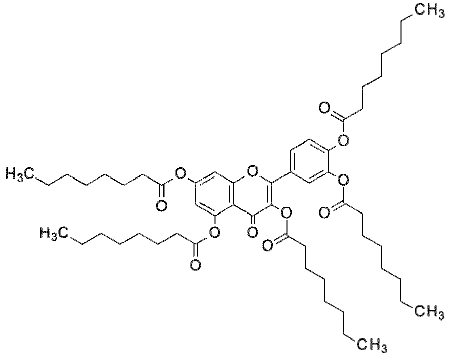

### Example 76: [2-octanoyloxy-4-[3,5,7-tri(octanoyloxy)-4-oxo-chromen-2-yl] phenyl] octanoate

To a mixture of 2-(3,4-dihydroxyphenyl)-3,5,7-trihydroxy-chromen-4-one (0.32 g) and octanoyl chloride (1.72 g) in THF (20 mL) was added triethylamine (1.07 g) at 25°C. Then the mixture was stirred at 55 °C for 12 h. A portion of the solvent was removed in vacuum and the precipitate was collected by filtration to give [2-octanoyloxy-4-[3,5,7-tri(octanoyloxy)-4-oxo-chromen-2-yl] phenyl] octanoate (0.20 g, 20%) as a white solid. ¹H NMR (400 MHz, CDCl₃): δ 7.709 - 7.655 (m, 2H), 7.329 - 7.301 (m, 2H), 6.837 (s, 1H), 2.723 (t, 2H), 2.612 - 2.539 (m, 8H), 1.751 (m, 10H), 1.412 -1.309 (m, 40H), 0.896 (m, 15H) ppm

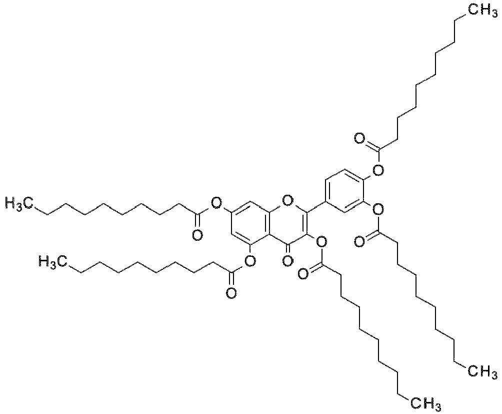

### Example 77: [2-decanoyloxy-4-[3,5,7-tris(decanoyloxy)-4-oxo-chromen-2-yl] phenyl] decanoate

To a mixture of 2-(3,4-dihydroxyphenyl)-3,5,7-trihydroxy-chromen-4-one (1 g) and decanoyl chloride (6.31 g) in THF (50 mL) was added triethylamine (3.35 g) at 25 °C, then the mixture was stirred at 55°C for 12 h. A portion of the solvent was removed in vacuum and the precipitate was collected by filtration to give [2-decanoyloxy-4-[3,5,7-tris(decanoyloxy)-4-oxo-chromen-2-yl] phenyl] decanoate (2.47 g, 69%) as a white solid. ¹H NMR (400 MHz, CDCl₃): δ 7.772 - 7.669 (m, 2H), 7.343 - 7.321 (m, 2H), 6.685 (s, 1H), 2.736 (t, 2H), 2.610 - 2.551 (m, 8H), 1.762 (m, 10H), 1.557 - 1.295 (m, 50H), 0.899 (m, 15H) ppm

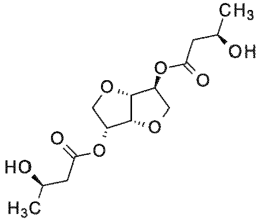

### Example 78: [(3S,3aR,6R,6aR)-6-[(3R)-3-hydroxybutanoyl]oxy-2,3,3a,5,6,6a-hexahydrofuro[3,2-b] furan-3-yl] (3R)-3-hydroxybutanoate

### Step 1: Methyl (3R)-3-benzyloxybutanoate

To a solution of methyl (3R)-3-hydroxybutanoate (10 g) and benzyl 2,2,2-trichloroethanimidate (23.6 g) in CH₂Cl₂ (300 mL) was added CF₃SO₃H (7.2 g) and the mixture was stirred at 25°C for 12 h. The reaction mixture was concentrated under reduced pressure to remove CH₂Cl₂. The residue was diluted with water (100 mL), extracted with four times with ethyl acetate (90 mL) and concentrated under reduced pressure. The residue was purified by column chromatography (SiO₂, petroleum ether / ethyl acetate, 20:1 to 9:1) to give methyl (3R)-3-benzyloxybutanoate (10 g, 56.7%) as colorless oil. LCMS: 209.1 (M+H⁺)

### Step 2: (3R)-3-benzyloxybutanoic acid

To a solution of methyl (3R)-3-benzyloxybutanoate (10 g) in methanol (100 mL) and water (50 mL) was added NaOH (2.88 g,). The mixture was stirred at 25 °C for 12 h. The residue was diluted with water (30 mL), extracted with ethyl acetate (90 mL) and concentrated under reduced pressure to give (3R)-3-benzyloxybutanoic acid (3 g, 32%) as colorless oil.

### Step 3: (3R)-3-benzyloxybutanoyl chloride

To a mixture of (3R)-3-benzyloxybutanoic acid (2.65 g) in CH₂Cl₂ (30 mL) was added (COCl)₂ (10.4 g) and DMF (0.099 g) in one portion at 25°C under N₂. Then the mixture was stirred at 25 °C for 12 h. The reaction mixture was concentrated under reduced pressure to give the crude (3R)-3-benzyloxybutanoyl chloride (2.55 g) as colorless oil which was used in the next step without further purification.

### Step 4: [(3S,3aR,6R,6aR)-6-[(3R)-3-benzyloxybutanoyl]oxy- 2,3,3a,5,6,6a-hexahydrofuro[3,2-b]furan-3- yl](3R)-3-benzyloxybutanoate

To a mixture of (3R,3aR,6S,6aR)-2,3,3a,5,6,6a-hexahydrofuro[3,2-b]furan-3,6-diol (0.37 g) and Na₂CO₃ (1.08 g) in acetonitrile (10 mL) was added (3R)-3-benzyloxybutanoyl chloride (2.23 g), then the mixture was stirred 12 h at 25°C. The reaction mixture was concentrated under reduced pressure and the residue was purified by prep-HPLC to give [(3S,3aR,6R,6aR)-6-[(3R)-3-benzyloxybutanoyl]oxy-2,3,3a,5,6,6a-hexahydrofuro[3,2-b]furan-3- yl](3R)-3-benzyloxybutanoate (0.27 g, 18%) as colorless oil. LCMS: 499.3 (M+H⁺)

### Step 5: [(3S,3aR,6R,6aR)-6-[(3R)-3-hydroxybutanoyl]oxy-2,3,3a,5,6,6a-hexahydrofuro[3,2-b] furan-3-yl] (3R)-3-hydroxybutanoate

To a solution of [(3S,3aR,6R,6aR)-6-[(3R)-3-benzyloxybutanoyl]oxy-2,3,3a,5,6,6a-hexahydrofuro[3,2-b]furan-3-yl](3R)-3-benzyloxybutanoate (0.27 g) in THF (5 mL) was added 10% Pd/C (0.5 g) under N₂. The suspension was degassed under vacuum and purged with H₂ several times. The mixture was stirred under H₂ (30 psi) at 25°C for 12 h. The reaction mixture was filtered and concentrated under reduced pressure. The residue was purified by column chromatography (SiO₂, petroleum ether / ethyl acetate, 1:1 to 0:1) to give [(3S,3aR,6R,6aR)-6-[(3R)-3-hydroxybutanoyl]oxy-2,3,3a,5,6,6a-hexahydrofuro[3,2-b] furan-3-yl] (3R)-3-hydroxybutanoate (0.089 g, 49%) as a colorless oil. ¹H NMR (400 MHz, CDCl₃): δ 5.524 (br, 1H), 5.203 (m, 1H), 4.486 (m, 1H), 4.500 (d, 1H), 4.412 (br, 2H), 3.991 - 3.949 (m, 3H), 3.868 (m, 1H), 2.95 (br, 1H), 2.7 (br, 1H), 2.541 - 2.460 (m, 4H), 1.251 (m, 6H) ppm

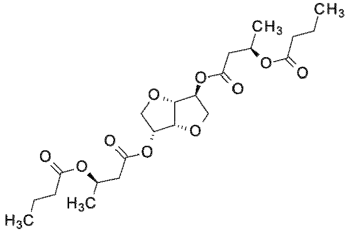

### Example 79: [(3S,3aR,6R,6aR)-6-[(3R)-3-butanoyloxybutanoyl]oxy-2,3,3a,5,6,6a-hexahydrofuro[3,2-b]furan-3-yl] (3R)-3-butanoyloxybutanoate

To a solution of (3R,3aR,6S,6aR)-2,3,3a,5,6,6a-hexahydrofuro[3,2-b]furan-3,6-diol (0.14 g) and Na₂CO₃ (0.30 g) in acetonitrile (3 mL) was added dropwise [(1R)-3-chloro-1-methyl-3-oxo-propyl] butanoate (0.92 g) under N₂ and the mixture was stirred at 25 °C for 12 h. The reaction mixture was filtered and concentrated. The residue was purified by column chromatography (SiO₂, petroleum ether / ethyl acetate, 100:1 to 50:1) to give [(3S,3aR,6R,6aR)-6-[(3R)-3-butanoyloxybutanoyl]oxy-2,3,3a,5,6,6a-hexahydrofuro[3,2-b]furan-3-yl] (3R)-3-butanoyloxybutanoate (0.063 g, 13%) as a brown oil. ¹H NMR (400 MHz, CDCl₃): δ 5.279 (m, 2H), 5.196 (m, 1H), 5.150 (m, 1H), 4.828 (t, 1H), 4.471 (m, 1H), 3.976 - 3.919 (m, 3H), 3.810, (m, 1H), 2.681 - 2.586 (m, 4H), 2.239 (m, 4H), 1.643 (m, 4H), 1.312 (m, 6H), 0.941 (t, 6H) ppm

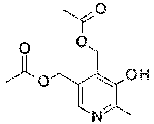

### Example 80: [4-(acetoxymethyl)-5-hydroxy-6-methyl-3-pyridyl] methyl acetate

To a solution of 4,5-bis(hydroxymethyl)-2-methyl-pyridin-3-ol (0.5 g) in CH₂Cl₂ (10 mL) was added triethylamine (0.63 g) and acetic anhydride (0.63 g) at 25°C. Then the mixture was stirred at 25 °C for 12 h. The reaction mixture was concentrated under reduced pressure and the residue was purified by prep-HPLC to give [4-(acetoxymethyl)-5-hydroxy-6-methyl-3-pyridyl] methyl acetate (0.036 g, 69%) as a white solid. LCMS: 254.1 (M+H⁺) ¹H NMR (400 MHz, CDCl₃): δ 8.224 (s, 1H), 8.122 (s, 1H), 5.222 (s, 2H), 5.194 (s, 2H), 2.529 (s, 3H), 2.141 (s, 3H), 2.108 (s, 3H) ppm

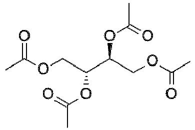

### Example 81: (2R, 3S)-butane-1,2,3,4-tetrayl tetraacetate

To a solution of (2R,3S)-butane-1,2,3,4-tetrol (0.5 g) and triethylamine (3.31 g) in CH₂Cl₂ (20 mL) was added Ac₂O (3.34 g). Then the mixture was stirred at 65°C for 16 h. The solvent was removed under reduced pressure and the residue was purified by column chromatography (SiO₂, petroleum ether / ethyl acetate, 0:1 to 20:1) to give (2R,3S)-butane-1,2,3,4-tetrayl tetraacetate (0.4 g, 34%) as a white solid. ¹H NMR (400 MHz, DMSO-d6): δ 5.178 - 5.134 (m, 2H), 4.257 (m, 2H), 4.165 (m, 2H), 2.029 (s, 6H),1.995 (s, 6H) ppm

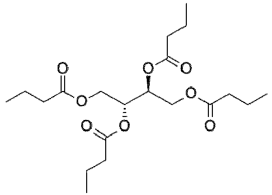

### Example 82: (2R,3S)-butane-1,2,3,4-tetrayl tetrabutyrate

To a solution of (2R,3S)-butane-1,2,3,4-tetrol (0.3 g) and K₂CO₃ (2.72 g) in acetonitrile (50 mL) was added butyryl chloride (2.09 g). Then the mixture was stirred at 60°C for 16 h. The solvent was removed under reduced pressure. The residue was purified by column chromatography (SiO₂, petroleum ether / ethyl acetate, 1:0 to 20:1) followed by prep-TLC (petroleum ether / ethyl acetate, 5:1) to give (2R,3S)-butane-1,2,3,4-tetrayl tetrabutyrate (0.1 g, 9.9%) as colorless oil. ¹H NMR (400 MHz, DMSO-d6): δ 5.197 - 5.153 (m, 2H), 4.268 (m, 2H), 4.120 (m, 2H), 2.296 - 2.206 (m, 8H), 1.517 - 1.463 (m, 8H), 0.866 - 0.818 (m, 12H) ppm

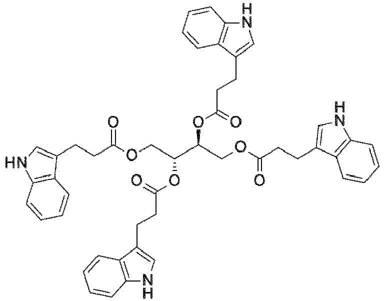

### Example 83: [(2R,3S)-2,3,4-tris[3-(1H-indol-3-yl)propanoyloxy]butyl] 3-(1H-indol-3-yl)propanoate

To a solution of (2R,3S)-butane-1,2,3,4-tetrol (0.3 g), *N,N*'-dicyclohexylcarbodiimide (3.05 g) and 4-dimethylaminopyridine (0.3 g) in DMF (10 mL) and CH₂Cl₂ (30 mL) was added 3-(1H-indol-3-yl) propanoic acid (2.79 g). The mixture was stirred at 25°C for 16 h. The solvent was removed in vacuum. The crude product was purified by reverse phase prep-HPLC (C18, water(0.05%HCI)-acetonitrile gradient) to give [(2R,3S)-2,3,4-tris[3-(1H-indol-3-yl)propanoyloxy]butyl] 3-(1H-indol-3-yl)propanoate (0.35 g 17%) as a yellow solid. LCMS: 807.3 (M+H⁺) ¹H NMR (400 MHz, DMSO-d6): δ 10.787 (s, 4H), 7.483 (M, 4H), 7.310 (m, 4H), 7.093 -6.937 (m, 12H), 5.243 (m, 2H), 4.278 (m, 2H), 4.136 (m, 2H), 2.961 - 2.910 (m, 8H), 2.679 - 2.601 (m, 8H) ppm

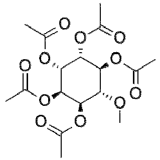

### Example 84 (1R,2R,3R,4S,5R,6R)-2,3,4,6-tetrakis(acetyloxy)-5-methoxycyclohexyl acetate

To a solution of (1R,2S,3R,4S,5S,6S)-6-methoxycyclohexane-1,2,3,4,5-pentol (0.3 g) in pyridine (10 mL) was added Ac₂O (1.26 g) and 4-dimethylaminopyridine (0.019 g). The mixture was stirred at 25°C for 16 h. The solvent was removed under reduced pressure and the residue was purified by column chromatography (SiO₂, petroleum ether / ethyl acetate, 40:1 to 10:1). The product was further purified by prep-TLC (petroleum ether: ethyl acetate, 5:1) to give (1R,2R,3R,4S,5R,6R)-2,3,4,6-tetrakis(acetyloxy)-5-methoxycyclohexyl acetate (0.16 g, 26%) as colorless oil. ¹H NMR (400 MHz, CDCl₃): δ 5.402 - 5.337 (m, 3H), 5.239 - 5.204 (m, 2H), 3.633 (m, 1H), 3.477 (s, 3H), 2.174 (s, 3H), 2.171 (s, 3H), 2.102 (s, 3H), 2.056 (s, 3H), 1.998 (s, 3H) ppm

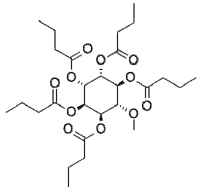

### Example 85: (1R,2R,3R,4S,5R,6R)-2,3,4,6-tetrakis(butanoyloxy)-5-methoxycyclohexyl butanoate

To a solution of (1R,2S,3R,4S,5S,6S)-6-methoxycyclohexane-1,2,3,4,5-pentol (0.15 g) and 4-dimethylaminopyridine (0.009 g) in Pyridine (5 mL) was added butyric anhydride (1.22 g). Then the mixture was stirred at 25°C for 16 h. The solvent was removed under reduced pressure and the residue was purified by column chromatography (SiO₂, petroleum ether/ ethyl acetate, 50:1 to 10:1). Then the product was further purified by prep-TLC (petroleum ether: ethyl acetate, 5:1) to give (1R,2R,3R,4S,5R,6R)-2,3,4,6-tetrakis(butanoyloxy)-5-methoxycyclohexyl butanoate (0.058 g, 14%) as a colorless oil. ¹H NMR (400 MHz, DMSO-d6): δ 5.248 - 5.212 (m, 3H), 5.103 - 5.005 (m, 2H), 3.666 (m, 1H), 3.366 (s, 3H), 2.434 - 2.154 (m, 10 h), 1.600 - 1.537 (m, 10H), 0.954 - 0.819 (m, 15 H) ppm

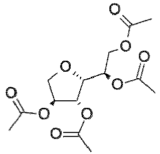

### Example 86: [(2R)-2-acetoxy-2-[(2R,3R,4S)-3,4-diacetoxytetrahydrofuran-2-yl]ethyl] acetate

To a mixture of (2R,3R,4S)-2-[(1R)-1,2-dihydroxyethyl]tetrahydrofuran-3,4-diol (0.3 g) in CH₂Cl₂ (10 mL) was added acetic anhydride (1.12 g) and triethylamine (1.11 g) in one portion at 25°C under N₂. The mixture was stirred at 25 °C for 12 h. The reaction mixture was concentrated under reduced pressure and the residue was purified by reverse phase prep-HPLC (C18, water(0.05%HCI)-acetonitrile gradient) to give [(2R)-2-acetoxy-2-[(2R,3R,4S)-3,4-diacetoxytetrahydrofuran-2-yl]ethyl] acetate (0.076 g, 12% yield) as a colorless oil. ¹H NMR (400 MHz, CDCl₃): δ 5.357 (m, 1H), 5.122 (m, 1H), 5.021 (m, 1H), 5.017 (m, 1H), 4.220 - 4.131 (m, 2H), 4.061 (m, 1H), 3.754 (m, 1H), 2.111 (s, 3H), 2.084 (s, 3H), 2.073 (s, 3H), 2.012 (s, 3H) ppm

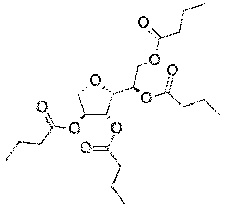

### Example 87: [(2R)-2-butanoyloxy-2-[(2R,3R,4S)-3,4-di (butanoyloxy)tetrahydrofuran-2-yl]ethyl] butanoate

To a mixture of (2R,3R,4S)-2-[(1R)-1,2-dihydroxyethyl]tetrahydrofuran-3,4-diol (0.3 g) in acetonitrile (10 mL) was added butanoyl chloride (1.17 g) and K₂CO₃(1.52 g) in one portion at 25°C under N₂. The mixture was stirred at 25°C for 12 h. The reaction mixture was concentrated under reduced pressure and the residue was purified by reverse phase prep-HPLC (C18, water (0.225% formic acid)-acetonitrile gradient) to give [(2R)-2-butanoyloxy-2-[(2R,3R,4S)-3,4-di (butanoyloxy)tetrahydrofuran-2-yl]ethyl] butanoate (0.067 g, 8%) as a yellow oil. ¹H NMR (400 MHz, CDCl₃): δ 5.376 (m, 1H), 5.212 (m, 1H), 5.063 (m, 1H), 4.591 (m, 1H), 4.272 - 4.112 (m, 3H), 3.787 (m, 1H), 2.239 - 2.228 (m, 8H), 1.682 - 1.608 (m, 8H), 0.988 -0.926 (m, 12H) ppm

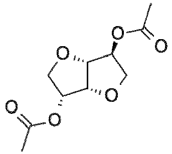

### Example 88: [(3S,3aR,6R,6aR)-6-acetoxy-2,3,3a,5,6,6a-hexahydrofuro[3,2-b] furan-3-yl] acetate

To a mixture of (3R,3aR,6S,6aR)-2,3,3a,5,6,6a-hexahydrofuro[3,2-b]furan-3,6-diol (1 g) in pyridine (10 mL) was added acetic anhydride (2.79 g) in one portion at 25°C under N₂. Then mixture was stirred at 25°C for 12 h. The reaction mixture was concentrated under reduced pressure and the residue was purified by column chromatography (SiO₂, petroleum ether / ethyl acetate, 50:1 to 10:1 to give [(3S,3aR,6R,6aR)-6-acetoxy-2,3,3a,5,6,6a-hexahydrofuro[3,2-b] furan-3-yl] acetate (0.871 g, 55%) as a colorless oil. ¹H NMR (400 MHz, CDCl₃):δ 5.177 - 5.119 (m, 2H), 4.822 (m, 1H), 4.479 (m, 1H), 3.986 - 3.921 (m, 3H), 3.798 (m, 1H), 2.069 (s, 3H), 2.098 (s, 3H) ppm

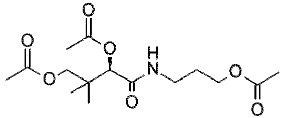

### Example 89: 3-[[(2R)-2,4-diacetoxy-3,3-dimethyl-butanoyl] amino]propyl acetate

To a solution of (2R)-2,4-dihydroxy-N-(3-hydroxypropyl)-3,3-dimethyl-butanamide (0.2 g) in pyridine (10 mL) was added Ac₂O (0.497 g) at 25 °C under N₂ and the mixture was stirred for 12 h at 25 °C. The mixture was concentrated under reduced pressure and the residue was purified by column chromatography (SiO₂, petroleum ether / ethyl acetate, 75:1 to 1:1) to give 3-[[(2R)-2,4-diacetoxy-3,3-dimethyl-butanoyl] amino]propyl acetate (0.21g, 62%) as a colorless oil. ¹H NMR (400 MHz, CDCl₃): δ 6.426 (br s, 1H), 5.007 (s, 1H), 4.145 - 4.048 (m, 4H), 3.857 (m, 1H), 3.322 - 3.278 (m, 2H), 2.176 (s, 3H), 2.077 (s, 3H), 2.071 (s, 3H), 1.879 - 1.798 (m, 2H), 1.087 (s, 3H), 1.037 (s, 3H) ppm

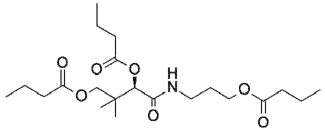

### Example 90: 3-[[(2R)-2,4-di(butanoyloxy)-3,3-dimethyl-butanoyl]amino] propyl butanoate

To a solution of (2R)-2,4-dihydroxy-N-(3-hydroxypropyl)-3,3-dimethyl-butanamide (0.2 g) in pyridine (10 mL) was added butanoyl butanoate (0.925 g) under N₂ and the mixture was stirred at 25 °C for 12 h. The reaction mixture was concentrated and the residue was purified by column chromatography (SiO₂, petroleum ether / ethyl acetate, 50:1 to 0:1) to give 3-[[(2R)-2,4-di(butanoyloxy)-3,3-dimethylbutanoyl]amino] propyl butanoate (0.287 g, 67%) as a colorless oil. ¹H NMR (400 MHz, CDCl₃): δ 6.416 (br t, 1H), 5.024 (s, 1H), 4.156 - 4.126 (m, 2H), 4.038 (m, 1H), 3.877 (m, 1H), 3.291 (m, 2H), 2.403 (m, 2H), 2.291 (m, 4H), 1.85 - 1.629 (m, 10H), 1.038 (s, 3H), 1.035 (s, 3H), 0.987 - 0.931 (m, 9H) ppm

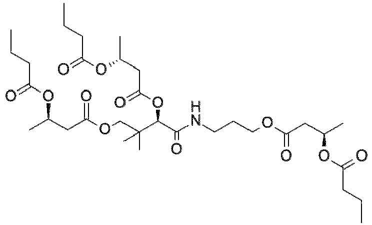

### Example 91: 3-[[(2R)-2,4-bis[[(3R)-3-butanoyloxybutanoyl]oxy]-3,3- dimethyl-butanoyl]amino]propyl (3R)-3-butanoyloxybutanoate

To a solution of (2R)-2,4-dihydroxy-N-(3-hydroxypropyl)-3,3-dimethyl-butanamide (0.2 g) and Na₂CO₃ (0.413 g) in acetonitrile (4 mL) was added [(1R)-3-chloro-1-methyl-3-oxo-propyl] butanoate (1.07 g) under N₂ and the mixture was stirred at 25 °C for 12 h. The reaction mixture was filtered and the filtrate was concentrated under reduced pressure. The residue was purified by column chromatography (SiO₂, petroleum ether / ethyl acetate, 80:1 to 3:1) to give 3-[[(2R)-2,4-bis[[(3R)-3-butanoyloxybutanoyl]oxy]-3,3-dimethyl-butanoyl]amino]propyl (3R)-3-butanoyloxybutanoate (0.11 g, 16%) as a colorless oil. ¹H NMR (400 MHz, CDCl₃): δ 6.664 (br t, 1H), 5.321 - 5.261 (m, 3H), 4.889 (s, 1H), 4.143 - 4.113 (m, 2H), 4.048 M, 1H), 3.823 (m, 1H), 3.302 (m 2H), 2.8 - 2.45 (m, 6H), 2.238 (m, 6H), 1.8 (m, 2H), 1.602 (m, 6H), 1.293 (m, 9H), 1.054 (s, 3H), 1.017 (s, 3H), 0.924 (m, 9H) ppm

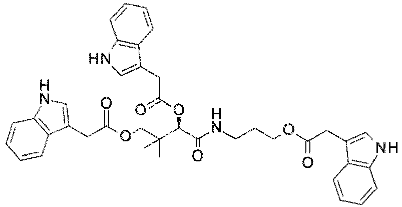

### Example 92: 3-[[(2R)-2,4-bis[[2-(1H-indol-3-yl)acetyl]oxy]-3,3-dimethyl-butanoyl]amino]propyl 2-(1H-indol-3-yl)acetate

To a solution of (2R)-2,4-dihydroxy-N-(3-hydroxypropyl)-3,3-dimethyl-butanamide (0.1 g), *N,N'-*dicyclohexylcarbodiimide (0.35 g), 4-dimethylaminopyridine (0.03 g) in THF (4 mL) was added 2-(1H-indol-3-yl)acetic acid (0.298 g) under N₂ and the mixture was stirred at 25 °C for 12 h. The mixture reaction was filtered and concentrated under reduced pressure. The residue was purified by prep-HPLC (C18, water (10 mM NH₄HCO₃)-acetonitrile gradient) to give 3-[[(2R)-2,4-bis[[2-(1H-indol-3-yl)acetyl]oxy]-3,3-dimethyl-butanoyl]amino]propyl 2-(1H-indol-3-yl)acetate (0.26 g, 78%) as an off-white solid. LCMS: 677.3 (M+H⁺) ¹H NMR (400 MHz, DMSO-d6): δ 10.945 (s, 3H), 7.947 (m, 1H), 7.496 (m, 3H), 7.349 (m, 3H), 7.262 (m, 3H), 7.071 (m, 3H), 6.968 (m, 3H), 4.737 (s, 1H), 4.006 (m, 2H), 3.847 (m, 4H), 3.736 (m, 4H), 3.136 - 3.088 (m, 2H), 1.697 (m, 2H), 0.889 (s, 3H), 0.859 (s, 3H) ppm

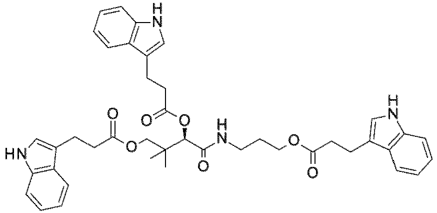

### Example 93: 3-[[(2R)-2,4-bis[3-(1H-indol-3-yl)propanoyloxy]-3,3-dimethyl-butanoyl]amino]propyl 3-(1H-indol-3-yl)propanoate

To a solution of (2R)-2,4-dihydroxy-N-(3-hydroxypropyl)-3,3-dimethyl-butanamide (0.2 g), *N,N'-*dicyclohexylcarbodiimide (0.70 g) and 4-dimethylaminopyridine (0.060 g) in THF (4 mL) was added 3-(1H-indol-3-yl) propanoic acid (0.645 g) and the mixture was stirred at 25 °C for 12 h. The reaction mixture was filtered and concentrated under reduced pressure. The residue was purified by reverse phase prep-HPLC (C18, water (10 mM NH₄HCO₃)-acetonitrile gradient) to give 3-[[(2R)-2,4-bis[3-(1H-indol-3-yl)propanoyloxy]-3,3-dimethyl-butanoyl]amino]propyl 3-(1H-indol-3-yl)propanoate (0.307 g, 43%) as an off-white solid. LCMS: 719.2 (M+H⁺) ¹H NMR (400 MHz, DMSO-d6): δ 10.797(br s, 3H), 7.981 (m, 1H), 7.485 (m, 3H), 7.208 (m, 3H), 7.107 - 6.942 (m, 9H), 4.718 (s, 1H), 4.005 - 3.837 (m, 3H), 2.950 (m, 2H), 2.937 (m, 6H), 2.781 (m, 2H), 2.693 - 2.639 (m, 4H), 1.687 (m, 2 H), 0.892 (m, 6H) ppm

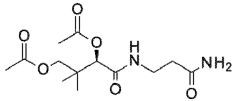

### Example 94: [(3R)-3-acetoxy-4-[(3-amino-3-oxo-propyl)amino]-2,2-dimethyl-4-oxo-butyl] acetate

To a solution of (2R)-N-(3-amino-3-oxo-propyl)-2,4-dihydroxy-3,3-dimethyl-butanamide (0.2 g) in pyridine (8 mL) was added Ac₂O (0.37 g) under N₂ and the mixture was stirred for 12 h at 25 °C. The mixture reaction was concentrated under reduced pressure. The residue was purified by column chromatography (SiO₂, petroleum ether / ethyl acetate, 50:1 to ethyl acetate / EtOH, 80:1) to give [(3R)-3-acetoxy-4-[(3-amino-3-oxo-propyl)amino]-2,2-dimethyl-4-oxo-butyl] acetate (0.135 g, 46%) as a colorless oil. ¹H NMR (400 MHz, CDCl₃): δ 6.885 (br t, 1H), 5.733 (br, 2H), 4.937(s, 1H), 4.033 (m, 1H), 3.847 (m, 1H), 3.600 - 3.500 (m, 2H), 2.500 - 2.389 (m, 2H), 2.155 (s, 3H), 2.075 (s, 3H), 1.071 (s, 3H), 1.035 (s, 3H) ppm

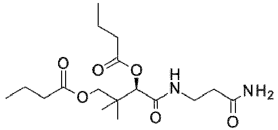

### Example 95: [(3R)-4-[(3-amino-3-oxo-propyl)amino]-3-butanoyloxy-2,2-dimethyl-4-oxo-butyl] butanoate

To solution of (2R)-N-(3-amino-3-oxo-propyl)-2,4-dihydroxy-3,3-dimethyl-butanamide (0.2 g) in pyridine (10 mL) was added butyric anhydride (0.58 g) under N₂ and the mixture was stirred at 25 °C for 12 h. The mixture reaction was concentrated under reduced pressure. The residue was purified by column chromatography (SiO₂, petroleum ether / ethyl acetate, 50:1 to 0:1) to give [(3R)-4-[(3-amino-3-oxo-propyl)amino]-3-butanoyloxy-2,2-dimethyl-4-oxo-butyl] butanoate (0.189 g,, 50%) as a colorless oil. ¹H NMR (400 MHz, CDCl₃): δ 6.825 (br t, 1H), 5.742 (br, 1H), 5.430 (br, 1H), 4.958 (s, 1H), 4.015 (m, 1H), 3.863 (m, 1H), 3.592 - 3.508 (m, 2H), 2.444 - 2.292 (m, 6H), 1.709 - 1.645 (m, 4H), 1.073 (s, 3H), 1.029 (s, 3H), 0.969 (t, 3H), (s, 3H), 0.950 (t, 3H) ppm

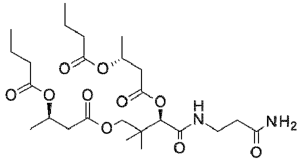

### Example 96: [(3R)-4-[(3-amino-3-oxo-propyl)amino]-3-[(3R)-3-butanoyloxybutanoyl]oxy-2,2-dimethyl-4-oxo-butyl] (3R)-3-butanoyloxybutanoate

To a solution of (2R)-N-(3-amino-3-oxo-propyl)-2,4-dihydroxy-3,3-dimethyl-butanamide (0.2 g) and Na₂CO₃ (0.39 g) in THF (5 mL) was added dropwise [(1R)-3-chloro-1-methyl-3-oxo-propyl] butanoate (0.706 g) under N₂. The solution was stirred at 25 °C for 12 h. The mixture reaction was filtered and concentrated under reduced pressure. The residue was purified by column chromatography (SiO₂, petroleum ether/ ethyl acetate, 50:1 to 0:1) to give [(3R)-4-[(3-amino-3-oxo-propyl)amino]-3-[(3R)-3-butanoyloxybutanoyl]oxy-2,2-dimethyl-4-oxo-butyl] (3R)-3-butanoyloxybutanoate (0.109 g, 21%) as a yellow oil. ¹H NMR (400 MHz, DMSO-d6): δ 8.030 (br t, 1H), 7.315 (br s, 1H), 6.822 (br s, 1H), 5.173 - 5.107 (m, 2H), 4.695 (s, 1H), 3.861 (m, 2H), 3.260 - 3.231 (m, 2H), 2.712 - 2.628 (m, 4H), 2.201 (m, 6H), 1.495 (m, 4H), 1.211 (m, 6H), 0.921 - 0.834 (m, 12H) ppm

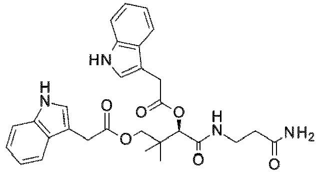

### Example 97: [(3R)-4-[(3-amino-3-oxo-propyl)amino]-3-[2-(1H-indol-3-yl) acetyl]oxy-2,2-dimethyl-4-oxobutyl] 2-(1H-indol-3-yl)acetate

### Step 1:

To a mixture of 3-aminopropanamide (4.79 g) in EtOH (100 mL) was added K₂CO₃ (10.62 g). The mixture was stirred at 25 °C for 30 min and then (3R)-3-hydroxy-4,4-dimethyl-tetrahydrofuran-2-one (5 g) was added. The mixture was degassed and purged with N₂ three times, and then stirred at 80°C for 10 hours under N₂ atmosphere. The reaction mixture was filtered and concentrated under reduced pressure and the residue was purified by column chromatography (SiO₂, ethyl acetate / methanol, 1:0 to 5:1) to give (2R)-N-(3-amino-3-oxo-propyl)-2,4-dihydroxy-3,3-dimethyl-butanamide (1.8 g, 21%) as a yellow oil. LCMS: 219.2 (M+H⁺)

### Step 2:

To a solution of (2R)-N-(3-amino-3-oxo-propyl)-2,4-dihydroxy-3,3-dimethyl-butanamide (0.200 g), *N,N*'-dicyclohexylcarbodiimide (0.416 g) and 4-dimethylaminopyridine (0.056 g) in CH₂Cl₂ (10 mL) was added dropwise 2-(1H-indol-3-yl)acetic acid (0.401 g) under N₂ and the mixture was stirred at 25 °C for 12 h. The mixture reaction was filtered and concentrated under reduced pressure and the residue was purified by prep-TLC (SiO₂, dichloromethane / methanol, 5:1) to give [(3R)-4-[(3-amino-3-oxopropyl)amino]-3-[2-(1H-indol-3-yl) acetyl]oxy-2,2-dimethyl-4-oxo-butyl] 2-(1H-indol-3-yl)acetate (0.053 g, 11%) as a yellow solid. ¹H NMR (400 MHz, DMSO-d6): δ 10.936 (br, 2H), 7.966 (br, 1H), 7.489 (br, 3H), 7.339 (br, 3H), 7.246 (m, 2H), 7.064 (m, 2H), 6.970 (m, 2H), 6.848 (m, 1H), 4.744 (s, 1H), 3.842 (br, 4H), 3.692 (br, 2H), 3.249 (br, 2H), 2.225 (br, 2H), 0.877 (s, 3H), 0.838 (s, 3H) ppm

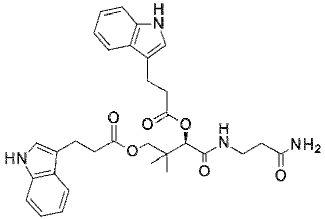

### Example 98: [(3R)-4-[(3-amino-3-oxo-propyl)amino]-3-[3-(1H-indol-3-yl)propanoyloxy]-2,2-dimethyl-4-oxobutyl] 3-(1H-indol-3-yl)propanoate

To a solution of (2R)-N-(3-amino-3-oxo-propyl)-2,4-dihydroxy-3,3-dimethyl-butanamide (0.200 g), *N,N*'-dicyclohexylcarbodiimide (0.415 g) and 4-dimethylaminopyridine (0.055 g) in THF (8 mL) was added 3-(1H-indol-3-yl) propanoic acid under N₂ and the mixture was stirred at 25 °C for 12 h. The mixture was combined with another batch for workup. The mixture reaction was filtered and concentrated. The residue was purified by prep-TLC (SiO₂, dichloromethane/methanol, 5:1) to give [(3R)-4-[(3-amino-3-oxopropyl)amino]-3-[3-(1H-indol-3-yl)propanoyloxy]-2,2-dimethyl-4-oxo-butyl] 3-(1H-indol-3-yl)propanoate (0.063 g) was obtained as a yellow solid. LCMS: 561.2 (M+H⁺) ¹H NMR (400 MHz, DMSO-d6): δ 10.796 (br, 2H), 7.975 (br, 1H), 7.508 (m, 2H), 7.321 (m, 3H), 7.132 - 6.965 (m, 9H), 6.85 (m, 1H), 4.740 (s, 1H), 3.948 - 3.831 (m, 2H), 3.230 (m, 2H), 2.950 (m, 4H), 2.798 - 2.674 (m, 4H), 2.226 (m, 2H), 0.917 (s, 3H), 0.886 (s, 3H) ppm

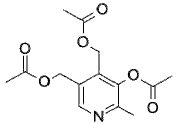

### Example 99: [5-acetoxy-4-(acetoxymethyl)-6-methyl-3-pyridyl] methyl acetate

To a solution of 4,5-bis(hydroxymethyl)-2-methyl-pyridin-3-ol (1 g) in CH₂Cl₂ (20 mL) was added acetic anhydride (1.81 g) and triethylamine (1.79 g) at 25°C. Then the mixture was stirred at 25 °C for 12 h. The reaction mixture was diluted with H₂O (30 mL), extracted three times with EtOAc (30 mL) and the combined organic phase were concentrated under reduced pressure. The residue was purified by column chromatography (SiO₂, petroleum ether/ethyl acetate, 10:1 to 5:1) to give [5-acetoxy-4-(acetoxymethyl)-6-methyl-3-pyridyl] methyl acetate (1.2 g, 68% yield) as a colorless oil. LCMS: 296.0 (M+H⁺) ¹H NMR (400 MHz, CDCl₃): δ 8.441 (s, 1H), 5.258(s, 1H), 5.133 (s, 1H), 2.412 (s, 3H), 2.376 (s, 3H), 2.073 (s, 3H), 2.026 (s, 3H) ppm

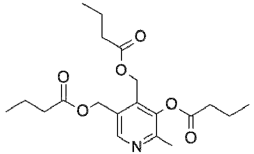

### Example 100: [5-butanoyloxy-4-(butanoyloxymethyl)-6-methyl-3-pyridyl]methyl butanoate

To a solution of 4,5-bis(hydroxymethyl)-2-methyl-pyridin-3-ol (1 g) in acetonitrile (50 mL) was added butanoyl chloride (1.89 g) and K₂CO₃ (3.27 g) at 25°C. Then the mixture was stirred at 50 °C for 12 h. The reaction mixture was concentrated under reduced pressure. The residue was purified by column chromatography (SiO₂, petroleum ether/ethyl acetate, 10:1 to 5:1) to give [5-butanoyloxy-4-(butanoyloxymethyl)-6-methyl-3-pyridyl]methyl butanoate (1.2 g, 50%) as a colorless oil. LCMS: 380.2 (M+H⁺) ¹H NMR (400 MHz, methanol-d4): δ 8.391 (s, 1H), 5.326(s, 1H), 5.173 (s, 1H), 2.683 (m, 2H), 2.366 (s, 3H), 2.327 - 2.253 (m, 4H), 1.75 (m, 2H), 1.596 (m, 4H), 1.088 (t, 3H), 0.924 (t, 3H), 0.918 (t, 3H) ppm

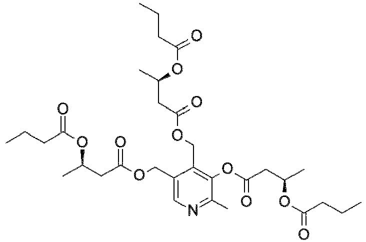

### Example 101: [5-[(3R)-3-butanoyloxybutanoyl]oxy-4-[[(3R)-3-butanoyloxybutanoyl]oxymethyl]-6-methyl-3-pyridyl]methyl (3R)-3-butanoyloxybutanoate

To a solution of 4,5-bis(hydroxymethyl)-2-methyl-pyridin-3-ol (0.2 g) and Na₂CO₃ (0.75 g) in acetonitrile (2 mL) was added dropwise [(1R)-3-chloro-1-methyl-3-oxo-propyl] butanoate (1.37 g) under N₂ and the mixture was stirred at 25 °C for 12 h. The mixture reaction was filtered and concentrated under reduced pressure. The residue was purified by reverse phase prep-HPLC (C18, water (10 mM NH₄HCO₃)-acetonitrile gradient) to give [5-[(3R)-3-butanoyloxybutanoyl]oxy-4-[[(3R)-3-butanoyloxybutanoyl]oxymethyl]-6-methyl-3-pyridyl]methyl (3R)-3-butanoyloxybutanoate (0.060 g, 7.8%) as a colorless oil. LCMS: 638.3 (M+H⁺) ¹H NMR (400 MHz, CDCl₃): δ 8.444 (s, 1H), 5.419 (m, 1H), 5.289- 5.096 (m, 6H), 3.05 - 2.85 (m, 2H), 2.7 - 2.45 (m, 4H), 2.393 (s, 3H), 2.287 (m, 2H), 2.163 (m, 2H), 1.7 - 1.5 (m, 6H), 1.423 (d, 3H), 1.287 -1.250 (m, 6H), 0.952 - 0.887 (m, 9H) ppm

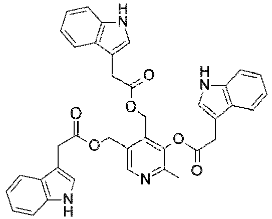

### Example 102: [5-[2-(1H-indol-3-yl)acetyl]oxy-4-[[2-(1H-indol-3-yl)acetyl]oxymethyl]-6-methyl-3-pyridyl]methyl 2-(1H-indol-3-yl)acetate

To a mixture of 4,5-bis(hydroxymethyl)-2-methyl-pyridin-3-ol (0.5 g) and 2-(1H-indol-3-yl)acetic acid (1.55 g) in CH₂Cl₂ (50 mL) was added *N,N*'-dicyclohexylcarbodiimide (1.83 g) and 4-dimethylaminopyridine (0.036 g) at 25°C Then the mixture was stirred at 25 °C for 12 hours. The reaction mixture was concentrated under reduced pressure to give a residue. The residue was purified by reverse phase prep-HPLC (C18, water (0.05%HCl)-acetonitrile gradient) to give [5-[2-(1H-indol-3-yl)acetyl]oxy-4-[[2-(1H-indol-3-yl)acetyl]oxymethyl]-6-methyl-3-pyridyl]methyl 2-(1H-indol-3-yl)acetate (1.6 g, 46%) as a white solid. ¹H NMR (400 MHz, CDCl₃): δ 8.339 (s, 1H), 8.0 - 7.9 (br 2H), 7.681 (m, 1H), 7.503 - 7.011 (m, 15H), 5.143 (s, 2H), 4.850 (s, 2H), 3.998 (s, 2H), 3.714 (s, 2H), 3.576 (s, 2H), 2.272 (s, 3H) ppm

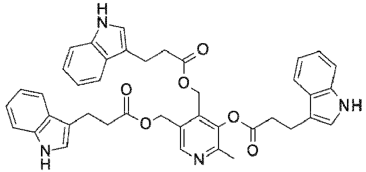

### Example 103: [5-[3-(1H-indol-3-yl)propanoyloxy]-4-[3-(1H-indol-3-yl)propanoyloxymethyl]-6-methyl-3-pyridyl]methyl3-(1H-indol-3-yl)propanoate

To a mixture of 4,5-bis(hydroxymethyl)-2-methyl-pyridin-3-ol (0.5 g), *N,N'-*dicyclohexylcarbodiimide (1.28 g) and 4-dimethylaminopyridine (0.036 g) in CH₂Cl₂ (30 mL) was added 3-(1H-indol-3-yl) propanoic acid (1.17 g) at 25°C. The mixture was stirred at 25°C for 12 h. The reaction mixture was concentrated under reduced pressure to give a residue which was purified by reverse phase prep-HPLC (C18, water (0.05%HCl)-acetonitrile gradient) to give [5-[3-(1H-indol-3-yl)propanoyloxy]-4-[3-(1H-indol-3-yl)propanoyloxymethyl]-6-methyl-3-pyridyl]methyl3-(1H-indol-3-yl)propanoate (1.2 g, 59%) as a colorless oil. LCMS: 683.2 (M+H⁺) ¹H NMR (400 MHz, CDCl₃): δ 8.306 (s, 1H), 8.0 - 7.85 (br 2H), 7.786 (br, 1H), 7.65 - 7.094 (m, 12H), 7.014 (s, 1H), 6.836 (s,1H), 6.793 (s, 1H), 5.065 (s, 2H), 4.806 (s, 2H), 3.238 (m, 2H), 3.033 (m, 6H), 2.726 (m, 2H), 2.604 (m, 2H), 2.274 (s, 3H) ppm

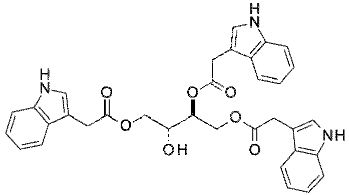

### Example 104: [(2R,3S)-2-hydroxy-3,4-bis[[2-(1H-indol-3-yl)acetyl]oxy]butyl] 2-(1H-indol-3-yl)acetate

To the solution of (2R,3S)-butane-1,2,3,4-tetrol (0.5 g), *N,N'*-dicyclohexylcarbodiimide (5.91 g) and 4-dimethylaminopyridine (0.10 g) in DMF (50 mL) was added 2-(1H-indol-3-yl)acetic acid (5.02 g). Then the solution was stirred at 100°C for 16 h. The solvent was removed under reduced pressure. The crude product was purified by reverse phase prep-HPLC (C18, water (0.05%HCl)-acetonitrile gradient) to give a solid after lyophilization. Saturated NaHCOs (20 ml) was added to the solid then the mixture was extracted three times with EtOAc (20mL). The combined organic phase was washed with brine (30 mL), dried with anhydrous Na₂SO₄, filtered and concentrated in vacuum to give [(2R,3S)-2-hydroxy-3,4-bis[[2-(1 H-indol-3-yl)acetyl]oxy]butyl] 2-(1H-indol-3-yl)acetate (0.1 g, 4%) as a yellow oil. LCMS: 594.2 (M+H⁺) ¹H NMR (400 MHz, DMSO-d6): δ 10.730 (br, 3H), 7.479 (m, 3H), 7.353 (m, 3H), 7.197 (m, 3H), 7.074 (m, 3H), 6.976 (m, 3H), 5.265 (br, 1H), 5.048 (br, 1H), 4.398 (m, 1H), 4.199 (m, 1H), 4.054 (m, 1H), 3.9 (br, 1H), 3.711 (s, 2H), 3.672 (s, 2H), 3.622 (s, 2H) ppm

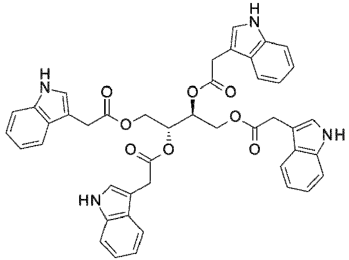

### Example 105: [(2R,3S)-2,3,4-tris[[2-(1H-indol-3-yl)acetyl]oxy]butyl] 2-(1H-indol-3-yl)acetate

To a solution of (2R,3S)-butane-1,2,3,4-tetrol (0.5 g), *N,N*'-dicyclohexylcarbodiimide (4.22 g) and 4-dimethylaminopyridine (0.05g,) in CH₂Cl₂ (20 mL) was added 2-(1H-indol-3-yl)acetic acid (3.59 g). Then the mixture was stirred at 25 °C for 16 h. The solvent was removed and the crude product was purified by prep-HPLC (C18, water(10 mM NH₄HCO₃)-acetonitrile gradient) followed by chromatography over silica gel (petroleum ether / ethyl acetate, 51:1:2) to give [(2R,3S)-2,3,4-tris[[2-(1H-indol-3-yl)acetyl]oxy]butyl] 2-(1H-indol-3-yl)acetate (0.5 g, 16% yield) as yellow solid. LCMS: 768 (M+H₃O⁺) ¹H NMR (400 MHz, DMSO-d6): δ 10.954 (s, 4H), 7.426 (m, 4H), 7.339 (m, 4H), 7.199 (m, 4H), 7.05 (m, 4H), 7.944 (m, 4H), 5.240 (m, 2H), 4.293 (m, 2H), 4.101 (m, 2H), 3.608 -3.586 (m, 8H) ppm

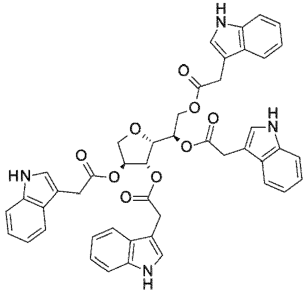

### Example 106: [(2R)-2-[(2R,3R,4S)-3,4-bis[[2-(1H-indol-3-yl)acetyl]oxy]tetrahydrofuran-2-yl]-2-[2-(1H-indol-3-yl)acetyl]oxy-ethyl] 2-(1H-indol-3-yl)acetate

To a solution of (2R,3R,4S)-2-[(1R)-1,2-dihydroxyethyl]tetrahydrofuran-3,4-diol (0.1 g), *N,N'-*dicyclohexylcarbodiimide (0.062 g) and 4-dimethylaminopyridine (0.074 g) in DMF (5 mL) and CH₂Cl₂ (15 mL) was added 2-(1H-indol-3-yl)acetic acid (0.534 g). The mixture was stirred at 25°C for 16 h under N₂ and the solvent was removed. The crude product was purified by prep-HPLC (phenyl stationary phase, water (10 mM NH₄HCO₃)-acetonitrile gradient) to give [(2R)-2-[(2R,3R,4S)-3,4-bis[[2-(1H-indol-3-yl)acetyl]oxy]tetrahydrofuran-2-yl]-2-[2-(1H-indol-3-yl)acetyl]oxy-ethyl] 2-(1H-indol-3-yl)acetate (0.053 g, 40%) as yellow solid. LCMS: 793.3(M+H⁺) ¹H NMR (400 MHz, CDCl₃): δ 8.003 (brs,1H), 7.872 (br s,1H), 7.816 (br s, 1H), 7.788 (brs,1H), 7.596 - 7.551 (m, 4H), 7.356 - 7.085 (m, 13H), 6.999 (br s, 1H), 6.940 (br s, 1H), 6.839 (br s, 1H), 5.172 (m, 2H), 4.996 (m, 1H), 4.582 (m, 1H), 4.062 (m, 3H), 3.789 (s, 2H), 3.629 (s, 2H), 3.616 - 3.606 (m 3H), 3.552 (m, 2H) ppm

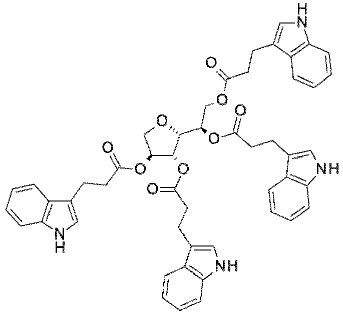

### Example 107: [(2R)-2-[(2R,3R,4S)-3,4-bis[3-(1H-indol-3-yl)propanoyloxy]tetrahydrofuran-2-yl]-2-[3-(1 H-indol-3-yl)propanoyloxy]ethyl] 3-(1H-indol-3-yl)propanoate

To the solution of (2R,3R,4S)-2-[(1R)-1,2-dihydroxyethyl]tetrahydrofuran-3,4-diol (0.1 g), *N,N-*dicyclohexylcarbodiimide (0.754 g) and 4-dimethylaminopyridine (0.074 g) in DMF (5 mL) and CH₂Cl₂ (15 mL) was added 3-(1H-indol-3-yl)propanoic acid (0.691 g). Then the mixture was stirred at 25°C for 16 h. The solvent was removed under reduced pressure and the crude product was purified by reverse phase prep-HPLC (C18, water(10 mM NH₄HCO₃)-acetonitrile gradient) to give [(2R)-2-[(2R,3R,4S)-3,4-bis[3-(1H-indol-3-yl)propanoyloxy]tetrahydrofuran-2-yl]-2-[3-(1H-indol-3-yl)propanoyloxy]ethyl] 3-(1H-indol-3-yl)propanoate (0.115 g, 22%) as yellow solid. LCMS: 849.3 (M+H⁺) ¹H NMR (400 MHz, CDCl₃): δ 7.382 (br s, 2H), 7.678 (br s, 2H), 7.520 (m, 4H), 7.265 - 7.003 (m, 12H), 6.888 (br, 2H), 6.838 (m, 1H), 6.783 (m, 1H), 5.100 (m, 2H), 4.730 (m, 1H), 4.452 (m, 1H), 4.969 (m, 1H), 3.850 (m, 2H), 3.471 (m, 1H), 3.044 - 2.922 (m, 8H), 2.675 - 2.910 (m, 8H) ppm

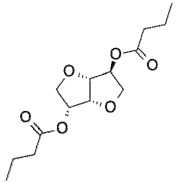

### Example 108: [(3S,3aR,6R,6aR)-6-butanoyloxy-2,3,3a,5,6,6a-hexahydrofuro[3,2-b]furan-3-yl] butanoate.

To a solution of (3R, 3aR, 6S, 6aR)-2, 3,3a,5,6,6a-hexahydrofuro[3,2-b]furan-3,6-diol (1 g) in acetonitrile (20 mL) was added K₂CO₃ (3.78 g) and followed by butanoyl chloride (2.92 g) in one portion at 25°C under N₂. The mixture was stirred at 25°C for 12 h. The reaction mixture was concentrated under reduced pressure and the residue was purified by reverse phase prep-HPLC (C18, water (0.05%HCl)-acetonitrile gradient) to give [(3S,3aR,6R,6aR)-6-butanoyloxy-2,3,3a,5,6,6a -hexahydrofuro[3,2-b]furan-3-yl] butanoate (0.174g, 8%) as colorless oil. ¹H NMR (400 MHz, CDCl₃): δ 5.182 - 5.132 (m, 2H), 4.815 (m, 1H), 4.459 (m, 1H), 3.933 (m, 3H), 3.791 (m, 1H), 2.356 - 2.262 (m, 4H), 1.669 - 1.583(m, 4H), 0.949 (t, 3H), 0.927 (t, 3H) ppm

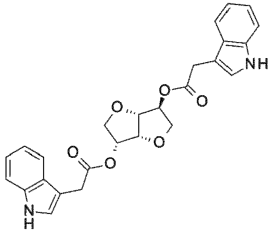

### Example 109: [(3S,3aR,6R,6aR)-6-[2-(1H-indol-3-yl)acetyl]oxy-2,3,3a,5,6,6a-hexahydrofuro[3,2-b]furan-3-yl] 2-(1H-indol-3-yl) acetate

To a mixture of (3R,3aR,6S,6aR)-2,3,3a,5,6,6a-hexahydrofuro[3,2-b]furan-3,6-diol (0.5 g) and 2-(1H-indol-3-yl) acetic acid (1.26 g) in CH₂Cl₂ (20 mL) was added *N,N*'-dicyclohexylcarbodiimide (1.48 g) and 4-dimethylaminopyridine (0.041 g) in one portion at 25°C under N₂. The mixture was stirred at 25 °C for 12 h. The reaction mixture was concentrated under reduced pressure and the residue was purified by reverse phase prep-HPLC (C18, water(0.05%HCI)-acetonitrile gradient) to give [(3S,3aR,6R,6aR)-6-[2-(1H-indol-3-yl)acetyl]oxy-2,3,3a,5,6,6a-hexahydrofuro[3,2-b]furan-3-yl] 2-(1H-indol-3-yl) acetate (0.395 g, 25%) as an off-white solid. LCMS: 461.0 (M+H⁺) ¹H NMR (400 MHz, CDCl₃): δ 8.069 (br s, 2H), 7.615 (m, 2H), 7.346 (m, 2H), 7.210 - 7.140 (m, 6H), 5.167 (m, 2H), 4.814 (m, 1H), 4.403 (m, 1H), 3.906 - 3.777 (m, 8H) ppm

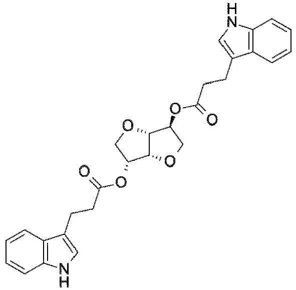

### Example 110: [(3S,3aR,6R,6aR)-6-[3-(1H-indol-3-yl)propanoyloxy]-2,3,3a,5,6,6a-hexahydrofuro[3,2-b]furan-3-yl] 3-(1H-indol-3-yl) propanoate

To a mixture of (3R,3aR,6S,6aR)-2,3,3a,5,6,6a-hexahydrofuro[3,2-b]furan-3,6-diol (0.5 g) and 3-(1H-indol-3-yl)propanoic acid (1.36 g) in CH₂Cl₂ (30 mL) was added *N,N*-dicyclohexylcarbodiimide (1.48 g) and 4-dimethylaminopyridine (0.041 g) in one portion at 25°C under N₂. The mixture was stirred at 25°C for 12 h. The reaction mixture was concentrated under reduced pressure and the residue was purified by reverse phase prep-HPLC (C18, water(0.05%HCI)-acetonitrile gradient) to give [(3S,3aR,6R,6aR)-6-[3-(1H-indol-3-yl)propanoyloxy]-2,3,3a,5,6,6a-hexahydrofuro[3,2-b]furan-3-yl] 3-(1H-indol-3-yl) propanoate (0.398 g, 24%) as a yellow solid. LCMS: 489.0 (M+H⁺) ¹H NMR (400 MHz, CDCl₃): δ 7.968 (br s, 2H), 7.604 (m, 2H), 7.363 (m, 2H), 7.209 - 7.135 (m, 4H), 7.015 (m, 2H), 5.167 5.107 (m, 2H), 4.693 (m, 1H), 4.314 (m, 1H), 3.891 - 3.859 (m, 3H), 3.739 (m, 1H), 3.135 2.094 (m, 4H), 2.820 - 2.727 (m, 4H) ppm

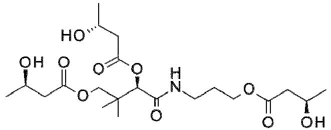

### Example 111: 3-[[(2R)-2,4-bis[[(3R)-3-hydroxybutanoyl]oxy]-3,3-dimethyl-butanoyl]amino]propyl (3R)-3-hydroxybutanoate

### Step 1:

To a solution of (2R)-2,4-dihydroxy-N-(3-hydroxypropyl)-3,3-dimethyl-butanamide (0.300 g), *N,N'-*dicyclohexylcarbodiimide (1.51 g) and 4-dimethylaminopyridine (0.054 g) in THF (5 mL) was added dropwise (3R)-3-benzyloxybutanoic acid (1.70 g) under N₂. The mixture was stirred at 25 °C for 12 h. The mixture reaction was filtered and concentrated and the residue was purified by column chromatography (SiO₂, petroleum ether / ethyl acetate, 20:1 to 0:1) followed by reverse phase prep-HPLC (C18, water (10 mM NH₄HCO₃)-acetonitrile gradient) to give 3-[[(2R)-2,4-bis[[(3R)-3-benzyloxybutanoyl]oxy]-3,3-dimethylbutanoyl]amino]propyl (3R)-3-benzyloxybutanoate (0.230 g, 19%) as a colorless oil. LCMS: 734.5 (M+H⁺)

### Step 2:

To a suspension of 10% Pd/C (0.200 g) in THF (30 mL) was added 3-[[(2R)-2,4-bis[[(3R)-3-benzyloxybutanoyl]oxy]-3,3-dimethyl-butanoyl]amino]propyl (3R)-3-benzyloxybutanoate (0.180 g). After degassing and purging with H₂ three times, the mixture was stirred at 65 °C under 50 psi for 12 hours. The reaction mixture was filtered and concentrated and the residue was purified by prep-TLC (SiO₂, ethyl acetate) to give 3-[[(2R)-2,4-bis[[(3R)-3-hydroxybutanoyl]oxy]-3,3-dimethyl-butanoyl]amino]propyl (3R)-3-hydroxybutanoate (0.051 g, 40%) as a colorless oil. ¹H NMR (400 MHz, CDCl₃): δ 7.188 (br s, 1H), 4.998 (s, 1H), 4.226 (br, 3H), 4.141 - 4.061 (m, 4H), 3.905 (m, 2H), 3.55 (br, 1H), 3.310 - 3.191 (m, 4H), 2.537 - 2.459 (m, 6H), 1.85 (m, 2H), 1.315 1.255 (m, 9H), 1.114 (s, 3H), 1.097 (s, 3H) ppm

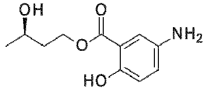

### Example 112: [(3R)-3-hydroxybutyl] 5-amino-2-hydroxy-benzoate

### Step 1: (3R)-3-benzyloxybutan-1-ol

To a suspension of LiAlH₄ (0.313 g) in dry THF (20 mL) was added dropwise a solution of (3R)-3-benzyloxybutanoic acid (2 g) in THF (20 mL) at 0 °C and the mixture was stirred at 0 °C for 2 h. The reaction mixture was added dropwise H₂O (1 mL) and 15% aq. NaOH (1 mL) and H₂O (3 mL), then dried over Na₂SO₄, filtered and concentrated. The residue was purified by column chromatography (SiO₂, petroleum ether / ethyl acetate, 50:1 to 20:1 to 15/1 to 10/1) to give (3R)-3-benzyloxybutan-1-ol (0.90 g, 43%) as a brown oil. LCMS: 181.2 (M+H⁺)

### Step 2: Benzyl 2-benzyloxy-5-nitro-benzoate

To a solution of 2-hydroxy-5-nitro-benzoic acid (1 g) in DMF (16 mL) was added Cs₂CO₃ (4.45 g), followed by benzyl bromide (2.10 g) added dropwise and the reaction mixture was stirred at 80 °C for 10 h. Water (20 mL) was added and the mixture was extracted three times with EtOAc (10 mL). The organic layer was washed three times with brine (10 mL) and dried over Na₂SO₄, filtered and concentrated. The residue was purified by column chromatography (SiOz, petroleum ether / ethyl acetate, 80:1 to 20:1) to give benzyl 2-benzyloxy-5-nitro-benzoate (1.3 g, 59%) as a yellow solid.

### Step 3: 2-benzyloxy-5-nitro-benzoic acid

To a solution of benzyl 2-benzyloxy-5-nitro-benzoate (0.800 g,) in THF (50 mL) was added a solution of LiOH (0.527 g) in H₂O (50 mL). The mixture was stirred at 25 °C for 12 h. The mixture was concentrated, and the residue was acidified to pH 5 with 2N HCl (30 mL). The yellow precipitate was collected by filtration, washed with petroleum ether / ethyl acetate (30 ml, 30:1) and dried under reduced pressure to give 2-benzyloxy-5-nitro-benzoic acid (0.600 g, 90%) as a yellow solid.

### Step 4: [(3R)-3-benzyloxybutyl] 2-benzyloxy-5-nitro-benzoate

To a solution of (3R)-3-benzyloxybutan-1-ol (0.195 g), *N,N*'-dicyclohexylcarbodiimide (0.335 g) and 4-dimethylaminopyridine (0.039 g) in CH₂Cl₂ (4 mL) was added 2-benzyloxy-5-nitro-benzoic acid (0.591 g). The mixture was degassed, purged with N₂ three times and stirred at 25 °C for 12 h. The reaction mixture was filtered and concentrated and the residue was purified by column chromatography (SiO₂, petroleum ether / ethyl acetate, 50:1 to 20:1) to give [(3R)-3-benzyloxybutyl] 2-benzyloxy-5-nitrobenzoate as a brown oil. LCMS: 458.2 (M+Na⁺).

### Step 5: [(3R)-3-hydroxybutyl] 5-amino-2-hydroxy-benzoate

To solution of [(3R)-3-benzyloxybutyl] 2-benzyloxy-5-nitro-benzoate (0.450 g) in THF (20 mL) was added 10% Pd/C (0.200 g). The mixture was degassed, purged three times with H₂ and stirred at 50 °C for 5 h at 50 psi. The reaction mixture was filtered and concentrated and the residue was purified by column chromatography (SiOz, petroleum ether / ethyl acetate, 50:1 to 0:1) to give [(3R)-3-hydroxybutyl] 5-amino-2-hydroxy-benzoate (0.174 g, 71%) as yellow a solid. LCMS: 226.1 (M+H⁺) ¹H NMR (400 MHz, CDCl₃): δ 10.820 (s, 1H), 7.448 (m, 1H), 6.116 (m, 1H), 5.986 (m, 1H), 4.591 (m, 1H), 4.271 (m, 2H), 3.769 (m, 1H), 1.728 (m, 2H), 1.106 (d, 3H) ppm

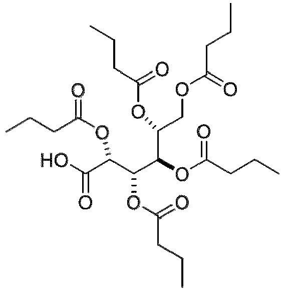

### Example 113: (2S,3S,4R,5S)-2,3,4,5,6- penta(butanoyloxy)hexanoic acid

To a mixture of (2R,3S,4R,5R)-2,3,4,5,6-pentahydroxyhexanoic acid, sodium salt (4 g) and butanoyl butanoate (19.3 g) was added HClO₄ (4.05 g) in one portion at 0°C under N₂. The mixture was stirred at 40 °C for 2 h. The mixture was poured on ice and extracted twice with CH₂Cl₂ (200 ml). Water (100ml) and triethylamine (8 ml) were added to the CH₂Cl₂ followed by stirring overnight to hydrolyze the anhydride. The organic layer was separated and washed with 1N HCl, dried over anhydrous Na₂SO₄, filtered and concentrated. The residue was purified by column chromatography (SiO₂, petroleum ether / ethyl acetate, 10:1 to 0:1) to give (2S,3S,4R,5S)-2,3,4,5,6-penta(butanoyloxy)hexanoic acid (6 g, 19%) as a colorless oil. LCMS: 545.2 (M-H⁻) ¹H NMR (400 MHz, CDCl₃): δ 5.633 (m, 1H), 5.492 (m, 1H), 5.089 (m, 2H), 4.401 (m, 1H), 4.096 (m, 1H), 2.415 -2.226 (m, 10H), 1.677 - 1.608 (m, 10H), 0.984 -0.929 (m, 15H) ppm

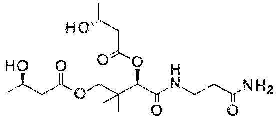

### Example 114: [(3R)-4-[(3-amino-3-oxo-propyl)amino]-3-[(3R)-3-hydroxybutanoyl]oxy-2,2-dimethyl-4-oxobutyl] (3R)-3-hydroxybutanoate

Step 1: To a solution of N-(3-amino-3-oxo-propyl)-2,4-dihydroxy-3,3-dimethyl-butanamide (0.300 g) and Na₂CO₃ (0.583 g) in THF (5 mL) was added dropwise (3R)-3-benzyloxybutanoyl chloride (1.17 g) under N₂. The mixture was stirred at 25 °C for 12 h. The reaction mixture was filtered and concentrated. The residue was purified by reverse phase prep-HPLC (C18, water (10 mM NH₄HCO₃)-acetonitrile gradient) to give [(3R)-4-[(3-amino-3-oxo-propyl)amino]-3-[(3R)-3-benzyloxybutanoyl]oxy-2,2-dimethyl-4-oxo-butyl] (3R)-3-benzyloxybutanoate (0.170 g,19.5%) as a colorless oil. LCMS: 571 (M+H⁺)

Step 2: To a suspension of 10% Pd/C (0.100 g) in THF (40 mL) was added [(3R)-4-[(3-amino-3-oxo-propyl)amino]-3-[(3R)-3-benzyloxybutanoyl]oxy-2,2-dimethyl-4-oxo-butyl] (3R)-3-benzyloxybutanoate (0.120 g), and the reaction mixture was degassed and purged with H₂ three times. The mixture was stirred at 65 °C for 12 h under H₂ (50 psi). The mixture reaction was filtered and concentrated. The residue was purified by column chromatography (SiO₂, petroleum ether/ethyl acetate, 10:1 to 0:1 to ethyl acetate/ethanol, 10:1 to 3:1) to give [(3R)-4-[(3-amino-3-oxo-propyl)amino]-3-[(3R)-3-hydroxybutanoyl]oxy-2,2-dimethyl-4-oxo-butyl] (3R)-3-hydroxybutanoate (0.070 g, 81%) as a colorless oil. LCMS: 391.2 (M+H⁺) ¹H NMR (400 MHz, CDCl₃): δ 7.540 (m, 1H), 5.995 (br s, 1H), 5.575 (br s, 1H), 4.956 (1H, m), 4.289 - 4.4.248 (m, 2H), 4.014 (d, 1H), 3.916 (d, 1H), 3.555, m, 1H), 3.403 (m, 1H), 2.595 - 2.362 (m, 6H), 1.302 (d, 3H), 1.267 (d, 3H), 1.096 (s, 3H), 1.087 (s, 3H) ppm

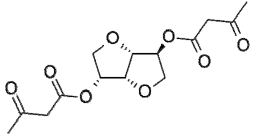

### Example 115: [(3S,3aR,6R,6aR)-6-(3-oxobutanoyloxy)-2,3,3a,5,6,6a-hexahydrofuro[3,2-b]furan-3-yl] 3-oxobutanoate

A solution of (3S,3aR,6R,6aR)-2,3,3a,5,6,6a-hexahydrofuro[3,2-b]furan-3,6-diol (1 g) and 2,2,6-trimethyl-1,3-dioxin-4-one (2.92 g) in Xylene (20 mL) was stirred at 150°C for 5 h. The solvent was removed under reduced pressure. The crude product was purified by silica gel chromatography (petroleum ether / ethyl acetate, 10:1 to 0:1) followed by reverse phase prep-HPLC (C18, water (0.05% HCl)-acetonitrile gradient). The product was further purified by silica gel chromatography (petroleum ether / ethyl acetate, 1:0 to 5:1) to give [(3S,3aR,6R,6aR)-6-(3-oxobutanoyloxy)-2,3,3a,5,6,6a-hexahydrofuro[3,2-b]furan-3-yl] 3-oxobutanoate (0.46 g, 21%) as a yellow oil. LCMS: 315.3 (M+H⁺) ¹H NMR (400 MHz, DMSO-d6): δ 5.146 (m,1H), 5.086 (m,1H), 4.412 (d, 1H), 3.877 - 3.618 (m, 9H), 2.189 (s, 3H), 2.174 (s, 3H) ppm

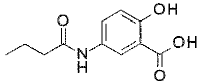

### Example 116: 5-(butanoylamino)-2-hydroxy-benzoic acid

To the solution of 5-amino-2-hydroxy-benzoic acid (1 g) and triethylamine (0.991 g) in dioxane (20 mL) and H₂O (10 mL) was added butyric anhydride (1.24 g), and the mixture was stirred at 20°C for 16 h. The dioxane was removed under reduced pressure and the pH was adjusted to 5-6 by adding aqueous 3N HCl at 0°C. The solid was filtered, washed three times with water (20 mL) and concentrated in vacuum. The crude product was purified by reverse phase prep-HPLC (C18, water(0.05%HCl)-acetonitrile gradient) to give 5-(butanoylamino)-2-hydroxy-benzoic acid (0.3 g, 20%) as a light pink solid. LCMS: 224.1 (M+H⁺) ¹H NMR (400 MHz, DMSO-d6): δ 11.015 (br, 1H), 9.807 (s, 1H), 8.106 (d, 1H), 7.652 (dd, 1H), 6.893 (d, 1H), 2.239 (m, 2H), 1.604 (m, 2H), 0.902 (t, 3H) ppm

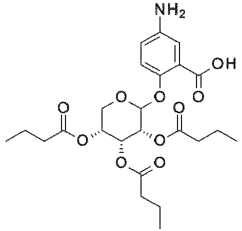

### Example 117: 5-amino-2-[(3R,4R,5R)-3,4,5-tri(butanoyloxy)tetrahydropyran-2-yl]oxy-benzoic acid

### Step 1. Ribose tetrabutyrate

To a stirred solution of D-(+)-ribose 1 (5 g) in anhydrous pyridine (24.2 mL) was added solution of butyryl chloride (23.70 g) in dichloromethane (50 mL) at 0-5 °C. The reaction mixture was brought to room temperature and stirred for 16 h. The mixture was diluted with dichloromethane (100 ml) and washed successively with water (100 mL), 2N aqueous HCl (300 mL), saturated sodium bicarbonate solution (300 mL) and brine (100 mL). The organic layer was dried over sodium sulphate and concentrated under reduced pressure. The residue was purified by column chromatography over silica gel (5-10% EtOAc-hexane gradient) to afford ribose tetrabutyrate as a colorless oil (7.5 g, 52%, mixture of α /β anomers).

### Step 2. Ribose tributyrate

Ammonium hydroxide (11 mL) was added slowly to a mixture of ribose tetrabutyrate 2 (7.5 g) in acetonitrile (60mL) at room temperature and the resulting reaction mixture was stirred for 5 h. The mixture was diluted with MTBE (75 mL) and stirred for 15 minutes. The organic layer was separated and concentrated under reduced pressure and the residue was partitioned between MTBE (100 mL) and water (75 mL). The MTBE layer was separated, dried over sodium sulphate and concentrated under reduced pressure. The residue was purified by column chromatography [using silica gel 100-200 mesh and 10-20% EtOAc-Hexane as eluting solvent] to afford ribose tributyrate as a colorless oil (1.1g, 17%).

### Step 3. 5-tert-butoxycarbonylamino-2-hydroxy-benzoic acid

To the stirred solution of 5-amino salicylic acid 4 (5g) in 1,4-dioxane and water (1:1; 100 mL) was added NaOH (1.3 g) and Boc-anhydride (7.83 g) at 0 °C and the resulting reaction mixture was stirred at room temperature for 1 h. The reaction mixture was concentrated under reduced pressure, the residue was diluted with EtOAc (50 mL) and the pH was adjusted to ~3-4 by dropwise addition of 0.5N aqueous HCl at 0 °C. The organic layer was separated and aqueous layer was extracted with EtOAc (50 mL). The combined organic layer was dried over sodium sulphate and concentrated under reduced pressure to provide 5-tert-butoxycarbonylamino-2-hydroxy-benzoic acid as off white solid (5.3 g, 64%).

### Step 4. 5-tert-butoxycarbonylmethyl-2-hydroxy-benzoic acid tert-butyl ester

To a stirred solution of 5-tert-butoxycarbonylamino-2-hydroxy-benzoic acid 5 (5.3 g) in DMF (50 mL) was added CDI (3.39 g) at 0-5 °C and stirred for 2 h. tert-Butanol (4.025 mL) and DBU (2.54 mL) were then added and the mixture was stirred at room temperature for 16 h. The mixture was diluted with water (100 mL) and extracted with EtOAc (200 mL). The organic layer was separated, dried over sodium sulphate and concentrated under reduced pressure. The residue was purified by column chromatography using silica gel [100-200 mesh; under gradient elution of 5-10% EtOAc-Hexane] to afford 5-tert-butoxycarbonylmethyl-2-hydroxy-benzoic acid tert-butyl ester as off white solid (2 g, 31%).

### Step 5. 5-tert-butoxycarbonylamino-2-(3,4,5-tris-butyryloxy-tetrahydro-pyran-2-yloxy)-benzoic acid tert-butyl ester

To a stirred solution of 5-tert-butoxycarbonylmethyl-2-hydroxy-benzoic acid tert-butyl ester 6 (0.850 g) and ribose tributyrate (1.04 g) in THF (5 mL) was sequentially added triphenylphosphine (1.03 g) and di-tert-butyl azodicarboxylate (0.948 g) at room temperature and the mixture was stirred for 16 h. The mixture was concentrated under reduced pressure and the residue was purified by column chromatography over silica gel (5 to 18 % EtOAc-Hexane gradient) to afford of crude 5-tert-butoxycarbonylamino-2-(3,4,5-tris-butyryloxy-tetrahydro-pyran-2-yloxy)-benzoic acid tert-butyl ester (1.3 g) which was used directly in the next step.

### Step 6. 5-amino-2-[(3R,4R,5R)-3,4,5-tri(butanoyloxy)tetrahydropyran-2-yl]oxy-benzoic acid

To a stirred solution of crude 5-tert-butoxycarbonylamino-2-(3,4,5-tris-butyryloxy-tetrahydropyran-2-yloxy)-benzoic acid tert-butyl ester 7 (1.3 g, crude from above experiment) in 1,4-dioxane (7 mL) was added 4N HCl in 1,4-dioxane (10 mL) at 0 °C and the resulting reaction mixture was stirred at room temperature for 16 h. Then reaction mixture was concentrated under reduced pressure and the residue was purified by reverse phase prep-HPLC to provide 5-amino-2-[(3R,4R,5R)-3,4,5-tri(butanoyloxy)tetrahydropyran-2-yl]oxy-benzoic acid (0.05g). LCMS: 496.5 (M+H⁺) ¹H NMR (400 MHz, DMSO-d6): δ 6.919 - 6.898 (m, 2H), 6.658 (m, 1H), 5.431 (m, 1H), 5.350 (m, 1H), 5.234 (m, 1H), 5.161 (m, 1H), 4.213 (m 1H), 3.749 (m, 1H), 2.497 - 2.268 (m, 4H), 2.197 (m, 1H), 1.620 - 1.487 (m, 6H), 0.926 - 0.888 (m, 9H) ppm

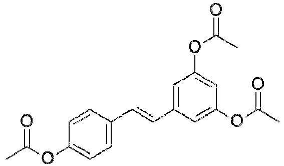

### Example 118: [(E)-2-(3,5-diacetoxyphenyl)vinyl]phenyl] acetate

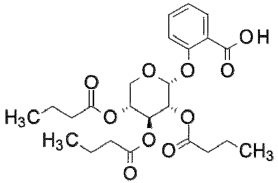

### Example 119: 2-[(2R,3R,4S,5R)-3,4,5-tri(butanoyloxy)tetrahydropyran-2-yl]oxybenzoic acid

Step 1. A mixture of xylose tributyrate (12 g), salicylic acid, benzyl ester (11.4 g) and triphenylphosphine (13.1 g) were dissolved in THF (240 mL) and stirred at 0 °C. To this mixture was added di-t-butyl azodicarboxylate (11.5 g) and stirring was continued at 0 °C for 1 h, then at room temperature, overnight. The reaction mixture was concentrated and purification by column chromatography using 0-30% ethyl acetate in hexanes provided the β-isomer (benzyl 2-[(2S,3R,4S,5R)-3,4,5-tri(butanoyloxy)tetrahydropyran-2-yl]oxybenzoate) (7 g, 32%) and the α-isomer (benzyl 2-[(2R,3R,4S,5R)-3,4,5-tri(butanoyloxy)tetrahydropyran-2-yl]oxybenzoate) (4.6 g, 21%).

Step 2. α-isomer, benzyl 2-[(2R,3R,4S,5R)-3,4,5-tri(butanoyloxy)tetrahydropyran-2-yl]oxybenzoate (2.8 g) was dissolved in methanol (40 mL) and stirred at room temperature. To this mixture was added 10% Pd/C (0.15 g). The suspension was stirred under hydrogen atmosphere at room temperature overnight. The reaction mixture was filtered through Celite and washed with methanol. The combined filtrate and washing were concentrated. The residue was purified by chromatography over silica gel (0-5% methanol in dichloromethane) to give 2-[(2R,3R,4S,5R)-3,4,5-tri(butanoyloxy)tetrahydropyran-2-yl]oxybenzoic acid (0.936 g, 40%). MS 479.2 (M-H) ¹H NMR (300 MHz CDCl₃): δ 8.175 (d, 1H), 7.542 (m, 1H), 7.363 (m, 1H), 7.247 (m, 1H), 5.725 (m, 2H), 5.132 (m, 2H), 4.007 (m, 1H), 3.765 (m, 1H), 2.324 - 2.249 (m, 6H), 1.606 - 1.582 (m, 6H), 0.921 - 0.865 (m, 9H) ppm

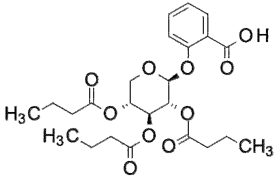

### Example 120: 2-[(2S,3R,4S,5R)-3,4,5-tri(butanoyloxy)tetrahydropyran-2-yl]oxybenzoic acid

β-isomer (benzyl 2-[(2S,3R,4S,5R)-3,4,5-tri(butanoyloxy)tetrahydropyran-2-yl]oxybenzoate (4.2 g) was dissolved in methanol (50 mL) and stirred at room temperature. To this mixture was added 10% Pd/C (0.2 g). The suspension was stirred under a hydrogen atmosphere at room temperature overnight. The reaction mixture was filtered through Celite and washed with methanol. The combined filtrate and washing were concentrated. The residue was purified by chromatography over silica gel (0-5% methanol in dichloromethane) to give 2-[(2S,3R,4S,5R)-3,4,5-tri(butanoyloxy)tetrahydropyran-2-yl]oxybenzoic acid (2.1 g, 60%).

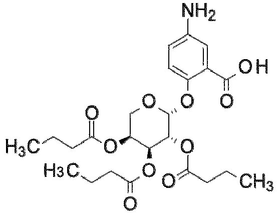

### Example 121: 5-amino-2-[(2R,3R,4S,5S)-3,4,5-tri(butanoyloxy)tetrahydropyran-2-yl]oxy-benzoic acid

Step 1: 2-Hydroxy-4-nitro-benzoate (20 g) and KHCO₃(13.1g) were suspended in DMF (100 mL). To the suspension was added benzyl bromide (22.4 g) and the reaction mixture was stirred at room temperature overnight. Water (150 mL) was added and the resulting mixture was extracted with ethyl acetate (250 mL). The organic phase was separated and washed twice with water, brine, and dried over Na₂SO₄. The solvent was removed under reduced pressure and the residue was purified by column chromatography (hexanes / ethyl acetate gradient). Recrystallization from 15% ethyl acetate in hexanes provided benzyl 2-hydroxy-4-nitro-benzoate (10.5 g).

Step 2: Benzyl 2-hydroxy-4-nitro-benzoate (8.5 g), arabinose tributyrate (7.5 g) and triphenylphosphine (8.2 g) were dissolved in THF (150 mL) and stirred at 0 °C. To this mixture was added di-t-butyl azodicarboxylate (7.2 g) and stirring was continued at 0 °C for 1 h, then at room temperature overnight. The reaction mixture was concentrated and purification by column chromatography (hexanes / ethyl acetate gradient) provided benzyl 5-nitro-2-[(2R,3R,4S,5S)-3,4,5-tri(butanoyloxy)tetrahydropyran-2-yl]oxy-benzoate (1.78 g, 14%).

Step 3: 5-nitro-2-[(2R,3R,4S,5S)-3,4,5-tri(butanoyloxy)tetrahydropyran-2-yl]oxy-benzoate (0.095 g) was dissolved in methanol (15 mL) and stirred at room temperature. To this mixture was added 10% Pd/C (0.05 g). The suspension was stirred under a hydrogen atmosphere at room temperature overnight. The reaction mixture was filtered through Celite and washed with methanol. The combined filtrate and washing were concentrated. The residue was purified by reverse phase chromatography (C-18, 0.1% trifluoroacetic acid in acetonitrile and 0.1% trifluoroacetic acid in water) to give 5-amino-2-[(2R,3R,4S,5S)-3,4,5-tri(butanoyloxy)tetrahydropyran-2-yl]oxy-benzoic acid (0.045 g, 59%). MS 494.2 (M-H) NMR (DMSO d6): δ 7.223 (m, 1H), 7.139 (m, 1H), 6.997 (s, 1H), 7.851 (d, 1H), 5.469 (m, 1H), 5.350 (m, 1H), 5.239 (m, 1H) 4.127 (d, 1H), 3.672 (d, 1H), 2.490 - 2.369 (M, 6H), 1.596 - 1.485 (m, 6H), 0.924 - 0.818 (m, 9H) ppm

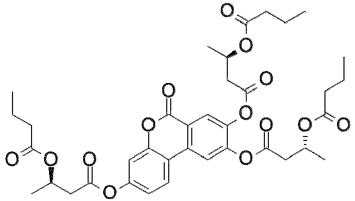

### Example 122: [8,9-bis[[(3R)-3-butanoyloxybutanoyl]oxy]-6-oxo-benzo[c]chromen-3-yl] (3R)-3-butanoyloxybutanoate

To a solution of 3,8,9-trihydroxybenzo[c]chromen-6-one (0.1 g) and K₂CO₃ (0.226 g) in acetonitrile (1 mL) was added [(1R)-3-chloro-1-methyl-3-oxo-propyl] butanoate (0.315 g) under N₂. The reaction mixture was stirred at 15 °C for 12 h. The mixture was filtered and concentrated. The residue was purified by reverse phase prep-HPLC (C18, [water (0.05%HCl)-acetonitrile]) to give [8, 9-bis[[(3R)-3-butanoyloxybutanoyl]oxy]-6-oxo -benzo[c]chromen-3-yl](3R)-3-butanoyloxybutanoate (0.050 g, 19%) as a yellow solid. LCMS 730.3 (M+H⁺) ¹H NMR (400 MHz, CDCl₃): δ 8.212 (s, 1H), 7.982 - 7.941 (m, 2H), 7.176 - 7.130 (m, 2H), 5.448 - 5.372 (m, 3H), 2.966 - 2.831 (m, 6H), 2.329 - 2.292 (m, 6H), 1.698 - 1.643 (m, 6H), 1.442 - 1.408 (m, 9H), 0.976 - 0.940 (m, 9H) ppm

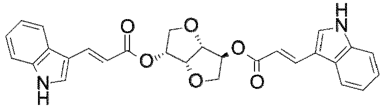

### Example 123: [(3R,3aR,6S,6aR)-6-[(E)-3-(1H-indol-3-yl)prop-2-enoyl]oxy-2,3,3a,5,6,6a-hexahydrofuro[3,2-b]furan-3-yl] (E)-3-(1H-indol-3-yl)prop-2-enoate

Step 1: To a solution of (E)-3-(1H-indol-3-yl)prop-2-enoic acid (21 g), Boc₂O (48.97 g) and triethylamine (28.38 g) in THF (560 mL) was added 4-dimethylaminopyridine (0.685 g), and the mixture was stirred at 15 °C for 12 h. The mixture reaction was concentrated and the residue was purified by column chromatography (SiOz, petroleum ether / ethyl acetate gradient) to give (E)-3-(1-tert-butoxycarbonylindol-3-yl)prop-2-enoic acid (13 g, 36% yield) as a yellow solid.

Step 2: To a solution of (3R,3aR,6S,6aR)-2,3,3a,5,6,6a-hexahydrofuro[3,2-b]furan-3,6-diol (0.200 g), N,N-dicyclohexylcarbodiimide (0.706 g) and 4-dimethylaminopyridine (0.083 g) in THF (10 mL) at 15 °C was added (E)-3-(1-tertbutoxycarbonylindol-3-yl)prop-2-enoic acid (0.983 g) and the reaction mixture was stirred at 15 °C for 12 h. The reaction mixture was filtered and concentrated. The residue was purified by column chromatography (SiO₂, petroleum ether / ethyl acetate gradient) to give tert-butyl3-[(E)-3-[[(3S,3aR,6R,6aR)-6-[(E)-3-(1-tert-butoxycarbonylindol-3-yl)prop-2-enoyl]xy-2,3,3a,5,6,6ahexahydrofuro[3,2-b]furan-3-yl]oxy]-3-oxo-prop-1-enyl]indole-1-caboxylate (0.600 g, 54% yield) as a yellow solid.

Step 3: tert-Butyl3-[(E)-3-[[(3R,3aR,6S,6aR)-6-[(E)-3-(1-tert-butoxycarbonylindol-3-yl)prop-2-enoyl]oxy-2,3,3a,5,6,6a-hexahydrofuro[3,2-b]furan-3-yl]oxy]-3-oxo-prop-1-enyl]indole-1-carboxylate (0.600 g) and trifluoroacetic acid (30 mL) were dissolved with CH₂Cl₂ (90 mL) and stirred at 15 °C for 1.5 h. The reaction mixture was washed 8 times with sat. NaHCOs (30 mL), dried over Na₂SO₄, filtered and concentrated. The residue was purified by prep-HPLC (C18, [water (0.05%HCl)-acetonitrile]) to gve [(3S,3aR,6R,6aR)-6-[(E)-3-(1H-indol-3-yl)prop-2-enoyl]oxy-2,3,3a,5,6,6a-hexahydrfuro[3,2-b]furan-3-yl](E)-3-(1H-indol-3-yl)prop-2-enoate (0.088 g, 20% yield) as a pink solid. LCMS: 483.2 (M-H⁺) ¹H NMR (400 MHz, DMSO-d6): δ 11.832 (s, 2H), 8.004 - 7.877 (m, 6H), 7.469 (m, 2H), 7.218 - 7.150 (m, 4H), 6.426 - 6.352 (m, 2H), 5.258 - 5.219 (m, 2H), 4.888 (m, 1H), 4.535 (m, 1H), 3.92 - 3.918 (m, 4H) ppm

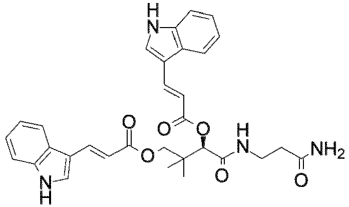

### Example 124: [(3R)-4-[(3-amino-3-oxo-propyl)amino]-3-[(E)-3-(1H-indol-3-yl)prop-2-enoyl]oxy-2,2-dimethyl-4-oxo-butyl] (E)-3-(1H-indol-3-yl)prop-2-enoate

Step 1: To a mixture of (2R)-N-(3-amino-3-oxo-propyl)-2,4-dihydroxy-3,3-dimethyl-butanamide (0.116 g) and (E)-3-(1-tert-butoxycarbonylindol-3-yl)prop-2-enoic acid (0.337 g) in THF (5 mL) was added *N,N*'-dicyclohexylcarbodiimide (0.242 g) and 4-dimethylaminopyridine (0.033 g) in one portion at 15°C under N₂. The mixture was stirred at 15 °C for 12 h. The reaction mixture was concentrated under reduced pressure and the residue was purified by column chromatography (SiO2, petroleum ether / ethyl acetate gradient) to give tert-butyl 3-[(E)-3-[(3R)-4-[(3-amino-3-oxo-propyl)amino]-3-[(E)-3-(1-tert-butoxycarbonylindol-3-yl)prop-2-enoyl]oxy-2,2-dimethyl-4-oxo-butoxy]-3-oxo-prop-1-enyl]indole-1-carboxylate (0.2 g, 30% yield) as a white solid.

Step 2: A solution of tert-butyl 3-[(E)-3-[(3R)-4-[(3-amino-3-oxo-propyl)amino]-3-[(E)-3-(1-tert-butoxycarbonylindol-3-yl)prop-2-enoyl]oxy-2,2-dimethyl-4-oxo-butoxy]-3-oxo-prop-1-enyl]indole-1-carboxylate (0.200 g) in trifluoroacetic acid (1.8 mL) and CH₂Cl₂ (1.2 mL) was stirred at 15 °C for 2 h. The reaction mixture was concentrated under reduced pressure to give a residue which was purified by reverse phase prep-HPLC (C₁₈, [water(0.05%HCl)-acetonitrile]) to give [(3R)-4-[(3-amino-3-oxopropyl)amino]-3-[(E)-3-(1H-indol-3-yl)prop-2-enoyl]oxy-2,2-dimethyl-4-oxo-butyl] (E)-3-(1H-indol-3-yl)prop-2-enoate (0.02 g, 13% yield) as a pink solid. LCMS: 557.2 (M+H⁺) ¹H NMR (400 MHz, CDCl₃): δ 8.454 (2 br s, 2H), 7.891 - 7.843 (m, 4H), 7.436 - 7.351 (m, 5H), 7.332 - 7.144 (m, 3H), 6.861 (m, 1H), 6.380 - 6.438 (m, 2H), 5.594 (br, 1H), 5.348 (br, 1H), 5.161 (s, 1H), 4.117 (s, 2H), 3.513 (m, 2H), 2.329 (m, 2H), 1.146 (s, 3H), 1.135 (s, 3H) ppm

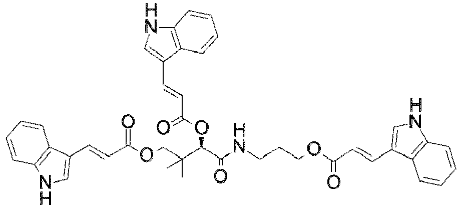

### Example 125: 3-[[(2R)-2,4-bis[[(E)-3-(1H-indol-3-yl)prop-2-enoyl]oxy]-3,3-dimethyl-butanoyl]amino]propyl (E)-3-(1H-indol-3-yl)prop-2-enoate

Step 1: To a solution of (2R)-2,4-dihydroxy-N-(3-hydroxypropyl)-3,3-dimethyl-butanamide (0.300 g), *N,N*-dicyclohexylcarbodiimide (1.06 g) and 4-dimethylaminopyridine (0.089 g), in THF (15 mL) was added (E)-3-(1-tertbutoxycarbonylindol- 3-yl)prop-2-enoic acid (1.47 g) and the mixture was stirred at 15 °C for 12 h. The mixture reaction was filtered and concentrated and the residue was purified by column chromatography (SiO₂, petroleum ether / ethyl acetate gradient) to give tert-butyl 3-[(E)-3-[3-[[(2R)-2,4-bis[[(E)-3-(1-tert-butoxycarbonylindol-3-yl)prop-2-enoyl]oxy]-3,3-dimethylbutanoyl] amino]propoxy]-3-oxo-prop-1-enyl]indole-1-carboxylate (1.2 g, 69% yield) as a yellow solid.

Step 2: To a solution of tert-butyl 3-[(E)-3-[3-[[(2R)-2,4-bis[[(E)-3-(1-tert-butoxycarbonylindol- 3-yl)prop-2-enoyl]oxy]-3,3-dimethylbutanoyl]amino]propoxy]-3-oxo-prop-1-enyl]indole-1-carboxylate (1.2 g) in CH₂Cl₂ (20 mL) was added dropwise trifluoroacetic acid (10.78 g) and the mixture was stirred at 15 °C for 2 h. The mixture reaction was concentrated and the residue was purified by reverse prep-HPLC (C18, [water(0.05%HCl)-acetonitrile]) to give 3-[[(2R)-2,4-bis[[(E)-3-(1H-indol-3-yl)prop-2-enoyl]oxy]-3,3-dimethyl-butanoyl]amino]propyl (E)-3-(1H-indol-3-yl)prop-2- enoate (0.370 g, 42% yield) as a pink solid. LCMS: 713.2 (M+H⁺) ¹H NMR (400 MHz, CDCl₃): δ 11.118 (m, 1H), 8.268 (brt, 1H), 8.057 (br, 1H), 8.018 (br, 1H), 7.980 (br (1H), 7.939 (br, 1H), 7.868 - 7.782 (m, 3H), 7.610 (m, 1H), 7.508 (br s, 1H), 7.415 - 7.395 (m, 3H), 7.201 - 7.072 (m, 6H), 6.561 - 6.357 (m, 3H), 5.165 (s, 1H), 4.294 - 4.132 (m, 4H), 3.413 - 3.310 (m, 2H), 2.019 - 1.923 (m, 3H), 1.229 (s, 6H) ppm

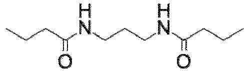

### Example 126: N-[3-(butanoylamino)propyl]butanamide

To a solution of propane-1,3-diamine (0.500 g) and K₂CO₃ (2.05 g) in acetonitrile (5 mL) was added dropwise butanoyl chloride (1.44 g) under N₂ and the mixture was stirred at 15 °C for 4 h. methanol (10 mL) was added and the mixture and stirred for 12 h. LCMS showed the product as major peak. The mixture reaction was filtered and concentrated. The residue was purified by reverse phase prep-HPLC (C₁₈, [water(0.05%HCl)-acetonitrile]) to give N-[3-(butanoylamino)propyl]butanamide (0.233 g, 15% yield) as a white solid. LCMS: 215.1 (M+H⁺) ¹H NMR (400 MHz, methanol-d4): δ 3.203 (t, 4H), 2.172 (t, 4H), 1.692 - 1.609 (m, 6H), o.945 (t, 6H) ppm

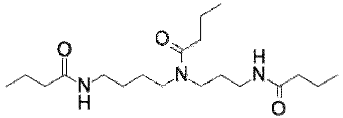

### Example 127: N-[4-[butanoyl-[3-(butanoylamino)propyl]amino]butyl]butanamide

To a solution of N'-(3-aminopropyl)butane-1,4-diamine (0.300 g) and triethylamine (0.648 g) in CH₂Cl₂ (5 mL) was added drop wise butanoyl chloride (0.671 g) under N₂ and the mixture was stirred at 15 °C for 12 h. The mixture was filtered and concentrated and the residue was purified by reverse phase prep-HPLC (C18, [water(0.05%HCl)-acetonitrile]) to give N-[4-[butanoyl-[3-(butanoylamino)propyl] amino]butyl]butanamide (0.264 g, 34% yield) as a yellow oil. LCMS: 356.2 (M+H⁺) ¹H NMR (400 MHz, methanol-d4): δ 3.403 - 3.356 (m, 4H), 3.260 - 3.133 (m, 4H), 2.394 - 2.356 (m, 2H), 2.253 - 2.154 (m, 4H), 1.809 - 1.504 (m, 12H), 0.990 - 0.936 (m, 9H) ppm

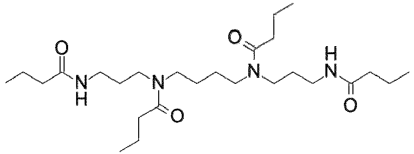

### Example 128: N-[3-[butanoyl-[4-[butanoyl-[3-(butanoylamino)propyl]amino]butyl] amino]propyl]butanamide

To a solution of N,N'-bis(3-aminopropyl)butane-1,4-diamine (0.300 g) and triethylamine (0.675 g) in CH₂Cl₂ (5 mL) was added butanoyl chloride (0.648 g) under N₂ and the mixture was stirred at 15 °C for 12 h. The mixture reaction was filtered and concentrated and the residue was purified by reverse phase prep-HPLC (C18, [water(0.05%HCl)-acetonitrile]) to give N-[3-[butanoyl-[4-[butanoyl-[3-(butanoylamino) propyl]amino]butyl]amino]propyl]butanamide (0.360 g, 48% yield) as yellow oil. LCMS: 483.5 (M+H⁺) ¹H NMR (400 MHz, methanol-d4): δ 3.393 (br, 8H), 3.246 - 3.185 (m, 4H), 2.396 - 2.338 (m, 4H), 2.232 - 2.202 (m, 4H), 1.772 -1.526 (m, 16 H), 0.989 - 0.940 (m, 12H) ppm

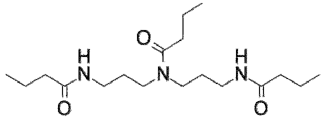

### Example 129: N-[3-[butanoyl-[3-(butanoylamino)propyl]amino]propyl]butanamide

To a solution of N'-(3-aminopropyl)propane-1,3-diamine (0.500 g) and triethylamine (1.35 g) in acetonitrile (5 mL) was added dropwise butanoyl chloride (1.24 g) under N₂ and the mixture was stirred at 15 °C for 12 h. The mixture reaction was filtered and concentrated and the residue was purified by prep-HPLC to giveN-[3-[butanoyl-[3-(butanoylamino)propyl] amino]propyl]butanamide (0.623 g, 45% yield) as a colorless oil. LCMS: 342.3 (M+H⁺) ¹H NMR (400 MHz, methanol-d4): δ 3.390 - 3.344 (m, 4H), 3.214 - 3.173 (m, 4H), 2.368 - 2.212 (m, 2H), 2.202 - 2.165 (m, 4H), 1.797 - 1.616 (m, 10H), 0.985 - 0.934 (m, 9H) ppm

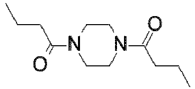

### Example 130: 1-(4-butanoylpiperazin-1-yl)butan-1-one

To a solution of piperazine (0.5 g) and triethylamine (1.17 g) in CH₂Cl₂ (10 mL) was added butanoyl chloride (2.47 g) at 0°C dropwise. Then the mixture was stirred at 15°C for 16 h. The solvent was removed under reduced pressureand the redidue was purified by reverse phase prep-HPLC (C18, [water(0.1 %trifluoroacetic acid)-acetonitrile]) to give 1-(4-butanoylpiperazin-1-yl)butan-1-one (0.38 g, 29% yield) as a colorless oil. LCMS: 227.2 (M+H⁺) ¹H NMR (400 MHz, CDCl₃): δ 3.678 (m, 4H), 3.517 (m, 4H), 2.375 (m, 4H), 1.674 (m, 4H), 0.995 (t, 6H) ppm

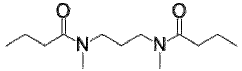

### Example 131: N-[3-[butanoyl(methyl)amino]propyl]-N-methyl-butanamide

To a solution of N,N'-dimethylpropane-1,3-diamine (0.5 g) and triethylamine (0.990 g) in CH₂Cl₂ (30 mL) was added butanoyl chloride (2.09 g) dropwise at 0°C. Then the solution was stirred at 15°C for 16 h. The solvent was removed under reduced pressure and the crude product was purified by reverse phase prep-HPLC (C18, [water(0.1%trifluoroacetic acid)-acetonitrile]) to give N-[3-[butanoyl(methyl)amino]propyl]-N-methyl-butanamide (0.358 g, 30% yield) as a colorless oil. LCMS: 243.2 (M+H⁺) ¹H NMR (400 MHz, CDCl₃, mixture of rotamers): δ 3.415 - 3.312 (m, 4H), 4.166 - 1.602 (2xs, 6H), 2.338- 2.283 (m, 4H), 1.805 - 1.632 (m, 6H), 0.969 (m, 6H) ppm

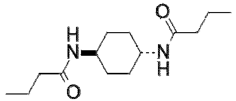

### Example 132: trans-N-[4-(butanoylamino)cyclohexyl]butanamide

To a solution of trans- cyclohexane-1,4-diamine (1 g) and triethylamine (1.77 g) in CH₂Cl₂ (20 mL) was added butanoyl chloride (2.33 g and the mixture was stirred at 15°C for 16 h. The solvent was removed under reduced pressure and the crude product was purified by reverse phase prep-HPLC (C18, [water(0.1 %trifluoroacetic acid)-acetonitrile]) to give trans-N-[4-(butanoylamino)cyclohexyl]butanamide (0.1 g, 4.5% yield) as a white solid. LCMS: 255.2 (M+H⁺) ¹H NMR (400 MHz, methanol-d4): δ 3.622 (br s, 2H), 2.130 (t, 4H), 1.909 (m, 4H), 1.650 - 1.595 (m, 4H), 1.348 - 1.320 (m, 4H), 0.936 (t, 6H) ppm

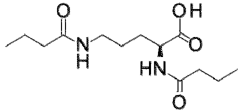

### Example 133: (2S)-2,5-bis(butanoylamino)pentanoic acid

Step 1: To a solution of methyl (2S)-2,5-diaminopentanoate dihydrochloride (2 g) and triethylamine (3.69 g) in THF (30 mL) was added butanoyl chloride (2.14 g) dropwise and the solution was stirred at 15°C for 16 h. The solution was filtered and the filtrate was concentrated in vacuum. The crude product was combined with another batch and purified by reverse phase prep-HPLC (C18, [water (0.1 %trifluoroacetic acid)-acetonitrile]) to give (2S)-2,5-bis(butanoylamino)pentanoic acid methyl ester (0.6 g) as a colorless oil.

Step 2: A solution of (2S)-2,5-bis(butanoylamino)pentanoic acid methyl ester (0.25 g) and NaOH (0.070 g) in methanol (25 mL) and H₂O (5 mL) was stirred at 15°C for 16 h. The solvent was removed in vacuum and the crude product was purified by reverse phase prep-HPLC (C18 [water(10 mM NH₄HCO₃)-acetonitrile]) to give (2S)-2,5-bis(butanoylamino)pentanoic acid (0.080 g, 34% yield) as a white solid. LCMS: 273.1 (M+H⁺) ¹H NMR (400 MHz, DMSO-d6): δ 3.889 (m, 1H), 2.992 - 2.902 (m, 2H), 2.044 (t, 2H), 1.978 (t, 2H), 1.639 - 1.602 (m, 1H), 1.510 - 1.419 (m, 5 H), 1.350 - 1.315 (m, 2H), 0.823 (t, 3H), 0.810 (t, 3H) ppm

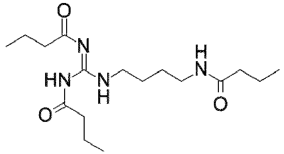

### Example 134: N-[4-[[(Z)-N,N'-di(butanoyl)carbamimidoyl]amino]butyl]butanamide

To a solution of 1-(4-aminobutyl)guanidine sulfate (0.500 g) and K₂CO₃ (1.82 g) in THF (10 mL) was added dropwise butanoyl chloride (0.724 g) under N₂ and stirred at 15 °C for 12 h. The mixture was filtered and concentrated and the residue was purified by column chromatography (SiO₂, petroleum ether / ethyl acetate gradient) to give N-[4-[[(Z)-N,N'-di(butanoyl)carbamimidoyl]amino]butyl]butanamide (0.050 g, 6.64% yield) as a white solid. LCMS: 341.2 (M+H⁺) ¹H NMR (400 MHz, CDCl₃): δ 13.242 (br s, 1H), 9.081 (br s, 1H), 5.550 (br s, 1H), 3.454 (m, 2H), 3.308 (m, 2H), 2.346 (m, 4H), 2.149 (m, 2H), 1.705 - 1.649 (m, 10H), 1.004 - 0.935 (m, 9H) ppm

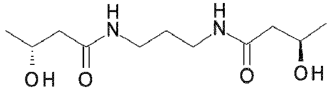

### Example 135: (3R)-3-hydroxy-N-[3-[[(3R)-3-hydroxybutanoyl]amino]propyl]butanamide

Step 1: To a mixture of propane-1,3-diamine (0.138 g) and (2,5-dioxopyrrolidin-1-yl) (3R)-3-[tert-butyl(diphenyl)silyl]oxybutanoate (1.8 g) in THF (10 mL) was added triethylamine (0.414.g) in one portion at 15 °C under N₂. The mixture was stirred at 15°C for 12 h. The reaction mixture was filtered and concentrated under reduced pressure and the residue was purified by column chromatography (SiO₂, petroleum ether / ethyl acetate, gradient) to give (3R)-3-[tert-butyl(diphenyl)silyl]oxy-N-[3-[[(3R)-3- [tert-butyl(diphenyl)silyl]oxybutanoyl]amino]propyl]butanamide (1 g, 67% yield) as a colorless oil.

Step 2: A mixture of (3R)-3-[tert-butyl(diphenyl)silyl]oxy-N-[3-[[(3R)-3-[tert-butyl(diphenyl)silyl] oxybutanoyl]amino]propyl]butanamide (0.35 g) and pyridine.HF (0.240 g) in THF (20 mL) was stirred at 15 °C for 12 h. The reaction mixture was filtered and concentrated under reduced pressure. The residue was was purified by reverse phase prep-HPLC (C18, [water(0.05%HCl) -acetonitrile]) to give (3R)-3-hydroxy-N-[3-[[(3R)-3-hydroxybutanoyl]amino]propyl]butanamide (0.014 g, 12% yield) as a colorless oil. LCMS: 247.1 (M+H⁺) ¹H NMR (400 MHz, DMSO-d6): δ 7.758 (br, 2H), 4.777 (br 2H), 3.960 - 3.882 (m, 2H), 3.004 (m, 4H), 2.186 - 2.032 (m, 4H), 1.492 (m, 2H), 1.026 (d, 6H) ppm

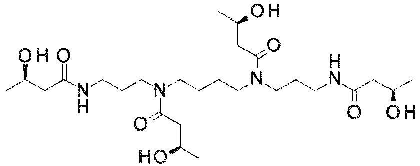

### Example 136: (3R)-3-hydroxy-N-[3-[[(3R)-3-hydroxybutanoyl]-[4-[[(3R)-3-hydroxybutanoyl]-[3-[[(3R)-3-hydroxybutanoyl]amino]propyl]amino]butyl]amino]propyl]butanamide

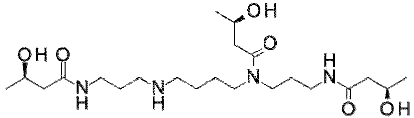

### Example 137: (3R)-3-hydroxy-N-[3-[4-[[(3R)-3-hydroxybutanoyl]-[3-[[(3R)-3-hydroxybutanoyl]amino]propyl]amino]butylamino]propyl]butanamide

Step 1: To a solution of N,N'-bis(3-aminopropyl)butane-1,4-diamine (0.2 g) and triethylamine (0.500 g) in THF (10 mL) was added (2,5-dioxopyrrolidin-1-yl) (3R)-3-[tert-butyl(diphenyl)silyl]oxybutanoate (2.17 g) and and the mixture was stirred at 15 °C for 12 h. The mixture reaction was concentrated and used in next step immediately.

Step 2: To a mixture of (3R)-3-[tert-butyl(diphenyl)silyl]oxy-N-[3-[[(3R)-3-[tert-butyl(diphenyl)silyl] oxybutanoyl]-[4-[[(3R)-3-[tert-butyl(diphenyl)silyl]oxybutanoyl]-[3-[[(3R)-3-[tert-butyl(diphenyl)silyl]oxybutanoyl]amino]propyl]amino]butyl]amino]propyl]butanamide (1.2 g) in THF (5 mL) was added pyridine.HF (0.079 g) in one portion at 15 °C under N₂. The mixture was stirred at 15 °Cfor 12 h. The reaction mixture was filtered and concentrated under reduced pressure to give a residue. The residue was purified by reverse phase prep-HPLC (C18, [water(0.05%HCl)-acetonitrile]) to give (3R)-3-hydroxy-N-[3-[[(3R)-3-hydroxybutanoyl]-[4-[[(3R)-3-hydroxybutanoyl]-[3-[[(3R)-3-hydroxybutanoyl]amino]propyl]amino]butyl]amino]propyl]butanamide (0.065 g, 15% yield) as a colorless oil and (3R)-3-hydroxy-N-[3-[4-[[(3R)-3-hydroxybutanoyl]-[3-[[(3R)-3-hydroxybutanoyl]amino]propyl]amino]butylamino]propyl]butanamide (0.090 g, 23% yield) as a colorless oil. (3R)-3-hydroxy-N-[3-[[(3R)-3-hydroxybutanoyl]-[4-[[(3R)-3-hydroxybutanoyl]-[3-[[(3R)-3-hydroxybutanoyl]amino]propyl]amino]butyl]amino]propyl]butanamide
LCMS: 547.3 (M+H⁺) ¹H NMR (400 MHz, DMSO-d6): δ 7.897 (br, 1H), 7.783 (br, 1H), 6.0 (br, 4H), 4.003 - 3.936 (m, 4H), 3.234 - 3.198 (m, 8H), 3.058 (m, 2H), 3.001 (m, 2H), 2.455 - 2.098 (m, 8H), 1.646 - 1.364 (m, 8H), 1.091 - 1.047 (m, 12H) ppm (3R)-3-hydroxy-N-[3-[4-[[(3R)-3-hydroxybutanoyl]-[3-[[(3R)-3-hydroxybutanoyl]amino]propyl]amino]butylamino]propyl]butanamide
LCMS: 461.3 (M+H⁺) ¹H NMR (400 MHz, DMSO-d6): δ 8.856 (br, 1H), 8.873 (br, 1H), 8.062 - 7.828 (m, 2H), 6.0 (br, 3H), 4.011 - 3.931 (m, 3H), 3.306 - 2.858 (m, 12H), 2.386 - 2.108 (m, 7H), 1.766 - 1.577 (8H), 1.147 -1.061 (9H) ppm

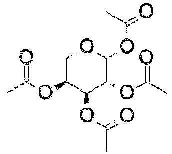

### Example 138: [(3S,4S,5R)-4,5,6-triacetoxytetrahydropyran-3-yl] acetate

To a solution of (3R,4S,5S)-tetrahydropyran-2,3,4,5-tetrol (2 g), triethylamine (10.91 g) and 4-dimethylaminopyridine (0.163 g,) in CH₂Cl₂ (20 mL) was added acetyl acetate (8.72 g), and the mixture was stirred at 15°C for 16 h. The solvent was removed under reduced pressure. The crude product was purified by silica gel chromatography (petroleum ether / ethyl acetate gradient) to give [(3S,4S,5R)-4,5,6-triacetoxytetrahydropyran-3-yl] acetate (1.5 g, 35% yield) as a yellow solid. LCMS: 341.1 (M+Na⁺) ¹H NMR (400 MHz, CDCl₃): δ 6.348 (d, 1H), 5.379 - 5.347 (m, 3H), 4.061 (d, 1H), 3.824 (m, 1H), 2.139 (s, 6H), 2.039 (s, 6H) ppm

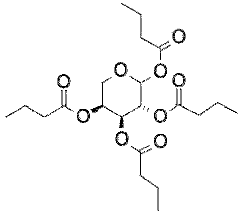

### Example 139: [(3S,4S,5R)-4,5,6-tri(butanoyloxy)tetrahydropyran-3-yl] butanoate

To a solution of (3R,4S,5S)-tetrahydropyran-2,3,4,5-tetrol (3 g), triethylamine (16.18 g) and 4-dimethylaminopyridine (0.488 g) in CH₂Cl₂ (30 mL) was added butanoic anhydride (19.34 g) at 0 °C. Then the solution was stirred 0 °C for 1 h and stirred at 15°C for another 15 h. The solvent was removed under reduced pressure. The crude product was purified by silica gel chromatography (petroleum ether) to give [(3S,4S,5R)-4,5,6-tri(butanoyloxy)tetrahydropyran-3-yl] butanoate (8 g, 93% yield) as yellow oil. LCMS: 453.3 (M+Na⁺) ¹H NMR (400 MHz, CDCl₃): δ 6.382 (d, 1H), 5.397 (m, 3H), 4.046 (d, 1H), 3.825 (m, 1H), 2.402 - 2.219 (m, 8H), 1.713 - 1.608 (m, 8H), 1.104 - 0.923 (m, 12H) ppm

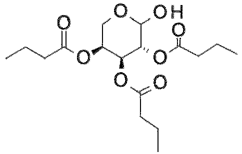

### Example 140: [(3S,4S,5R)-4,5-di(butanoyloxy)-6-hydroxy-tetrahydropyran-3-yl] butanoate

To a solution of [(3S,4S,5R)-4,5,6-tri(butanoyloxy)tetrahydropyran-3-yl] butanoate (1 g) in THF (20 mL) and H₂O (1 mL) was added methanamine in THF (2 M, 1.51 mL) and the mixture was stirred at 15 °C for 24 h. The solvent was removed under reduced pressure. The crude product was purified by silica gel chromatography (petroleum ether / ethyl acetate gradient) to give [(3S,4S,5R)-4,5-di(butanoyloxy)-6-hydroxy-tetrahydropyran-3-yl] butanoate (0.15 g, 17.7% yield) as yellow oil. LCMS: 383.1 (M+Na⁺) ¹H NMR (400 MHz, CDCl₃) (mixture of anomers): δ 5.411 (d, 1H, major anomer), 5.370 (dd, 1H, major anomer), 5.300 (br, 1H, major anomer), 5.235 (br, 1H, minor anomer), 5.154 (dd, 1H, major anomer), 5.040 (m, 1H) 5.557 (br, 1H, minor anomer) 4.139 (d, 1H, major anomer) 3.959 (dd, 1H, minor anomer), 3.642 (dd, 1H, major anomer), 3.620 (d, 1H, minor anomer), 3.350 (br d, 1H, minor anomer), 2.619 (br, 2H, minor anomer), 2.330 - 2.116 (m, 6H, major and minor anomer), 1.666 - 1.496 (m, 6H, major and minor anomer), 0.931 - 0.834 (9H, major and minor anomer).

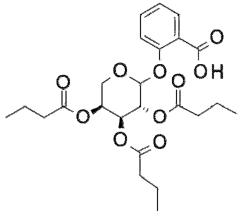

### Example 141: 2-[(3R,4S,5S)-3,4,5-tri(butanoyloxy)tetrahydropyran-2-yl]oxybenzoic acid

Step 1: A solution of *N,N*'-dicyclohexylcarbodiimide (8 g) in THF (50 mL) was added to a solution of salicylic acid (5 g) and 4-dimethylaminopyridine (0.17 g) in t-BuOH (100 mL) dropwise and the solution was stirred at 15 °C for 16 h. The crude product was purified by silica gel chromatography eluted with petroleum ether to give salicylic acid t-butyl ester (3 g, 43% yield) as colorless oil.

Step 2: To a solution of [(3S,4S,5R)-4,5-di(butanoyloxy)-6-hydroxy-tetrahydropyran-3-yl] butanoate (0.7 g) and salicylic acid t-butyl ester (0.358 g) in THF (30 mL) was added PPh₃ (0.728 g) and DTAD (0.671 g) in portions and the mixture was stirred at 15°C for 16 h. The solvent was removed under reduced pressure. The crude product was purified by reverse phase prep-HPLC (C18, [water(0.1 %trifluoroacetic acid)-acetonitrile]) to give tert-butyl 2-[(3R,4S,5S)-3,4,5-tri(butanoyloxy)tetrahydropyran-2-yl]oxybenzoate (0.4 g, 38% yield) as yellow oil.

Step 3: To a solution of tert-butyl 2-[(3R,4S,5S)-3,4,5-tri(butanoyloxy)tetrahydropyran-2-yl]oxybenzoate (0.2 g) in CH₂Cl₂ (10 mL) was added trifluoroacetic acid (3.08 g) and the mixture was stirred at 15°C for 16 h under N₂. The solvent was removed under reduced pressure. The crude product was purified by reverse phase prep-HPLC (C18, [water(0.1%trifluoroacetic acid)-acetonitrile]) to give 2-[(3R,4S,5S)-3,4,5-tri(butanoyloxy)tetrahydropyran-2-yl]oxybenzoic acid (0.030 g, 16% yield) as yellow oil. LCMS: 503.1 (M+Na⁺) ¹H NMR (400 MHz, CDCl₃): δ 8.202 (dd, 1H), 7.567 (m, 1H), 7.234 (m, 2H), 5.475 (m, 1H), 5.424 - 5.351 (m, 3H), 4.067 (dd, 1H), 3.783 (dd, 1H), 2.445 - 2-297 (m, 6H), 1.726 - 1.543 (m, 6H), 0.997 - 0.920 (m, 9H) ppm

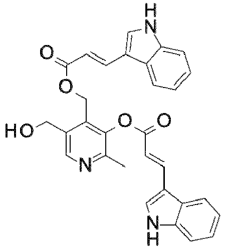

### Example 142: [5-(hydroxymethyl)-3-[(E)-3-(1H-indol-3-yl)prop-2-enoyl]oxy-2-methyl-4-pyridyl]methyl (E)-3-(1H-indol-3-yl)prop-2-enoate

Step 1: A solution of (E)-3-(1-tert-butoxycarbonylindol-3-yl)prop-2-enoic acid (0.6 g), pyridoxine (2.04 g), *N,N*'-dicyclohexylcarbodiimide (1.46 g) and 4-dimethylaminopyridine (0.433 g) in CH₂Cl₂ (30 mL) and DMF (10 mL) was stirred at 15°C for 16 h. The solvent was removed under reduced pressure. The crude product was purified by reverse phase prep-HPLC (C18, [water(0.1 %trifluoroacetic acid)-acetonitrile]) to give tert-butyl 3-[(E)-3-[[3-[(E)-3-(1-tert-butoxycarbonylindol-3-yl)prop-2-enoyl]oxy-5-(hydroxymethyl)-2-methyl-4-pyridyl]methoxy]-3-oxo-prop-1-enyl]indole-1-carboxylate (0.7 g, 28% yield) as a white solid.

Step 2: To the solution of tert-butyl 3-[(E)-3-[[3-[(E)-3-(1-tert-butoxycarbonylindol-3-yl)prop-2-enoyl]oxy-5-(hydroxymethyl)-2-methyl-4-pyridyl]methoxy]-3-oxo-prop-1-enyl]indole-1-carboxylate (0.2 g) in CH₂Cl₂ (20 mL) was added trifluoroacetic acid (0.032 g) and the solution was stirred at 15°C for 16 h. The solvent was removed under reduced pressure. The crude product was purified by reverse phase prep-HPLC (C18, [water(10 mM NH₄HCO₃)-acetonitrile]) to give [5-(hydroxymethyl)-3-[(E)-3-(1H-indol-3-yl)prop-2-enoyl]oxy-2-methyl-4-pyridyl]methyl (E)-3-(1H-indol-3-yl)prop-2-enoate (0.025 g) as a yellow solid. LCMS: 508.2 (M+H⁺) ¹H NMR (400 MHz, DMSO-d6): δ 8.407 (s, 1H), 8.130 (s, 1H), 8.090 (s, 1H), 7.737 (br, 1H), 7.840 - 7.714 (m, 3H), 7.457 - 7.417 (m, 2H), 7.175 (dd, 2H), 7.050 (dd, 2H), 6.620 (d, 1H), 6.252 (d, 1H), 5.228 (s, 2H), 4.717 (s, 2H), 2.362 (s, 3H) ppm

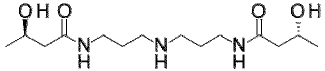

### Example 143: (3R)-3-hydroxy-N-[3-[3-[[(3R)-3-hydroxybutanoyl]amino]propylamino]propyl] butanamide

Step 1: To a mixture of N'-(3-aminopropyl)butane-1,4-diamine (0.15 g) and (2,5-dioxopyrrolidin-1-yl) (3R)-3-[tert-butyl(diphenyl)silyl]oxybutanoate (1.45 g) in THF (5 mL) was added triethylamine (0.334 g) in one portion at 15°C under N₂. The mixture was stirred at 15 °C for 12 h. The reaction mixture was filtered and concentrated under reduced pressure to give (3S)-3-[tert-butyl(diphenyl)silyl]oxy-N-[3-[3-[[(3R)-3-[tert-butyl(diphenyl) silyl]oxybutanoyl]amino]propylamino]propyl]butanamide (1 g) as a colorless oil which was used in the next step without further purification.

Step 2: To a mixture of (3S)-3-[tert-butyl(diphenyl)silyl]oxy-N-[3-[3-[[(3R)-3-[tert-butyl(diphenyl)silyl]oxybutanoyl]amino]propylamino]propyl]butanamide (1 g) in THF (5 mL) was added pyridine hydrofluoride (0.635 g) in one portion at 15°C under N₂. The mixture was stirred at 15 °C for 12 h. The reaction mixture was filtered and concentrated under reduced pressure to give a residue which was purified by reverse phase prep-HPLC [water(0.05%HCl)-acetonitrile] to give (3S)-3-hydroxy-N-[3-[3-[[(3R)-3- hydroxybutanoyl]amino]propylamino]propyl]butanamide (0.11 g, 28% yield) as a colorless oil. LCMS: 304.2 (M+H⁺) ¹H NMR (400 MHz, DMSO-d6): δ 8.726 (br s, 2H), 0.803 (br, 2H), 3.957 (m, 2H), 3.149 - 3.079 (m, 3H), 2.842 - 2.813 (m, 4H), 2.111 -2.076 (m, 4H), 1.755 - 1.685 (m, 4H), 1.049 (d, 6H) ppm

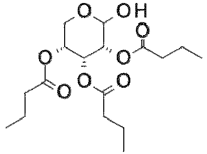

### Example 144: [(3R,4R,5R)-4,5-di(butanoyloxy)-6-hydroxy-tetrahydropyran-3-yl] butanoate

Step 1: To a mixture of (3R,4R,5R)-tetrahydropyran-2,3,4,5-tetrol (15 g), triethylamine (80.88 g) and 4-dimethylaminopyridine (2.44 g) in CH₂Cl₂ (30 mL) was added butanoyl butanoate (94.83 g) at 0 °C. The mixture was stirred at 15 °C for 12 h. The reaction mixture was filtered and concentrated under reduced pressure and the residue was purified by column chromatography (SiO₂, petroleum ether / ethyl acetate gradient) to give [(3R,4R,5R)-4,5,6-tri(butanoyloxy)tetrahydropyran-3-yl] butanoate (30 g) as a colorless oil.

Step 2: To a solution of [(3R,4R,5R)-4,5,6-tri(butanoyloxy)tetrahydropyran-3-yl] butanoate (6 g) in THF (60 mL) and H₂O (10 mL) was added MeNH₂ (2 M in THF, 16.03 mL) and the mixture was stirred at 15°C for 12 h. The mixture was combined with additional batches and concentrated. The residue was purified by column chromatography (SiO₂, petroleum ether / ethyl acetate gradient) to give [(3R,4R,5R)-4,5-di(butanoyloxy)-6-hydroxy-tetrahydropyran-3-yl]butanoate (2.8 g) as a brown oil. LCMS: 383.1 (M+Na⁺) ¹H NMR (400 MHz, methanol-d4): δ 5.533 (br, 1H), 5.071 (m, 1H), 4.990 (m, 1H), 4.849 (m, 1H), 4.041 (dd, 1H), 3.751 (dd, 1H), 2.373 - 2.292 (m, 6H), 1.697 1.626 (m, 6H), 0.989 - 0.930 (m, 9H) ppm

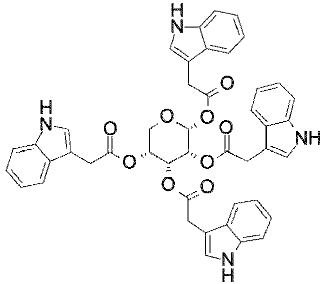

### Example 145: [(3R,4R,5R,6R)-4,5,6-tris[[2-(1H-indol-3-yl)acetyl]oxy]tetrahydropyran-3-yl] 2-(1H-indol-3-yl)acetate

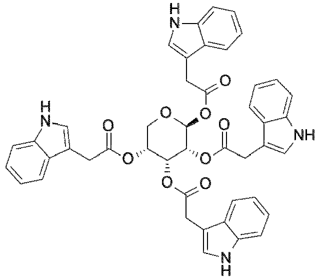

### Example 146: [(3R,4R,5R,6S)-4,5,6-tris[[2-(1H-indol-3-yl)acetyl]oxy]tetrahydropyran-3-yl] 2-(1H-indol-3-yl)acetate

To a solution of (3R,4R,5R)-tetrahydropyran-2,3,4,5-tetrol (0.200 g) and 2-(1H-indol-3-yl)acetic acid (1.87 g) in CH₂Cl₂ (20 mL) was added (1-Cyano-2-ethoxy-2-oxoethylidenaminooxy)dimethylamino-morpholino-carbenium hexafluorophosphate (COMU) (3.42 g) and Hunig's base (1.38 g). The mixture was stirred at 50 °C for 12 h. The mixture was concentrated and the residue was purified by reverse phase prep-HPLC (C18, [water (10 mM NH₄HCO₃)-acetonitrile]) to give two isomers (150 mg and 600 mg) as yellow solids. [(3R,4R,5R,6R)-4,5,6-tris[[2-(1H-indol-3-yl)acetyl]oxy]tetrahydropyran-3-yl] 2-(1H-indol-3-yl)acetate
LCMS: 777.2 (M-H) ¹H NMR (400 MHz, methanol-d4): δ 7.452 (m, 3H), 7.330 - 7.263 (m, 5H), 7.101 -6.939 (m, 12H), 6.093 (s, 1H), 5.235 (d, 1H), 5.056 (dd, 1H), 4.116 (dd, 1H), 3.762 (dd, 1H), 3.656 (dd, 1H), 3.592 (m, 2H), 3.475 - 3.379 (m, 4H), 3.245 (s, 2H) ppm [(3R,4R,5R,6S)-4,5,6-tris[[2-(1H-indol-3-yl)acetyl]oxy]tetrahydropyran-3-yl] 2-(1H-indol-3-yl)acetate
LCMS: 777.1 (M-H) ¹H NMR (400 MHz, methanol-d4): δ 7.5 - 7.296 (m, 5H), 7.112 -6.952 (m, 12H), 5.801 (d, 1H), 5.502 (m, 1H), 4.909 (m, 2H), 3.642 - 3.263 (m, 10H) ppm

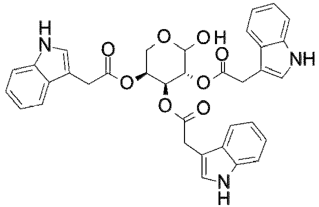

### Example 147: [(3S,4S,5R)-6-hydroxy-4,5-bis[[2-(1H-indol-3-yl)acetyl]oxy]tetrahydropyran-3-yl] 2-(1H-indol-3-yl)acetate

Step 1: A solution of (3R,4S,5S)-tetrahydropyran-2,3,4,5-tetrol (2 g), 2-(1H-indol-3-yl)acetic acid (18.67 g), COMU (34.23 g) and DIPEA (13.77 g) in CH₂Cl₂ (200 mL) was stirred at 50°Cfor 16 h under N₂. The solvent was removed under reduced pressure. The crude product was purified by reverse phase prep-HPLC (C18, [water (10 mM NH₄HCO₃)-acetonitrile]) to give [(3S,4S,5R)-4,5,6-tris[[2-(1H-indol-3-yl)acetyl]oxy]tetrahydropyran-3-yl] 2-(1H-indol-3-yl)acetate (3 g, 29% yield) as yellow solid.

Step 2: A solution of [(3S,4S,5R)-4,5,6-tris[[2-(1H-indol-3-yl)acetyl]oxy]tetrahydropyran-3-yl] 2-(1H-indol-3-yl)acetate (1 g) and methanamine in THF (2 M, 1.28 mL) in THF(10 mL) and H₂O (2 mL) was stirred at 15°C for 16 h. The solvent was removed under reduced pressure. The crude product was purified by reverse phase prep-HPLC (C18, [water(10 mM NH₄HCO₃)-acetonitrile]) to give [(3S,4S,5R)-6-hydroxy-4,5-bis[[2-(1H-indol-3-yl)acetyl]oxy]tetrahydropyran-3-yl] 2-(1H-indol-3-yl)acetate (0.185 g, 11% yield) as a yellow solid. LCMS: 639.3 (M+H₂O⁺): 639.3 ¹H NMR (400 MHz, methanol-d4) (mixture of anomers): δ 7.258 -6.477 (m, 15H), 5.339 - 5.127 (m), 4.998 -4.928, (m), 4.606 (d), 4.133 (m), 3.815 (m), 3.572 - 520 (m), 3.457 (br s), 3.303 (br s), 2.934 (br s), 2.881 (br s) ppm

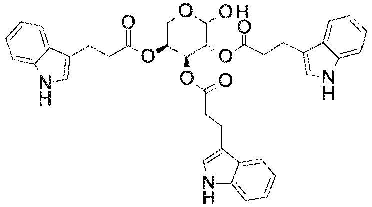

### Example 148: [(3S,4S,5R)-6-hydroxy-4,5-bis[3-(1H-indol-3-yl)propanoyloxy]tetrahydropyran-3-yl] 3-(1H-indol-3-yl )propanoate

Step 1: A solution of (3R,4S,5S)-tetrahydropyran-2,3,4,5-tetrol (2 g), 3-(1H-indol-3-yl)propanoic acid (17.6 g), COMU (34.2 g), DIPEA (13.77 g) in CH₂Cl₂ (50 mL) was stirred at 50°C for 16 h. The solvent was removed under reduced pressure. The crude product was purified by reverse phase prep-HPLC (C18, [water(10 mM NH₄HCO₃)-acetonitrile]) to give [(3S,4S,5R)-4,5,6-tris[3-(1H-indol-3-yl)propanoyloxy]tetrahydropyran-3-yl] 3-(1H-indol-3-yl)propanoate (2 g) as a brown solid.

Step 2: A solution of [(3S,4S,5R)-4,5,6-tris[3-(1H-indol-3-yl)propanoyloxy]tetrahydropyran-3-yl] 3-(1H-indol-3-yl)propanoate (1 g) and methanamine in THF (2 M, 1.08 mL) in THF (10 mL) and H₂O (2 mL) was stirred at 15°C for 16 h. The solvent was removed under reduced pressure. The crude product was purified by reverse phase prep-HPLC (C18 [water (0.04%NH₃ H₂O +10 mM NH₄HCO₃)-acetonitrile]) to give [(3S,4S,5R)-6-hydroxy-4,5-bis[3-(1H-indol-3-yl)propanoyloxy]tetrahydropyran-3-yl]3-(1H-indol-3-yl)propanoate (0.18 g, 22% yield) as a yellow solid. LCMS: 681.2 (M+18) ¹H NMR (400 MHz, CDCl₃) (mixture of anomers, signals reported for major anomer): δ 7.763 - 6.599 (m, 18H), 5.410 - 5.075) (m, 4H), 4.148 (m, 1H), 3.638 (m, 1H), 3.122 - 2.183 (m, 12H) ppm

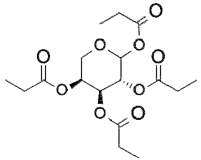

### Example 149: [(3S,4S,5R)-4,5,6-tri(propanoyloxy)tetrahydropyran-3-yl] propanoate

To a solution of (3R,4S,5S)-tetrahydropyran-2,3,4,5-tetrol (10 g) in pyridine (100 mL) was added propionic anhydride (52 g) at 15 °C. The mixture was stirred at 15 °C for 16 hr. The reaction mixture was concentrated under reduced pressure. The residue was purified by column chromatography (SiO₂, petroleum ether / ethyl acetate gradient) to give [(3S,4S,5R)-4,5,6-tri(propanoyloxy)tetrahydropyran-3-yl] propanoate (21 g, 84% yield) as a white solid. LCMS: 397.1 (M+Na⁺) ¹H NMR (400 MHz, CDCl₃) (mixture of anomers, signals for major anomer reported): δ 6.371 (m, 1H), 5.691 (d, 1H), 5.407 - 5.379 (m, 3H), 4.024 (m, 1H), 3.835 (m, 1H), 2.433 - 2.259 (m, 8H), 1.181 - 1.095 (m, 12H) ppm

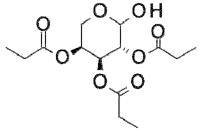

### Example 150: [(3S,4S,5R)-6-hydroxy-4,5-di(propanoyloxy)tetrahydropyran-3-yl] propanoate

To a solution of [(3S,4S,5R)-4,5,6-tri(propanoyloxy)tetrahydropyran-3-yl] propanoate (44 g) in THF (500 mL) was added MeNH₂ (40%, 18.25 g, 2 eq) at 15 °C. The mixture was stirred at 15 °C for 16 hr. The reaction mixture was concentrated under reduced pressure and the residue was purified by column chromatography (SiO₂, petroleum ether / ethyl acetate gradient) to give [(3S,4S,5R)-6-hydroxy-4,5-di(propanoyloxy)tetrahydropyran-3-yl] propanoate (15 g, 39% yield) as a yellow oil. LCMS: 336.1 (M+18) ¹H NMR (400 MHz, CDCl₃) (mixture of anomers, signals for major anomer reported): δ 5.479 (m, 1H), 5.372 (m, 1H), 5.370 (m, 1H), 5.228 (m, 1H), 4.216 (m, 1H), 3.707 (m, 1H), 2.432 -2.258 (m, 6H), 1.195 - 1.106 (3 x s, 9H) ppm

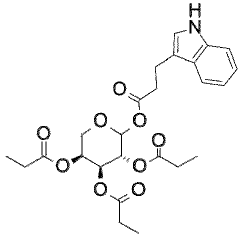

### Example 151: [(3R,4S,5S)-3,4,5-tri(propanoyloxy)tetrahydropyran-2-yl] 3-(1H-indol-3-yl)propanoate

To a solution of [(3S,4S,5R)-6-hydroxy-4,5-di(propanoyloxy)tetrahydropyran-3-yl] propanoate (0.200 g), 3-(1H-indol-3-yl)propanoic acid (0.238 g) and 4-dimethylaminopyridine (0.115 g) in THF (2 mL) was added N,N-dicyclohexylcarbodiimide (0.194 g) at 15 °C. The mixture was stirred at 15 °C for 16 hr. The reaction mixture was concentrated under reduced pressure and the residue was purified by prep-TLC (SiO₂, petroleum ether / ethyl acetate, 1:1) to give [(3R,4S,5S)-3,4,5-tri(propanoyloxy)tetrahydropyran-2-yl] 3-(1H-indol-3-yl)propanoate (0.043 g, 13.9% yield) as a colorless solid. LCMS: 512.2 (M+Na⁺) ¹H NMR (400 MHz, CDCl₃) (mixture of anomers, signals for major anomer reported): δ 8.033 (br, 1H), 7.619 (m, 1H), 7.369 (m, 1H), 7.208 - 7.028 (m, 3H), 7.362 (m, 1H), 5.382 - 5.517 (m, 3H), 3.822 (m, 1H), 3.718 (m, 1H), 3.155 (m, 2H), 2.760 (m, 2H), 2.456 2.104 (m, 6H), 1.180 (s, 3H), 1.116 (s, 3H), 1.023 (s, 3H) ppm

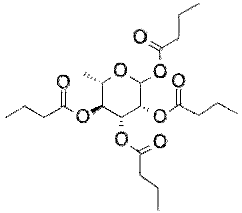

### Example 152: [(2S,3S,4R,5R)-4,5,6-tri(butanoyloxy)-2-methyl-tetrahydropyran-3-yl] butanoate

To a mixture of (3R,4R,5R,6S)-6-methyltetrahydropyran-2,3,4,5-tetrol (1 g), triethylamine (4.93 g) and 4-dimethylaminopyridine (0.149 g) in CH₂Cl₂ (30 mL) was added butanoyl butanoate (5.78 g) in one portion at 0°C under N₂. The mixture was stirred at 15 °C for 12 h. The reaction mixture was filtered and concentrated under reduced pressure to give a residue. The residue was purified by column chromatography (SiO₂, petroleum ether / ethyl acetate gradient) to give [(2S,3S,4R,5R)-4,5,6-tri(butanoyloxy)-2-methyl-tetrahydropyran-3-yl] butanoate (1.5 g) as a colorless oil. LCMS: 462.3 (M+18) ¹H NMR (400 MHz, CDCl₃): δ 6.017 (d, 1H), 5.330 5.276 (m, 2H), 5.263 (m, 1H), 3.927 (m, 1H), 2.400 - 2.193 (m, 8H), 1.705 -1.638 (m, 8H), 1.218 (d, 3H), 1.002 - 0.901 (m, 12H) ppm

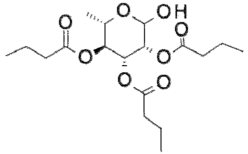

### Example 153: [(2S,3S,4R,5R)-4,5-di(butanoyloxy)-6-hydroxy-2-methyl-tetrahydropyran-3-yl] butanoate

Step 1: To a mixture of (3R,4R,5R,6S)-6-methyltetrahydropyran-2,3,4,5-tetrol (20 g), triethylamine (98.6 g) and 4-dimethylaminopyridine (2.98 g) in CH₂Cl₂ (150 mL) was added butanoyl butanoate (115.6 g) in one portion at 0 °C under N₂. The mixture was stirred at 15 °C for 12 h. The reaction mixture was filtered and concentrated under reduced pressure to give a residue which was purified by column chromatography (SiO₂, petroleum ether / ethyl acetate gradient) to give [(2S,3S,4R,5R)-4,5,6-tri(butanoyloxy)-2-methyl- tetrahydropyran-3-yl] butanoate (25 g, crude) as a colorless oil.

Step 2: To a mixture of [(2S,3S,4R,5R)-4,5,6-tri(butanoyloxy)-2-methyl-tetrahydropyran-3-yl] butanoate (25 g) in THF (250 mL) was added MeNH₂ in THF (2 M, 36.6 mL) in one portion at 15°C under N₂. The mixture was stirred at 15 °C for 12 h. The reaction mixture was filtered and concentrated under reduced pressure to give a residue. The residue was purified by column chromatography (SiO₂, petroleum ether / ethyl acetate gradient) to give [(2S,3S,4R,5R)-4,5-di(butanoyloxy)-6-hydroxy-2-methyltetrahydropyran-3-yl] butanoate (16 g, 72% yield) as a colorless oil. ¹H NMR (400 MHz, CDCl₃): δ 5.311 (m, 1H), 5.225 (m, 1H), 5.077 -4.886 (m, 2H), 4.074 (m, 1H), 3.401 (m, 1H), 2.386 - 2.111 (m, 6H), 1.624 - 1.482 (m, 6H), 1.142 (d, 3H), 0.931 - 0.831 (m, 9H) ppm

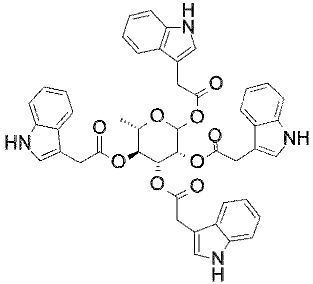

### Example 154: [(2S,3S,4R,5R)-4,5,6-tris[[2-(1H-indol-3-yl)acetyl]oxy]-2-methyl-tetrahydropyran-3-yl] 2-(1H-indol-3-yl)acetate

To a mixture of 2-(1H-indol-3-yl)acetic acid (4.27 g) and (3R,4R,5R,6S)-6-methyltetrahydropyran-2,3,4,5-tetrol (0.5 g) in CH₂Cl₂ (30 mL) was added Hünig's base (3.15 g) and COMU (7.83 g) in one portion at 15°C under N2. The mixture was stirred at 15 °C for 12 h. The reaction mixture was filtered and concentrated under reduced pressure to give a residue. The residue was purified by prep-HPLC [water(10 mM NH₄HCO₃)-acetonitrile] to give two isomers of [(2S,3S,4R,5R)-4,5,6-tris[[2-(1H-indol-3-yl)acetyl]oxy]-2-methyl-tetrahydropyran-3-yl] 2-(1H-indol-3-yl)acetate.
Isomer 1: LCMS 791.1 (M-H⁺) ¹H NMR (400 MHz, DMSO-d6): δ 10.978 - 10.929 (br m, 4H), 7.552 - 6.907 (m, 20H), 6.050 (s, 1H), 5.412 (d, 1H), 5.279 (dd, 1H), 5.007 (t, 1H), 3.833 (m, 1H), 3.669 - 3.552 (m, 6H), 3.180 - 3.009 M, 2H), 1.022 (d, 3H) ppm
Isomer 2: LCMS 791.1 (M-H⁺) ¹H NMR (400 MHz, DMSO-d6): δ 10.998 - 10.938 (br m, 4H), 7.497 - 6.993 (m, 20H), 5.860 (d, 1H), 5.192 - 5.127 (m, 2H), 5.018 (t, 1H), 3.910 (d, 1H), 3.809 (d, 1H), 3.681 - 3.544 (m, 6H), 3.172 (s, 2H), 0.836 (d, 3H) ppm

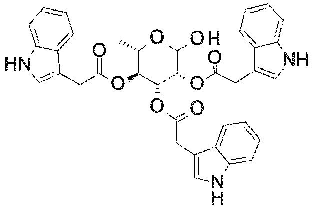

### Example 155: [(2S,3S,4R,5R)-6-hydroxy-4,5-bis[[2-(1H-indol-3-yl)acetyl]oxy]-2-methyl-tetrahydropyran-3-yl] 2-(1H-indol-3-yl)acetate

To a mixture of [(2S,3S,4R,5R)-4,5,6-tris[[2-(1H-indol-3-yl)acetyljoxy]-2-methyl-tetrahydropyran-3-yl] 2-(1H-indol-3-yl)acetate (0.02 g,) isomers in THF (1 mL) and H₂O (0.2 mL) was added MeNH₂ in THF (2 M, 16.40 µL) in one portion at 15°C under N₂. The mixture was stirred at 15 °C for 12h. The reaction mixture was filtered and concentrated under reduced pressure to give a residue which was purified by reverse phase prep-HPLC [water(0.04% NH₃·H₂O + 10 mM NF₄HCO₃)-acetonitrile] to give [(2S,3S,4R,5R)-6-hydroxy-4,5-bis[[2-(1H-indol-3-yl)acetyl]oxy]- 2-methyl-tetrahydropyran-3-yl] 2-(1H-indol-3-yl)acetate (6 mg, 37% yield) as a yellow solid. LCMS: 634.2 (M-H⁺) ¹H NMR (400 MHz, DMSO-d6) (mixture of anomers): δ 10.970 - 10.900 (m, 3H), 7.512 - 6.995 (m, 15H), 5.168 - 4.930 (m, 4H), 3.950 - 3.557 (m, 7H), 1.019 -0.969 (m 3H) ppm

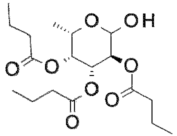

### Example 156: [(2S,3R,4R,5S)-4,5-di(butanoyloxy)-6-hydroxy-2-methyl-tetrahydropyran-3-yl] butanoate

To a mixture of [(2S,3R,4R,5S)-4,5,6-tri(butanoyloxy)-2-methyl-tetrahydropyran-3-yl] butanoate (6 g) in THF (60 mL) and H₂O (6 mL) was added MeNH₂ in THF (2 M, 10.12 mL) in one portion at 15°C under N₂. The mixture was stirred at 15 °C for 12 h. The reaction mixture was concentrated under reduced pressure to give a residue. The residue was purified by column chromatography (SiO₂, petroleum ether / ethyl acetate gradient) to give [(2S,3R,4R,5S)-4,5-di(butanoyloxy)-6-hydroxy-2-methyltetrahydropyran-3-yl] butanoate (7.9 g, 39% yield) as a yellow oil. LCMS: 392.2 (M+18) ¹H NMR (400 MHz, DMSO-d6) (mixture of anomers, major anomer reported): δ 6.935 (d, 1H), 5.303 (m, 1H), 5.190 (m, 2H), 4.912 (m, 1H), 4.318 (m, 1H), 2.401 - 2.253 (m, 6H), 1.596 - 1.500 (m, 6H), 1.013 - 0.817 (m, 12H) ppm

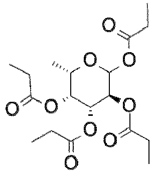

### Example 157: [(2S,3R,4R,5S)-2-methyl-4,5,6-tri(propanoyloxy)tetrahydropyran-3-yl] propanoate

To a solution of (2S,3R,4R,5S)-2,3,4,5-tetrahydroxyhexanal (1 g) in pyridine (10 mL) was added propanoyl propanoate (4.36 g). The mixture was stirred at 15 °C for 12 hr. The reaction mixture was filtered and concentrated under reduced pressure and the residue was purified by column chromatography (SiO₂, petroleum ether / ethyl acetate gradient) to give [(2S,3R,4R,5S)-2-methyl-4,5,6-tri(propanoyloxy)tetrahydropyran-3-yl] propanoate (0.8 g, 34% yield) as a colorless oil. LCMS: (M+H₂O⁺) and 406.2 (M+Na⁺) 411.1 ¹H NMR (400 MHz, CDCl₃) (mixture of anomers, signals for major anomer reported): δ 3.367 (m, 1H), 3.353 (m, 3H), 4.292 (m, 1H) 2.294 - 2.241 (m, 8H), 1.224 - 0.934 (m, 15H)

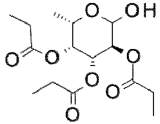

### Example 158: [(2S,3R,4R,5S)-6-hydroxy-2-methyl-4,5-di(propanoyloxy)tetrahydropyran-3-yl] propanoate

To a solution of [(2S, 3R,4R,5S)-2-methyl-4,5,6-tri(propanoyloxy)tetrahydropyran-3-yl] propanoate (5 g) in THF (50 mL) was added MeNH₂ in H₂O (40%, 1.80 g). The mixture was stirred at 15 °C for 12 hr. The reaction mixture was concentrated under reduced pressure and the residue was purified by column chromatography (SiO₂, petroleum ether / ethyl acetate gradient) to give [(2S,3R,4R,5S)-6-hydroxy-2-methyl-4,5-di(propanoyloxy) tetrahydropyran-3-yl]propanoate (18 g, 83% yield) as a colorless oil. LCMS: 350.1 (M+H₂O⁺) & 355.1 (M+Na⁺) ¹H NMR (400 MHz, CDCl³) (mixture of anomers, approx. 1:1): δ 5.477 - 5.052 (m), 4.678 (m), 4.437 (m), 3.881 (m), 2.492 - 2.232 (m, 6H), 1.220 - 0.950 (m, 12H) ppm

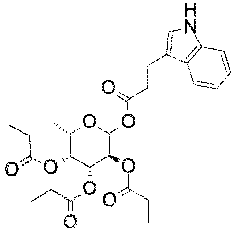

### Example 159 : [(3S,4R,5R,6S)-6-methyl-3,4,5-tri(propanoyloxy)tetrahydropyran-2-yl] 3-(1H-indol-3-yl)propanoate

To a mixture of [(2S,3R,4R,5S)-6-hydroxy-2-methyl-4,5-di(propanoyloxy)tetrahydropyran-3-yl] propanoate (0.5 g) and 3-(1H-indol-3-yl)propanoic acid (0.427 g) in THF (5 mL) was added N,N'-dicyclohexylcarbodiimide (0.621 g) and 4-dimethylaminopyridine (0.092 g) in one portion at 15°C under N₂. The mixture was stirred at 15 °C for 12 h. The reaction mixture was filtered and concentrated under reduced pressure and the residue was purified by prep-TLC (SiO₂, petroleum ether/ ethyl acetate, 5:1) to give [(3S,4R,5R,6S)-6-methyl-3,4,5-tri(propanoyloxy)tetrahydropyran -2-yl]3-(1H-indol-3-yl) propanoate (0.35 g, 44% yield) as a colorless oil. LCMS: 504.2 (M+H⁺) & 521.2 (M+H₂O⁺) & 526.2 (M+Na⁺) ¹H NMR (400 MHz, CDCl₃) (mixture of anomers, signals for major anomer reported): δ 8.056 (br, 1H), 7.623 (m, 1H), 7.359 (m, 1H), 7.190 - 7.018 (m, 3H), 6.327 (m, 1H), 5.380 - 5.257 (m, 3H), 3.969 (m, 1H), 3.134 (m, 2H), 2.834 (m, 2H), 2.440 (m, 2H), 2.249 (m, 2H), 2.110 (m, 2H), 1.214 - 1.016 (m, 12H) ppm

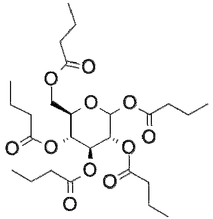

### Example 160: [(2R,3R,4S,5R)-3,4,5,6-tetra(butanoyloxy)tetrahydropyran-2-yl]methyl butanoate

To a solution of (2R,3S,4R,5R)-2,3,4,5,6-pentahydroxyhexanal (20 g) in CH₂Cl₂ (500 mL) was added butanoyl chloride (94.6 g). The mixture was stirred at 15°C for 0.5 h, and then pyridine (70.25 g) was slowly added to the solution dropwise. The mixture was stirred at 15°C for another 15.5 h. The solvent was removed under reduced pressure and the crude product was purified by silica gel chromatography (petroleum ether / ethyl acetate gradient) to give [(2R,3R,4S,5R)-3,4,5,6-tetra(butanoyloxy)tetrahydropyran-2-yl]methyl butanoate (58 g, 98% yield) as a yellow oil. ¹H NMR (400 MHz, CDCl₃): δ 6.357 (d, 1H), 5.503 (t, 1H), 5.163 - 5.089 (m, 2H), 4.202 (m, 1H), 4.127 4.093 (m, 2H), 2.460 -2.207 (m, 10H), 1.656 -1.569 (m, 10H), 0.990 - 0.893 (m, 15H) ppm

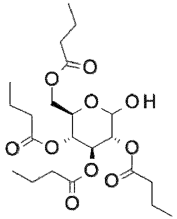

### Example 161: [(2R,3R,4S,5R)-3,4,5-tri(butanoyloxy)-6-hydroxy-tetrahydropyran-2-yl]methyl butanoate

To a solution of [(2R,3R,4S,5R)-3,4,5,6-tetra(butanoyloxy)tetrahydropyran-2-yl]methyl butanoate (10 g) in THF (85 mL) and H₂O (5 mL) was added methanamine in THF (2 M, 12.25 mL), and then the mixture was stirred at 15°C for 16 h. The solvent was removed under reduced pressure. The crude product was purified by chromatography over silica gel (petroleum ether / ethyl acetate gradient) to give [(2R,3R,4S,5R)-3,4,5-tri(butanoyloxy)-6-hydroxy-tetrahydropyran-2-yl]methyl butanoate (10 g, 57 % yield) as a yellow oil. LCMS: 483.3 (M+Na⁺) ¹H NMR (400 MHz, CDCl₃) (mixture of anomers, signals for major anoner reported): δ 5.570 (t, 1H), 5.464 (br, 1H), 5.117 (m, 1H), 4.928 (m, 1H), 4.215 -4.132 (m, 3H), 2.351 - 2.216 (8H), 1.649 -1.571 (m, 8H), 0.967 - 0.895 (m, 12H) ppm

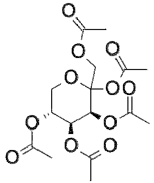

### Example 162: [(3S,4S,5R)-2,3,4,5-tetraacetoxytetrahydropyran-2-yl]methyl acetate

To a solution of (3S, 4S, 5R)-2-(hydroxymethyl) tetrahydropyran-2, 3, 4, 5-tetrol (6 g) in pyridine (50 mL) was added Ac₂O (65.40 g), and the mixture was stirred at 15 °C for 12 h. The reaction mixture was concentrated under reduced pressure. The residue was purified by column chromatography (SiO₂, petroleum ether / ethyl acetate gradient) to give [(3S,4S,5R)-2,3,4,5-tetraacetoxytetrahydropyran-2-yl]methyl acetate (6 g, 43.9% yield) as a yellow solid. LCMS: 493.1 (M+Na⁺) ¹H NMR (400 MHz, CDCl₃): δ 5.461 (d, 1H), 5.327 (dd, 1H), 5.250 (m, 1H), 4.797 (d, 1H), 4.415 (d, 1H), 4.102 (dd, 1H), 3.496 (t, 1H), 2.165 (s, 3H0, 2.134 (s, 3H), 2.050 (s, 3H), 2.019 (s, 3H), 1.999 (s, 3H) ppm

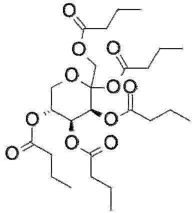

### Example 163: [(3S,4S,5R)-2,3,4,5-tetra(butanoyloxy)tetrahydropyran-2-yl]methyl butanoate

This compound was prepared using the approach described in Example 162, with the exception that butyric anhydride was used instead of acetic anhydride. LCMS: 553.2 (M+Na⁺) ¹H NMR (400 MHz, CDCl₃): δ 5.012 (dd, 1H), 5.373 (m, 1H), 5.287 (m, 1H), 4.847 (d, 1H), 4.348 (d, 1H), 4.096 (m, 1H), 3.514 (t, 1H), 2.443 - 2.207 (m, 10H), 1.716 - 1.597 (m, 10H), 1.003 - 0.910 (m, 15H) ppm

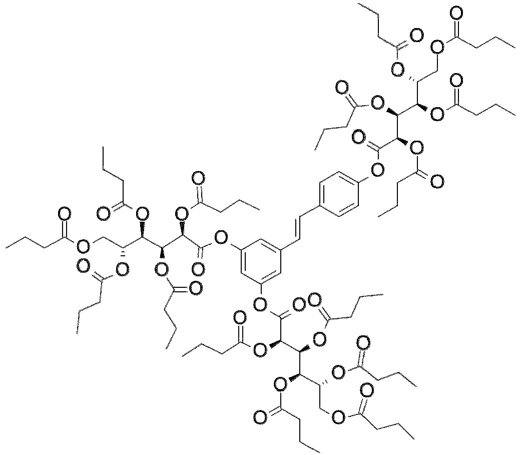

### Example 164: [4-[(E)-2-[3,5-bis[[(2R,3S,4R,5R)-2,3,4,5,6-penta(butanoyloxy)hexanoyl]oxy]phenyl]vinyl]phenyl] (2R,3S,4R,5R)-2,3,4,5,6-penta(butanoyloxy)hexanoate

To a solution of 5-[(E)-2-(4-hydroxyphenyl)vinyl]benzene-1,3-diol (0.050 g), *N,N'-*dicyclohexylcarbodiimide (0.180 g) and 4-dimethylaminopyridine (0.008 g) in THF (5 mL) was added (2R,3S,4R,5R)-2,3,4,5,6-penta(butanoyloxy)hexanoic acid (0.479 g) and the mixture was stirred at 40 °C for 12 h. The mixture reaction was filtered and concentrated. The residue was purified by column chromatography (SiO₂, petroleum ether / ethyl acetate gradient) to give [4-[(E)-2-[3,5-bis[[(2R,3S,4R,5R)-2,3,4,5,6-penta(butanoyloxy)hexanoyl] oxy]phenyl]vinyl]phenyl] (2R, 3S, 4R, 5R)-2,3,4,5,6-penta(butanoyloxy)hexanoate (0.080 g,16% yield) as a colorless oil. ¹H NMR (400 MHz, CDCl₃): δ 7.529 - 7.481 (m, 2H), 7.177 - 7.089 (m, 5H), 6.669 (m, 1H), 6.825 (m, 1H), 5.781 (m, 2H), 5.495 - 5.483 (m , 2H), 5.341- 5.305 (m, 2H), 5.289 - 5.184 (m, 2H), 5.053 (d, 1H), 4.728 (dd, 1H), 4.375 (m, 2H), 4.156 - 3.989 (m, 4H), 2.474 - 2.221 (m, 30H), 1.597 - 1.566 (m, 30H), 0.923 - 0.847 (m, 45H) ppm

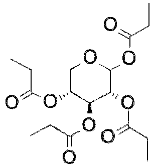

### Example 165: [(3R,4S,5R)-4,5,6-tri(propanoyloxy)tetrahydropyran-3-yl] propanoate

To a solution of (2R,3S,4R)-2,3,4,5-tetrahydroxypentanal (10 g) in pyridine (100 mL) was added propanoyl propanoate (47.68 g) and the mixture was stirred at 15 °C for 12 h. The mixture reaction was concentrated. The residue was purified by column chromatography (SiO₂, petroleum ether / ethyl acetategradient) to give [(3R,4S,5R)-4,5,6-tri(propanoyloxy)tetrahydropyran-3-yl] propanoate (40 g) as colorless oil from 2 batches. LCMS: 397.1 (M+Na⁺) ¹H NMR (400 MHz, CDCl₃) (mixture of anomers): δ 6.291 (d), 5.745 (d), 5.500 (t), 5.242 (t), 5.089 -4.977 (m), 4.151 (dd), 3.936 (dd), 3.715 (t), 3.539 (dd), 2.519 -2.254 (m), 1.211 - 1.084 (m) ppm

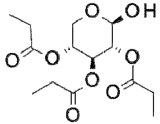

### Example 166: [(3R,4S,5R,6R)-6-hydroxy-4,5-di(propanoyloxy)tetrahydropyran-3-yl] propanoate

To a solution of [(3R,4S,5R)-4,5,6-tri(propanoyloxy)tetrahydropyran-3-yl] propanoate (20.0 g) in THF (200 mL) was added MeNH₂ in H₂O (7.47 g) and the mixture was stirred at 15 °C for 12 h. The mixture reaction was concentrated. The residue was purified by column chromatography (SiO₂, petroleum ether / ethyl acetate gradient) to give [(3R,4S,5R)-6-hydroxy-4,5-di(propanoyloxy)tetrahydropyran-3-yl] propanoate (6 g, 32% yield) as a white solid. ¹H NMR (400 MHz, CDCl₃) (mixture of anomers): δ 5.550 (t), 5.398 (d), 5.282 (t), 5.046 - 4.964 (m), 4.884 (dd), 4.868 (m), 4.685 (d), 4.140 (dd), 3.958 - 3.626 (m), 3.387 (dd), 2.368 - 2.277 (m), 1.169 - 1.066 (m) ppm

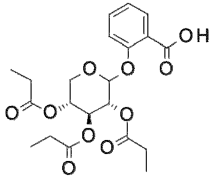

### Example 167: 2-[(3R,4S,5R)-3,4,5-tri(propanoyloxy)tetrahydropyran-2-yl]oxybenzoic acid

Step 1: To a solution of [(3R,4S,5R)-6-hydroxy-4,5-di(propanoyloxy)tetrahydropyran-3-yl] propanoate (0.500 g), tert-butyl 2-hydroxybenzoate (0.610 g) and PPh₃ (0.824 g) in THF (10 mL) was added tert-butyl (NE)-N-tert-butoxycarbonyliminocarbamate (0.723 g) at 0 °C. The reaction mixture was stirred for 12 h at 15 °C. The mixture reaction was concentrated. The residue was purified by column chromatography (SiO₂, petroleum ether / ethyl acetate) to give tert-butyl 2-[(3R, 4S, 5R)-3,4,5-tri(propanoyloxy)tetrahydropyran-2-yl]oxybenzoate (0.320 g, 37% yield) as a brown solid.

Step 2: To a solution of trifluoroacetic acid (15.40 g) in CH₂Cl₂ (30 mL) was added tert-butyl 2-[(3R,4S,5R)-3,4,5-tri(propanoyloxy)tetrahydropyran-2-yl]oxybenzoate (0.300 g) and the mixture was stirred at 15 °C for 2 h. The mixture reaction was concentrated. The residue was purified by reverse phase prep-HPLC (C18, [water(10 mM NH₄HCO₃)-acetonitrile]) to give 2-[(3R,4S,5R)-3,4,5-tri(propanoyloxy)tetrahydropyran-2-yl]oxybenzoic acid (0.010 g, 3.4% yield) as a yellow oil. LCMS: 437.1 (M-H⁺) ¹H NMR (400 MHz, DMSO-d6): δ 7.566 (br 1H), 7.325 (m, 1H), 7.132 (m, 1H), 7.038 (m, 1H), 5.940 (m, 1H), 5.594 (t, 1H), 5.032 (m, 2H), 4.019 (br, 1H), 3.732 (m, 1H), 2.338 - 2.236 (m, 6H), 1.019 - 0.963 (m, 9H) ppm

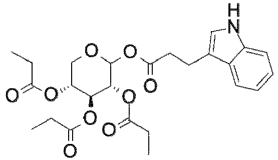

### Example 168: [(3R,4S,5R)-3,4,5-tri(propanoyloxy)tetrahydropyran-2-yl] 3-(1H-indol-3-yl)propanoate

To a solution of [(3R,4S,5R)-6-hydroxy-4,5-di(propanoyloxy)tetrahydropyran-3-yl] propanoate (0.050 g), *N,N*-dicyclohexylcarbodiimide (0.032 g) and 4-dimethylaminopyridine (0.0058 g) in THF (5 mL) was added 3-(1H-indol-3-yl)propanoic acid (0.0297 g). The mixture was stirred at 15 °C for 12 h. The mixture reaction was filtered and concentrated. The residue was purified by prep-TLC (SiO₂, petroleum ether / ethyl acetate, 1:1) to give [(3R,4S,5R)-3,4,5-tri(propanoyloxy)tetrahydropyran-2-yl] 3-(1H-indol-3-yl)propanoate (0.020 g, 23% yield) as a yellow solid. LCMS: 507.2 (M+H₃O⁺) ¹H NMR (400 MHz, CDCl₃) (approx. 1:1 mixture of anomers): δ 7.997 (br), 7.883 (br), 7.492 (m), 7.362 (d), 7.203 (m), 7.147 - 7.104 (m), 7.020 (m), 6.288 (d), 5.772 (d), 5.458 (t), 5.219 (t), 5.068 - 4.956 (m), 4.025 (m), 3.444 (m), 3.085 - 3.011 (m), 2.794 (m), 2.688 (m), 2.260 - 1.986 (m), 1.066 - 0.914 (m) ppm

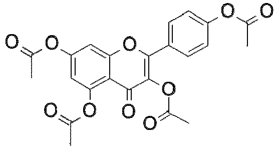

### Example 169: [4-(3,5,7-triacetoxy-4-oxo-chromen-2-yl)phenyl] acetate

To a mixture of 3,5,7-trihydroxy-2-(4-hydroxyphenyl)chromen-4-one (2 g) in pyridine (15 mL) was added acetyl acetate (30 g), and then the mixture was stirred at 15 °C for 12 hr under N₂ atmosphere. The solvent was removed under reduced pressure and the residue was poured into crushed ice with vigorous stirring. The solid precipidate was collected by filtration and washed with cold water and then with methanol. The desired compound [4-(3,5,7-triacetoxy-4-oxo-chromen-2-yl)phenyl] acetate (2.1 g, 65% yield) was obtained as a white solid. LCMS: 455.0 (M+H⁺) ¹H NMR (400 MHz, CDCl₃): δ 7.858 (d, 2H), 7.339 (d, 1H), 7.278 7.257 (m, 2H), 6.883 (d, 1H), 2.447 (s, 3H), 2.357 (s, 6H), 2.333 (s, 3H) ppm

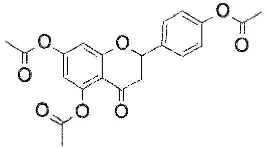

### Example 170: [4-(5,7-diacetoxy-4-oxo-chroman-2-yl)phenyl] acetate

5,7-dihydroxy-2-(4-hydroxyphenyl)chroman-4-one (0.500 g) was dissolved with pyridine (10 mL), and then acetyl acetate (0.844 g) was added to the mixture reaction. The reaction mixture was stirred at 15°C for 12 h. The mixture reaction was concentrated under reduced pressure. The residue was purified by column chromatography (SiO₂, petroleum ether / ethyl acetate gradient) to give [4-(5,7-diacetoxy-4-oxo- chroman-2-yl)phenyl] acetate (0.300 g, 39% yield) as a white solid. LCMS: 416.1 (M+H₃O⁺) ¹H NMR (400 MHz, CDCl₃): δ 7.468 (d, 2H), 7.166 (d, 2H), 6.793 (d, 1H), 6.551 (d, 1H), 5.497 (dd, 1H), 3.039 (dd, 1H), 2.783 (dd, 1H), 2.393 (s, 3H), 2.326 (s, 3H), 2.308 (s, 3H) ppm

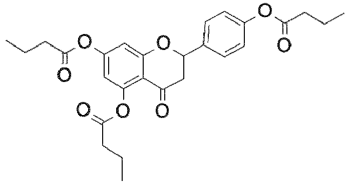

### Example 171: [4-[5,7-di(butanoyloxy)-4-oxo-chroman-2-yl]phenyl] butanoate

To a solution of 5,7-dihydroxy-2-(4-hydroxyphenyl)chroman-4-one (0.500 g) in pyridine (10 mL), was added butanoyl butanoate (1.02 g). The reaction mixture was stirred at 15°C for 12 h. The mixture was concentrated. The residue was purified by column chromatography (SiO₂, petroleum ether / ethyl acetate gradient) to give [4-[5,7-di(butanoyloxy)-4-oxo-chroman-2-yl]phenyl] butanoate (0.325 g, 34% yield) as a white solid. LCMS: 500.2 (M+H₃O⁺) ¹H NMR (400 MHz, CDCl₃): δ 7.463 (d, 2H), 7.158 (d, 2H), 6.786 (d, 1H), 6.536 (d, 1H), 5.483 (m, 1H), 3.031 (m, 1H), 2.662 (m, 1H), 2.586 - 2.524 (m, 6H), 1.837 - 1.785 (m, 6H), 1.089 - 1.021 (m, 9H) ppm

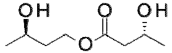

### Example 172: [(3R)-3-hydroxybutyl] (3R)-3-hydroxybutanoate

Step 1: To a solution of methyl (3R)-3-hydroxybutanoate (10 g) in CH₂Cl₂ (400 mL) was added imidazole (23.05 g) and tert-butyl-chloro-dimethyl-silane (25.52 g) at 0 °C and the mixture was stirred at 15 °C for 12 h. Water (100 mL) was added and the mixture was extracted three times with CH₂Cl₂ (50 mL). The organic layer was washed three times with brine (30 mL), dried over Na₂SO₄, filtered and concentrated. The residue was purified by column chromatography (SiO₂, petroleum ether / ethyl acetate gradient) to give methyl (3R)-3-[tert-butyl (dimethyl)silyl]oxybutanoate (16 g, 73% yield) as a colorless oil.

Step 2: To a solution of LiOH.H₂O (2.71 g) in THF (60 mL) and H₂O (40 mL) was added a solution of methyl (3R)-3-[tert-butyl (dimethyl) silyl]oxybutanoate (5 g) in THF (15 mL), and the mixture was stirred at 15 °C for 12 h. The mixture was concentrated under reduced pressure. The residue was diluted with CH₂Cl₂ (20 mL), washed twice with 0.5M HCl (20 mL), then washed three times with brine (10 mL), dried over Na₂SO₄, filtered and concentrated to give (3R)-3-[tert- butyl (dimethyl) silyl]oxybutanoic acid (4.7 g) as a colorless oil which was used without further purification.

Step 3: To a solution of LiAlH₄ (1.63 g) in THF (100 mL) was added methyl (3R)-3-[tertbutyl(dimethyl)silyl]oxybutanoate (10 g) at -30 °C, and stirred for 1 h at the same temperature. The reaction was quenched by adding H₂O (2 mL) at -30 °C. Then 10% aqueous NaOH (2 mL) and H₂O (6 mL) was added into the mixture. The mixture was extracted three times with CH₂Cl₂ (50 mL), dried over Na₂SO₄, filtered and concentrated. The residue was purified by column chromatography (SiO₂, ethyl acetate) to give (3R)-3-[tert- butyl (dimethyl)silyl]oxybutan-1-ol (3 g) as a colorless oil.

Step 4: To a solution of (3R)-3-[tert-butyl(dimethyl)silyl]oxybutan-1-ol (3 g), *N,N'-*dicyclohexylcarbodiimide (5.45 g) and 4-dimethylaminopyridine (0.538 g) in CH₂Cl₂ (30 mL) was added (3R)-3-[tert-butyl(dimethyl)silyl]oxybutanoic acid (3.6 g) at 15 °C, and then stirred for 12 h. The reaction mixture was filtered and concentrated. The residue was purified by column chromatography (SiO₂, petroleum ether / ethyl acetate gradient) to give [(3R)-3-[tert- butyl (dimethyl)silyl]oxybutyl](3R)-3-[tert-butyl(dimethyl)silyl]oxybutanoate (2.4 g, 32.3% yield) as a colorless oil.

Step 5: To a solution of [(3R)-3-[tert-butyl(dimethyl)silyl]oxybutyl](3R)-3-[tert-butyl(dimethyl)silyl] oxybutanoate (2.4 g) in THF (20 mL) was added pyridine hydrofluoride (2.64 g), and the mixture was stirred at 15 °C for 12 h. The reaction mixture was combined with another batch, filtered and concentrated. The residue was purified by column chromatography (SiO₂, petroleum ether / ethyl acetate gradient) to give [(3R)-3-hydroxybutyl] (3R)-3-hydroxybutanoate as a colorless oil (0.900 g). ¹H NMR (methanol-d4): δ 4.209 -4.126 (m, H), 3.856 (m, 1H), 2.435 (m, 2H), 1.767 - 1.705 (m, 2H), 1.206 (d, 3H), 1.189 (d, 3H) ppm

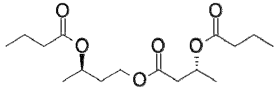

### Example 173: [(3R)-3-butanoyloxybutyl] (3R)-3-butanoyloxybutanoate

To a solution of [(3R)-3-hydroxybutyl] (3R)-3-hydroxybutanoate (0.400 g), K₂CO₃ (0.784 g) in acetonitrile (5 mL) was added butanoyl chloride (0.532 g), and the mixture was stirred at 15 °C for 12 h. The reaction mixture was concentrated and the residue was purified by column chromatography (SiO₂, petroleum ether / ethyl acetate gradient) to give [(3R)-3-butanoyloxybutyl] (3R)-3-butanoyloxybutanoate (0.220 g, 27.5% yield) as a colorless oil. ¹H NMR (400 MHz, CDCl₃): δ 5.197 (m, 1H), 4.965 (m, 1H), 4.045 (m, 2H), 2.528 (m, 1H), 2.449 (m, 1H), 2.222 - 2.158 (m, 4H), 1.799 (m, 2H), 1.602 - 1.546 (m, 4H), 1.222 (d, 3H), 1.182 (d, 3H), 0.884 (t, 3H), 0.874 (t, 3H) ppm

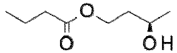

### Example 174: [(3R)-3-hydroxybutyl] butanoate

To a solution of (3R)-butane-1,3-diol (10 g) and triethylamine (12.35 g) in CH₂Cl₂ (10 mL) was added butanoyl butanoate (19.31 g) and the mixture was stirred at 25 °C for 12 h. The mixture reaction was concentrated. The residue was purified by column chromatography (SiO₂, petroleum ether / ethyl acetate gradient) to give [(3R)-3-hydroxybutyl] butanoate (12 g, 61% yield) as a colorless oil. LCMS: 161.1 (M+H⁺) ¹H NMR (400 MHz, CDCl₃): δ 1.777 - 1.632 (m, 4H), 1.230 (d, 3H), 0.954 (t, 3H) ppm

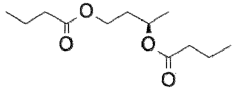

### Example 175: [(3R)-3-butanoyloxybutyl] butanoate

To a solution of (3R)-butane-1,3-diol (6 g) and K₂CO₃ (23.92 g) in acetonitrile (50 mL) was added butanoyl chloride (18.44 g) and the mixture was stirred at 15 °C for 12 h. The mixture reaction was concentrated. The residue was purified by column chromatography (SiO₂, petroleum ether / ethyl acetate gradient) to give [(3R)-3-butanoyloxybutyl] butanoate (11 g, 64.57% yield) as a colorless oil.
LCMS: 248.1 (M+H₃O⁺) ¹H NMR (400 MHz, CDCl₃): δ 5.022 (m, 1H), 4.101 (m, 2H), 2.300 - 2.247 (m, 4H), 1.885 (m, 2H), 1.679 - 1.594 (m, 4H), 1.260 (d, 3H), 0.949 (t, 6H) ppm

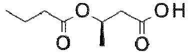

### Example 176: (3R)-3-butanoyloxybutanoic acid

Step 1: To a mixture of methyl (3R)-3-hydroxybutanoate (20 g) in methanol (133 mL) and H₂O (67 mL) was added NaOH (8.13 g) in one portion at 15°C under N₂. The mixture was stirred at 15 °C for 12 h. The reaction mixture was concentrated under reduced pressure to give [(3R)-3-hydroxybutanoyl]oxysodium (20 g, 93.69% yield) was obtained as a white solid.

Step 2: A mixture of [(3R)-3-hydroxybutanoyl]oxysodium (19 g) bromomethylbenzene (25.77 g) in DMF (50 mL) was degassed and purged three times with N₂,and then the mixture was stirred at 60 °C for 12 hr under N₂ atmosphere. The reaction mixture was filtered and extracted with three times with ethyl acetate (70 mL), and the combined organic phase was concentrated under reduced pressure. The residue was purified by column chromatography (SiOz, petroleum ether / ethyl acetate gradient) to give benzyl (3R)-3-hydroxybutanoate (19.5 g, 66% yield) as a colorless oil.

Step 3: To a mixture of benzyl (3R)-3-hydroxybutanoate (19.5 g), pyridine (19.06 g) and 4-dimethylaminopyridine (0.368 g) in CH₂Cl₂ (10 mL) was added butanoyl chloride (14.98 g) in one portion at 15°C under N₂. The mixture was stirred at 15°C for 12 h. The reaction mixture was filtered and concentrated under reduced pressure. The residue was purified by column chromatography (SiO₂, petroleum ether / ethyl acetate gradient) to give benzyl (3R)-3-butanoyloxybutanoate (18.4 g, 69% yield) as a colorless oil.

Step 4: To a solution of benzyl (3R)-3-butanoyloxybutanoate (18.4 g) in EtOAc (184 mL) was added 10% Pd/C (1.00 equiv.) under N₂ atmosphere. The suspension was degassed and purged three times with H₂. The mixture was stirred under H₂ (15psi) at 15 °C for 2 hr. The reaction mixture was filtered and concentrated under reduced pressure to give (3R)-3-butanoyloxybutanoic acid (8 g, 58% yield) as a colorless oil. LCMS: 175.1 (M+H⁺) &197.0 (M+Na⁺) ¹H NMR (400 MHz, CDCl₃): δ 5.288 (m, 1H), 2.688 (dd, 1H), 2.552 (dd, 1H), 2.260 (t, 2H), 1.647 (m, 2H), 1.318 (d, 3H), 0.935 (t, 3H) ppm

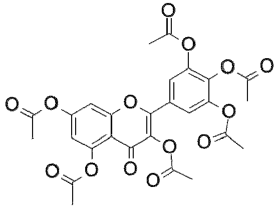

### Example 177: [3,5-diacetoxy-4-oxo-2-(3,4,5-triacetoxyphenyl)chromen-7-yl] acetate

To a solution of 3,5,7-trihydroxy-2-(3,4,5-trihydroxyphenyl)chromen-4-one (1 g) in pyridine (10 mL) was added acetyl acetate (15.26 g), then the mixture was stirred at 15°C for 16 h. The solvent was removed and the mixture was poured into ice water under stirring. The solid was filtered, washed with water and dried in vacuum to give [3,5-diacetoxy-4-oxo-2-(3,4,5-triacetoxyphenyl)chromen-7-yl] acetate (1.1 g, 61% yield) as a gray solid. LCMS 571.1 (M+H⁺) ¹H NMR (400 MHz, CDCl₃): δ 7.260 (s, 2H), 7.349 (d, 1H), 6.886 (d, 1H), 2.441 (s, 3H), 2.372 (s, 3H), 2.353 (s, 3H), 2.341 (s, 3H), 2.333 (s, 6H) ppm

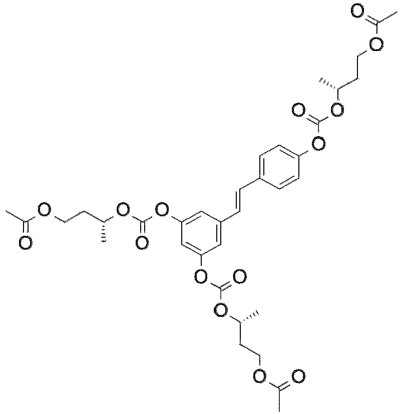

### Example 178: [(3R)-3-[4-[(E)-2-[3,5-bis[[(1R)-3-acetoxy-1-methyl-propoxy]carbonyloxy]phenyl]vinyl]phenoxy]carbonyloxybutyl] acetate

Step 1: To a solution of (3R)-butane-1,3-diol (2.4 g) in pyridine (20 mL) was added Ac₂O (2.17 g) and the mixture was stirred at 15 °C for 12 h. The mixture reaction was concentrated. The residue was purified by column chromatography (SiO₂, petroleum ether / ethyl acetate gradient) to give [(3R)-3-hydroxybutyl] acetate (1.4 g, 35.8% yield) as a colorless oil.

Step 2: To a solution of triphosgene (0.269 g) in THF (5 mL) was added a solution of [(3R)-3-hydroxybutyl] acetate (0.300 g) and triethylamine (0.230 g) in THF (5 mL) at 0 °C and the mixture was stirred for 1 h at 15 °C. A -0.23 M of solution (15 mL) was obtained. The mixture reaction was used in the next step directly.

Step 3: To a solution of 5-[(E)-2-(4-hydroxyphenyl)vinyl]benzene-1,3-diol (0.090 g) and triethylamine (0.218 g) in THF (3 mL) was added a solution of [(3R)-3-chlorocarbonyloxybutyl] acetate (0.23 M, 10 mL) in THF. The reaction mixture was stirred for 5 h at 15 °C. The mixture reaction was filtered and concentrated. The residue was purified by prep-TLC (SiO₂, petroleum ether / ethyl acetate, 4:1) to give [(3R)-3-[4-[(E)-2-[3,5-bis[[(1R)-3-acetoxy-1-methyl-propoxy]carbonyloxy]phenyl]vinyl]phenoxy]carbonyloxybutyl] acetate (0.085 g, 28.8% yield as a colorless oil. LCMS: 725.1 (M+Na⁺) ¹H NMR (400 MHz, CDCl₃): δ 7.520 (d, 2H), 7.244 - 7.192 (m, 4H), 7.114 (d, 1H), 7.036 - 6.979 (m, 2H), 5.019 (m, 3H), 4.226 (m, 6H), 2.099 - 1.995 (m, 6H), 2.055 (s, 6H), 1.442 - 1.422 (m, 9H) ppm

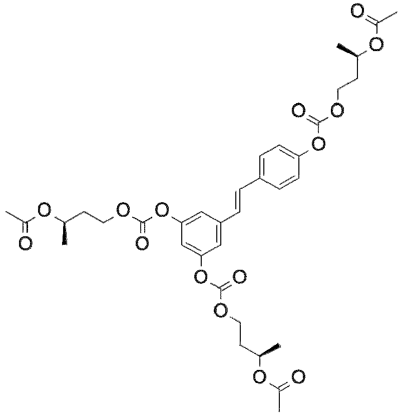

### Example 179: [(1R)-3-[4-[(E)-2-[3,5-bis[[(3R)-3-acetoxybutoxy]carbonyloxy]phenyl]vinyl]phenoxy]carbonyloxy-1-methyl-propyl] acetate

Step 1: To a solution of NaH (2.35 g, 60%) in THF (100 mL) was added (3R)-3-[tert-butyl (dimethyl)silyl]oxybutan-1-ol (10 g) at 0 °C. The mixture was stirred at 15 °C for 1.5 h. Benzyl bromide (10.04 g) was added and the mixture was stirred at 15 °C for 16 h. The reaction mixture was filtered and concentrated under reduced pressure and the residue was purified by column chromatography (SiO₂, petroleum ether) to give [(1R)-3-benzyloxy-1-methyl-propoxy]-tert-butyl-dimethyl-silane (11 g, 55% yield) as a colorless oil.

Step 2: To a solution of [(1R)-3-benzyloxy-1-methyl-propoxy]-tert-butyl-dimethyl-silane (10 g) in THF (100 mL) was added pyridine hydrofluoride (8.41 g) at 15 °C. The mixture was stirred for 2 h at 50 °C. The reaction mixture was combined with another batch and concentrated under reduced pressure. The residue was diluted with H₂O (50 mL) and extracted four times with ethyl acetate (50 mL). The combined organic phase was washed with brine (50 mL), dried over Na₂SO₄, filtered and concentrated under reduced pressure. The residue was purified by column chromatography (SiO₂, petroleum ether / ethyl acetate gradient) to give (2R)-4-benzyloxybutan-2-ol (5.54 g) as a colorless oil.

Step 3: To a solution of (2R)-4-benzyloxybutan-2-ol (5.54 g) in pyridine (50 mL) was added Ac₂O (4.71 g) at 15 °C. The mixture was stirred for 12 h at 15 °C. The reaction mixture was concentrated under reduced pressure and the residue was purified by column chromatography (SiO₂, petroleum ether / ethyl acetate gradient) to give [(1R)-3-benzyloxy-1-methyl-propyl] acetate (4.7 g, 57% yield) as a colorless oil.

Step 4: To a solution of [(1R)-3-benzyloxy-1-methyl-propyl] acetate (2 g) in THF (20 mL) was added 10% Pd/C (0.027 g). The mixture was stirred under H₂ (30 psi) for 16 h at 30 °C. The reaction mixture was filtered and concentrated under reduced pressure and the residue was purified by column chromatography (SiO₂, petroleum ether / ethyl acetate gradient) to give [(1R)-3-hydroxy-1-methyl-propyl] acetate (1.07 g, 65% yield) as a colorless oil.

Step 5: To a solution of [(1R)-3-hydroxy-1-methyl-propyl] acetate (0.300 g) in THF (5 mL) was added a solution of triphosgene (0.337 g) and triethylamine (0.230 g) in THF (5 mL) at 0 °C. The mixture was stirred for 1 h at 15 °C. The mixture reaction was filtered and used to next step directly.
Step 6: To a solution of 5-[(E)-2-(4-hydroxyphenyl)vinyl]benzene-1,3-diol (0.080 g) and triethylamine (0.194 g) in THF (3 mL) was added a solution of [(1R)-3-chlorocarbonyloxy-1-methyl-propyl] acetate (0.2 M, 10 mL) in THF. The reaction mixture was stirred for 5 h at 15 °C. The mixture reaction was filtered and concentrated. The residue was purified by prep-TLC (SiO₂, petroleum ether / ethyl acetate, 4/1) to give (3R)-3-[4-[(E)-2-[3,5-bis[[(1R)-3-acetoxy-1-methyl-propoxy] carbonyloxy]phenyl]vinyl]phenoxy]carbonyloxybutyl] acetate (0.056 g, 21% yield) as a acolorless oil. LCMS: 703.1 (M+H⁺) ¹H NMR (400 MHz, CDCl₃): δ 7.513 (d, 2H), 7.232 - 7.181 (m, 4H), 7.154 (d, 1H), 7.129 - 7.002 (m, 2H), 5.106 (m, 3H), 4.340 (m, 6H), 2.072 (s, 9H), 2.078 - 1.995 (m, 6H), 1.323 - 1.282 (m, 9H) ppm

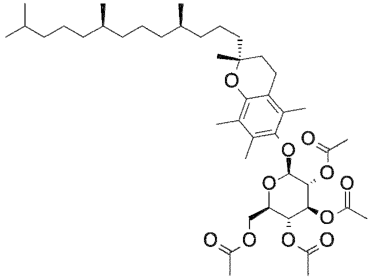

### Example 180: [(2R,3R,4S,5R,6S)-3,4,5-triacetoxy-6-[(2R)-2,5,7,8-tetramethyl-2-[(4R,8R)-4,8,12-trimethyltridecyl]chroman-6-yl]oxy-tetrahydropyran-2-yl]methyl acetate

Step 1: 3-Bromopyridin-2-ol (5 g) was added to aqueous NaOH (0.34 M, 84.52 mL) and aqueous AgNO₃ (0.68 M, 42.26 mL) at 15 °C. The mixture was stirred for 10 min. The reaction mixture was filtered and the solid was washed with H₂O (800 mL) and cooled methanol (200 mL) and dried under reduced pressure to give silver 3-bromopyridin-2-olate (6.5 g, 80.5% yield) as a white solid.

Step 2: To a solution of [(1R,2R,3S,4R,5S)-2,3,4-triacetoxy-5-bromo-cyclohexyl]methyl acetate (0.488 g) in toluene (10 mL) was added silver 3-bromopyridin-2-olate (1 g) at 15 °C. The mixture was stirred for 3 hr at 120 °C. The reaction mixture was filtered and concentrated under reduced pressure and the residue was purified by column chromatography (SiO₂, petroleum ether / ethyl acetate, 1:1) to give [(1R,2R,3S,4S,5S)-2,3,4-triacetoxy-5-[(3-bromo-2-pyridyl)oxy]cyclohexyl]methyl acetate (0.500 g, 75% yield) as a white solid.

Step 3: To a solution of [(1R,2R,3S,4S,5S)-2,3,4-triacetoxy-5-[(3-bromo-2-pyridyl)oxy]cyclohexyl]methyl acetate (0.350 g) and α-tocopherol(0.598 g) in CH₂Cl₂ (5 mL) was added BF₃.Et₂O (47%, 0.629 g, 3 eq) at 15 °C. The mixture was stirred for 5 hr at 15 °C. The reaction mixture was quenched with sodium bicarbonate solution (5 mL), and extracted three times with dichloromethane (10 mL). The combined organic layers were washed with brine (10 mL), dried over Na₂SO₄, filtered and concentrated under reduced pressure. The residue was purified by prep-TLC (SiO₂, petroleum ether / Ethyl acetate, 5:1) to give [(2R,3R,4S,5R,6S)-3,4,5-triacetoxy-6-[(2R)-2,5,7,8-tetramethyl-2-[(4R,8R)-4,8,12-trimethyltridecyl]chroman-6-yl]oxy-tetrahydropyran-2-yl]methyl acetate (0.400 g, 75.7% yield) as a white solid. ¹H NMR (400 MHz, CDCl₃): δ 5.362 - 5.179 (m, 3H), 4.724 (d, 1H), 4.191 -4.049 (m, 3H), 3.536 (m, 1H), 2.568 (m, 2H), 2.152 (s, 3H), 2.120 (s, 3H), 2.105 (s, 3H), 2.082 (s, 3H), 2.054 - 2.027 (m, 9H), 1.838 -1.737 (m, 2H), 1.572 -1.042 (m, 24H), 0.882 - 0.842 (m, 12H) ppm

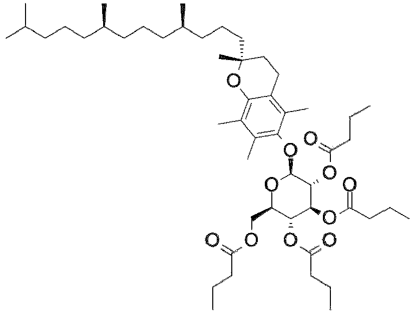

### Example 181: [(2R,3R,4S,5R,6S)-3,4,5-tri(butanoyloxy)-6-[(2R)-2,5,7,8-tetramethyl-2-[(4R,8R)-4,8,12-trimethyltridecyl]chroman-6-yl]oxy-tetrahydropyran-2-yl]methyl butanoate

Step 1: To a solution of [(2R,3R,4S,5R,6S)-3,4,5-triacetoxy-6-[(2R)-2,5,7,8-tetramethyl-2-[(4R,8R)-4,8,12-trimethyltridecyl]chroman-6-yl]oxy-tetrahydropyran-2-yl]methyl acetate (2.7 g) in methanol (30 mL) was added NaOMe in methanol (25%, 192 mg) at 15 °C. The mixture was stirred for 3 hr at 15 °C. The reaction mixture was neutralized with cation exchange resin, filtered and concentrated under reduced pressure. The residue was purified by column chromatography (SiO₂, petroleum ether / ethyl acetate, 3:1 to ethyl acetate / methanol, 20:1 gradient) to give (2R,3S,4S,5R,6S)-2-(hydroxymethyl)-6-[(2R)-2,5,7,8-tetramethyl-2-[(4R,8R)-4,8,12-trimethyltridecyl]chroman-6-yl]oxy-tetrahydropyran-3,4,5-triol (1.3 g, 62% yield) as a yellow solid.

Step 2:To a solution of (2R,3S,4S,5R,6S)-2-(hydroxymethyl)-6-[(2R)-2,5,7,8-tetramethyl-2-[(4R,8R)-4,8,12-trimethyltridecyl]chroman-6-yl]oxy-tetrahydropyran-3,4,5-triol in CH₂Cl₂ (5 mL) was added pyridine (0.107 g) and butanoyl chloride (0.144 g) at 15 °C. The mixture was stirred for 16 hr at 15 °C. The reaction mixture was filtered and concentrated under reduced pressure. The residue was purified by prep-TLC (SiO₂, petroleum ether / ethyl acetate,4:1) to give [(2R,3R,4S,5R,6S)-3,4,5-tri(butanoyloxy)-6-[(2R)-2,5,7,8-tetramethyl-2-[(4R,8R)-4,8,12-trimethyltridecyl]chroman-6-yl]oxy-tetrahydropyran-2-yl]methyl butanoate (0.075 g, 51% yield) as a colorless oil. ¹H NMR (400 MHz, CDCl₃): δ 5.362 (m, 1H), 5.284 (m, 1H), 5.201 (m, 1H), 4.733 (d, 1H), 4.107 (m, 2H), 3.529 (m, 1H), 2.562 (m, 2H), 2.336 (m, 2H), 2.28 - 2.21 (m, 6H), 2.147 (s, 3H), 2.100 (s, 3H), 2.076 (s, 3H), 1.852 - 1.01 (m, 37H), 0.963 0.842 (m, 24H) ppm

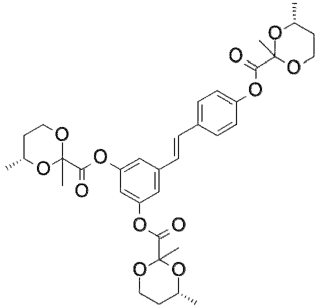

### Example 182: [4-[(E)-2-[3,5-bis[[(4R)-2,4-dimethyl-1,3-dioxane-2-carbonyl]oxy]phenyl]vinyl]phenyl] (4R)-2,4-dimethyl-1,3-dioxane-2-carboxylate

Step 1: To a solution of (3R)-butane-1,3-diol (2 g) and methyl 2-oxopropanoate (4.53 g) in acetonitrile (100 mL) was added BF₃.Et₂O (47%, 13.40 g, 2 eq) dropwise, then the mixture was stirred at 15°C for 16 h. The pH of the solution was adjusted to 7-8 with sat. NaHCO₃ and the aqueous phase was extracted three times with ethyl acetate (30 mL). The combined organic phase was washed with brine (20 mL), dried with anhydrous Na₂SO₄, filtered and concentrated in vacuum. The crude product was purified by silica gel chromatography (petroleum ether / ethyl acetate gradient) to give methyl (4R)-2,4-dimethyl-1,3-dioxane-2-carboxylate (2.5 g, 64.7% yield) as a yellow oil.

Step 2: To a solution of methyl (4R)-2,4-dimethyl-1,3-dioxane-2-carboxylate (2.5 g) in methanol (40 mL) and H₂O (10 mL) was added NaOH (1.15 g), and the mixture was stirred at 80°C for 16 h. The methanol was removed and the pH of the mixture was adjusted to pH=2-3 with aqueous HCl (6M). The aqueous phase was extracted four times with ethyl acetate (30 mL). The combined organic phase was washed with brine (30 mL), dried with anhydrous Na₂SO₄, filtered and concentrated in vacuum to give (4R)-2,4-dimethyl-1,3-dioxane-2-carboxylic acid (1.5 g, 65% yield) as a yellow oil.

Step 3: A solution of resveratrol (0.2 g), (4R)-2,4-dimethyl-1,3-dioxane-2-carboxylic acid (0.561 g), *N,N'*-dicyclohexylcarbodiimide (0.723 g) and 4-dimethylaminopyridine (0.054 g) in CH₂Cl₂ (30 mL) was stirred at 15°C for 16 h. The solid was removed by filtration and the solution was concentrated in vacuum. The crude product was purified by reverse phase prep-HPLC (C18, [water(0.1% trifluoroacetic acid)-acetonitrile]0) to give [4-[(E)-2-[3,5-bis[[(4R)-2,4-dimethyl-1,3-dioxane-2-carbonyl]oxy]phenyl]vinyl]phenyl] (4R)-2,4-dimethyl-1,3-dioxane-2-carboxylate (0.1 g, 17% yield) as a colorless oil. LCMS: 672.3 (M+18) ¹H NMR (400 MHz, CDCl₃): δ 7.552 (d, 2H), 7.211 (d, 2H), 7.170 - 7.104 (m, 3H), 7.034 (m, 1H), 6.944 (m, 1H), 4.163 - 4.005 (m, 9H), 1.975 - 1.709 (m, 12H), 1.549 - 1.513 (m, 3H), 1.318 (d, 9H) ppm

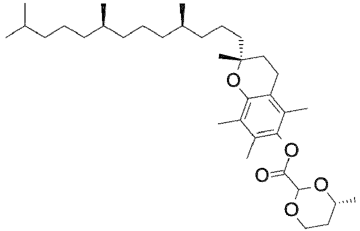

### Example 183: [(2R)-2,5,7,8-tetramethyl-2-[(4R,8R)-4,8,12-trimethyltridecyl]chroman-6-yl] (4R)-4-methyl-1,3-dioxane-2-carboxylate

The solution of α-tocopherol (1 g), (4R)-2,4-dimethyl-1,3-dioxane-2-carboxylic acid (0.169 g), EDCI (0.223 g) and 4-dimethylaminopyridine (0.071 g) in CH₂Cl₂ (10 mL) was stirred at 15°C for 16 h. The solvent was removed and the crude product was purified by prep-TLC (petroleum ether / ethyl acetate. 5:1) to give [(2R)-2,5,7,8-tetramethyl-2-[(4R,8R)-4,8,12-trimethyltridecyl]chroman-6-yl] (4R)-4-methyl-1,3-dioxane-2-carboxylate (0.12 g, 9% yield) as a yellow oil. LCMS: 576.4 (M+18) ¹H NMR (400 MHz, CDCl₃): δ 5.297 (s, 1H), 4.306 (dd, 1H), 3.972 - 3.900 (m, 2H), 2.575 (t, 2H), 2.075 (s, 3H), 2.024 (s, 3H), 1.983 (s, 3H), 1.95 - 1.00 (m, 32H), 0.874 - 0.835 (m, 12H) ppm

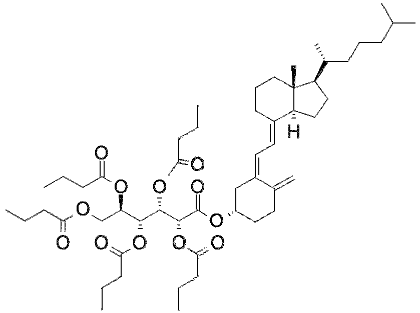

### Example 184: [(1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexyl] (2R,3S,4R,5R)-2,3,4,5,6-penta(butanoyloxy)hexanoate

To a solution of (1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexanol (0.200 g) and (2R,3S,4R,5R)-2,3,4,5,6-penta(butanoyloxy)hexanoic acid (0.341 g) in CH₂Cl₂ (2 mL) was added *N,N'-*dicyclohexylcarbodiimide (0.129 g) and 4-dimethylaminopyridine (0.013 g) at 15 °C. The mixture was stirred at 15 °C for 16 h. The reaction mixture was filtered and concentrated under reduced pressure and the residue was purified by prep-TLC (SiO₂, petroleum ether / ethyl acetate, 5:1) to give [(1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexyl] (2R,3S,4R,5R)-2,3,4,5,6-penta(butanoyloxy)hexanoate (0.300 g, 63% yield) as a colorless oil. ¹H NMR (400 MHz, CDCl₃): δ 6.231 (d, 1H), 6.014 (d, 1H), 5.641 (dd, 1H), 5.511 (m, 1H), 5.262 (d, 1H), 5.121 - 5.066 (m, 2H), 4.967 (m, 1H), 4.847 (d, 1H), 4.317 (dd, 1H), 4.111 (m, 1H), 2.818 (m, 1H), 2.597 (m, 1H), 2.274 -2.185 (m, 13H), 2.055 - 1.955 (m, 5H), 1.733 - 1.129 (m, 29H), 1.001 - 0.870 (m, 24H) ppm

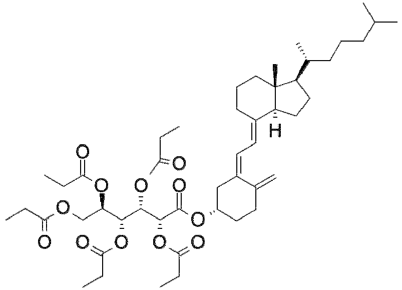

### Example 185: [(1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexyl] (2R,3S,4R,5R)-2,3,4,5,6-penta(propanoyloxy)hexanoate

To a solution of (2R,3S,4R,5R)-2,3,4,5,6-penta(propanoyloxy) hexanoic acid (0.5 g) in CH₂Cl₂ (5 mL) was added (1S,3Z)-3-[(2E)-2-[(1R,3aS,7aR)-1-[(1R)-1,5-dimethylhexyl] -7a-methyl-2,3,3a,5,6,7-hexahydro-1H-inden-4-ylidene]ethylidene]-4-methylene-cyclohexanol (0.484 g), *N,N'-*dicyclohexylcarbodiimide (0.433 g) and 4-dimethylaminopyridine (0.038 g). The mixture was stirred at 15 °C for 12 hr. The reaction mixture was filtered and concentrated under reduced pressure to give a residue. The residue was purified by column chromatography (SiO₂, petroleum ether / ethyl acetate gradient) to give [(1S,3Z)-3-[(2E)-2-[(1R,3aS,7aR)-1-[(1R)-1,5- dimethylhexyl]-7a-methyl-2,3,3a,5,6,7-hexahydro-1H-inden-4-ylidene]ethylidene]-4-methylene-cyclohexyl] (2R,3S,4R,5R)-2,3,4,5,6-penta (propanoyloxy) hexanoate (0.24 g, 25% yield) as a colorless oil. ¹H NMR (400 MHz, CDCl₃): δ 6.228 (d, 1H), 6.104 (d, 1H), 5.649 (m, 1H), 5.523 (m, 1H), 5.272 (d, 1H), 5.123 - 5.068 (m, 2H), 4.989 (m, 1H), 4.852 (m, 1H), 4.299 (m, 1H), 4.129 (m, 1H), 2.834 (m, 1H), 2.65 - 0.95 (m, 68H), 0.94 - 0.869 (m, 12H) ppm

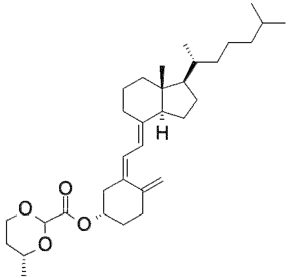

### Example 186: [(1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexyl] (4R)-4-methyl-1,3-dioxane-2-carboxylate

The solution of (1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexanol (0.3 g), (4R)-4-methyl-1,3-dioxane-2-carboxylic acid (0.228 g), *N,N'*-dicyclohexylcarbodiimide (0.322 g) and 4-dimethylaminopyridine (0.095 g) in CH₂Cl₂ (20 mL) was stirred at 15°C for 16 h. The solid was filtered and the filtrate was concentrated in vacuum. The crude product was purified by prep-TLC (petroleum ether/ ethyl acetate, 5:1) to give [(1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexyl] (4R)-4-methyl-1,3-dioxane-2-carboxylate (0.090 g, 21% yield) as a yellow solid. LCMS: 513.3 (M+H⁺) ¹H NMR (400 MHz, CDCl₃): δ 6.230 (d, 1H), 6.030 (d, 1H), 5.068 - 5.035 (m, 2H), 4.990 (s, 1H), 4.844 (s, 1H), 4.233 (m, 1H), 3.868 - 3.801 (m, 2H), 2.798 (m, 1H), 2.612 (m, 1H), 2.442 (m, 2H), 2.2 (m, 1H), 2.050 - 0.095 (m, 29H), 0.925 (d, 3H), 0.875 (d, 3H), 0.870 (d, 3H), 0.546 (s, 3H) ppm

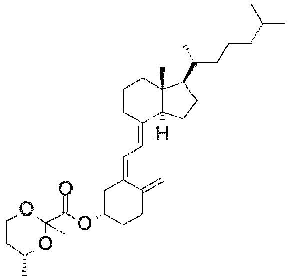

### Example 187: [(1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexyl](4R)-2,4-dimethyl-1,3-dioxane-2-carboxylate

A solution of (1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexanol (0.3 g), (4R)-2,4-dimethyl-1,3-dioxane-2-carboxylic acid (0.250 g), *N,N'*-dicyclohexylcarbodiimide (0.322 g) and 4-dimethylaminopyridine (0.048 g) in CH₂Cl₂ (20 mL) was stirred at 15°C for 16 h. The solvent was removed under reduced pressure. The crude product was purified by silica gel chromatography (petroleum ether / ethyl acetate gradient) to give [(1S,3Z)-3-[(2E)-2-[(1R,3aS,7aR)-1-[(1R)-1,5-dimethylhexyl]-7a-methyl-2,3,3a,5,6,7-hexahydro-1H-inden-4-ylidene]ethylidene]-4-methylene-cyclohexyl] (4R)-2,4-dimethyl-1,3-dioxane-2-carboxylate (0.050 g, 12% yield) as yellow oil. LCMS: 549.4 (M+Na+) ¹H NMR (400 MHz, CDCl₃): δ 6.130 (d, 1H), 5.938 (d, 1H), 5.026 - 4.978 (m, 2H), 4.784 (d, 1H), 3.843 - 3.772 (m, 3H), 2.730 (m, 1H), 2.552 - 0.995 (35H), 0.848 (d, 3H), 0.799 (d, 3H), 0.795 (d, 3H0, 0.469 (s, 3H) ppm

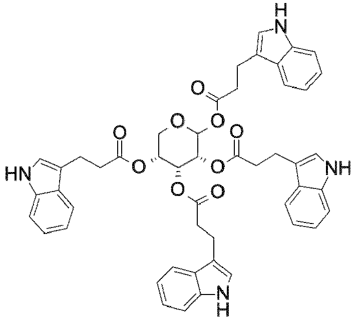

### Example 188: [(3R,4R,5R)-4,5,6-tris[3-(1H-indol-3-yl)propanoyloxy]tetrahydropyran-3-yl]-3-(1H-indol-3-yl)propanoate

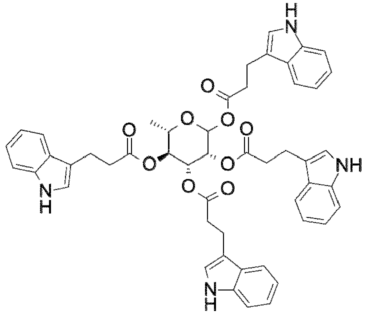

### Example 189: [(2S,3S,4R,5R)-4,5,6-tris[3-(1H-indol-3-yl)propanoyloxy]-2-methyl-tetrahydropyran-3-yl] 3-(1 H-indol-3-yl)propanoate

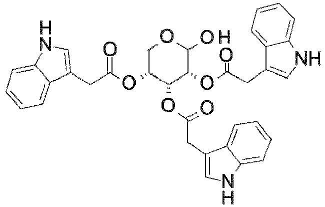

### Example 190: [(3R,4R,5R)-6-hydroxy-4,5-bis[[2-(1 H-indol-3-yl)acetyl]oxy]tetrahydropyran-3-yl] 2-(1H-indol-3-yl)acetate

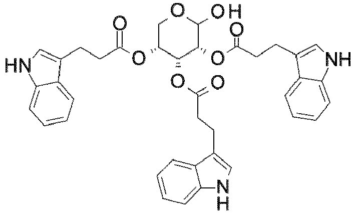

### Example 191: [(3R,4R,5R)-6-hydroxy-4,5-bis[3-(1H-indol-3-yl)propanoyloxy]tetrahydropyran-3-yl] 3-(1H-i ndol-3-yl)propanoate

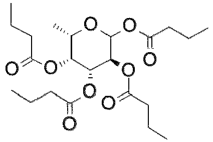

Example 192: [(2S,3R,4R,5S)-4,5,6-tri(butanoyloxy)-2-methyl-tetrahydropyran-3-yl] butanoate

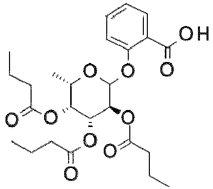

### Example 193: 2-[(3S,4R,5R,6S)-3,4,5-tri(butanoyloxy)-6-methyl-tetrahydropyran-2-yl]oxybenzoic acid

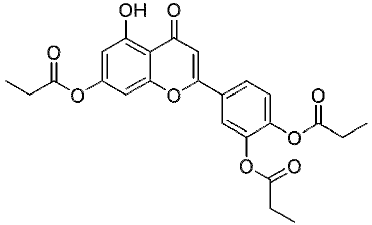

### Example 194: [4-[4-oxo-5,7-di(propanoyloxy)chromen-2-yl]-2-propanoyloxy-phenyl] propanoate

Propionic anhydride (1.33 mL, 10.4 mmol) was added dropwise to a stirred solution of luteolin (0.3 g, 1.04 mmol) in anhydrous pyridine (2.5 mL, 31.2 mmol) at 0°C under N₂ atmosphere. The resulting stirred solution was allowed to come to room temperature and reaction was monitored to completion by LCMS. The solution was diluted with 30 mL ethyl acetate and washed with H₂O (30 mL), 1M HCl (30 mL), H₂O (30 mL), and saturated NaHCO₃ (30 mL). The organic layer was dried over sodium sulfate, filtered, and concentrated by rotary evaporation. The crude residue was purified by flash chromatography (silica, 10-100% ethyl acetate in hexanes) and fractions were concentrated by rotary evaporation to yield compound 194 (0.073 g, 15% yield) as an off-white solid. ¹H NMR (DMSO-d6, 400 MHz): δ 12.75 (s, 1H), 8.07 (m, 2H), 7.5 (m, 1H), 7.15 (s, 1H), 7.12 (d, 1H), 6.66 (d, 1H), 2.59-2.66 (m, 6H), 1.11-1.17 (m, 9H) ppm

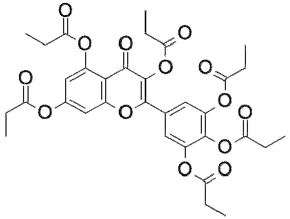

### Example 195: [4-oxo-3,5-di(propanoyloxy)-2-[3,4,5-tri(propanoyloxy)phenyl]chromen-7-yl] propanoate

Propionic anhydride (2 mL, 15.6 mmol) was added dropwise to a stirred solution of myricetin (0.5 g, 1.56 mmol) in anhydrous pyridine (2.78 mL, 47.1 mmol) at 0°C under N₂ atmosphere. The resulting stirred solution was allowed to come to room temperature and reaction was monitored to completion by LCMS. The solution was diluted with 30 mL ethyl acetate and washed with H₂O (30 mL), 1M HCl (30 mL), H₂O (30 mL), and saturated NaHCO₃ (30 mL). The organic layer was dried over sodium sulfate, filtered, and concentrated by rotary evaporation. The crude residue was purified by flash chromatography (silica, 10-100% ethyl acetate in hexanes) and fractions were concentrated by rotary evaporation to yield Compound 195 (0.31 g, 30% yield) as a white solid. ¹H NMR (DMSO-d6, 400 MHz): δ 7.77 (s, 2H), 7.64 (d, 1H), 7.16 (d, 1H), 2.60-2.70 (m, 12H), 1.07-1.17 (m, 18H) ppm

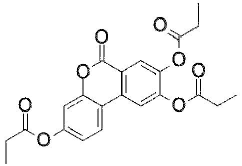

### Example 196: [6-oxo-8,9-di(propanoyloxy)benzo[c]chromen-3-yl] propanoate

Propionic anhydride (2.61 mL, 20.4 mmol) was added dropwise to a stirred solution of urolithin C (0.5 g, 2.04 mmol) in anhydrous pyridine (4.92 mL, 61.2 mmol) at 0°C under N₂ atmosphere. The resulting stirred solution was allowed to come to room temperature and reaction was monitored to completion by LCMS. The solution was diluted with 30 mL ethyl acetate and washed with H₂O (30 mL), 1M HCl (30 mL), H₂O (30 mL), and saturated NaHCO₃ (30 mL). The organic layer was dried over sodium sulfate, filtered, and concentrated by rotary evaporation. The crude residue was purified by flash chromatography (silica, 10-100% ethyl acetate in hexanes) and fractions were concentrated by rotary evaporation to yield Compound 196 (0.05 g, 6% yield) as a pink solid. ¹H NMR (DMSO-d6, 400 MHz): δ 8.4 (s, 1H), 8.35 (d, 1H), 8.14 (s, 1H), 7.31 (d, 1H), 7.23 (m, 1H) , 2.73 -2.63 (m, 6H), 1.21 - 1.14 (m, 9H) ppm

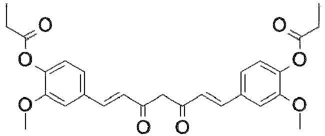

### Example 197: [2-methoxy-4-[(1E,6E)-7-(3-methoxy-4-propanoyloxy-phenyl)-3,5-dioxo-hepta-1,6-dienyl]phenyl] propanoate

Propionic anhydride (1.72 mL, 13.5 mmol) was added dropwise to a stirred solution of curcumin (0.5 g, 1.35 mmol) in anhydrous pyridine (3.25 mL, 40.5 mmol) at 0°C under N₂ atmosphere. The resulting stirred solution was allowed to come to room temperature and reaction was monitored to completion by LCMS. The solution was diluted with 30 mL ethyl acetate and washed with H₂O (30 mL), 1M HCl (30 mL), H₂O (30 mL), and saturated NaHCO₃ (30 mL). The organic layer was dried over sodium sulfate, filtered, and concentrated by rotary evaporation. The crude residue was purified by flash chromatography (silica, 10-100% ethyl acetate in hexanes) and fractions were concentrated by rotary evaporation to yield 197 (0.350 g, 54% yield) as a yellow solid. ¹H NMR (DMSO-d6, 400 MHz): δ 7.79 (m, 1H), 7.66 (m, 2 H), 7.51 (s, 2H), 7.33 (m, 2H), 7.16 (d, 2H), 6.19 (m, 1H), 3.83 (s, 6H), 2.62 (m, 4H), 1.13-1.24 (m, 6H) ppm

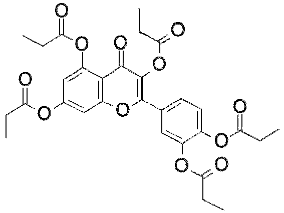

### Example 198: [4-[4-oxo-3,5,7-tri(propanoyloxy)chromen-2-yl]-2-propanoyloxy-phenyl] propanoate

Propionic anhydride (2.1 mL, 16.5 mmol) was added dropwise to a stirred solution of quercetin (0.5 g, 1.65 mmol) in anhydrous pyridine (3.98 mL, 49.5 mmol) at 0°C under N₂ atmosphere. The resulting stirred solution was allowed to come to room temperature and reaction was monitored to completion by LCMS. The solution was diluted with 30 mL ethyl acetate and washed with H₂O (30 mL), 1M HCl (30 mL), H₂O (30 mL), and saturated NaHCO₃ (30 mL). The organic layer was dried over sodium sulfate, filtered, and concentrated by rotary evaporation. The crude residue was purified by flash chromatography (silica, 10-100% ethyl acetate in hexanes) and fractions were concentrated by rotary evaporation to yield Compound 198 (0.1 g, 10% yield) as a white solid. ¹H NMR (DMSO-d6, 400 MHz): δ 7.85 (m, 2 H), 7.66 (d, 1H), 7.54 (d, 1H), 7.18 (d, 1H), 2.62-2.89 (m, 10H), 1.09-1.19 (m, 20H) ppm

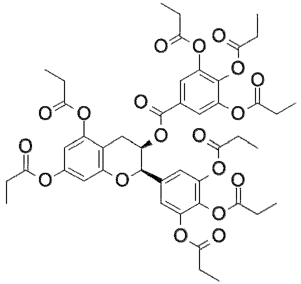

### Example 199: [(2R,3R)-5,7-di(propanoyloxy)-2-[3,4,5-tri(propanoyloxy)phenyl]chroman-3-yl] 3,4,5-tri(propanoyloxy)benzoate

Propionic anhydride (2.78 mL, 21.8 mmol) was added dropwise to a stirred solution of epigallocatechin gallate (0.5 g, 1.09 mmol) in anhydrous pyridine (2.61 mL, 32.6 mmol) at 0°C under N₂ atmosphere. The resulting stirred solution was allowed to come to room temperature and reaction was monitored to completion by LCMS. The solution was diluted with 30 mL ethyl acetate and washed with H₂O (30 mL), 1M HCl (30 mL), H₂O (30 mL), and saturated NaHCO₃ (30 mL). The organic layer was dried over sodium sulfate, filtered, and concentrated by rotary evaporation. The crude residue was purified by flash chromatography (silica, 10-100% ethyl acetate in hexanes) and fractions were concentrated by rotary evaporation to yield Compound 199 (0.695 g, 70% yield) as a white solid. 1H-NMR (DMSO-d6, 400 MHz): δ 7.54 (s, 2H), 7.38 (s, 2H), 6.79 (m, 1H), 6.66 (m, 1H), 5.66 (m, 1H), 5.54 (s, 1H), 3.13-3.17 (m, 1 H), 2.96 (d, 1H), 2.5-2.65 (m, 16H), 1.0-1.2 (m, 24 H) ppm

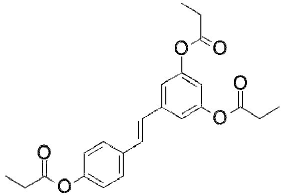

### Example 200: [4-[(E)-2-[3,5-di(propanoyloxy)phenyl]vinyl]phenyl] propanoate

Propionic anhydride (0.56 mL, 4.4 mmol) was added dropwise to a stirred solution of resveratrol (0.1 g, 0.44 mmol) in anhydrous pyridine (1 mL, 12.4 mmol) at 0°C under N₂ atmosphere. The resulting stirred solution was allowed to come to room temperature and reaction was monitored to completion by LCMS. The solution was diluted with 30 mL ethyl acetate and washed with H₂O (30 mL), 1M HCl (30 mL), H₂O (30 mL), and saturated NaHCO₃ (30 mL). The organic layer was dried over sodium sulfate, filtered, and concentrated by rotary evaporation. The crude residue was purified by flash chromatography (silica, 10-60% ethyl acetate in hexanes) and fractions were concentrated by rotary evaporation to yield compound 200 (0.075 g, 43% yield) as a white solid. ¹H-NMR (DMSO-d₆, 400 MHz): δ 7.65 (d, 2H), 7.2-7.3 (m, 4H), 7.13 (d, 2H), 6.92 (t, 1H), 2.6 (m, 6H), 1.1-1.3 (m, 9H) ppm

### Biological Assays

In some preferred instances, a multibiotic agent is hydrolyzed in the small intestine to release active agents targeting a disease or condition. For example, multibiotic agents for treating multiple sclerosis, NAFLD/NASH, or type 2 diabetes are preferably stable though the stomach (SGF assay, assay 2) and are hydrolyzed to release individual agents in the small intestine (SIF assay, assay 3).

In some preferred instances, a multibiotic agent is hydrolyzed in the colon to release active agents targeting a disease or condition. For example, multibiotic agents for treating colorectal cancer, IBS, ulcerative colitis, C. difficile infection, chronic diarrhea, and constipation (e.g., constipation secondary to a neurodegenerative disorder (e.g., Parkinson's disease)) are preferably stable through the stomach (SGF assay, assay 2) and, in some instances, through the small intestine (SIF assay, assay 3), and are hydrolyzed to release individual agents in the fecal assay, assay 4.

Multibiotic agents are typically stable under a range of physiological pH levels and cleaved selectively at a desired site of action (for example, in the GI tract, e.g., in the stomach, small intestine, or large intestine) by enzymes present in the local microenvironment. Multibiotic agents are tested for chemical stability at a range of pH levels as well as their ability to be degraded in representative in vitro systems. Data for select multibiotic agents are shown below.

Assay 1. Stability of multibiotic agents in a buffer. This assay can be used for the assessment of the stability of a multibiotic agent at different physiological pH levels.

Compounds were diluted in DMSO, and the resulting solution was added in the appropriate quantity to phosphate buffer (pH levels 2, 4, 6, and 8) to reach a total sample concentration of 2 µM. Compounds were incubated at RT, and aliquots were removed at time points 0, 60, 120, 360 and 1440 minutes and analyzed for purity by LC/MS/MS.

Assay 2. Stability of multibiotic agents in Simulated Intestinal Fluid (SIF). This assay can be used to assess the stability of a multibiotic agent in a small intestine.

Phosphate buffer was prepared by dissolving 0.42 g of sodium hydroxide pellets and 3.95 g of monobasic sodium phosphate monohydrate and 6.19 g of sodium chloride in ultrapure water (MilliQ^{®}, Millipore Sigma, Darmstadt, Germany). The pH was adjusted to 6.7 using aq. HCl and aq. NaOH, as necessary, and the solution was diluted with ultrapure water to produce 1L of the pH 6.7 buffer.

112 mg FaSSIF powder (Biorelevant^{™}, London, UK) was dissolved in 50 mL of the pH 6.7 buffer. 2 to 3 mL of the resulting solution were then added to 500 mg pancreatin (Millipore Sigma, Darmstadt, Germany). The resulting mixture was agitated by finger tapping the vessel containing the mixture until milky suspension formed. At this time, the remainder of the 50 mL FaSSiF/pH 6.7 buffer solution was added. The resulting suspension was flipped upside down 10 times to produce SIF, which was used fresh.

Test compounds are dissolved in DMSO stock to 1 mM. An aliquot of the DMSO stock solution is removed and diluted in the SIF media in 15 mL falcon tubes to produce a mixture with a tested compound concentration of 1 µM. A 1 mL aliquot is immediately removed and diluted once with 1 volume of acetonitrile for T0 timepoint. The mixture is sealed and agitated at 37 ⁰C in an incubator. Aliquots (1 mL) are removed at regular intervals and immediately quenched by the addition of 1 volume of acetonitrile. The resulting samples are analyzed by LC/MS to determine degradation rates.

Assay 3. Stability of multibiotic agents in Simulated Gastric Fluid (SGF). This assay can be used to assess the stability of a multibiotic agent in a stomach.

Medium was prepared by dissolving 2 g of sodium chloride in 0.6 L in ultrapure water (MilliQ^{®}, Millipore Sigma, Darmstadt, Germany). The pH was adjusted to 1.6 with 1N hydrochloric acid, and the volume was then adjusted to 1 L with purified water.

60 mg FaSSIF powder (Biorelevant^{™}, London, UK) were dissolved in 500 mL buffer (above). Pepsin was added (0.1 mg/mL) (Millipore Sigma, Darmstadt, Germany), and the solution was stirred. The resulting SGF media were used fresh for each experiment.

Test compounds were dissolved in DMSO stock to 1 mM. An aliquot of the DMSO stock solution was removed and diluted in the SGF Media in 15 mL falcon tubes to generate a total compound concentration of 1 µM. A 1 mL aliquot was immediately removed and diluted once with 1 volume of acetonitrile for T0 timepoint. The mixture was sealed and mixed at 37 °C in an incubator. Aliquots (1 mL) were removed at regular intervals and immediately quenched by the addition of 1 volume of acetonitrile. The resulting samples were analyzed by LC/MS to determine degradation rates in SGF.

Assay 4. Fecal Incubation Stability. This assay can be used to assess the stability of a multibiotic agent in a large intestine. All experiments perfomed in an anaerobic chamber containing 90% nitrogen, 5% hydrogen and 5% carbon dioxide. Fecal matter in a slurry (15% (w/v)) was added to 96 well plates containing YCFA media or other suitable media (1.6 mL). Compounds were added to each individual well to reach a final analyte concentration of 1 or 10 µM, and the material was mixed by pipetting. At predetermined timepoints a sample was removed, quenched with acetonitrile, and analyzed by LC/MS.

In Table 1 below, examples 20, 21, 117 and 121 are examples of the invention. The remaining examples are disclosed for illustrative purposes.

**Table 1**

| **Example** | **Assay 1 (% Remaining @ 24 hours)** | **Assay 2 (SGF) (% Remaining @ 4 hours)** | **Assay 3 (SIF) (% @ Remaining 4 hours)** | **Assay 4 (% Remaining at 24 h)** |
|---|---|---|---|---|
| 1 | | C | C | |
| 2 | | C | C | |
| 3 | | A | A | |
| 7 | | C | B | |
| 8 | | C | C | |
| 9 | | | C | |
| 14 | | C | C | |
| 15 | | C | C | C |
| 16 | | C | A | A |
| 20 | | C | C | A |
| 21 | D | C | C | |
| 24 | D | | | |
| 29 | D | | | |
| 51 | | C | C | |
| 52 | | | | A |
| 55 | | C | B | |
| 56 | | C | C | |
| 57 | | C | | |
| 69 | | C | | |
| 70 | | C | | |
| 71 | | C | C | C |
| 72 | | C | B | |
| 73 | | C | B | |
| 74 | | C | A | |
| 75 | | C | B | |
| 78 | | C | B | |
| 79 | | C | B | B |
| 80 | | C | C | |
| 81 | | C | C | |
| 82 | | C | B | |
| 84 | | C | C | |
| 85 | | C | C | |
| 86 | | C | C | |
| 87 | | C | C | |
| 88 | | C | C | |
| 89 | | C | C | |
| 90 | | C | A | |
| 91 | | C | A | |
| 92 | | C | C | |
| 93 | | | A | |
| 94 | | C | C | |
| 95 | | C | C | |
| 96 | | C | C | |
| 97 | | C | C | |
| 98 | | C | A | |
| 99 | | C | C | |
| 100 | | C | C | |
| 101 | | C | C | |
| 102 | | C | B | |
| 103 | | C | A | |
| 104 | | C | B | |
| 105 | | C | B | |
| 106 | | C | C | |
| 107 | | C | B | |
| 108 | | C | B | |
| 109 | | | A | |
| 110 | | C | A | |
| 111 | | C | C | |
| 112 | | C | C | |
| 113 | | C | B | |
| 114 | | C | C | |
| 115 | | C | B | |
| 116 | | C | C | |
| 117 | | C | C | |
| 118 | | C | C | |
| 120 | | | A | |
| 121 | | | C | |
| 122 | | C | A | |
| 123 | | C | C | |
| 124 | | C | C | |
| 125 | | C | C | |
| 126 | | C | C | |
| 127 | | C | C | |
| 128 | | C | C | |
| 129 | | C | C | |
| 130 | | C | C | |
| 131 | | C | C | |
| 132 | | C | C | |
| 133 | | C | C | |
| 134 | | C | C | |
| 135 | | C | C | |
| 136 | | C | C | |
| 137 | | C | C | |
| 138 | | C | C | |
| 139 | | C | C | |
| 140 | | C | C | |
| 141 | | C | A | |
| 142 | | C | C | |
| 143 | | C | C | |
| 144 | | C | A | |
| 145 | | C | A | |
| 146 | | C | A | |
| 147 | | C | B | |
| 148 | | C | A | |
| 149 | | C | C | |
| 152 | | C | C | |
| 155 | | C | C | |
| 156 | | C | A | |
| 157 | | C | A | |
| 160 | | C | A | |
| 161 | | C | A | |
| 162 | | C | B | |
| 163 | | B | A | |
| 164 | | B | B | |
| 165 | | C | A | |
| 166 | | C | B | |
| 168 | | C | | |
| 169 | | C | A | |
| 170 | | C | A | |
| 171 | | B | A | |
| 172 | | C | C | |
| 173 | | C | A | |
| 177 | | C | A | |
| 178 | | C | A | |
| 180 | | C | C | |
| 182 | | C | C | |
| 183 | | C | A | |
| 184 | | B | A | |
| 186 | | C | C | |
| 187 | | B | C | |

| | | | | |
|---|---|---|---|---|
| In Table 1, A: <25% of the tested compound remaining; B: 25-75% of the tested compound remaining; C: >75% of the tested compound remaining; and D: no change in the amount of the tested compound. | | | | |

The data in Table 1 demonstrate that, for example, no apparent degradation was observed for the compounds of Examples 21, 24, and 29 in Assay 1. Table 1 further demonstrates that, for example, compounds of Examples 7, 16, 55, 72-75, 78, 79, 82, 90, 91, 93, 98, 102-105, 107, 108, 110, 113, 115, 122, 141, 144-148, 156, 157, 160-163, 165, 166, 169-171, 173, 177, 178, 183, and 184 can be delivered at least to the small intestine.

The Ketone Body Release Assay disclosed below is for illustrative purposes and is not part of the invention.

### Ketone Body Release Assay

Phosphate buffer was prepared by dissolving 0.42 g of sodium hydroxide pellets, 3.95 g of monobasic sodium phosphate monohydrate, and 6.19 g of sodium chloride in ultrapure water (MilliQ^{®}, Millipore Sigma, Darmstadt, Germany). The pH of the resulting solution was adjusted to 6.7 using aq. HCl and aq. NaOH, as necessary, and the solution was diluted with ultrapure water to produce 1L of the pH 6.7 buffer.

112 mg FaSSIF powder (Biorelevant^{™}, London, UK) was dissolved in 50 mL of the pH 6.7 buffer. 2 to 3 mL of this solution were then added to 500 mg pancreatin (Millipore Sigma, Darmstadt, Germany). The resulting mixture was agitated by finger tapping the vessel containing the mixture until milky suspension formed. At this time, the remainder of the 50 mL FaSSiF/pH 6.7 buffer solution was added. The resulting suspension was flipped upside down 10 times to produce SIF and was used fresh.

Test compounds were weighed into 15 mL falcon tubes and mixed with the SIF to 5 mL total volume. Upon addition of the media, incubation was started. The amount of β-hydroxybutyrate released was measured over time using Ketone Meter (Precision Xtra Blood Glucose & Ketone Monitoring System, Abbott, East Columbus, OH). 1.5 µL Aliquots were removed from the tube at T=0 h, 0.5 h, 4 h, and 16 h, and the ketone concentrations were measured in each aliquot.

The results of this assay are shown in Table 2.

**Table 2.**

| Compound | T0 (mM) | T0.5 h (mM) | T4h (mM) | T16h (mM) | T24h (mM) |
|---|---|---|---|---|---|
| 5 | 0 | 0 | 0 | 0 | |
| 6 | 0 | 0.1 | 0.15 | 0.15 | |
| 72 | 0 | 0 | 0 | 0 | |
| 73 | 0 | 0 | 0 | 0 | |
| 78 | 0 | 0.56 | 1.33 | 2.66 | |
| 79 | 0 | 0 | 0.15 | 1.3 | |
| 91 | 0 | 0 | 0.2 | 0.5 | |
| 96 | 0 | 0 | 0 | | 0.9 |
| 111 | 0 | 0 | 0 | | 0 |

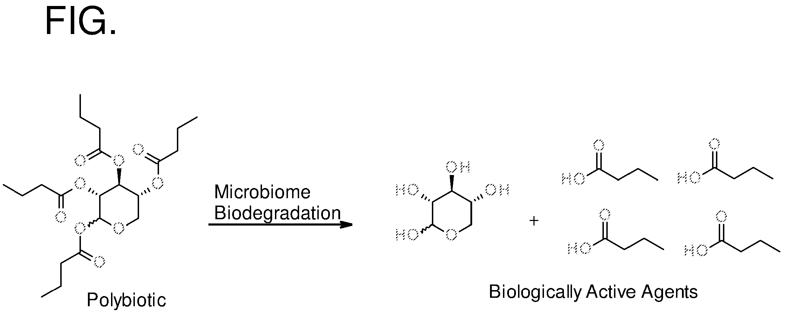

## Claims

1. A multibiotic agent, wherein the multibiotic agent is selected from the group consisting of the compounds of the following structure: 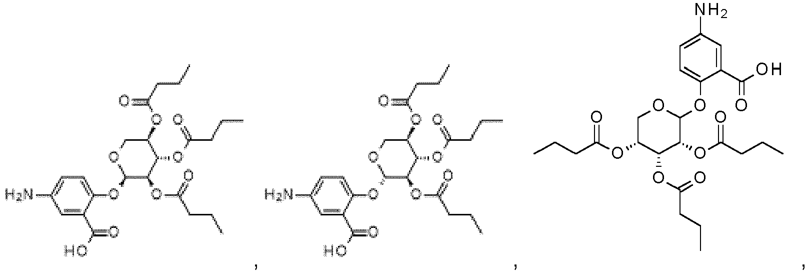 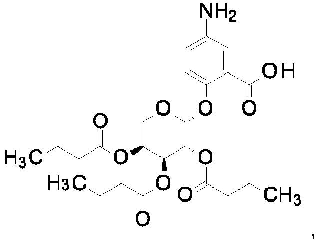 and salts thereof.

2. The multibiotic agent of claim 1, wherein the multibiotic agent is a compound of the following structure: 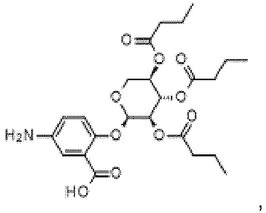 or a salt thereof.

3. The multibiotic agent of claim 1, wherein the multibiotic agent is a compound of the following structure: 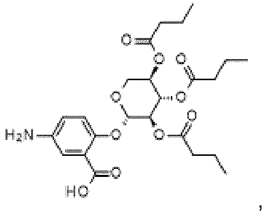 or a salt thereof.

4. The multibiotic agent of claim 1, wherein the multibiotic agent is a compound of the following structure: 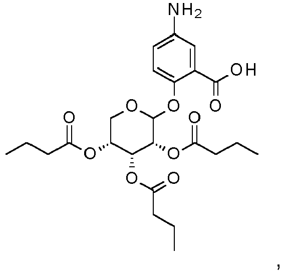 or a salt thereof.

5. The multibiotic agent of claim 1, wherein the multibiotic agent is a compound of the following structure: 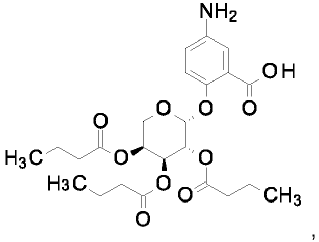 or a salt thereof.

6. A pharmaceutical composition comprising the multibiotic agent of any one of claims 1 to 5 and a pharmaceutically acceptable excipient.

7. A nutraceutical composition comprising the multibiotic agent of any one of claims 1 to 5 and a physiologically acceptable excipient.

8. The multibiotic agent of any one of claims 1 to 5 for use in delivering a biologically active compound to a target site in a subject.

9. The multibiotic agent for use of claim 8, wherein the multibiotic agent is administered orally.

10. The multibiotic agent for use of claim 8, wherein the multibiotic agent is substantially activated by a microbiota of the subject.

## Patentansprüche

1. Multibiotikum, wobei das Multibiotikum aus der Gruppe ausgewählt ist bestehend aus den Verbindungen der folgenden Struktur: 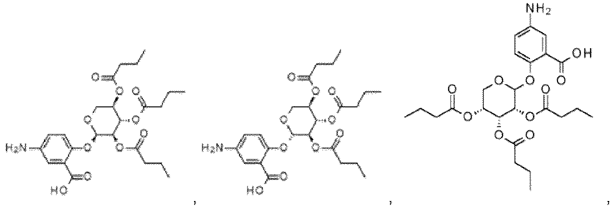 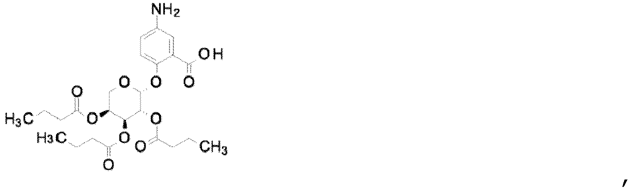 und Salzen davon.

2. Multibiotikum nach Anspruch 1, wobei das Multibiotikum eine Verbindung der folgenden Struktur ist: 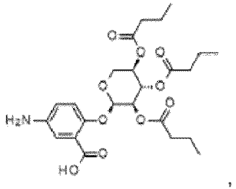 oder ein Salz davon.

3. Multibiotikum nach Anspruch 1, wobei das Multibiotikum eine Verbindung der folgenden Struktur ist: 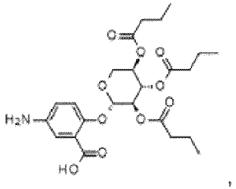 oder ein Salz davon.

4. Multibiotikum nach Anspruch 1, wobei das Multibiotikum eine Verbindung der folgenden Struktur ist: 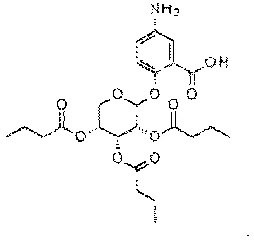 oder ein Salz davon.

5. Multibiotikum nach Anspruch 1, wobei das Multibiotikum eine Verbindung der folgenden Struktur ist: 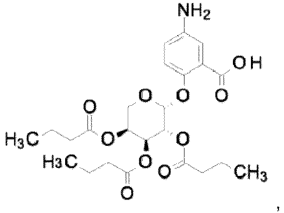 oder ein Salz davon.

6. Pharmazeutische Zusammensetzung, umfassend das Multibiotikum nach einem der Ansprüche 1 bis 5 und einen pharmazeutisch verträglichen Hilfsstoff.

7. Nutrazeutische Zusammensetzung, umfassend das Multibiotikum nach einem der Ansprüche 1 bis 5 und einen physiologisch verträglichen Hilfsstoff.

8. Multibiotikum nach einem der Ansprüche 1 bis 5 zur Verwendung bei der Abgabe einer biologisch aktiven Verbindung an eine Target-Stelle in einer Person.

9. Multibiotikum zur Verwendung nach Anspruch 8, wobei das Multibiotikum oral verabreicht wird.

10. Multibiotikum zur Verwendung nach Anspruch 8, wobei das Multibiotikum im Wesentlichen durch eine Mikrobiota der Person aktiviert wird.

## Revendications

1. Agent multibiotique, dans lequel l'agent multibiotique est choisi dans le groupe constitué par les composés de la structure suivante : 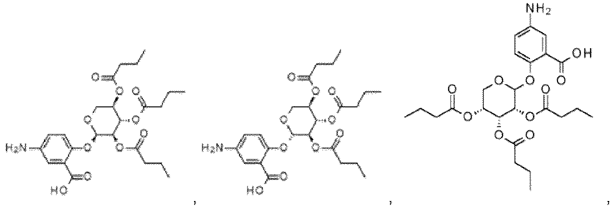 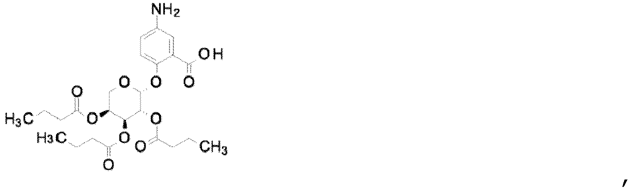 et leurs sels.

2. Agent multibiotique selon la revendication 1, dans lequel l'agent multibiotique est un composé de la structure suivante : 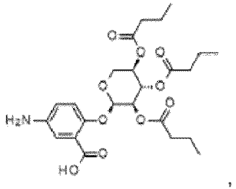 ou un de ses sels.

3. Agent multibiotique selon la revendication 1, dans lequel l'agent multibiotique est un composé de la structure suivante : 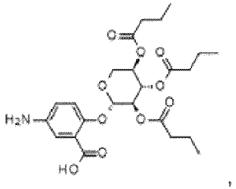 ou un de ses sels.

4. Agent multibiotique selon la revendication 1, dans lequel l'agent multibiotique est un composé de la structure suivante : 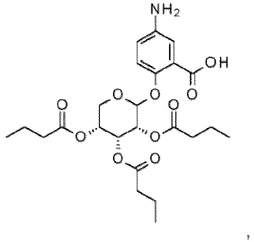 ou un de ses sels.

5. Agent multibiotique selon la revendication 1, dans lequel l'agent multibiotique est un composé de la structure suivante : 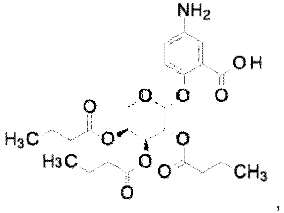 ou un de ses sels.

6. Composition pharmaceutique comprenant l'agent multibiotique selon l'une quelconque des revendications 1 à 5 et un excipient pharmaceutiquement acceptable.

7. Composition nutraceutique comprenant l'agent multibiotique selon l'une quelconque des revendications 1 à 5 et un excipient physiologiquement acceptable.

8. Agent multibiotique selon l'une quelconque des revendications 1 à 5 destiné à être utilisé dans la délivrance d'un composé biologiquement actif à un site cible chez un sujet.

9. Agent multibiotique destiné à être utilisé selon la revendication 8, dans lequel l'agent multibiotique est administré oralement.

10. Agent multibiotique destiné à être utilisé selon la revendication 8, dans lequel l'agent multibiotique est essentiellement activé par un microbiote du sujet.
